# EUROPEAN PATENT APPLICATION

(11) **EP 3 608 417 A1**
(43) Date of publication of application: **12.02.2020**
(21) Application number: 18899006.3
(22) Date of filing: 30.08.2018
(51) Int. Cl.: C12Q 1/48, C12N 9/10, C12N 15/63, C12P 7/22, C12P 7/42, C12P 17/06

(54) **NOVEL MALONYL-COA BIOSENSOR BASED ON TYPE III POLYKETIDE SYNTHASE, AND USE THEREOF**

(30) Priority: 08.06.2018 KR 20180066323
(71) Applicant: Korea Advanced Institute of Science and Technology, Daejeon 34141 (KR)
(72) Inventor: LEE, Sang Yup, Yuseong-gu Daejeon 34049 (KR); YANG, Dongsoo, Yuseong-gu Daejeon 34141 (KR)
(74) Representative: Mewburn Ellis LLP
(86) International application number: PCT/KR2018/010087
(87) International publication number: WO 2019/235688

(57) **Abstract**

The present invention relates to a recombinant microorganism for malonyl-CoA detection in which a type III polyketide synthase-encoding gene is inserted in the genome or in which a recombinant vector containing the gene is introduced; a method of screening a malonyl-CoA production-inducing substance using the recombinant microorganism; a method of screening a gene which is involved in increased malonyl-CoA production; and a method comprising knocking down the gene, screened by the method, in a microorganism, thus increasing the production of malonyl-CoA in the microorganism, and producing a useful substance in the microorganism using malonyl-CoA as a precursor. The use of the biosensor according to the present invention provides single-step signal generation, utilization in various microorganisms, utilization in self-fluorescent microorganisms, a simple construction method, and a simple screening method. In addition, when the present invention is combined with high-throughput screening, it has advantages in that strains having increased malonyl-CoA producing ability can be screened very easily and rapidly (∼3 days) and can be applied directly to the malonyl-CoA-based production of useful compounds.

## Description

### TECHNICAL FIELD

The present invention relates to a novel malonyl-CoA biosensor based on type III polyketide synthase and the use thereof, and more particularly to a recombinant microorganism for malonyl-CoA detection into which a recombinant vector containing a type III polyketide synthase-encoding gene is introduced; a method of screening a malonyl-CoA production-inducing substance using the recombinant microorganism; a method of screening a gene which is involved in increased malonyl-CoA production; and a method comprising knocking down the gene, screened by the method, in a microorganism, thus increasing the production of malonyl-CoA in the microorganism, and producing a useful substance in the microorganism using malonyl-CoA as a precursor.

### BACKGROUND ART

Due to global environmental concerns, depletion of limited resources, and increased demand for environmentally friendly energy sources, the construction of cell factories based on reproducible organisms has been of increasing interest. These cell factories can produce desired metabolites (bioenergy, environmentally friendly chemical substances, new drug substances, etc.) through intracellular metabolic networks optimized for the production of the desired metabolites, and various molecular biology techniques are required for this technological process.

Among various substances which can be produced by microbial cell factories, malonyl-CoA is particularly important because it is a key precursor for producing very useful substances, such as polyketides, phenylpropanoids and biofuels. However, malonyl-CoA causes various essential metabolic reactions (such as fatty acid biosynthesis) in cells, and thus the amount of malonyl-CoA that can be utilized in metabolic engineering is very small. Furthermore, high-performance instruments such as LC-MS/MS are required to measure the intracellular concentration of malonyl-CoA, and a sampling method that takes a long time and requires very careful attention is required to measure malonyl-CoA which disappears after rapid production in cells and is sensitive to ambient environmental conditions. Therefore, transcription factor-based malonyl-CoA biosensors have been developed from previous studies in order to more quickly and easily measure the intracellular concentration of malonyl-CoA (Xu P, Li L, Zhang F, Stephanopoulos G, Koffas M (2014), Proc Natl Acad Sci USA 111:11299-11304; Li S, Si T, Wang M, Zhao H (2015), ACS Synth Biol 4:1308-1315). However, the fluorescent protein-based sensors based on transcription factors as described above have disadvantages in that they have to undergo various signaling steps and cannot be used in self-fluorescent microorganisms such as *Pseudomonas.*

Accordingly, the present inventors have made efforts to solve the above-described problems, and as a result, have developed a novel malonyl-CoA biosensor based on type III polyketide synthase, which utilizes the property that type III polyketide synthases, including RppA, convert malonyl-CoA into a colored substance through a single-step reaction, and have found that the use of the biosensor can easily detect malonyl-CoA, makes it possible to screen a malonyl-CoA-producing strain, a malonyl-CoA production-inducing substance, and a gene which is involved in increased malonyl-CoA production, and makes it possible to easily construct a method of producing various useful substances using malonyl-CoA as either a substrate or a precursor, thereby completing the present invention.

### DISCLOSURE OF INVENTION

### TECHNICAL PROBLEM

It is an object of the present invention to provide a novel biosensor for malonyl-CoA detection and various methods of using the biosensor, and a method of increasing the production of malonyl-CoA in the microorganism by use of the methods, and producing a useful substance in the microorganism using malonyl-CoA as a substrate or a precursor.

### TECHNICAL SOLUTION

To achieve the above object, the present invention provides a recombinant microorganism for malonyl-CoA detection in which a type III polyketide synthase-encoding gene is inserted in the genome of the microorganism or into which a recombinant vector containing the type III polyketide synthase-encoding gene is introduced.

The present invention also provides a method for screening a malonyl-CoA production-inducing substance, comprising the steps of:
(a) culturing the above-described recombinant microorganism;
(b) adding a candidate substance to the recombinant microorganism;
(c) comparing the color of a culture supernatant of the recombinant microorganism after addition of the candidate substance with the color of a culture supernatant of the recombinant microorganism without addition of the candidate substance; and
(d) selecting the candidate substance as the malonyl-CoA production-inducing substance, when the culture supernatant of the recombinant microorganism after addition of the candidate substance shows a deeper red color than the culture supernatant of the recombinant microorganism without addition of the candidate substance.

The present invention also provides a method for screening a gene involved in increased malonyl-CoA production, the method comprising the steps of:
(a) introducing a gene regulation library, which changes gene expression in a recombinant microorganism, into the above-described recombinant microorganism, thereby constructing a recombinant microorganism library in which gene expression in the recombinant microorganism is changed;
(b) culturing the constructed recombinant microorganism library and the recombinant microorganism into which the gene regulation library is not introduced, measuring the absorbance of the culture supernatants , and comparing the color of the culture supernatants; and
(c) selecting the gene introduced in the recombinant microorganism library, when the culture supernatant of the recombinant microorganism library shows a deeper red color than the culture supernatant of the recombinant microorganism into which the gene regulation library is not introduced.

The present invention also provides a method for producing a recombinant microorganism having increased malonyl-CoA producing ability, the method comprising regulating expression of the gene, screened by the above-described method, in a microorganism inherently having malonyl-CoA producing ability or externally introduced with malonyl-CoA producing ability.

The present invention also provides a recombinant microorganism having increased malonyl-CoA producing ability wherein expression of one or more genes selected from the group consisting of the following genes:
*fabF* (3-oxoacyl-[acyl-carrier-protein] synthase II);
*yfcY* (beta-ketoacyl-CoA thiolase);
*xapR* (transcriptional activator of *xapAB*);
*cytR* (transcriptional repressor for *deo* operon, *udp, cdd, tsx, nupC* and *nupG*) ;
*fabH* (3-oxoacyl-[acyl-carrier-protein] synthase III);
*mqo* (malate dehydrogenase);
*yfiD* (pyruvate formate lyase subunit);
*fmt* (10-formyltetrahydrofolate:L-methionyl-tRNA(fMet)N-formyltransferase);
*pyrF* (orotidine-5'-phosphate decarboxylase);
araA (L-arabinose isomerase);
*fadR* (negative regulator for fad regulon and positive regulator of *fabA*);
*pabA* (aminodeoxychorismate synthase, subunit II);
*purB* (adenylosuccinate lyase); and
*hycI* (protease involved in processing C-terminal end of HycE), in a microorganism having malonyl-CoA producing ability is decreased compared to that in a wild-type microorganism.

The present invention also provides a method of producing a useful substance using malonyl-CoA as a substrate or an intermediate, the method comprising the steps of:
(a) constructing a recombinant microorganism in which a gene involved in production of the useful substance is additionally introduced, or expression of the gene is increased, or the gene involved in production of the useful substance is additionally deleted, or expression of the gene is decreased;
(b) culturing the constructed microorganism; and
(c) recovering the useful substance from the cultured microorganism.

The present invention also provides a recombinant microorganism for producing 6-methylsalicylic acid in which genes that encode 6-methylsalicylic acid synthase (6MSAS) and 4'-phosphopantetheinyl transferase (Sfp) are additionally introduced in the above-described recombinant microorganism or expression of the genes is increased.

The present invention also provides a method of producing 6-methylsalicylic acid, the method comprising the steps of:
(a) culturing the above-described recombinant microorganism; and
(b) recovering the 6-methylsalicylic acid from the cultured microorganism.

The present invention also provides a recombinant microorganism for producing aloesone in which a gene that encodes aloesone synthase is additionally introduced in the above-described recombinant microorganism or expression of the gene is increased.

The present invention also provides a method of producing aloesone, the method comprising the steps of:
(a) culturing the above-described recombinant microorganism; and
(b) recovering the aloesone from the cultured microorganism.

The present invention also provides a recombinant microorganism for producing resveratrol in which genes that encode tyrosine ammonia-lyase (TAL), 4-coumarate:CoA ligase (4CL) and stilbene synthase (STS) are additionally introduced in the above-described recombinant microorganism or expression of the genes is increased.

The present invention also provides a method of producing resveratrol, the method comprising the steps of:
(a) culturing the above-described recombinant microorganism; and
(b) recovering the resveratrol from the cultured microorganism.

The present invention also provides a recombinant microorganism for producing naringenin in which genes that encode tyrosine ammonia-lyase (TAL), 4-coumarate:CoA ligase (4CL), chalcone synthase (CHS), and chalcone isomerase (CHI) are additionally introduced in the above-described recombinant microorganism or expression of the genes is increased.

The present invention also provides a method of producing naringenin, the method comprising the steps of:
(a) culturing the above-described recombinant microorganism; and
(b) recovering the naringenin from the cultured microorganism.

### BRIEF DESCRIPTION OF THE DRAWINGS

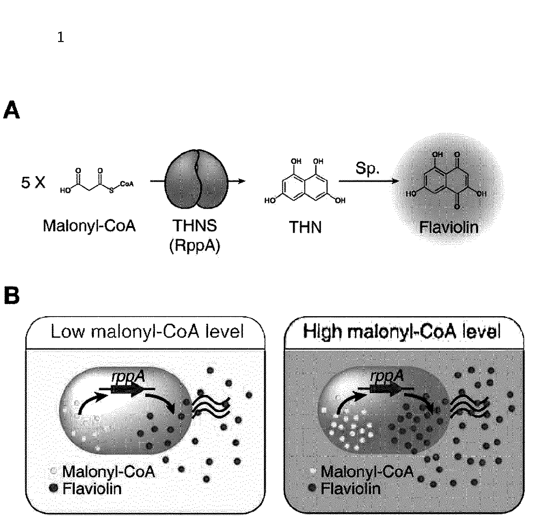

FIG. 1 shows the operational mechanism of a malonyl-CoA biosensor based on type III polyketide synthase (RppA). (A) RppA can convert five molecules of malonyl-CoA into one molecule of red-colored **flaviolin.** At this time, RppA is responsible for the conversion of malonyl-CoA to 1,3,6,8-tetrahydroxynaphthalene (THN), which is then spontaneously converted to flaviolin. (B) As the red color becomes deeper by the RppA biosensor, the intracellular level of malonyl-CoA is higher.

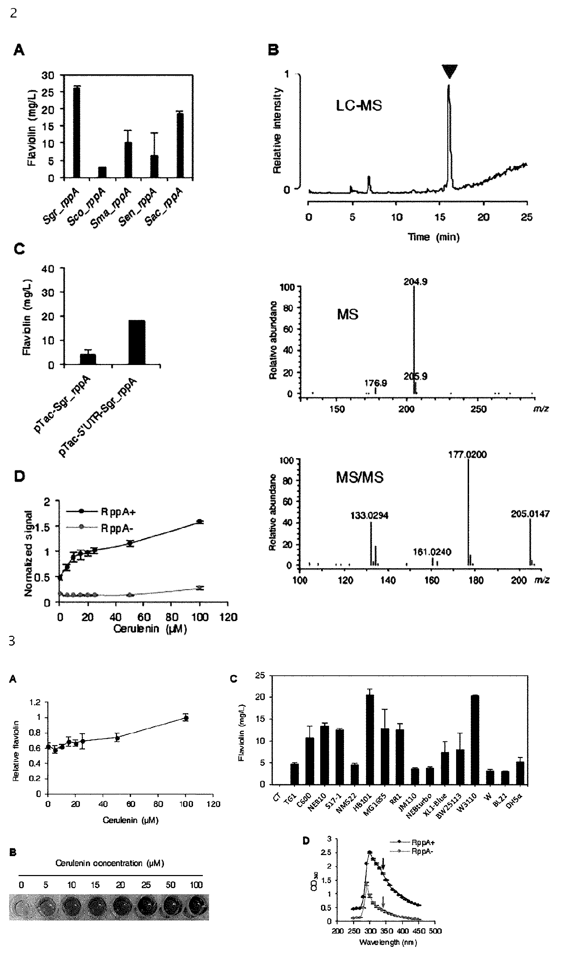

FIG. 2 shows flaviolin biosynthesis. (A) Flaviolin production by *rppA* from five different strains. (B) Production of flaviolin confirmed by LC-MS and MS/MS analysis. (C) Optimization of flaviolin production. Flaviolin production was increased by optimization of 5'UTR. (D) Examination of the applicability of an RppA biosensor. Changes in signals due to changes in intracellular levels of malonyl-CoA by an indirect method using cerulenin. RppA⁺ denotes an RppA-expressing strain, and RppA⁻ denotes a strain that does not express RppA.
FIG. 3 shows the applicability and scalability of an RppA biosensor. (A) Flaviolin production increases as the amount of cerulenin added increases, and (B) this can be confirmed even by the naked eyes. (C) Flaviolin production from all 16 *E. coli* strains is possible. (D) In the case of an RppA-expressing strain (RppA+) and a control strain (RppA- ) that does not express RppA, the noise from the control at 340 nm (indicated by the arrow) was the lowest and the signal from the RppA+ strain was the strongest, and thus the absorbance at 340 nm was used as the signal of an RppA biosensor.

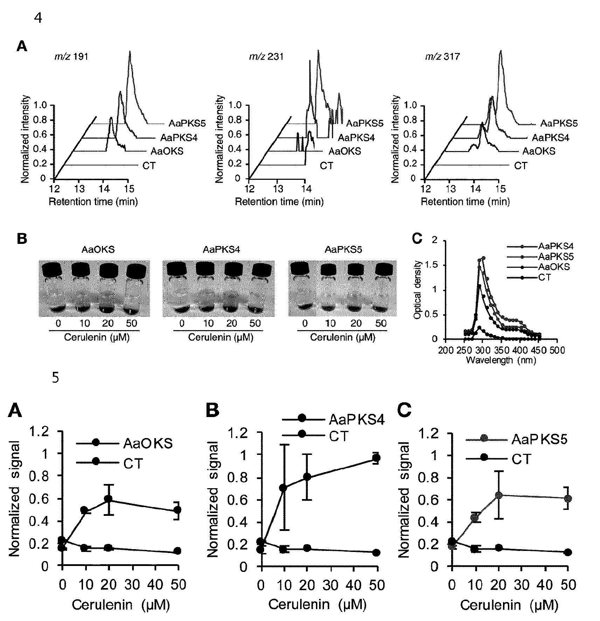

FIG. 4 shows characterization of three type III polyketide synthases (AaOKS, AaPKS4, and AaPKS5) as malonyl-CoA biosensors. (A) Extracted ion chromatograms of culture supernatants of *E. coli* BL21(DE3) strains expressing AaOKS, AaPKS4 and AaPKS5 generated by LC-MS (negative scan mode). The products predicted from the chromatograms are as follows: 5,7-dihydroxy-2-methylchromone (m/z 191); aloesone (m/z 231); SEK4 and SEK4b (m/z 317). (B) shows the colors of culture supernatants of *E. coli* strains expressing AaOKS, AaPKS4 and AaPKS5 when different concentrations of cerulenin was added to the medium, and (C) shows the optical density (absorbance) of the culture supernatants at different wavelengths. CT, control strain (BL21 (DE3) pET-30a(+)). At this time, the signal of malonyl-CoA biosensor strains at 300 nm was the strongest compared to the control, and thus in the case of AaOKS, AaPKS4 and AaPKS5, the absorbance at 300 nm was used as the signal. The data points represent the average values of three biological replicates.
FIG. 5 indicates that three type III polyketide synthases (AaOKS, AaPKS4, and AaPKS5) can be used as malonyl-CoA biosensors. (A) AaOKS, (B) AaPKS4, and (C) AaPKS5 indicate increased normalized signals caused by an increase in the malonyl-CoA level (controlled by addition of cerulenin). Each signal indicates each case that harbors or does not harbor PKS.

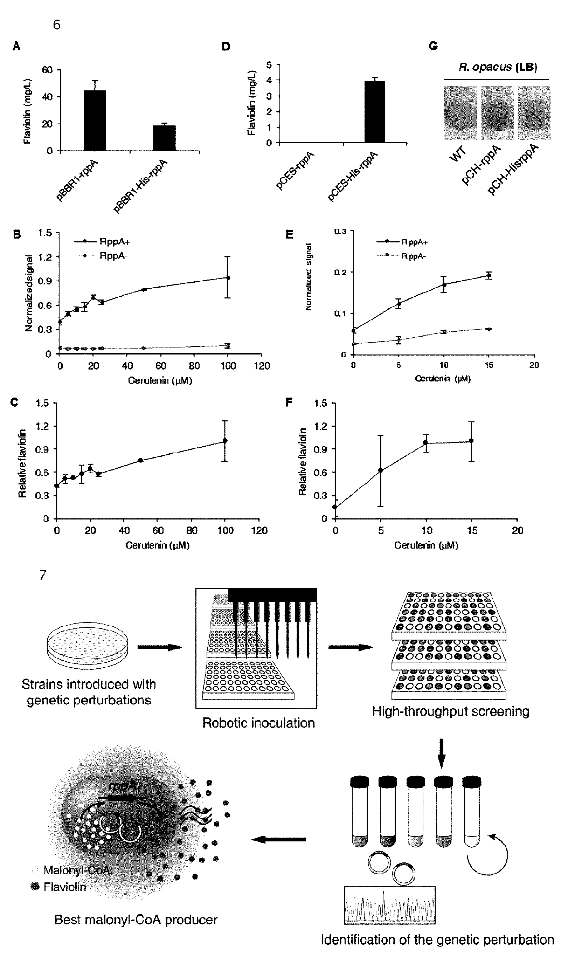

FIG. 6 shows the results of testing an RppA biosensor in *Pseudomonas putida, Corynebacterium glutamicum,* and *Rhodococcus opacus.* (A) shows the production of flaviolin when the indicated plasmid was transformed into a *P. putida* strain. (B) shows the cerulenin concentration-dependent signal intensity of a *P. putida* pBBR1-rppA sensor strain, and (C) shows relative flaviolin production at this time. (D) shows the production of flaviolin when the indicated plasmid was transformed into a *C*. *glutamicum* strain. (E) shows the cerulenin concentration-dependent signal intensity of a C. *glutamicum* pCES-His-rppA sensor strain, and (F) shows relative flaviolin production at this time. (G) shows the color of medium when the indicated plasmid was transformed into an *R. opacus* strain.
FIG. 7 shows a process of screening strains, which show increased malonyl-CoA production, by high-throughput screening using an RppA biosensor.

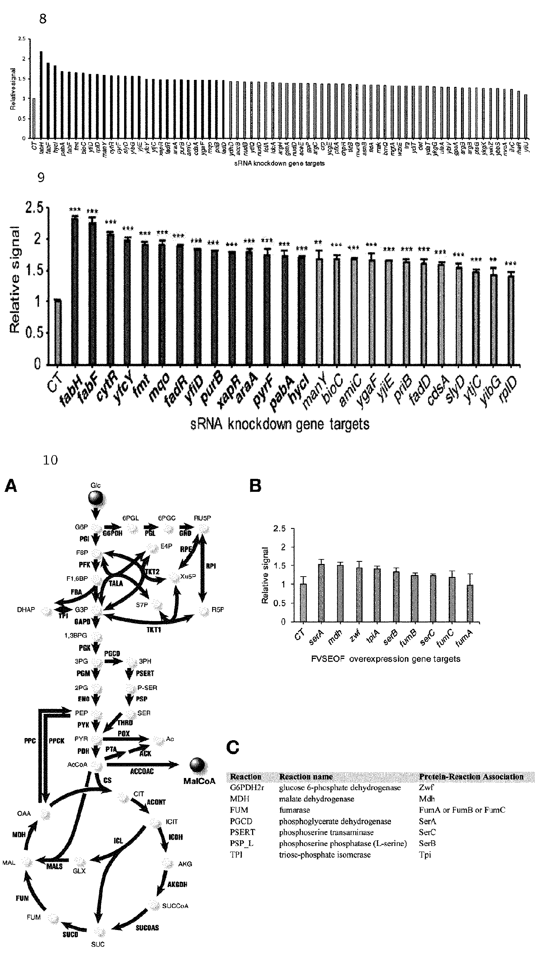

FIG. 8 shows the initial results of screening strains which show increased malonyl-CoA levels when an *E*. *coli* genome-scale synthetic control sRNA library was introduced and high-throughput screening was performed using an RppA biosensor.
FIG. 9 shows signals generated when 26 initially screened synthetic-control sRNAs involved in increased malonyl-CoA production were transformed into *E. coli* sensor strains. At this time, 14 synthetic-control sRNAs showing an increase in signal of 70% or more compared to the control were selected as final targets.
FIG. 10 shows FVSEOF simulation performed to identify increased malonyl-CoA production. (A) shows the biosynthetic pathway related to malonyl-CoA biosynthesis. Letters in bold denote reactions, and plain letters denote metabolites. Grey arrows denote metabolic fluxes, and red arrows denote metabolic fluxes identified as overexpression targets. (B) shows biosensor signals appearing when overexpressing the gene targets identified by FVSEOF. (C) shows lists the gene targets identified by FVSEOF.

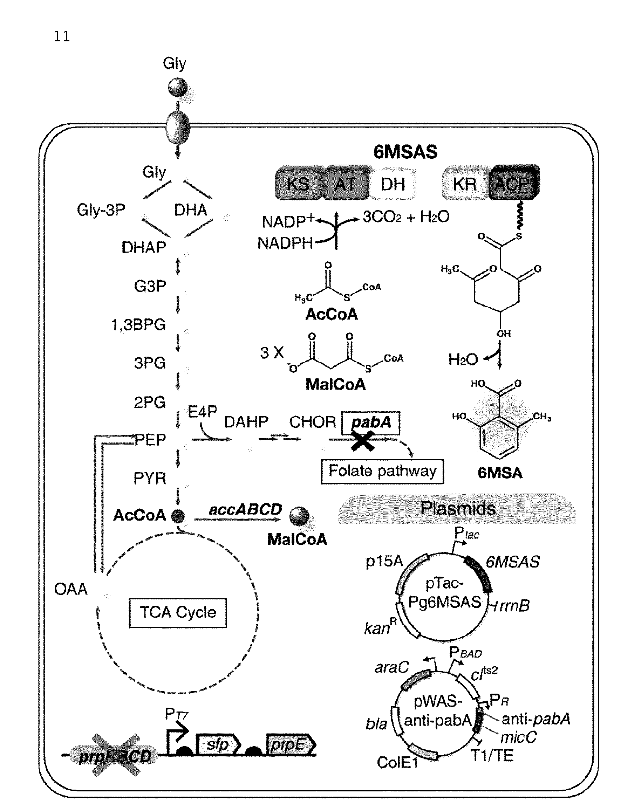

FIG. 11 shows the biosynthetic pathway of 6-methylsalicylic acid. Here, red X denotes knocked-out genes, and blue X denotes knocked-down genes. *bla,* beta-lactamase gene; *kan*^{R}*,* kanamycin-resistance gene; p15A, replication origin; ColE1, replication origin; P*_{tac}*, tac promoter; P*_{BAD}*, arabinose-inducible promoter; P*_{R}*, P*_{R}* promoter; *rrnB, rrnBT1T2* terminator; T1/TE, terminator. Gly, glycerol; Gly-3P, glycerol 3-phosphate; DHA, dihydroxyacetone; DHAP, dihydroxyacetone phosphate; G3P, glyceraldehyde 3-phosphate; 1,3BPG, 1,3-bisphosphoglycerate; 3PG, 3-phosphoglycerate; 2PG, 2-phosphoglycerate; PEP, phosphoenolpyruvate; PYR, pyruvate; OAA, oxaloacetate; E4P, D-erythrose 4-phosphate; DAHP, 3-deoxy-D-arabinoheptulosonate 7-phosphate; CHOR, chorismate; AcCoA, acetyl-CoA; MalCoA, malonyl-CoA; 6MSAS, 6MSA synthase; KS, ketosynthase; AT, acyltransferase; DH, dehydratase; KR, ketoreductase; ACP, acyl carrier protein.

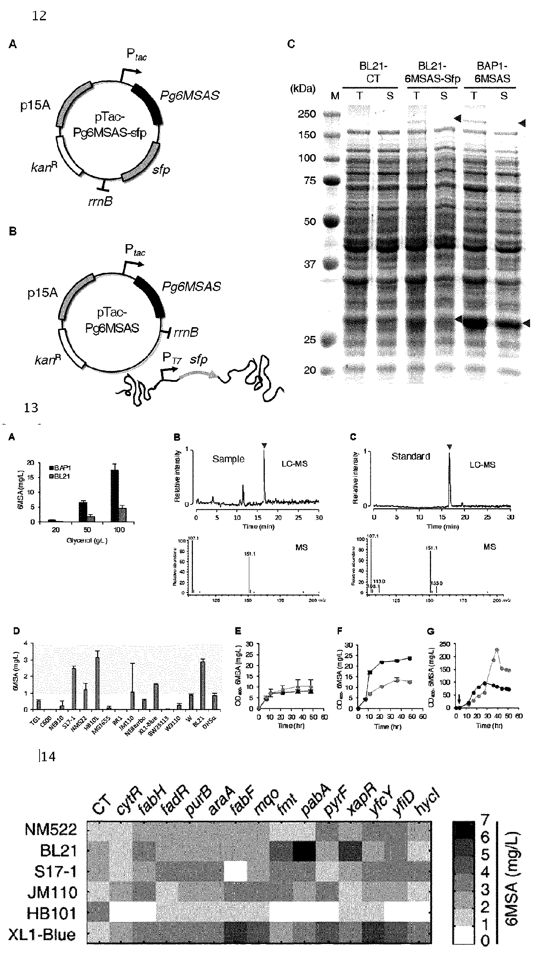

FIG. 12 shows different gene expression cassettes for production of 6-methylsalicylic acid. (A) shows the plasmid pTac-Pg6MSAS-sfp, and (B) shows *sfp* expressed with the plasmid pTac-Pg6MSAS on the genome of an *E. coli* BAP1 strain. (C) shows the results of SDS-PAGE performed to analyze the expression of 6-methylsalicylic acid synthase (Pg6MSAS) and 4'-phosphopantetheinyl transferase (Sfp). Pg6MSAS (188 kDa), Sfp (26.1 kDa).
FIG. 13 shows the production of 6-methylsalicylic acid from modified *E. coli* strains. (A) shows the production of 6-methylsalicylic acid from various concentrations of glucose/glycerol in a BL21-based strain and a BAP1-based strain. (B) shows the results of LC-MS analysis of 6-methylsalicylic acid produced from *E. coli,* and (C) shows the results of LC-MS analysis of a commercially available 6-methylsalicylic acid compound. (D) shows test-tube scale 6-methylsalicylic acid production in 16 *E. coli* strains. Blue sections indicate a production of 1 mg/L or more. (E) shows time-course 6-methylsalicylic acid production in an *E. coli* BL21(DE3) pTac-Pg6MSAS-sfp pWAS-anti-pabA (*pabA*-knockdown sRNA plasmid) strain. (F) shows time-course 6-methylsalicylic acid production in a BAP1 pTac-Pg6MSAS pWAS-anti-pabA strain. (G) shows fed-batch fermentation of the same strain. The red arrow denotes the onset of IPTG induction. In (E), (F) and (G), the blue lines and points denote cell growth (OD₆₀₀), and the red lines and points denote 6-methylsalicylic acid concentrations.
FIG. 14 shows 6-methylsalicylic acid production when 14 synthetic control sRNAs selected in Example 2.1 were introduced into 6 screened *E. coli* strains and test tube-scale culture of the produced strains was performed.

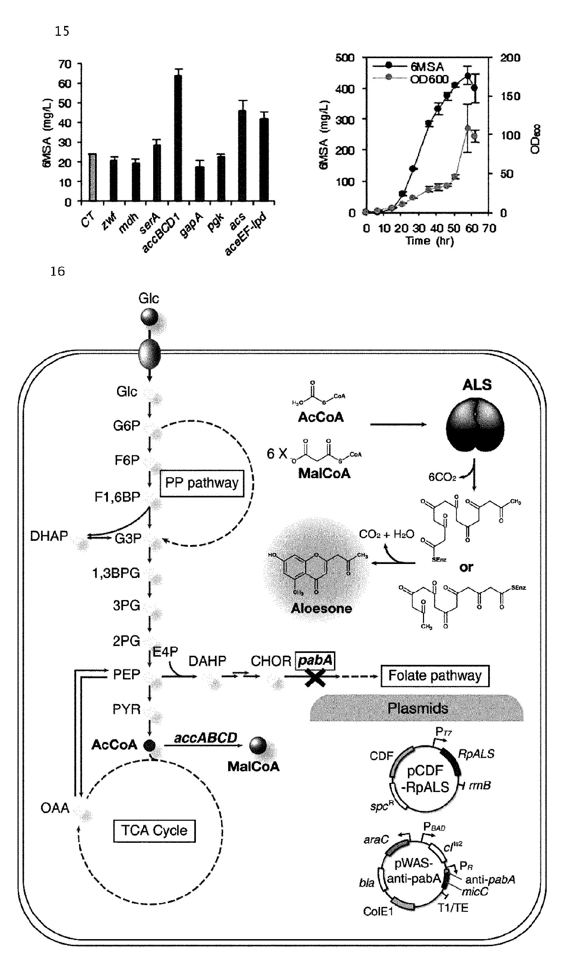

The left graph of FIG. 15 shows the concentration of 6-methylsalicylic acid that appeared in flask culture when the genes at the bottom of the left graph were overexpressed in *E*. *coli* BAP1 pTac-Pg6MSAS pWAS-anti-pabA, a strain showing the highest 6-methylsalicylic acid productivity in FIG. 12. The right graph shows the results of fed-batch fermentation of *E*. *coli* BAP1 pTac-Pg6MSAS pWAS-anti-pabA pBBR1-accBCD1, a strain showing the highest productivity.

FIG. 16 shows the aloesone biosynthetic pathway. Here, red X denotes knocked-out genes, and blue X denotes knocked-down genes. Glc, glucose; G6P, glucose 6-phosphate; F6P, fructose 6-phosphate; F1,6BP, fructose 1,6-bisphosphate; DHAP, dihydroxyacetone phosphate; G3P, glyceraldehyde 3-phosphate; 1,3BPG, 1,3-bisphosphoglycerate; 3PG, 3-phosphoglycerate; 2PG, 2-phosphoglycerate; PEP, phosphoenolpyruvate; PYR, pyruvate; OAA, oxaloacetate; E4P, D-erythrose 4-phosphate; DAHP, 3-deoxy-D-arabinoheptulosonate 7-phosphate; CHOR, chorismate; AcCoA, acetyl-CoA; MalCoA, malonyl-CoA; ALS, aleosone synthase. *bla,* beta-lactamase gene; *spc*^{R}*,* spectinomycin-resistance gene; CDF, replication origin; ColE1, replication origin; P*_{T7}*, *T7* promoter; P*_{BAD}*, arabinose-inducible promoter; P*_{R}*, P*_{R}* promoter; *rrnB, rrnBT1T2* terminator; T1/TE, terminator.

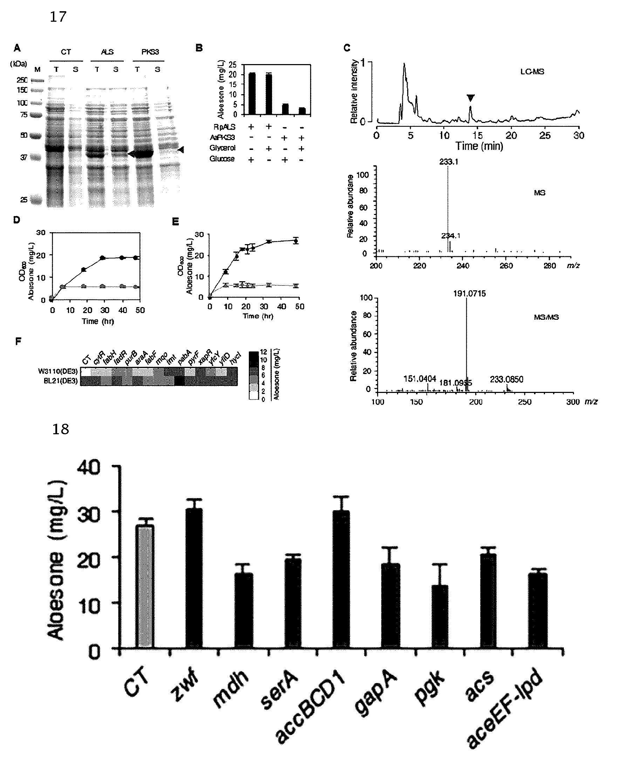

FIG. 17 shows aloesone production in modified *E. coli* strains. (A) shows the results of SDS-PAGE performed to analyze the expression of aloesone synthase (RpALS, 43 kDa) and aloesone synthase (AaPKS3, 44 kDa). (B) shows the production of aloesone when RpALS/AaPKS3 was expressed using glucose/glycerol as a carbon source. (C) shows the LC-MS and MS/MS spectra of aloesone produced in *E. coli.* (D) shows time-course aloesone production in a BL21(DE3) pCDF-RpALS strain, and (E) shows time-course aloesone production in a BL21(DE3) pCDF-RpALS pWAS-anti-pabA strain. In (D) and (E), the blue lines and points depict cell growth (OD₆₀₀), and the red lines and points depict the aloesone concentration. (F) shows the results of test tube-scale culture of strains produced by introducing 14 synthetic-control sRNAs, selected in Example 2.1, into two types of *E. coli* strains.

FIG. 18 shows the concentration of aloesone that appeared in flask culture when the genes at the bottom of the graph were overexpressed in BL21(DE3) pCDF-RpALS pWAS-anti-pabA, a strain showing the highest aloesone productivity in FIG. 17(E).

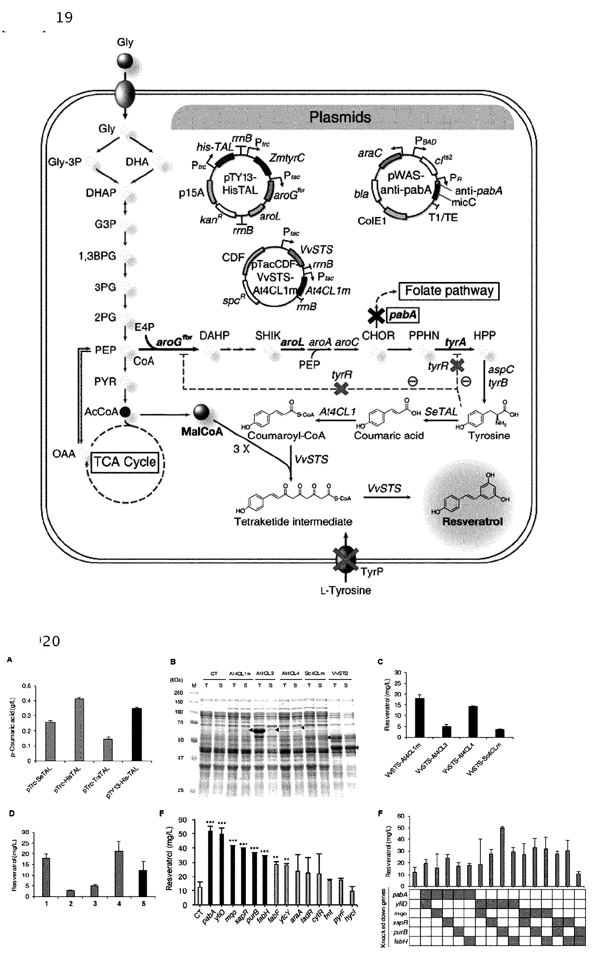

FIG. 19 shows the resveratrol biosynthetic pathway. Here, red X denotes knocked-out genes, and blue X denotes knocked-down genes. Black arrows along with genes written in bold letters denote overexpressed metabolic fluxes. Red dotted lines (along with - signs in circles) indicate transcriptional repression. Black dotted lines (along with + signs in circles) indicate transcriptional activation. Gly, glycerol; Gly-3P, glycerol 3-phosphate; DHA, dihydroxyacetone; DHAP, dihydroxyacetone phosphate; G3P, glyceraldehyde 3-phosphate; 1,3BPG, 1,3-bisphosphoglycerate; 3PG, 3-phosphoglycerate; 2PG, 2-phosphoglycerate; PEP, phosphoenolpyruvate; PYR, pyruvate; OAA, oxaloacetate; E4P, D-erythrose 4-phosphate; DAHP, 3-deoxy-D-arabinoheptulosonate 7-phosphate; SHIK, shikimate; CHOR, chorismate; PPHN, prephenate; HPP, 4-hydroxyphenylpyruvate; FOR, formate; AcCoA, acetyl-CoA; MalCoA, malonyl-CoA. *bla,* beta-lactamase gene; *kan*^{R}*,* kanamycin-resistance gene; *spc*^{R}*,* spectinomycin-resistance gene; p15A, replication origin; ColE1, replication origin; CDF, replication origin; P*_{tac}, tac* promoter; P*_{BAD}*, arabinose-inducible promoter; P*_{R}*, P*_{R}* promoter; P*_{trc}, trc* promoter; *rrnB, rrnBT1T2* terminator; T1/TE, terminator.

FIG. 20 shows the production of *p*-coumaric acid and resveratrol by modified *E. coli* strains. (A) shows the production of *p*-coumaric acid by various plasmids constructed to examine the effects of N-terminal protein tags. Each of the vectors except for pTY13-HisTAL was transformed into a BTY5.13 strain, and pTY13-HisTAL was transformed into a BTY5 strain. (B) shows the results of SDS-PAGE performed to analyze the expression of each protein. At4CL1m (61.1 kDa), At4CL3 (61.3 kDa), At4CL4 (62.6 kDa), Sc4CLm (55.3 kDa), STS (42.8 kDa). CT depicts a control. M depicts a protein size marker. (C) shows the results of resveratrol production by combinations of STS and different 4CLs. (D) shows the production of resveratrol by different plasmid construction strategies. Here, 1 to 5 depict plasmids, and each of the plasmids is as follows. 1, pTac-VvSTS-At4CL1m; 2, pTac-At4CL1m-opr-VvSTS (expressing two genes in an operon); 3, pTac-At4CL1m-fus-VvSTS (expressing a fusion protein of At4CL1m and STS); 4 and 5, pTacCDF-VvSTS-At4CL1m. Here, the plasmids corresponding to 1 to 4 were introduced into *E. coli* BL21(DE3) and were cultured in the presence of 2 mM p-coumaric acid and 20 g/L glycerol. The plasmid corresponding to 5 was introduced into a BTY5 pTY13-HisTAL and was cultured in the presence of 20 g/L of glycerol. The strain corresponding to 5 was used as a resveratrol-producing base strain and indicated by the deeper blue bar. (E) shows the production of resveratrol obtained by transforming 14 synthetic-control sRNAs, selected in Example 2.1, into the base resveratrol-producing strain (BTY5 pTY13-HisTAL pTacCDF-VvSTS-At4CL1m), followed by flask culture. sRNAs corresponding to six strains displayed as deeper red graphs showed more than 2.5-fold increase in resveratrol production compared to the control, and thus were used in combinatorial double knockdown tests. (F) shows the results of combinatorial double knockdown tests using combinations of the previously selected six sRNAs. **P* < 0.05, ***P* < 0.01, ****P* < 0.001 (two-tailed Student's *t*-test).

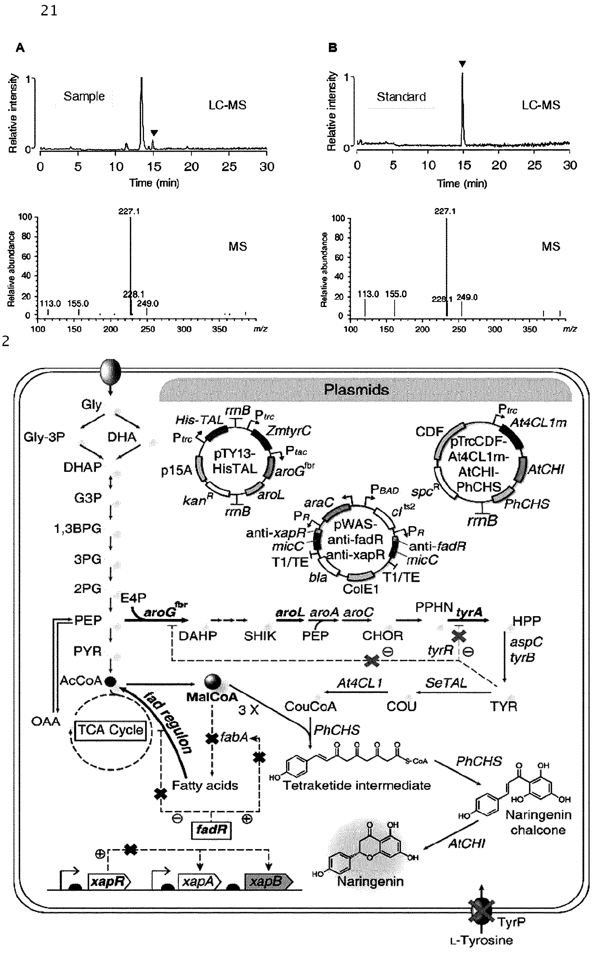

FIG. 21 shows the results of LC-MS performed to determine the authenticity of resveratrol produced from modified *E. coli.* (A) shows the LC-MS spectrum of resveratrol produced from modified *E. coli,* and (B) shows the LC-MS spectrum of a commercially available resveratrol compound.

FIG. 22 shows the naringenin biosynthetic pathway. Here, red X denotes knocked-out genes, and blue X denotes knocked-down genes. Black arrows along with genes written in bold letters denote overexpressed metabolic fluxes. Red dotted lines (along with - signs in circles) indicate transcriptional repression. Black dotted lines (along with + signs in circles) indicate transcriptional activation. *bla,* beta-lactamase gene; *kan*^{R}*,* kanamycin-resistance gene; *spc*^{R}*,* spectinomycin-resistance gene; p15A, replication origin; ColE1, replication origin; CDF, replication origin; P*_{tac}*, *tac* promoter; P*_{BAD}*, arabinose-inducible promoter; P*_{R}*, P*_{R}* promoter; P*_{trc}, trc* promoter; *rrnB, rrnBTlT2* terminator; T1/TE, terminator. Gly, glycerol; Gly-3P, glycerol 3-phosphate; DHA, dihydroxyacetone; DHAP, dihydroxyacetone phosphate; G3P, glyceraldehyde 3-phosphate; 1,3BPG, 1,3-bisphosphoglycerate; 3PG, 3-phosphoglycerate; 2PG, 2-phosphoglycerate; PEP, phosphoenolpyruvate; PYR, pyruvate; OAA, oxaloacetate; E4P, D-erythrose 4-phosphate; DAHP, 3-deoxy-D-arabinoheptulosonate 7-phosphate; SHIK, shikimate; CHOR, chorismate; PPHN, prephenate; HPP, 4-hydroxyphenylpyruvate; FOR, formate; AcCoA, acetyl-CoA; MalCoA, malonyl-CoA; TYR, L-tyrosine; COU, *p-*coumaric acid; CouCoA, *p*-coumaroyl-CoA.

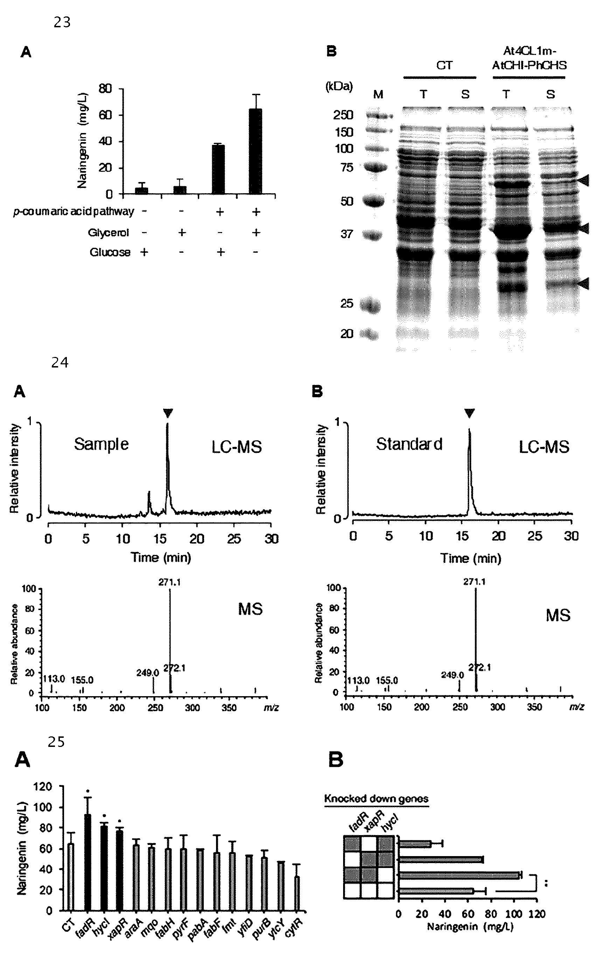

FIG. 23 shows naringenin production in modified *E. coli.* (A) shows naringenin production in strains containing a pTrcCDF-At4CL1m-AtCHI-PhCHS plasmid. Here, in the case of strains having a *p*-coumaric acid pathway, the vector was transformed into a BTY5 pTY13-HisTAL strain, and in other cases, the vector was transformed into a BL21(DE3) strain, and then 2 mM *p*-coumaric acid was added to medium. 20 g/L of glucose or glycerol was added. (B) shows SDS-PAGE analysis of heterologous enzymes. At4CL1m, PhCHS (42.6 kDa), AtCHI (26.6 kDa) .

FIG. 24 shows the results of LC-MS performed to determine the authenticity of naringenin produced from modified *E. coli.* (A) shows the LC-MS spectrum of naringenin produced from modified *E. coli,* and (B) shows the LC-MS spectrum of a commercially available naringenin compound.

FIG. 25 shows naringenin production in modified *E. coli.* (A) shows the amount of naringenin produced by transforming 14 synthetic-control sRNAs, selected in Example 2.1, into a base naringenin strain (BTY5 pTY13-HisTAL pTrcCDF-At4CL1m-AtCHI-PhCHS), and then performing flask culture. sRNAs corresponding to three strains displayed as deeper red bars showed an increase in naringenin production of 15% or more compared to the control, and thus were used in combinatorial double knockdown tests. (B) shows the results of combinatorial double knockdown tests using combinations of the previously selected three sRNAs. **P* < 0.05, ***P* < 0.01 (two-tailed Student's *t*-test).

### BEST MODE FOR CARRYING OUT THE INVENTION

Unless defined otherwise, all the technical and scientific terms used herein have the same meaning as those generally understood by one of ordinary skill in the art to which the invention pertains. Generally, the nomenclature used herein and the experiment methods, which will be described below, are those well known and commonly employed in the art.

In the present invention, it has been found that the use of a recombinant microorganism introduced with type III polyketide synthase makes it possible to measure the malonyl-CoA concentration in a manner significantly improved in terms of time, cost, convenience and the like, compared to a conventional method of measuring the malonyl-CoA concentration.

Therefore, in one aspect, the present invention is directed to a recombinant microorganism for malonyl-CoA detection in which a type III polyketide synthase-encoding gene is inserted in the genome of the microorganism or in which a recombinant vector containing the type III polyketide synthase-encoding gene is introduced.

In the present invention, the type III polyketide synthase may be:
RppA derived from a microorganism selected from the group consisting of *Streptomyces griseus, Streptomyces coelicolor, Streptomyces avermitilis, Saccharopolyspora erythraea, Streptomyces peucetius,* and *Streptomyces aculeolatus;*
PhlD(polyketide synthase) derived from *Pseudomonas fluorescens;*
DpgA(polyketide synthase) derived from *Amycolatopsis mediterranei;*
ALS(aloesone synthase) derived from *Rheum palmatum;* or
PCS (5,7-dihydroxy-2-methylchromone synthase), OKS (octaketide synthase), PKS3 (aloesone synthase), PKS4 (octaketide synthase 2) or PKS5(octaketide synthase 3), which is derived from *Aloe arborescens,* but is not limited thereto.

Type III polyketide synthase RppA derived from the above-mentioned six *Actinomycetes* strains produces flaviolin as a product using malonyl-CoA as a substrate. In addition, other type III polyketide synthases also use malonyl-CoA as a substrate, but PhlD produces phloroglucinol as a product, DpgA produces dihydroxyphenylacetate as a product, PCS produces 5,7-dihydroxy-2-methylchromone as a product, and OKS, PKS4 or PKS5 produces SEK4 and SEK4b as a product. ALS and PKS3 produce aloesone as a product.

The amino acid sequence of each RppA enzyme used in the present invention is as follows:

Streptomyces griseus RppA [SEQ ID NO: 93]: matlcrpaia
vpehvitmqq tldlaretha ghpqrdlvlr liqntgvqtr hlvqpiektl
ahpgfevrnq vyeaeaktrv pevvrralan aetepseidl ivyvsctgfm
mpsltawiin smgfrpetrq lpiaqlgcaa ggaainrahd fcvaypdsnv
livscefcsl cyqptdigvg sllsnglfgd alsaavvrgq ggtgmrlern
gshlvpdted wisyavrdtg fhfqldkrvp gtmemlapvl ldlvdlhgws
vpnmdffivh aggprilddl chfldlppem frysratlte rgniassvvf
dalarlfddg gaaesaqgli agfgpgitae vavgswakeg lgadvgrdld
eleltagval sg

Streptomyces coelicolor RppA [SEQ ID NO: 94]: matlcrpsvs
vpehvitmee tlelarrrht dhpqlplalr lientgvrtr hivqpiedtl
ehpgfedrnk vyereaksrv paviqraldd aellatdidv iiyvsctgfm
mpsltawlin emgfdsttrq ipiaqlgcaa ggaainrahd fctaypeana
livacefcsl cyqptdlgvg sllcnglfgd giaaavvrgr ggtgvrlern
gsylipkted wimydvkatg fhflldkrvp atmeplapal kelagehgwd
asdldfyivh aggprilddl stflevdpha frfsratlte ygniasavvl

dalrrlfdeg gveegargll agfgpgitae mslgcwqtad vrrgirqdvt
rtaargvsrr vrqa
Streptomyces avermitilis RppA [SEQ ID NO: 95]:
matlckpavs vpehvitmee tlelarsrhp dhpqlplalr lientgvhtr
hivqpieetl khpgfeernh vyeaeakarv pavvqralde aelltdidv
iiyvsctgfm mpsltaylin smdfssdtrq ipiaqlgcaa ggsainrahd
fctaypqana livacefcsl cyqptdlgvg sllsnglfgd giaaaavrgk
ggtgitlern asylipktde wisydvratg fhflldkrvp gtmeplapal
qelasqhgwd asdldfyiih aggprilddl skflrvppea frfsratlte
ygniasavvl dalrrlfdeg gaehaargml agfgpgitae mslgrwhrtd ea

Saccharopolyspora erythraea RppA [SEQ ID NO: 96]:
mavlctpava vpehvitvee tldlarrvha dhpqlplvlr lisntgvrer
hlirpiedtl ehpgfevrnr iyeeqakqrv pavvrealds aelgpedidl
ivyvsctgfm mpsltawlin smgfrmstrq lpiaqlgcaa ggaainrahd
fctaypdana livscefcsl cyqptdddig sllsnglfgd avgaavvrgh
ggtgvrlern assmipeted wisyavkatg fhfqldkrvp ktmeplapal
ralaedhrwd vagldfyvih aggprilddl tkflgvpsea frhsratlaq
ygniasavvl dalrriieeg rlesgargmi agfgpgitae msvgtwvphd
vllhgehstt sapggnr

Streptomyces peucetius RppA [SEQ ID NO: 97]: mrvpvavddl
vapstmgerh tvidrgtsva avhtalpphr yaqsdlteli adlclepgad
rallrrlhts agvrtrhlal pieqyaglgd fgqanaawlt vglalaeeal
sgaldaaglt aadidllvct sitgvaapsl darlavrmgm radvkrvpvf
glgcvggaag lgrlhdyllg hpddtavlls velcsltlqr dgslanlvag
alfgdgaaav varggdagrr gagwpmvaat rghlypdteh llgwrigasg

frvvvdagip dvvrthlggd lrnflathgl vpddigtwic hpggpkvlaa
vgdalelpdg aldsswrsla gvgnlssasv lrvledvatr crpdpgtwgv
llamgpgfca efvllrw

Streptomyces aculeolatus RppA [SEQ ID NO: 98]:
mprlckpavs apeytitmee tlefakqaha gkpqlplalr lirntgvlkr
hivqpiektl ghpglternl iyeaeskkmc ppvieealqn admtardida
iiyvsctgfl mpsltawlin kmgfrsdtrq ipiaqlgcaa ggaavnrahd
fclahpgsnv livacelcsl cyqptaddig sllsdglfgd avaaavvrgn
ggvgievern asylipntee wisysvrdtg fhfqldrrvp gtmeplapvl
refakdhswd agkldfyivh aggprilddl arfldvdrqv frhswstlte
ygniasavvf daarrlfeeg sakpdatgmi agfgpgitae malgtwgtdg tgtsn

The amino acid sequences of other type III polyketide synthases used in the present invention are as follows:

Pseudomonas fluorescens type III polyketide synthase
(PhlD)[SEQ ID NO: 99]:
MSTLCKPSLL FPHYKITQQQ MIDHLEQLHD DHPRMALAKR MIQNTQVNER
YLVLPIDELA VHTGFTHRSI VYEREARRMS SIAARQAIEN AGLTTDDIRM
VAVTSCTGFM MPSLTAHLIN DLGLRTSTVQ LPIAQLGCVA GAAAINRAND
FASLSPDNHA LIVSLEFSSL CYQPQDTKLH AFISAALFGD AVSACVMRAD
DKAPGFKIAK TGSYFLPDSE HYIKYDVKDS GFHFTLDKAV MNSIKDVAPM
MEELNFETFN QHCAQNDFFI FHTGGRKILD ELVLQLDLEP GRVAQSRDSL
SEAGNIASVV VFDVLKRQFD SGPANGATGM LAAFGPGFTA EMAVGKWVA

*Amycolatopsis orientalis* type III polyketide synthase
(DpgA)[SEQ ID NO: 100]:

MDVSMTTGIE LTEELSVLNG LTEITRFAGV GTAVSETSYS QTELLDILDV
EDPKIRSVFL NSAIDRRFLT LPPENPGGGR LAEPQGDLLD KHKKIAVDMG
CRALEACLKS AGATLSDLRH LCCVTSTGFL TPGLSALIIR EMGIDPHCSR
SDIVGMGCNA GLNALNVVSG WSAAHPGELG VVLCSEACSA AYALDGTMRT
AVVNSLFGDG SAALAVISGD GRVAGPRVLK FASYIITDAV DAMRYDWDRD
QDRFSFFLDP QIPYVVGAHA EIVVDRLLSG TGLRRSDIGH WLVHSGGKKV
VDAVVVNLGL SRHDVRHTTG VLRDYGNLSS GSFLFSYERL SEEDVTRPGD
YGVLMTMGPG STIEMALIQW

Rheum palmatum type III polyketide synthase (aloesone
synthase, ALS) [SEQ ID NO: 101]:
madvlqeirn sqkasgpatv laigtahppt cypqadypdf yfrvcksehm
tklkkkmqfi cdrsgirqrf mfhteenlgk npgmctfdgp slnarqdmli
mevpklgaea aekaikewgq dksrithlif ctttsndmpg adyqfatlfg
lnpgvsrtmv yqqgcfaggt vlrlvkdiae nnkgarvlvv cseivafafr
gphedhidsl igqllfgdga aalvvgtdid esverpifqi msatqatipn
slhtmalhlt eagltfhlsk evpkvvsdnm eelmleafkp lgitdwnsif
wqvhpggrai ldkieeklel tkdkmrdsry ilseygnlts acvlfvmdem
rkrsfregkq ttgdgyewgv aiglgpgltv etvvlrsvpi p

Aloe arborescens type III polyketide synthase (5,7-
dihydroxy-2-methylchromone synthase, PCS)[SEQ ID NO: 102]:
MSSLSNSLPL MEDVQGIRKA QKADGTATVM AIGTAHPPHI FPQDTYADVY
FRATNSEHKV ELKKKFDHIC KKTMIGKRYF NYDEEFLKKY PNITSYDEPS
LNDRQDICVP GVPALGTEAA VKAIEEWGRP KSEITHLVFC TSCGVDMPSA
DFQCAKLLGL HANVNKYCIY MQGCYAGGTV MRYAKDLAEN NRGARVLVVC
AELTIMMLRA PNETHLDNAI GISLFGDGAA ALIIGSDPII GVEKPMFEIV

CTKQTVIPNT EDVIHLHLRE TGMMFYLSKG SPMTISNNVE ACLIDVFKSV
GITPPEDWNS LFWIPHPGGR AILDQVEAKL KLRPEKFRAA RTVLWDYGNM
VSASVGYILD EMRRKSAAKG LETYGEGLEW GVLLGFGPGI TVETILLHSL PLM

Aloe arborescens type III polyketide synthase
(octaketide synthase, OKS)[SEQ ID NO: 103]:
MSSLSNASHL MEDVQGIRKA QRADGTATVM AIGTAHPPHI FPQDTYADFY
FRATNSEHKV ELKKKFDRIC KKTMIGKRYF NYDEEFLKKY PNITSFDEPS
LNDRQDICVP GVPALGAEAA VKAIAEWGRP KSEITHLVFC TSCGVDMPSA
DFQCAKLLGL RTNVNKYCVY MQGCYAGGTV MRYAKDLAEN NRGARVLVVC
AELTIIGLRG PNESHLDNAI GNSLFGDGAA ALIVGSDPII GVEKPMFEIV
CAKQTVIPNS EDVIHLHMRE AGLMFYMSKD SPETISNNVE ACLVDVFKSV
GMTPPEDWNS LFWIPHPGGR AILDQVEAKL KLRPEKFRAT RTVLWDCGNM
VSACVLYILD EMRRKSADEG LETYGEGLEW GVLLGFGPGM TVETILLHSL PLM

Aloe arborescens type III polyketide synthase (aloesone synthase, PKS3)[SEQ ID NO: 104]:

mgslsdstpl mkdvqgirka qkadgtatvm aigtahpphi isqdsyadfy
frvtnsehkv elkkkfdric kktmigkryf nfdeeflkky pnitsfdkps
lndrhdicip gvpalgaeaa vkaieewgrp kseithlvfc tsggvdmpsa
dfqcakllgl rtnvnkyciy mqgcyaggtv mryakdlaen nrgarvlmvc
aeltiialrg pndshidnai gnslfgdgaa alivgsdpii gvekpmfeiv
cakqtvipns eevihlhlre sglmfymtkd saatisnnie aclvdvfksv
gmtppedwns lfwiphpggr aildqveakl klrpekfsat rtvlwdygnm
isacvlyild emrrksaaeg letygeglew gvllgfgpgm tietillhsl ppv

*Aloe arborescens* type III polyketide synthase
(octaketide synthase 2, PKS4)[SEQ ID NO: 105]:

MGSLSNYSPV MEDVQAIRKA QKADGTATVM AIGTAHPPHI FPQDTYADFY
FRATNSEHKV ELKKKFDRIC KKTMIGKRYF NYDEEFLKKY PNITSFDEPS
LNDRQDICVP GVPALGAEAA VKAIAEWGRP KSEITHLVFC TSCGVDMPSA
DFQCAKLLGL RTNVNKYCVY MQGCYAGGTV MRYAKDLAEN NRGARVLVVC
AELTIIGLRG PNESHLDNAI GNSLFGDGAA ALIVGSDPII GVERPMFEIV
CAKQTVIPNS EDVIHLHMRE AGLMFYMSKD SPETISNNVE ACLVDVFKSV
GMTPPEDWNS LFWIPHPGGR AILDQVEARL KLRPEKFGAT RTVLWDCGNM
VSACVLYILD EMRRKSVADG LATYGEGLEW GVLLGFGPGM TVETILLHSL PPV

Aloe arborescens type III polyketide synthase
(octaketide synthase 3, PKS5)[SEQ ID NO: 106]:
MGSIAESSPL MSRENVEGIR KAQRAEGTAT VMAIGTAHPP HIFPQDTYAD
FYFRATNSEH KVELKKKFDR ICKKTMIGKR YFNYDEEFLK KYPNITSFDE
PSLNDRQDIC VPGVPALGKE AALKAIEEWG QPLSKITHLV FCTSCGVDMP
SADFQLAKLL GLNTNVNKYC VYMQGCYAGG TVLRYAKDLA ENNRGSRVLV
VCAELTIIGL RGPNESHLDN AIGNSLFGDG AAALIVGADP IVGIEKPIFE
IVCAKQTVIP DSEDVIHLHL REAGLMFYMS KDSPETISNN VEGCLVDIFK
SVGMTPPADW NSLFWIPHPG GRAILDEVEA RLKLRPEKFR ATRHVLWEYG
NMVSACVLYI LDEMRNKSAA DGLGTYGEGL EWGVLLGFGP GMTVETILLH SLPPV

The amino acid sequence of the above-described type III polyketide synthase illustrates some feasible enzymes for constructing the recombinant microorganism for malonyl-CoA detection according to the present invention. The present invention has a technical feature in that malonyl-CoA detection is possible by introduction of type III polyketide synthase. Thus, it will be obvious to those skilled in the art that the present invention makes it possible to construct recombinant microorganisms introduced with type III polyketide synthases other than the above-described type III polyketide synthase.

In the present invention, the recombinant microorganism may be characterized in that an gene encoding the type III polyketide synthase is operably linked to a promoter selected from the group consisting of tac, trc, T7, BAD, λPR an Anderson synthetic promoter, but is not limited thereto.

In the present invention, the recombinant microorganism may be selected from the strain group consisting of *E. coli, Rhizobium, Bifidobacterium, Rhodococcus, Candida, Erwinia, Enterobacter, Pasteurella, Mannheimia, Actinobacillus, Aggregatibacter, Xanthomonas, Vibrio, Pseudomonas, Azotobacter, Acinetobacter, Ralstonia, Agrobacterium, Rhodobacter, Zymomonas, Bacillus, Staphylococcus, Lactococcus, Streptococcus, Lactobacillus, Clostridium, Corynebacterium, Streptomyces, Bifidobacterium, Cyanobacterium,* and *Cyclobacterium,* but the microorganism to which the present invention is applied is not limited thereto. Preferably, the recombinant microorganism of the present invention may be constructed by introducing a recombinant vector into a microorganism selected from the group consisting of *E. coli, Pseudomonas* sp., *Corynebacterium* sp., and *Rhodococcus* sp., but is not limited thereto. As *E. coli,* one selected from among the *E. coli* strains shown in Table 2 below is preferably used, but those skilled in the art will appreciate that other *E. coli* strains expected to exhibit similar effects may also be used. The recombinant microorganism of the present invention may be constructed by using *Pseudomonas putida* as *Pseudomonas* sp., *Corynebacterium glutamicum* as *Corynebacterium* sp., and *Rhodococcus opacus* as *Rhodococcus* sp., but this is an example of a representative microorganism, and the microorganism to which the present invention is applied is not limited thereto.

In the meantime, in the present invention, when the recombinant microorganism for malonyl-CoA detection is used in which a type III polyketide synthase-encoding gene is inserted in the genome of the microorganism or in which a recombinant vector containing the type III polyketide synthase-encoding gene is introduced, the increase and decrease in the production of the malony-CoA could be easily confirmed through the concentration-dependent color development from the malony-CoA, and a malonyl-CoA production-inducing substance may be easily screened.

Therefore, in another aspect, the present invention is directed to a method for screening a malonyl-CoA production-inducing substance, comprising the steps of:
(a) culturing the recombinant microorganism;
(b) adding a candidate substance to the recombinant microorganism;
(c) comparing the color of a culture supernatant of the recombinant microorganism after addition of the candidate substance with the color of a culture supernatant of the recombinant microorganism without addition of the candidate substance; and
(d) selecting the candidate substance as the malonyl-CoA production-inducing substance, when the culture supernatant of the recombinant microorganism after addition of the candidate substance shows a deeper red color than the culture supernatant of the recombinant microorganism without addition of the candidate substance.

In the present invention, step (c) may comprise clearly observing a change in the color by the naked eyes, or measuring absorbance and quantitatively expressing a change in the absorbance.

Thus, the comparison between the colors in step (c) may be made by the naked eyes.

In addition, the comparison between the colors in step (c) may be made by measuring absorbance, and step (d) may comprises selecting the candidate substance as the malonyl-CoA production-inducing substance, when the absorbance measured after addition of the candidate substance is higher than that measured without addition of the candidate substance.

In the present invention, the absorbance (OD value) at 280 to 450 nm, preferably 300 to 340 nm, may be measured, and the OD value increases as the malonyl-CoA concentration increases.

The malonyl-CoA production-inducing substance can induce the production of the malonyl-CoA by a mechanism that regulates expression of the gene involved in increased malonyl-CoA production.

Therefore, in still another aspect, the present invention is directed to a method for screening a gene involved in increase of malonyl-CoA production, the method comprising the steps of:
(a) introducing a gene regulation library, which changes gene expression in a recombinant microorganism, into the above-described recombinant microorganism, thereby constructing a recombinant microorganism library in which gene expression in the recombinant microorganism is changed;
(b) culturing the constructed recombinant microorganism library, measuring the absorbance of the culture supernatant of the recombinant microorganism library, culturing the recombinant microorganism before introduction of the gene regulation library, and comparing the color of the culture supernatant of the recombinant microorganism library with the color of the culture supernatant of the recombinant microorganism before introduction of the gene regulation library; and
(c) selecting the gene introduced in the recombinant microorganism library, when the culture supernatant of the recombinant microorganism library shows a deeper red color than the culture supernatant of the recombinant microorganism before introduction of the gene regulation library.

In the present invention, step (b) may comprise clearly observing a change in the color by the naked eyes, or measuring absorbance and quantitatively expressing a change in the absorbance.

Thus, the comparison of the color in step (c) may be made by the naked eyes.

In addition, the comparison between the colors in step (b) is made by measuring absorbance, and step (c) may comprise selecting the gene introduced in the recombinant microorganism library as a gene involved in increase of malonyl-CoA production, when the absorbance of the culture supernatant of the recombinant microorganism library is higher than that of the culture supernatant of the recombinant microorganism before introduction of the gene regulation library.

In the present invention, the absorbance (OD value) at 280 to 450 nm, preferably 300 to 340 nm, may be measured, and the OD value increases as the malonyl-CoA concentration increases.

In the present invention, the gene regulation library may be a library selected from an sRNA library, a genomic library, a cDNA library, a gRNA library, and an oligonucleotide library for construction of knockout or mutant strains, but is not limited thereto.

Specifically, to achieve the object of the present invention, the gene regulation library may be suitably selected from among the following:
an sRNA library, or an oligonucleotide library for construction of knockout or mutant strains for inhibiting endogenous gene expression in the recombinant microorganism;
a genomic library or a cDNA library which is a library for endogenous or exogenous gene overexpression; and
a gRNA (guide RNA which is used in CRISPR (Clustered Regularly Interspaced Short Palindromic Repeats)-Cas9 technology) library which is a library enabling both gene expression inhibition and gene overexpression.

That is, the addition of the synthetic-control sRNA library described in Example 2 below is an example to describe the present invention in detail, and presents the method of using the same, and it is possible to introduce any other libraries known to exhibit similar functions.

Meanwhile, when the gene screened by the method is knocked down in a microorganism, malonyl-CoA production in the microorganism can be increased. Therefore, in yet another aspect, the present invention is directed to a method for producing a recombinant microorganism having increased malonyl-CoA producing ability, the method comprising regulating expression of the gene, screened by the method, in a microorganism having malonyl-CoA producing ability.

The microorganism having malonyl-CoA producing ability means a microorganism inherently having malonyl-CoA producing ability or externally introduced with malonyl-CoA producing ability.

In the present invention, the screened gene may be one or more genes selected from the group consisting of:
*fabF* (3-oxoacyl-[acyl-carrier-protein] synthase II);
*yfcY* (beta-ketoacyl-CoA thiolase);
*xapR* (transcriptional activator of *xapAB*);
*cytR* (transcriptional repressor for *deo* operon, *udp, cdd, tsx, nupC* and *nupG*) ;
*fabH* (3-oxoacyl-[acyl-carrier-protein] synthase III);
*mqo* (malate dehydrogenase);
*yfiD* (pyruvate formate lyase subunit);
*fmt* (10-formyltetrahydrofolate:L-methionyl-tRNA(fMet)N-formyltransferase);
*pyrF* (orotidine-5'-phosphate decarboxylase);
araA (L-arabinose isomerase);
*fadR* (negative regulator for fad regulon and positive regulator of *fabA*);
*pabA* (aminodeoxychorismate synthase, subunit II);
*purB* (adenylosuccinate lyase); and
*hycI* (protease involved in processing C-terminal end of HycE),
and expression of the gene may be reduced, but is not limited thereto.

In the present invention, *fabF* encodes 3-oxoacyl-[acyl-carrier-protein] synthase II, *yfcY* encodes beta-ketoacyl-CoA thiolase, *xapR* encodes transcriptional activator of *xapAB, cytR* encodes transcriptional repressor for *deo* operon, *udp, cdd, tsx, nupC* and *nupG, fabH* encodes 3-oxoacyl-[acyl-carrier-protein] synthase III, *mqo* encodes malate dehydrogenase, *yfiD* encodes pyruvate formate lyase subunit, *fmt* encodes 10-formyltetrahydrofolate:L-methionyl-tRNA(fMet)N-formyltransferase, *pyrF* encodes orotidine-5'-phosphate decarboxylase, araA encodes L-arabinose isomerase, *fadR* encodes negative regulator for fad regulon and positive regulator of *fabA, pabA* encodes aminodeoxychorismate synthase, subunit II, *purB* encodes adenylosuccinate lyase, and *hycI* encodes protease involved in processing C-terminal end of HycE, respectively.

The amino acid sequence of each of enzymes, which are screened and used in the present invention, is as follows, but may have a substitution, deletion or addition of one or more amino acids in the amino acid sequences, and in this case, the resulting mutant amino acid sequence may exhibit a function equal to or better than that in the present invention. For example, it will be obvious that if the structural configuration of the mutant amino acid sequence is identical or similar to the wild-type amino acid sequence, and thus exhibits the same or higher effect on a substrate, the mutant amino acid sequence also falls within the scope of the present invention. In the same context, it will be obvious that besides enzymes used in the present invention, other microorganism-derived enzymes having an identical or similar function can be properly applied to the present invention, and the application of a corresponding enzyme within a range that can be easily applied by a person of ordinary skill in the art falls within the scope of the present invention.

FabF[SEQ ID NO: 107]:

MSKRRVVVTGLGMLSPVGNTVESTWKALLAGQSGISLIDHFDTSAYATKFAGLVKD

FNCEDIISRKEQRKMDAFIQYGIVAGVQAMQDSGLEITEENATRIGAAIGSGIGGLGLIEEN

HTSLMNGGPRKISPFFVPSTIVNMVAGHLTIMYGLRGPSISIATACTSGVHNIGHAARIIAY

GDADVMVAGGAEKASTPLGVGGFGAARALSTRNDNPQAASRPWDKERDGFVLGDGAGMLVLE

EYEHAKKRGAKIYAELVGFGMSSDAYHMTSPPENGAGAALAMANALRDAGIEASQIGYVNAH

GTSTPAGDKAEAQAVKTIFGEAASRVLVSSTKSMTGHLLGAAGAVESIYSILALRDQAVPPT
INLDNPDEGCDLDFVPHEARQVSGMEYTLCNSFGFGGTNGSLIFKKI

YfcY[SEQ ID NO: 108]:

MGQVLPLVTRQGDRIAIVSGLRTPFARQATAFHGIPAVDLGKMVVGELLARSEIPA

EVIEQLVFGQVVQMPEAPNIAREIVLGTGMNVHTDAYSVSRACATSFQAVANVAESLMAGTI

RAGIAGGADSSSVLPIGVSKKLARVLVDVNKARTMSQRLKLFSRLRLRDLMPVPPAVAEYST

GLRMGDTAEQMAKTYGITREQQDALAHRSHQRAAQAWSDGKLKEEVMTAFIPPYKQPLVEDN

NIRGNSSLADYAKLRPAFDRKHGTVTAANSTPLTDGAAAVILMTESRAKELGLVPLGYLRSY

AFTAIDVWQDMLLGPAWSTPLALERAGLTMSDLTLIDMHEAFAAQTLANIQLLGSERFAREA

LGRAHATGEVDDSKFNVLGGSIAYGHPFAATGARMITQTLHELRRRGGGFGLVTACAAGGLG

AAMVLEAE

XapR[SEQ ID NO: 109]:

MERVYRTDLKLLRYFLAVAEELHFGRAAARLNMSQPPLSIHIKELENQLGTQLFIR

HSRSVVLTHAGKILMEESRRLLVNANNVLARIEQIGRGEAGRIELGVVGTAMWGRMRPVMRR

FLRENPNVDVLFREKMPAMQMALLERRELDAGIWRMATEPPTGFTSLRLHESAFLVAMPEEH

HLSSFSTVPLEALRDEYFVTMPPVYTDWDFLQRVCQQVGFSPVVIREVNEPQTVLAMVSMGI

GITLIADSYAQMNWPGVIFRPLKQRIPADLYIVYETQQVTPAMVKLLAALTQ

CytR[SEQ ID NO: 110]:













FabH[SEQ ID NO: 111]:

MYTKIIGTGSYLPEQVRTNADLEKMVDTSDEWIVTRTGIRERHIAAPNETVSTMGF
EAATRAIEMAGIEKDQIGLIVVATTSATHAFPSAACQIQSMLGIKGCPAFDVAAACAGFTYA
LSVADQYVKSGAVKYALVVGSDVLARTCDPTDRGTIIIFGDGAGAAVLAASEEPGIISTHLH
ADGSYGELLTLPNADRVNPENSIHLTMAGNEVFKVAVTELAHIVDETLAANNLDRSQLDWLV
PHQANLRIISATAKKLGMSMDNVVVTLDRHGNTSAASVPCALDEAVRDGRIKPGQLVLLEAF
GGGFTWGSALVRF

Mqo[SEQ ID NO: 112]:



















YfiD[SEQ ID NO: 113]:

MITGIQITKAANDDLLNSFWLLDSEKGEARCIVAKAGYAEDEVVAVSKLGDIEYRE

VPVEVKPEVRVEGGQHLNVNVLRRETLEDAVKHPEKYPQLTIRVSGYAVRFNSLTPEQQRDV

IARTFTESL

Fmt[SEQ ID NO: 114]:

MSESLRIIFAGTPDFAARHLDALLSSGHNVVGVFTQPDRPAGRGKKLMPSPVKVLA

EEKGLPVFQPVSLRPQENQQLVAELQADVMVVVAYGLILPKAVLEMPRLGCINVHGSLLPRW

RGAAPIQRSLWAGDAETGVTIMQMDVGLDTGDMLYKLSCPITAEDTSGTLYDKLAELGPQGL

ITTLKQLADGTAKPEVQDETLVTYAEKLSKEEARIDWSLSAAQLERCIRAFNPWPMSWLEIE

GQPVKVWKASVIDTATNAAPGTILEANKQGIQVATGDGILNLLSLQPAGKKAMSAQDLLNSR

REWFVPGNRLV

PyrF[SEQ ID NO: 115]:

MTLTASSSSRAVTNSPVVVALDYHNRDDALAFVDKIDPRDCRLKVGKEMFTLFGPQ

FVRELQQRGFDIFLDLKFHDIPNTAAHAVAAAADLGVWMVNVHASGGARMMTAAREALVPFG

KDAPLLIAVTVLTSMEASDLVDLGMTLSPADYAERLAALTQKCGLDGVVCSAQEAVRFKQVF

GQEFKLVTPGIRPQGSEAGDQRRIMTPEQALSAGVDYMVIGRPVTQSVDPAQTLKAINASLQ

RSA

AraA[SEQ ID NO: 116]:

MTIFDNYEVWFVIGSQHLYGPETLRQVTQHAEHVVNALNTEAKLPCKLVLKPLGTT

PDEITAICRDANYDDRCAGLVVWLHTFSPAKMWINGLTMLNKPLLQFHTQFNAALPWDSIDM

DFMNLNQTAHGGREFGFIGARMRQQHAVVTGHWQDKQAHERIGSWMRQAVSKQDTRHLKVCR

FGDNMREVAVTDGDKVAAQIKFGFSVNTWAVGDLVQVVNSISDGDVNALVDEYESCYTMTPA

TQIHGKKRQNVLEAARIELGMKRFLEQGGFHAFTTTFEDLHGLKQLPGLAVQRLMQQGYGFA

GEGDWKTAALLRIMKVMSTGLQGGTSFMEDYTYHFEKGNDLVLGSHMLEVCPSIAAEEKPIL

DVQHLGIGGKDDPARLIFNTQTGPAIVASLIDLGDRYRLLVNCIDTVKTPHSLPKLPVANAL

WKAQPDLPTASEAWILAGGAHHTVFSHALNLNDMRQFAEMHDIEITVIDNDTRLPAFKDALR

WNEVYYGFRR

ArgC[SEQ ID NO: 117]:

MLNTLIVGASGYAGAELVTYVNRHPHMNITALTVSAQSNDAGKLISDLHPQLKGIV

DLPLQPMSDISEFSPGVDVVFLATAHEVSHDLAPQFLEAGCVVFDLSGAFRVNDATFYEKYY

GFTHQYPELLEQAAYGLAEWCGNKLKEANLIAVPGCYPTAAQLALKPLIDADLLDLNQWPVI

NATSGVSGAGRKAAISNSFCEVSLQPYGVFTHRHQPEIATHLGADVIFTPHLGNFPRGILET

ITCRLKSGVTQAQVAQVLQQAYAHKPLVRLYDKGVPALKNVVGLPFCDIGFAVQGEHLIIVA

TEDNLLKGAAAQAVQCANIRFGYAETQSLI

FadR[SEQ ID NO: 118]:

MVIKAQSPAGFAEEYIIESIWNNRFPPGTILPAERELSELIGVTRTTLREVLQRLA

RDGWLTIQHGKPTKVNNFWETSGLNILETLARLDHESVPQLIDNLLSVRTNISTIFIRTAFR

QHPDKAQEVLATANEVADHADAFAELDYNIFRGLAFASGNPIYGLILNGMKGLYTRIGRHYF

ANPEARSLALGFYHKLSALCSEGAHDQVYETVRRYGHESGEIWHRMQKNLPGDLAIQGR

NudD[SEQ ID NO: 119]:

MFLRQEDFATVVRSTPLVSLDFIVENSRGEFLLGKRTNRPAQGYWFVPGGRVQKDE

TLEAAFERLTMAELGLRLPITAGQFYGVWQHFYDDNFSGTDFTTHYVVLGFRFRVSEEELLL

PDEQHDDYRWLTSDALLASDNVHANSRAYFLAEKRTGVPGL

PabA[SEQ ID NO: 120]:

MILLIDNYDSFTWNLYQYFCELGADVLVKRNDALTLADIDALKPQKIVISPGPCTP

DEAGISLDVIRHYAGRLPILGVCLGHQAMAQAFGGKVVRAAKVMHGKTSPITHNGEGVFRGL

ANPLTVTRYHSLVVEPDSLPACFDVTAWSETREIMGIRHRQWDLEGVQFHPESILSEQGHQL

LANFLHR

PurB[SEQ ID NO: 121]:

MELSSLTAVSPVDGRYGDKVSALRGIFSEYGLLKFRVQVEVRWLQKLAAHAAIKEV

PAFAADAIGYLDAIVASFSEEDAARIKTIERTTNHDVKAVEYFLKEKVAEIPELHAVSEFIH

FACTSEDINNLSHALMLKTARDEVILPYWRQLIDGIKDLAVQYRDIPLLSRTHGQPATPSTI

GKEMANVAYRMERQYRQLNQVEILGKINGAVGNYNAHIAAYPEVDWHQFSEEFVTSLGIQWN

PYTTQIEPHDYIAELFDCVARFNTILIDFDRDVWGYIALNHFKQKTIAGEIGSSTMPHKVNP

IDFENSEGNLGLSNAVLQHLASKLPVSRWQRDLTDSTVLRNLGVGIGYALIAYQSTLKGVSK

LEVNRDHLLDELDHNWEVLAEPIQTVMRRYGIEKPYEKLKELTRGKRVDAEGMKQFIDGLAL

PEEEKARLKAMTPANYIGRAITMVDELK

HycI[SEQ ID NO: 122]:

MTDVLLCVGNSMMGDDGAGPLLAEKCAAAPKGNWVVIDGGSAPENDIVAIRELRPT
RLLIVDATDMGLNPGEIRIIDPDDIAEMFMMTTHNMPLNYLIDQLKEDIGEVIFLGIQPDI
VGFYYPMTQPIKDAVETVYQRLEGWEGNGGFAQLAVEEE

In the present invention, the microorganism may be selected from the group consisting of *E. coli, Rhizobium, Bifidobacterium, Rhodococcus, Candida, Erwinia, Enterobacter, Pasteurella, Mannheimia, Actinobacillus, Aggregatibacter, Xanthomonas, Vibrio, Pseudomonas, Azotobacter, Acinetobacter, Ralstonia, Agrobacterium, Rhodobacter, Zymomonas, Bacillus, Staphylococcus, Lactococcus, Streptococcus, Lactobacillus, Clostridium, Corynebacterium, Streptomyces, Bifidobacterium, Cyanobacterium,* and *Cyclobacterium,* but the microorganism to which the present invention can be applied is not limited thereto. Preferably, the microorganism of the present invention may be constructed by introducing a recombinant vector into a microorganism selected from the group consisting of *E. coli, Pseudomonas* sp., *Corynebacterium* sp., and *Rhodococcus* sp., but is not limited thereto. As *E. coli,* one selected from among the *E. coli* strains shown in Table 2 below is preferably used, but those skilled in the art will appreciate that other *E. coli* strains expected to exhibit similar effects may also be used. The recombinant microorganism of the present invention may be constructed by using *Pseudomonas putida* as *Pseudomonas* sp., *Corynebacterium glutamicum* as *Corynebacterium* sp., and *Rhodococcus opacus* as *Rhodococcus* sp., but this is an example of a representative microorganism, and the microorganism to which the present invention is applied is not limited thereto.

In addition, the use of the screened gene can produce a useful substance using malonyl-CoA as a substrate or an intermediate in high yield by only a very simple genetic manipulation.

Therefore, in a first further aspect, the present invention is directed to a recombinant microorganism having increased malonyl-CoA producing ability wherein expression of one or more genes selected from the group consisting of the following genes:
*fabF* (3-oxoacyl-[acyl-carrier-protein] synthase II);
*yfcY* (beta-ketoacyl-CoA thiolase);
*xapR* (transcriptional activator of *xapAB*);
*cytR* (transcriptional repressor for *deo* operon, *udp, cdd, tsx, nupC* and *nupG*) ;
*fabH* (3-oxoacyl-[acyl-carrier-protein] synthase III);
*mqo* (malate dehydrogenase);
*yfiD* (pyruvate formate lyase subunit);
*fmt* (10-formyltetrahydrofolate:L-methionyl-tRNA(fMet)N-formyltransferase);
*pyrF* (orotidine-5'-phosphate decarboxylase);
*araA* (L-arabinose isomerase);
*fadR* (negative regulator for fad regulon and positive regulator of *fabA*);
*pabA* (aminodeoxychorismate synthase, subunit II);
*purB* (adenylosuccinate lyase); and
*hycI* (protease involved in processing C-terminal end of HycE), in a microorganism having malonyl-CoA producing ability is decreased compared to that in a wild-type microorganism.

The microorganism having malonyl-CoA producing ability means a microorganism inherently having malonyl-CoA producing ability or externally introduced with malonyl-CoA producing ability.

The present invention is also directed to a method of producing a useful substance using malonyl-CoA as a substrate or an intermediate, the method comprising the steps of:
(a) constructing a recombinant microorganism in which a gene involved in production of the useful substance is additionally introduced, or expression of the gene is increased, or a gene involved in production of the useful substance is additionally deleted, or expression of the gene is decreased;
(b) culturing the constructed microorganism; and
(c) recovering the useful substance from the cultured microorganism.

In the present invention, the useful substance may be one or more useful substances selected from among:
a polyketide compound composed of actinorhodin, doxorubicin, daunorubicin, oxytetracycline, rapamycin, lovastatin, SEK4, SEK4b, SEK34, SEK15, SEK26, FK506, DMAC, aklavinone, aklanonic acid, epsilon-rhodomycinone, aloesin, aloenin, barbaloin, 5,7-dihydroxy-2-methylchromone, erythromycin, rifamycin, avermectin, geldanamycin, ivermectin, doxycycline, anthramycin, penicillic acid, calicheamicin, epothilone, tetracenomycin, frenolicin, triacetic acid lactone, 6-methylsalicylic acid, and aloesone;
a phenylpropanoid compound composed of pinocembrin, dihydrokaempferol, eriodictyol, dihydroquercetin, daidzein, genistein, apigenin, luteolin, kaempferol, quercetin, catechin, pelargonidin, cyanidin, afzelechin, myricetin, fisetin, galangin, hesperetin, tangeritin, delphinidin, epicatechin, chrysin, resveratrol, and naringenin;
a biofuel group composed of pentane, hexane, heptane, octane, nonane, decane, undecane, dodecane, tridecane, tetradecane, pentadecane, hexadecane, heptadecane, octadecane, nonadecane, icosane, pentanol, hexanol, heptanol, octanol, nonanol, decanol, undecanol, dodecanol, tridecanol, tetradecanol, pentadecanol, hexadecanol, heptadecanol, octadecanol, nonadecanol, icosanol, methyl caprate, methyl laurate, methyl myristate, methyl palmitate, methyl palmitoleate, methyl stearate, methyl oleate, methyl linoleate, methyl linolenate, methyl arachidate, methyl paullinate, methyl erucate, ethyl caprate, ethyl laurate, ethyl myristate, ethyl palmitate, ethyl palmitoleate, ethyl stearate, ethyl oleate, ethyl linoleate, ethyl linolenate, ethyl arachidate, ethyl paullinate, and methyl erucate;
a lipid compound composed of ceramide, palmitate, and sphingosine; and
3-hydroxypropionic acid, but is not limited thereto. In other words, the biosensor (i.e., recombinant microorganism) of the present invention can be applied to high-valued products such as other any malonyl-CoA-derived natural substances, compounds, biofuels, and the like, besides the above-described useful substances.

The present invention is also directed to a recombinant microorganism for producing 6-methylsalicylic acid in which genes that encode 6-methylsalicylic acid synthase (6MSAS) and 4'-phosphopantetheinyl transferase (Sfp) are additionally introduced or expression of the genes is increased in the recombinant microorganism.

The 6MSAS may be derived from *Penicillium patulum* (or *Penicillium griseofulvum), Aspergillus terreus, Aspergillus aculeatus, Aspergillus niger, Aspergillus westerdijkiae,* Byssochlamys nivea, *Glarea lozoyensis, Penicillium expansum,* or *Streptomyces antibioticus,* but is not limited thereto.

The Sfp may be derived from *Bacillus subtilis, Corynebacterium ammoniagenes, Escherichia coli, Homo sapiens, Mycobacterium tuberculosis, Pseudomonas aeruginosa, Ricinus communis, Saccharomyces cerevisiae, Spinacia oleracea, Stigmatella aurantiaca, Streptomyces coelicolor, Streptomyces pneumonia, Streptomyces verticillus,* or *Vibrio harveyi,* but is not limited thereto.

The gene whose expression is decreased compared to that in a wild-type microorganism may be one or more selected from the group consisting of *pabA, fabF, xapR* and *ytcY,* but are not limited thereto.

The recombinant microorganism for producing 6-methylsalicylic acid may be characterized in that genes that encode one or more enzymes selected from the group consisting of glucose-6-phosphate dehydrogenase (Zwf), malate dehydrogenase (Mdh), phosphoglycerate dehydrogenase (SerA), acetyl-CoA carboxylase (AccBC and AccD1), glyceraldehyde 3-phosphate dehydrogenase (GapA), phosphoglycerate kinase (Pgk), acetyl-CoA synthetase (Acs), and pyruvate dehydrogenase (PDH: AceEF and Lpd) are additionally introduced or expression of the genes is increased in the recombinant microorganism.

The Zwf, Mdh, SerA, GapA, Pgk, Acs, AceE, AceF and Lpd may be derived from *Escherichia coli (E. coli),* but are not limited thereto. AccBC and AccD1 may be derived from *Corynebacterium glutamicum,* but are not limited thereto and can be extended to enzymes corresponding to other microorganism-derived acetyl-CoA carboxylases.

The amino acid sequence of each of enzymes, which are screened and used in the present invention, is as follows, but may have a substitution, deletion or addition of one or more amino acids in the amino acid sequences, and in this case, the resulting mutant amino acid sequence may exhibit a function equal to or better than that in the present invention. For example, it will be obvious that if the structural configuration of the mutant amino acid sequence is identical or similar to the wild-type amino acid sequence, and thus exhibits the same or higher effect on a substrate, the mutant amino acid sequence also falls within the scope of the present invention. In the same context, it will be obvious that besides enzymes used in the present invention, other microorganism-derived enzymes having an identical or similar function can be properly applied to the present invention, and the application of a corresponding enzyme within a range that can be easily applied by a person of ordinary skill in the art falls within the scope of the present invention.

Zwf[SEQ ID NO: 171]:

MAVTQTAQACDLVIFGAKGDLARRKLLPSLYQLEKAGQLNPDTRIIGVGRADWDKA

AYTKVVREALETFMKETIDEGLWDTLSARLDFCNLDVNDTAAFSRLGAMLDQKNRITINYF

AMP

PSTFGAICKGLGEAKLNAKPARVVMEKPLGTSLATSQEINDQVGEYFEECQVYRID
HYLGKETVLNLLALRFANSLFVNNWDNRTIDHVEITVAEEVGIEGRWGYFDKAGQMRDMIQ
NHLLQILCMIAMSPPSDLSADSIRDEKVKVLKSLRRIDRSNVREKTVRGQYTAGFAQGKKV
PGYLEEEGANKSSNTETFVAIRVDIDNWRWAGVPFYLRTGKRLPTKCSEVVVYFKTPELNL
FKESWQDLPQNKLTIRLQPDEGVDIQVLNKVPGLDHKHNLQITKLDLSYSETFNQTHLADA
YERLLLETMRGIQALFVRRDEVEEAWKWVDSITEAWAMDNDAPKPYQAGTWGPVASVAMIT
RDGRSWNEFE

Mdh[SEQ ID NO: 172]:

MKVAVLGAAGGIGQALALLLKTQLPSGSELSLYDIAPVTPGVAVDLSHIPTAVKIK

GFSGEDATPALEGADVVLISAGVARKPGMDRSDLFNVNAGIVKNLVQQVAKTCPKACIGII

TNPVNTTVAIAAEVLKKAGVYDKNKLFGVTTLDIIRSNTFVAELKGKQPGEVEVPVIGGHS

GVTILPLLSQVPGVSFTEQEVADLTKRIQNAGTEVVEAKAGGGSATLSMGQAAARFGLSLV

RALQGEQGVVECAYVEGDGQYARFFSQPLLLGKNGVEERKSIGTLSAFEQNALEGMLDTLK

KDIALGEEFVNK

SerA[SEQ ID NO: 173]:

MAKVSLEKDKIKFLLVEGVHQKALESLRAAGYTNIEFHKGALDDEQLKESIRDAHF

IGLRSRTHLTEDVINAAEKLVAIGCFCIGTNQVDLDAAAKRGIPVFNAPFSNTRSVAELVI

GELLLLLRGVPEANAKAHRGVWNKLAAGSFEARGKKLGIIGYGHIGTQLGILAESLGMYVY

FYDIENKLPLGNATQVQHLSDLLNMSDVVSLHVPENPSTKNMMGAKEISLMKPGSLLINAS

RGTVVDIPALCDALASKHLAGAAIDVFPTEPATNSDPFTSPLCEFDNVLLTPHIGGSTQEA

QENIGLEVAGKLIKYSDNGSTLSAVNFPEVSLPLHGGRRLMHIHENRPGVLTALNKIFAEQ

GVNIAAQYLQTSAQMGYVVIDIEADEDVAEKALQAMKAIPGTIRARLLY

GapA[SEQ ID NO: 174]:

MTIKVGINGFGRIGRIVFRAAQKRSDIEIVAINDLLDADYMAYMLKYDSTHGRFDG

TVEVKDGHLIVNGKKIRVTAERDPANLKWDEVGVDVVAEATGLFLTDETARKHITAGAKKV

VMTGPSKDNTPMFVKGANFDKYAGQDIVSNASCTTNCLAPLAKVINDNFGIIEGLMTTVHA

TTATQKTVDGPSHKDWRGGRGASQNIIPSSTGAAKAVGKVLPELNGKLTGMAFRVPTPNVS

VVDLTVRLEKAATYEQIKAAVKAAAEGEMKGVLGYTEDDVVSTDFNGEVCTSVFDAKAGIA

LNDNFVKLVSWYDNETGYSNKVLDLIAHISK

Pgk[SEQ ID NO: 175]:

MSVIKMTDLDLAGKRVFIRADLNVPVKDGKVTSDARIRASLPTIELALKQGAKVMV

TSHLGRPTEGEYNEEFSLLPVVNYLKDKLSNPVRLVKDYLDGVDVAEGELVVLENVRFNKG

EKKDDETLSKKYAALCDVFVMDAFGTAHRAQASTHGIGKFADVACAGPLLAAELDALGKAL

KEPARPMVAIVGGSKVSTKLTVLDSLSKIADQLIVGGGIANTFIAAQGHDVGKSLYEADLV

DEAKRLLTTCNIPVPSDVRVATEFSETAPATLKSVNDVKADEQILDIGDASAQELAEILKN

AKTILWNGPVGVFEFPNFRKGTEIVANAIADSEAFSIAGGGDTLAAIDLFGIADKISYIST

GGGAFLEFVEGKVLPAVAMLEERAKK

Acs[SEQ ID NO: 176]:

MSQIHKHTIPANIADRCLINPQQYEAMYQQSINVPDTFWGEQGKILDWIKPYQKVK

NTSFAPGNVSIKWYEDGTLNLAANCLDRHLQENGDRTAIIWEGDDASQSKHISYKELHRDV

CRFANTLLELGIKKGDVVAIYMPMVPEAAVAMLACARIGAVHSVIFGGFSPEAVAGRIIDS

NSRLVITSDEGVRAGRSIPLKKNVDDALKNPNVTSVEHVVVLKRTGGKIDWQEGRDLWWHD

LVEQASDQHQAEEMNAEDPLFILYTSGSTGKPKGVLHTTGGYLVYAALTFKYVFDYHPGDI

YWCTADVGWVTGHSYLLYGPLACGATTLMFEGVPNWPTPARMAQVVDKHQVNILYTAPTAI

RALMAEGDKAIEGTDRSSLRILGSVGEPINPEAWEWYWKKIGNEKCPVVDTWWQTETGGFM

ITPLPGATELKAGSATRPFFGVQPALVDNEGNPLEGATEGSLVITDSWPGQARTLFGDHER

FEQTYFSTFKNMYFSGDGARRDEDGYYWITGRVDDVLNVSGHRLGTAEIESALVAHPKIAE

AAVVGIPHNIKGQAIYAYVTLNHGEEPSPELYAEVRNWVRKEIGPLATPDVLHWTDSLPKT

RSGKIMRRILRKIAAGDTSNLGDTSTLADPGVVEKLLEEKQAIAMPS

AceE[SEQ ID NO: 177]:

MSERFPNDVDPIETRDWLQAIESVIREEGVERAQYLIDQLLAEARKGGVNVAAGTG
ISNYINTIPVEEQPEYPGNLELERRIRSAIRWNAIMTVLRASKKDLELGGHMASFQSSATI
YDVCFNHFFRARNEQDGGDLVYFQGHISPGVYARAFLEGRLTQEQLDNFRQEVHGNGLSSY
PHPKLMPEFWQFPTVSMGLGPIGAIYQAKFLKYLEHRGLKDTSKQTVYAFLGDGEMDEPES
KGAITIATREKLDNLVFVINCNLQRLDGPVTGNGKIINELEGIFEGAGWNVIKVMWGSRWD
ELLRKDTSGKLIQLMNETVDGDYQTFKSKDGAYVREHFFGKYPETAALVADWTDEQIWALN
RGGHDPKKIYAAFKKAQETKGKATVILAHTIKGYGMGDAAEGKNIAHQVKKMNMDGVRHIR
DRFNVPVSDADIEKLPYITFPEGSEEHTYLHAQRQKLHGYLPSRQPNFTEKLELPSLQDFG
ALLEEQSKEISTTIAFVRALNVMLKNKSIKDRLVPIIADEARTFGMEGLFRQIGIYSPNGQ
QYTPQDREQVAYYKEDEKGQILQEGINELGAGCSWLAAATSYSTNNLPMIPFYIYYSMFGF
QRIGDLCWAAGDQQARGFLIGGTSGRTTLNGEGLQHEDGHSHIQSLTIPNCISYDPAYAYE
VAVIMHDGLERMYGEKQENVYYYITTLNENYHMPAMPEGAEEGIRKGIYKLETIEGSKGKV
QLLGSGSILRHVREAAEILAKDYGVGSDVYSVTSFTELARDGQDCERWNMLHPLETPRVPY
IAQVMNDAPAVASTDYMKLFAEQVRTYVPADDYRVLGTDGFGRSDSRENLRHHFEVDASYV
VVAALGELAKRGEIDKKVVADAIAKFNIDADKVNPRLA

AceF[SEQ ID NO: 178]:

MAIEIKVPDIGADEVEITEILVKVGDKVEAEQSLITVEGDKASMEVPSPQAGIVKE

IKVSVGDKTQTGALIMIFDSADGAADAAPAQAEEKKEAAPAAAPAAAAAKDVNVPDIGSDE

VEVTEILVKVGDKVEAEQSLITVEGDKASMEVPAPFAGTVKEIKVNVGDKVSTGSLIMVFE

VAGEAGAAAPAAKQEAAPAAAPAPAAGVKEVNVPDIGGDEVEVTEVMVKVGDKVAAEQSLI

TVEGDKASMEVPAPFAGVVKELKVNVGDKVKTGSLIMIFEVEGAAPAAAPAKQEAAAPAPA

AKAEAPAAAPAAKAEGKSEFAENDAYVHATPLIRRLAREFGVNLAKVKGTGRKGRILREDV

QAYVKEAIKRAEAAPAATGGGIPGMLPWPKVDFSKFGEIEEVELGRIQKISGANLSRNWVM

IPHVTHFDKTDITELEAFRKQQNEEAAKRKLDVKITPVVFIMKAVAAALEQMPRFNSSLSE

DGQRLTLKKYINIGVAVDTPNGLVVPVFKDVNKKGIIELSRELMTISKKARDGKLTAGEMQ

GGCFTISSIGGLGTTHFAPIVNAPEVAILGVSKSAMEPVWNGKEFVPRLMLPISLSFDHRV

IDGADGARFITIINNTLSDIRRLVM

Lpd[SEQ ID NO: 179]:

















AccBC[SEQ ID NO: 180]:

VSVETRKITKVLVANRGEIAIRVFRAARDEGIGSVAVYAEPDADAPFVSYADEAFA

LGGQTSAESYLVIDKIIDAARKSGADAIHPGYGFLAENADFAEAVINEGLIWIGPSPESIR

SLGDKVTARHIADTAKAPMAPGTKEPVKDAAEVVAFAEEFGLPIAIKAAFGGGGRGMKVAY

KMEEVADLFESATREATAAFGRGECFVERYLDKARHVEAQVIADKHGNVVVAGTRDCSLQR

RFQKLVEEAPAPFLTDDQRERLHSSAKAICKEAGYYGAGTVEYLVGSDGLISFLEVNTRLQ

VEHPVTEETTGIDLVREMFRIAEGHELSIKEDPAPRGHAFEFRINGEDAGSNFMPAPGKIT

SYREPQGPGVRMDSGVVEGSEISGQFDSMLAKLIVWGDTREQALQRSRRALAEYVVEGMPT

VIPFHQHIVENPAFVGNDEGFEIYTKWIEEVWDNPIAPYVDASELDEDEDKTPAQKVVVEI

NGRRVEVALPGDLALGGTAGPKKKAKKRRAGGAKAGVSGDAVAAPMQGTVIKVNVEEGAEV

NEGDTVVVLEAMKMENPVKAHKSGTVTGLTVAAGEGVNKGVVLLEIK

AccD1[SEQ ID NO: 181]:

MTISSPLIDVANLPDINTTAGKIADLKARRAEAHFPMGEKAVEKVHAAGRLTARER

LDYLLDEGSFIETDQLARHRTTAFGLGAKRPATDGIVTGWGTIDGREVCIFSQDGTVFGGA

LGEVYGEKMIKIMELAIDTGRPLIGLYEGAGARIQDGAVSLDFISQTFYQNIQASGVIPQI
SVIMGACAGGNAYGPALTDFVVMVDKTSKMFVTGPDVIKTVTGEEITQEELGGATTHMVTA
GNSHYTAATDEEALDWVQDLVSFLPSNNRSYTPLEDFDEEEGGVEENITADDLKLDEIIPD
SATVPYDVRDVIECLTDDGEYLEIQADRAENVVIAFGRIEGQSVGFVANQPTQFAGCLDID
SSEKAARFVRTCDAFNIPIVMLVDVPGFLPGAGQEYGGILRRGAKLLYAYGEATVPKITVT
MRKAYGGAYCVMGSKGLGSDINLAWPTAQIAVMGAAGAVGFIYRKELMAADAKGLDTVALA
KSFEREYEDHMLNPYHAAERGLIDAVILPSETRGQISRNLRLLKHKNVTRPARKHGNMPL

The present invention is also directed to a method of producing 6-methylsalicylic acid, the method comprising the steps of:
(a) culturing the above-described recombinant microorganism; and
(b) recovering the 6-methylsalicylic acid from the cultured microorganism.

The method of producing 6-methylsalicylic acid of the present invention may comprises culturing the recombinant microorganism by adding 1-50 g/L of glucose or 1-100 g/L of glycerol as a carbon source.

The present invention is also directed to a recombinant microorganism for producing aloesone in which a gene that encodes aloesone synthase is additionally introduced or expression of the gene is increased in the recombinant microorganism. In the present invention, the aloesone synthase may be *ALS* or *PKS3,* the ALS may be derived from *Rheum palmatum,* and the PKS3 may be derived from *Aloe arborescens,* but aloesone synthase other than the aloesone synthase can be applied to the present invention.

The gene whose expression is decreased compared to that in a wild-type microorganism may be *pabA,* but are not limited thereto.

The recombinant microorganism for producing aloesone may be characterized in that genes that encode one or more enzymes selected from the group consisting of glucose-6-phosphate dehydrogenase (Zwf), malate dehydrogenase (Mdh), phosphoglycerate dehydrogenase (SerA), acetyl-CoA carboxylase (AccBC and AccD1), glyceraldehyde 3-phosphate dehydrogenase (GapA), phosphoglycerate kinase (Pgk), acetyl-CoA synthetase (Acs), and pyruvate dehydrogenase (PDH: AceEF and Lpd) are additionally introduced or expression of the genes is increased in the recombinant microorganism.

The Zwf, Mdh, SerA, GapA, Pgk, Acs, AceE, AceF and Lpd may be derived from *Escherichia coli (E. coli),* but are not limited thereto. AccBC and AccD1 may be derived from *Corynebacterium glutamicum,* but are not limited thereto and can be extended to enzymes corresponding to other microorganism-derived acetyl-CoA carboxylases.

The present invention is also directed to a method of producing aloesone, the method comprising the steps of:
(a) culturing the above-described recombinant microorganism; and
(b) recovering the aloesone from the cultured microorganism.

The method of producing aloesone of the present invention may comprises culturing the recombinant microorganism by adding 1-50 g/L of glucose or 1-100 g/L of glycerol as a carbon source.

The present invention is also directed to a recombinant microorganism for producing resveratrol in which genes that encode tyrosine ammonia-lyase (TAL), 4-coumarate:CoA ligase (4CL) and stilbene synthase (STS) are additionally introduced or expression of the genes is increased in the recombinant microorganism.

TAL may be derived from *Rhodobacter capsulatus, Clitoria ternatea, Fragaria x ananassa, Rhodobacter sphaeroides, Zea mays* or *Saccharothrix espanaensis,*

4CL may be derived from *Arabidopsis thaliana, Streptomyces coelicolor, Acetobacterium woodii, Agastache rugose, Avena sativa, Camellia sinensis, Centaurium erythraea, Cephalocereus senilis, Cocos nucifera, Eriobotrya japonica, Erythrina cristagalli, Forsythia sp., Fragaria x ananassa, Glycine max, Gossypium hirsutum, Hibiscus cannabinus, Larix cajanderi, Larix gmelinii, Larix kaempferi, Larix kamtschatica, Larix sibirica, Larix sukaczewii, Lithospermum erythrorhizon, Lolium perenne, Lonicera japonica, Metasequoia glyptostroboides, Nicotiana tabacum, Ocimum basilicum, Ocimum tenuiflorum, Oryza sativa, Paulownia tomentosa, Petroselinum crispum, Phyllostachys bambusoides, Physcomitrella patens, Picea abies, Pinus radiate, Pinus taeda, Pisum sativum, Platycladus orientalis, Polyporus hispidus, Populus tomentosa, Populus tremuloides, Populus x canadensis, Prunus avium, Pueraria montana, Robinia pseudoacacia, Ruta graveolens, Saccharomyces cerevisiae, Salix babylonica, Solanum lycopersicum, Solanum tuberosum, Sorbus aucuparia, Triticum aestivum* or *Vitis vinifera,* and

STS may be derived from *Arachis hypogaea, Pinus densiflora, Pinus massoniana, Pinus strobus, Polygonum cuspidatum, Psilotum nudum* or *Vitis vinifera,* but are not limited thereto.

The 4-coumarate:CoA ligase may be either a mutant enzyme in which amino acids are mutated to I250L/N404K/I461V in the amino acid sequence represented by SEQ ID NO: 128 or a mutant enzyme in which amino acids are mutated to A294G/A318G in the amino acid sequence represented by SEQ ID NO: 131, but is not limited thereto.

The gene whose expression is decreased compared to that in a wild-type microorganism may be one or more selected from the group consisting of *pabA, yfiD, mqo, xapR, purB, fabH, fabF, ytcY, argC, nudD, araA, fadR, cytR, fmt* and *pyrF,* but are not limited thereto.

The present invention is also directed to a method of producing resveratrol, the method comprising the steps of:
(a) culturing the above-described recombinant microorganism; and
(b) recovering the resveratrol from the cultured microorganism.

The method of producing resveratrol of the present invention may comprises culturing the recombinant microorganism by adding 1-50 g/L of glucose or 1-100 g/L of glycerol as a carbon source.

The present invention is also directed to a recombinant microorganism for producing naringenin in which genes that encode tyrosine ammonia-lyase (TAL), 4-coumarate:CoA ligase (4CL), chalcone synthase (CHS), and chalcone isomerase (CHI) are additionally introduced in the above-described recombinant microorganism or expression of the genes is increased.

TAL may be derived from *Rhodobacter capsulatus, Clitoria ternatea, Fragaria x ananassa, Rhodobacter sphaeroides, Zea mays* or *Saccharothrix espanaensis,*

4CL may be derived from *Arabidopsis thaliana, Streptomyces coelicolor, Acetobacterium woodii, Agastache rugose, Avena sativa, Camellia sinensis, Centaurium erythraea, Cephalocereus senilis, Cocos nucifera, Eriobotrya japonica, Erythrina cristagalli, Forsythia sp., Fragaria x ananassa, Glycine max, Gossypium hirsutum, Hibiscus cannabinus, Larix cajanderi, Larix gmelinii, Larix kaempferi, Larix kamtschatica, Larix sibirica, Larix sukaczewii, Lithospermum erythrorhizon, Lolium perenne, Lonicera japonica, Metasequoia glyptostroboides, Nicotiana tabacum, Ocimum basilicum, Ocimum tenuiflorum, Oryza sativa, Paulownia tomentosa, Petroselinum crispum, Phyllostachys bambusoides, Physcomitrella patens, Picea abies, Pinus radiate, Pinus taeda, Pisum sativum, Platycladus orientalis, Polyporus hispidus, Populus tomentosa, Populus tremuloides, Populus x canadensis, Prunus avium, Pueraria montana, Robinia pseudoacacia, Ruta graveolens, Saccharomyces cerevisiae, Salix babylonica, Solanum lycopersicum, Solanum tuberosum, Sorbus aucuparia, Triticum aestivum* or *Vitis vinifera,*

CHS may be derived from *Freesia hybrid cultivar, Medicago sativa, Physcomitrella patens, Plagiochasma appendiculatum, Triticum aestivum, Vitis vinifera, Citrus sinensis, Arabidopsis thaliana, Avena sativa, Daucus carota, Fagopyrum esculentum, Glycine max, Glycyrrhiza echinata, Humulus lupulus, Hypericum androsaemum, Petroselinum crispum, Physcomitrella patens, Rubus idaeus, Scutellaria baicalensis, Xanthisma gracile, Cosmos sulphureus, Gerbera hybrid cultivar, Hordeum vulgare, Juglans sp., Phaseolus vulgaris, Pueraria montana, Secale cereal, Silene sp., Sinapis alba, Spinacia oleracea, Stellaria longipes, Tulipa hybrid cultivar, Verbena sp.,* or *Petunia x hybrida,* and

CHI may be derived from *Perilla frutescens, Ginkgo biloba, Trigonella foenumgraecum, Medicago sativa, Scutellaria baicalensis, Glycine max, Cephalocereus senilis, Citrus sinensis, Glycyrrhiza echinata, Glycyrrhiza uralensis, Lilium candidum, Morella rubra, Petunia x hybrid, Phaseolus vulgaris, Soja hispida, Tulipa hybrid cultivar, Arabidopsis thaliana* or *Nicotiana tabacum,* but are not limited thereto.

4-coumarate:CoA ligase may be a mutant enzyme in which amino acids are mutated to I250L/N404K/I461V in the amino acid sequence represented by SEQ ID NO: 128, but is not limited thereto.

The gene whose expression is decreased compared to that in a wild-type microorganism may be one or more selected from the group consisting of *fadR, hycI* and *xapR.*

The present invention is also directed to a method of producing naringenin, the method comprising the steps of:
(a) culturing the above-described recombinant microorganism; and
(b) recovering the naringenin from the cultured microorganism.

The method of producing naringenin of the present invention may comprises culturing the recombinant microorganism by adding 1-50 g/L of glucose or 1-100 g/L of glycerol as a carbon source.

In the present invention, "regulation" of a gene includes all gene deletion, expression inhibition, expression promotion, knockdown, promoter replacement, and inducing expression of the gene by a technique such as a regulatory mechanism, and is meant to include evolving or mutating one or more of enzymes present in the biosynthetic pathway.

In the present invention, "knockdown" means significantly reducing the expression level of a gene so as to reduce the function of the gene, unlike "knockout" that completely blocks gene expression. It can be regulated at the gene transcript level or at the protein level. However, since the present invention is significant in inhibiting or reducing the expression of a gene encoding an enzyme involved in the corresponding pathway, the use of any of "knockdown" and "knockout" can achieve the desired object.

As used herein, the term "vector" means a DNA construct containing a DNA sequence operably linked to a suitable control sequence capable of effecting the expression of the DNA in a suitable host. The vector may be a plasmid, a phage particle, or simply a potential genomic insert. Once incorporated into a suitable host, the vector may replicate and function independently of the host genome, or may in some instances, integrate into the genome itself. In the present specification, "plasmid" and "vector" are sometimes used interchangeably, as the plasmid is the most commonly used form of vector. For the purpose of the present invention, the plasmid vector is preferably used. A typical plasmid vector which can be used for this purpose contains: (a) a replication origin by which replication occurs efficiently such that several hundred plasmid vectors per host cell are created; (b) an antibiotic-resistant gene by which host cells transformed with the plasmid vector can be selected; and (c) restriction enzyme cutting sites into which foreign DNA fragments can be inserted. Even if suitable restriction enzyme cutting sites are not present in the vector, the use of a conventional synthetic oligonucleotide adaptor or linker enables the easy ligation between the vector and the foreign DNA fragments. After ligation, the vector should be transformed into suitable host cells. The transformation can be easily achieved by the calcium chloride method or electroporation (Neumann, et al., EMBO J., 1:841, 1982).

The promoter of the vector may be constructive or inductive, and may be further modified to achieve the effects of the present invention. Furthermore, the expression vector includes a selective marker for selecting a host cell containing the vector, and a replicable expression vector includes a replication origin. The vector may be self-replicating, or may be integrated into the host genome DNA. Preferably, a gene inserted into the vector is irreversibly fused into the genome of the host cell, such that the expression of the gene in the cells is stably continued for a long period of time).

A nucleotide sequence is "operably linked" when it is placed into a functional relationship with another nucleotide sequence. The nucleotide sequence may be a gene and a control sequence (s) linked to be capable of expressing the gene when it binds to a control sequence(s) (e.g., transcription-activating protein). For example, DNA for a pre-sequence or a secretory leader is operably linked to DNA encoding polypeptide when expressed as pre-protein participating in secretion of polypeptide; a promoter or an enhancer is operably linked to a coding sequence when affecting the transcription of the sequence; and a ribosome binding site(RBS) is operably linked to a coding sequence when affecting the transcription of the sequence, or to a coding sequence when arranged to facilitate translation. Generally, the term "operably linked" means that the DNA linked sequences are contiguous, and in the case of the secretory leader, are contiguous and present in a reading frame. However, an enhancer is not necessarily contiguous. The linkage between these sequences is performed by ligation at a convenient restriction enzyme site. However, when the site does not exist, a synthetic oligonucleotide adaptor or a linker is used according to a conventional method.

As is well known in the art, in order to increase the expression level of a transfected gene in a host cell, a corresponding gene should be operably linked to transcription and translation expression control sequences which are operated in a selected expression host. Preferably, the expression control sequences and the corresponding gene are included in one recombinant vector together with a bacterial selection marker and a replication origin. When a host cell is a eukaryotic cell, a recombinant vector should further include an expression marker which is useful in a eukaryotic expression host.

The host cell transformed by the aforementioned recombinant vector constitutes another aspect of the present invention. As used herein, the term "transformation" means that DNA can be replicated as a factor outside of chromosome or by means of completion of the entire chromosome by introducing DNA as a host.

Of course, it should be understood that all vectors and expression control sequences do not equally function to express DNA sequences according to the present invention. Similarly, all hosts do not equally function with respect to the same expression system. However, one skilled in the art may appropriately select from among various vectors, expression control sequences, and hosts without either departing from the scope of the present invention or bearing excessive experimental burden. For example, a vector must be selected considering a host, because the vector must be replicated in the host. Specifically, the copy number of the vector, the ability of regulating the copy number and the expression of other protein encoded by the corresponding vector (e.g., the expression of an antibiotic marker) should also be considered.

In addition, the gene introduced in the present invention may be introduced into the genome of a host cell and present as a chromosomal factor. It will be obvious to those skilled in the art to which the present invention pertains that even when the gene is inserted into the genomic chromosome of a host cell, it has the same effect as when the recombinant vector is introduced into the host cell as described above.

### EXAMPLES

Hereinafter, the present invention will be described in further detail with reference to examples. It will be obvious to a person having ordinary skill in the art that these examples are for illustrative purposes only and are not to be construed to limit the scope of the present invention.

### Example 1. Construction of Type III Polyketide Synthase-Based Malonyl-CoA Biosensor

### 1.1. Performance Test for Various Type III Polyketide Synthases

The restriction enzymes used in this Example and the following Examples were purchased from New England Labs (USA) or Enzynomics (Korea), and the PCR polymerase was purchased from Biofact (Korea). Others are marked separately. In addition, the introduced exogenous genes in this Example and the following Examples are summarized in Table 1 below together with the accession numbers in a database containing information thereon and the sequences thereof. In addition, unless otherwise specified, the *E. coli* strain used in cloning was a DH5α strain which was cultured in a LB medium (per liter: 10 g tryptone, 5 g yeast extract and 10g NaCl) or on an LB agar plate (1/5%, v/v). Furthermore, if necessary, a suitable concentration of an antibiotic (50µg/mL kanamycin, 100µg/mL ampicillin and/or 100µg/mL spectinomycin) was added.

In the present invention, red-colored flaviolin was produced from five molecules of malonyl-CoA using type III polyketide synthase RppA, and used as an indicator of intracellular malonyl-CoA level (FIG. 1). To this end, the performances of various III polyketide synthases were compared and tested. Type III polyketide synthase-encoding *rppA* genes from a total of five *Actinomycetes* strains (*Streptomyces griseus, Streptomyces coelicolor, Streptomyces avermitilis, Saccharopolyspora erythraea, Streptomyces aculeolatus*) were cloned, and each of the genes was transformed into an *E. coli* BL21(DE3) strain. Then, each of the constructed strains was inoculated into a flask containing M9 minimal medium supplemented with 10 g/L of glucose, and then treated with 0.5 mM isopropyl-β-D-1-thiogalactopyranoside (IPTG) in an initial exponential phase. Next, each strain was cultured for 48 hours, and the production of flaviolin was observed. At this time, the highest flaviolin production was observed in the strain in which the *S. griseus-based* enzyme Sgr_RppA was expressed (25.7 mg/L; FIG. 2A). The constructed plasmids were based on pET-30a(+) (Novagen) and named pET-Sgr_rppA, pET-Sco_rppA, pET-Sma_rppA, pET-Sen_rppA, and pET-Sac_rppA, respectively. For construction of pET-Sgr_rppA, the *rppA* gene was amplified using the genomic DNA of *S. griseus* as a template and the primer pair of SEQ ID NO: 1 and SEQ ID NO: 2. The gene was cloned into the pET-30a(+) plasmid at EcoRI and BamHI sites using Gibson assembly (Gibson DG, et al. (2009), Nature Methods 6:343-345). Other plasmids were also constructed by the same method as described above. The M9 minimal medium contained, per liter, the following components: 12.8 g Na₂HPO₄·7H₂O, 3 g KH₂PO₄, 0.5 g NaCl, 1 g NH₄Cl, 2 mM MgSO₄, 0.1 mM CaCl₂.
[SEQ ID NO: 1] 5'-CTTTAAGAAGGAGATATACATATGGCGACCCTGTGCCGACC-3'
[SEQ ID NO: 2] 5'-CTTGTCGACGGAGCTCGAATTCATTAGCCGGACAGCGCAACGC-3'

**[Table 1]**

| Information on exogenous genes proposed in the present invention | | |
|---|---|---|
| **Gene** | **Organism** | **Accession Number *** |
| *Sgr_rppA* | *Streptomyces griseus* | BAE07216 |
| *Sco_rppA* | *Streptomyces coelicolor* | CAC01488 |
| *Sma_rppA* | *Streptomyces avermitilis* | BAC74842 |
| *Sen_rppA* | *Saccharopolyspora erythraea* | AAL78053 |
| *Sac_rppA* | *Streptomyces aculeolatus* | ABS50451 |
| *whiE_*ORFII | *Streptomyces coelicolor* | CAB45605 |
| *AaPCS* | *Aloe arborescens* | AAX35541 |
| *AaOKS* | *Aloe arborescens* | AAT48709 |
| *AaPKS4* | *Aloe arborescens* | ACR19997 |
| *AaPKS5* | *Aloe arborescens* | ACR19998 |
| *sfp* | *Bacillus subtilis* | ABV89950 |
| *Pg6MSAS* | *Penicillium griseofulvum* | CAA39295 |
| *RpALS* | *Rheum palmatum* | AAS87170 |
| *AaPKS3* | *Aloe arborescens* | ABS72373 |
| *SeTAL* | *Saccharothrix espanaensis* | CCH33126 |
| *At4CL1* | *Arabidopsis thaliana* | AAQ86588 |
| *At4CL3* | *Arabidopsis thaliana* | AAQ86589 |
| *At4CL4* | *Arabidopsis thaliana* | Q9LU36 |
| *Sc4CL* | *Streptomyces coelicolor* | CAB95894 |
| *PhCHS* | *Petunia x hybrida* | AGZ04577 |
| *AtCHI* | *Arabidopsis thaliana* | NP_191072 |
| *VvSTS*^{†} | *Vitis vinifera* | CAA54221 |

| | | |
|---|---|---|
| * depicts NCBI numbers for enzymes ^{†} codon-optimized for *E. coli* K-12 MG1655. | | |

In FIG. 1A, malonyl-CoA is converted to THN by the RppA enzyme. Then, THN is converted to flaviolin by a spontaneous oxidation reaction. WhiE_ORFIII was reported to involved in this final step (Austin MB, et al. (2004), J Biol Chem 279:45162-45174.), and hence, in the present invention, the corresponding gene was additionally cloned into pET-30a(+), but it caused reduced flaviolin production. This demonstrates that the *rppA* single gene is sufficient for flaviolin production. The produced flaviolin was confirmed by LC-MS and MS/MS (FIG. 2B). Thereafter, the rppA expression platform was established in a tac promoter-based plasmid for its use in all *E. coli* strains beyond the conventional T7 promoter-based system. To this end, a pTacCDFS plasmid based on a pCDFDuet-1 (Novagen) plasmid was first constructed, which contains a CDF replication origin and has spectinomycin antibiotic resistance and a tac promoter-based gene expression cassette. To this end, a DNA fragment containing the tac promoter was amplified by PCR using a pTac15K plasmid (Lee SY, et al. (2008), US patent 20110269183) as a template and the primers of SEQ ID NO: 3 and SEQ ID NO: 4, and the corresponding plasmid was linearized using a pCDFDuet-1 plasmid as a template and the primers of SEQ ID NO: 5 and SEQ ID NO: 6. The produced two DNA fragments were assembled using Gibson assembly, thereby constructing a pTacCDFS plasmid. A pTrcCDFS plasmid was also constructed for further use, and this plasmid is the same as the pTacCDFS plasmid, except that it contains the trc promoter. In addition, the pTrcCDFS plasmid was constructed in the same manner as the pTacCDFS plasmid, except that the DNA fragment containing the trc promoter was amplified by PCR using the pTrc99A plasmid (Lee SY, et al. (2008), US patent 20110269183) as a template. Thereafter, the *rppA* expression plasmid pTac-Sgr_rppA based on the pTacCDFS plasmid was constructed by the following method. First, the *rppA* gene was amplified from the genomic DNA of *S. griseus* using the primers of SEQ ID NO: 7 and SEQ ID NO: 8, and the pTacCDFS plasmid was linearized using the primers of SEQ ID NO: 9 and SEQ ID NO: 10. The two produced DNA fragments were assembled using Gibson assembly, thereby constructing a pTac-Sgr_rppA plasmid. Furthermore, for optimized expression of *rppA,* optimization of the 5' untranslated region (5'UTR) was performed. The 5'UTR DNA sequence was designed using the previously reported UTR designer (url: https://sbi.postech.ac.kr/utr_designer/). For construction of the corresponding primer pTac-5'UTR-Sgr_rppA, inverse PCR was performed using pTac-Sgr_rppA as a template and the primers of SEQ ID NO: 9 and SEQ ID NO: 11. The produced linearized plasmid was treated with DpnI to remove the template, and ligated by T4 polynucleotide kinase (PNK) (Enzynomics, Korea) and T4 ligase (Elpis Biotech, Korea) treatment. The resulting plasmid was introduced into an *E. coli* BL21(DE3) strain and cultured in M9 minimal medium, thereby producing 17.8 mg/L of flaviolin (FIG. 2C).
[SEQ ID NO: 3] 5'-CGACTCCTGCATTAGGAAATGACTGCACGGTGCACCAATG-3'
[SEQ ID NO: 4] 5'- GCGTTTCACTTCTGAGTTCG-3'
[SEQ ID NO: 5] 5'-CGAACTCAGAAGTGAAACGCCTGAAACCTCAGGCATTTGAG-3'
[SEQ ID NO: 6] 5'- ATTTCCTAATGCAGGAGTCG-3'
[SEQ ID NO: 7] 5'-CAATTTCACACAGGAAACAGAATGGCGACCCTGTGCCGACC-3'
[SEQ ID NO: 8] 5'-CTCTAGAGGATCCCCGGGTACCATTAGCCGGACAGCGCAACGC-3'
[SEQ ID NO: 9] 5'- GGTACCCGGGGATCCTCTAGAG-3'
[SEQ ID NO: 10] 5'- TCTGTTTCCTGTGTGAAATTG-3'
[SEQ ID NO: 11] 5'-GATGCTCCTTTCTTGTTATTGAAATTGTTATCCGCTCACAATTC-3'

### 1.2. Examination of Applicability of Type III Polyketide Synthase-Based Malonyl-CoA Biosensor

After construction of a malonyl-CoA biosensor based on *S. griseus* RppA, the following experiment was performed in order to examine the applicability of the biosensor. First, the signal of the RppA malonyl-CoA biosensor was defined as the absorbance of culture supernatant at 340 nm (FIG. 3D). Generally, for characterizing a biosensor, different concentrations of a target compound has to be added to examine whether an increased signal occur. However, because malonyl-CoA is a substance that cannot pass through the cell membrane, adding malonyl-CoA to medium is meaningless. Thus, cerulenin (Sigma-aldrich, USA) known to block fatty acid biosynthetic pathways was used to indirectly characterize the biosensor (Omura S (1976), Bacteriol Rev 40:681-697.). **Cerulenin** blocks fatty acid biosynthetic pathways, and thus leads to the accumulation of malonyl-CoA, a fatty acid precursor. Thus, the concentration of malonyl-CoA in *E*. *coli* according to addition of different concentrations of cerulenin as described in previous studies was measured (Xu P, Li L, Zhang F, Stephanopoulos G, Koffas M (2014), Proc Natl Acad Sci U S A 111:11299-11304). An *E. coli* BL21(DE3) strain containing the plasmid pTac-5'UTR-Sgr_rppA was inoculated into 3 mL of LB medium in a 14-mL disposable Falcon round-bottom tube. Next, the strain was cultured in an incubator at 30°C and 220 rpm for 16 hours, and then inoculated into 3 mL of modified R/2 medium (pH 6.8) in another 14-mL disposable Falcon round-bottom tube. The modified R/2 medium was prepared to contain, per liter, the following components: 3g yeast extract, 6.75g KH₂PO4, 2g (NH₄)₂HPO₄, 0.8g MgSO₄·7H₂O, 3g (NH₄)₂SO₄, 0.85g citric acid, 5mL trace metal solution [per 1 L of 0.1 M HCl: 10g FeSO₄·7H₂O, 2.25g ZnSO₄·7H₂O, 0.58g MnSO₄·5H₂O, 1g CuSO₄·5H₂O, 0.1g (NH₄)₆MO₇O₂₄·4H₂O, 0.02g Na₂B₄O₇·10H₂O, 2g CaCl₂·2H₂O]. 20 g/L of glucose, 100 µg/mL of spectinomycin, 0.2 mM IPTG, and a suitable concentration of cerulenin were added thereto. The concentrations of cerulenin added were as follows: 0, 5, 10, 15, 20, 25, 50, and 100 µM. The inoculated cells were cultured in an incubator at 30°C and 220 rpm for 16 hours, and then the growth (OD₆₀₀) of the cells and the absorbance at 340 nm of the culture supernatant were measured. The results of the measurement are shown in FIG. 2D. At this time, it could be seen that the RppA-expressing strain generated a normalized signal, which obviously increased as the concentration of cerulenin increased, compared to the strain that does not express RppA. As shown in FIG. 3A, it could be confirmed that the normalized production of flaviolin also increased with increasing cerulenin concentration, as observed by the naked eyes (FIG. 3B). In addition, it was confirmed that when the pTac-5'UTR-Sgr_rppA plasmid was introduced into 16 different *E. coli* strains (Table 2), flaviolin was produced from all the strains. This demonstrates that the malonyl-CoA biosensor proposed in the present invention is applicable to all *E. coli* strains (FIG. 3C) .

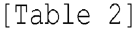

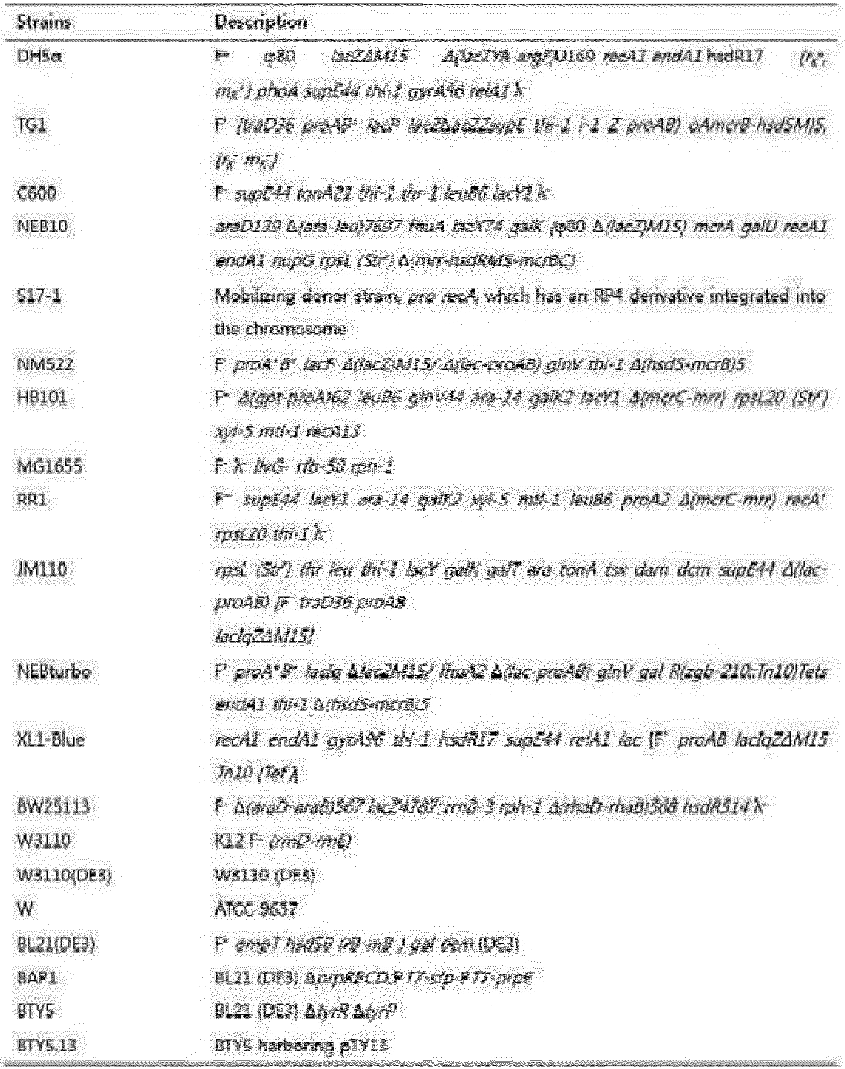

Furthermore, in order to demonstrate that other type III polyketide synthases in addition to the *S. griseus* RppA-based malonyl-CoA biosensor are also applicable as malonyl-CoA biosensors, three type III polyketide synthases were tested. To this end, *Aloe arborescens* type III polyketide synthase (octaketide synthase, OKS) [SEQ ID NO: 103], *Aloe arborescens* type III polyketide synthase (octaketide synthase 2, PKS4) [SEQ ID NO: 105], and *Aloe arborescens* type III polyketide synthase (octaketide synthase 3, PKS5) [SEQ ID NO: 106] were tested, the plasmids pET-AaOKS, pET-AaPKS4 and pET-AaPKS5, each containing a gene encoding each of the enzymes, were constructed as follows. First, AaOKS was amplified by PCR using a synthesized AaOKS gene (Integrated DNA Technologies, Inc., USA) as a template and the primers of SEQ ID NO: 137 and SEQ ID NO: 138, and then inserted into the a pET-30a(+) plasmid at NdeI and EcoRI sites. Other plasmids were also constructed in the same method as described above, except that AaPKS4 was amplified using the primers of SEQ ID NO: 141 and SEQ ID NO: 142 and AaPKS5 was amplified using the primers of SEQ ID NO: 143 and SEQ ID NO: 144.
[SEQ ID NO: 135] 5'-CTTTAAGAAGGAGATATACATATGAGTTCACTCTCCAACTCTC-3'
[SEQ ID NO: 136] 5'-CTTGTCGACGGAGCTCGAATTCATTACATGAGAGGCAGGCTGTG-3'
[SEQ ID NO: 137] 5'-CTTTAAGAAGGAGATATACATATGAGTTCACTCTCCAACGCTTC-3'
[SEQ ID NO: 138] 5'-CTTGTCGACGGAGCTCGAATTCATTACATGAGAGGCAGGCTGTG-3'
[SEQ ID NO: 139] 5'-CTTTAAGAAGGAGATATACATATGGGTTCACTCTCCGACTCTAC-3'
[SEQ ID NO: 140] 5'-CTTGTCGACGGAGCTCGAATTCATTACACAGGAGGCAGGCTATG-3'
[SEQ ID NO: 141] 5'-CTTTAAGAAGGAGATATACATATGGGTTCACTCTCCAACTAC-3'
[SEQ ID NO: 142] 5'-CTTGTCGACGGAGCTCGAATTCATTACACAGGGGGCAGGCTG-3'
[SEQ ID NO: 143] 5'-CTTTAAGAAGGAGATATACATATGGGTTCGATCGCCGAG-3'
[SEQ ID NO: 144] 5'-CTTGTCGACGGAGCTCGAATTCATTAGACAGGAGGCAGGCTG-3'
[SEQ ID NO: 145] 5'-GCTCACCATCATTGGGCTCCGTGGCCCCAATG-3'
[SEQ ID NO: 146] 5'- CATTGGGGCCACGGAGCCCAATGATGGTGAGC-3'

When an *E. coli* BL21 (DE3) strain containing each of the constructed plasmids was cultured, change in the color of the strains expressing AaOKS, AaPKS4 and AaPKS5 could be observed. Thus, polyketide synthases that contributed to the color changes were analyzed by LC-MS analysis (FIG. 4A). As noted above, malonyl-CoA experiments using cerulenin were performed, and as a result, it was demonstrated that the signal generated from each strain increased as the intracellular malonyl-CoA concentration increased (FIGS. 4 and 5) . Here, the signal indicates the absorbance at 300 nm of the culture supernatant (FIG. 4C). Therefore, in the present invention, it was demonstrated that not only the RppA enzyme, all other type III polyketide synthases capable of producing colored polyketide-based compounds from malonyl-CoA, can be used as malonyl-CoA biosensors.

In the following Examples, additional experiments were performed using RppA among the above-described type III polyketide synthases.

### 1.3. Examination of Scalability to Other Industrially Useful Strains

After the applicability in *E. coli* was demonstrated in the above-described Examples, the scalability of this RppA malonyl-CoA biosensor to other industrially useful strains was examined. Examples of strains which are used in this examination include, in addition to *E. coli, Pseudomonas putida* which is a representative Gram-negative bacterium, and *Corynebacterium glutamicum* and *Rhodococcus opacus,* which are representative gram-positive bacteria. First, a plasmid for producing flaviolin in a *P. putida* strain was constructed using pBBR1MCS2 as a base plasmid (Kovach ME, et al. (1995), Gene 166:175-176). First, the pBBR1MCS2 plasmid was linearized using the primers of SEQ ID NO: 12 and SEQ ID NO: 13. Then, an *rppA* expression cassette containing the tac promoter was amplified by PCR using the pTac-Sgr_rppA plasmid as a template and the primers of SEQ ID NO: 14 and SEQ ID NO: 15. The obtained two DNA fragments were assembled using Gibson assembly, thereby constructing a pBBR1-rppA plasmid. Furthermore, in order to examine the effect of the N-terminal poly His-tag on enzyme expression and on flaviolin production, pTac-His-Sgr_rppA was first constructed. At this time, a pTacCDFS plasmid, linearized by PCR using the primers of SEQ ID NO: 9 and SEQ ID NO: 10, and a His-tagged *rppA* gene amplified by PCR from the genomic DNA of *S. griseus* using the primers of SEQ ID NO: 16 and SEQ ID NO: 8 were assembled into a single plasmid (pTac-His-Sgr_rppA) using Gibson assembly. The constructed plasmid was amplified again by PCR using the primers of SEQ ID NO: 14 and SEQ ID NO: 15, and assembled with the linearized pBBR1MCS2 plasmid using Gibson assembly, thereby constructing a pBBR1-His-rppA plasmid. At this time, each of pBBR1-rppA and pBBR1-His-rppA plasmids was transformed into a *P. putida* strain, and then each of the resulting strains was inoculated into a test tube containing 5 mL of LB medium, and then cultured at 30°C and 220 rpm for 18 hours. Next, 0.8 g/L MgSO₄·7H₂O, 10 g/L glucose, and an antibiotic were added to 5 mL LB, and then subculture was performed. After culture under the same conditions for 25 hours, sampling was performed. As a result, a larger amount of flaviolin was produced in the strain containing the pBBR1-rppA plasmid (44.7 mg/L; FIG. 6A). Accordingly, using the screened *P. putida* pBBR1-rppA strain, characterization of the malonyl-CoA biosensor was performed using cerulenin in the same manner as described in Example 1.2 above. As a result, it could be observed that the normalized signal and the normalized production of flaviolin also increased as the cerulenin concentration in the corresponding strain increased (FIGS. 6B and 6C). Here, the subculture was performed in 2 mL of MR-MOPS medium supplemented with 20 g/L glucose, 25 µg/mL kanamycin, 0.5 mM IPTG and a suitable concentration of cerulenin, and the composition of the MR-MOPS medium (pH 7.0) contained, per liter, the following components: 6.67g KH₂PO₄, 4g (NH₄)₂HPO₄, 0.8 g citric acid, 5mL trace metal solution, 20.09g MOPS (3-(N-morpholino)propanesulfonic acid), 0.8g/L MgSO₄·7H₂O. As a result, it was demonstrated that the RppA malonyl-CoA biosensor successfully acts in *P. putida.*
[SEQ ID NO: 12] 5'-CGAACTCAGAAGTGAAACGCTGCCTAATGAGTGAGCTAAC -3'
[SEQ ID NO: 13] 5'-CATTGGTGCACCGTGCAGTCAAAATTCGCGTTAAATTTTTG-3'
[SEQ ID NO: 14] 5'- GACTGCACGGTGCACCAATG-3'
[SEQ ID NO: 15] 5'- GCGTTTCACTTCTGAGTTCG-3'
[SEQ ID NO: 16] 5'-CAATTTCACACAGGAAACAGAATGCACCATCACCATCACCATGCGACCCTGTGCCGACC-3'

In addition, in order to apply this biosensor to a C. *glutamicum* strain, an *rppA* expression plasmid was constructed using pCES-H36 as a base plasmid (Yim SS, An SJ, Kang M, Lee J, Jeong KJ (2013), Biotechnol Bioeng 110:2959-2969). To this end, the corresponding plasmid was linearized using a pCES-H36-GFP plasmid as a template and the primers of SEQ ID NO: 17 and SEQ ID NO: 18. A tac promoter-based *rppA* expression cassette was amplified by PCR using pTac-Sgr_rppA as a template and the primers of SEQ ID NO: 19 and SEQ ID NO: 20, and an N-terminal His-tagged *rppA* expression cassette based on the tac promoter was amplified by PCR using pTac-His-Sgr_rppA as a template and the primers of SEQ ID NO: 21 and SEQ ID NO: 20. The two different *rppA* expression cassettes were assembled with the above-described linearized pCES-H36 plasmid using Gibson assembly, thereby constructing pCES-rppA and pCES-His-rppA plasmids. The constructed plasmids were transformed into a C. *glutamicum* strain, and then inoculated into 5 mL of LB medium. Each of the resulting strains was cultured in an incubator at 30°C and 220 rpm for 18 hours, and then subcultured in 5 mL of LB medium for 48 hours, after which sampling was performed. At this time, 3.9 mg/L of flaviolin was produced only in the strain containing the N-terminal His-tagged *rppA* expression cassette (pCES-His-rppA) (FIG. 6D). Accordingly, using the selected C. *glutamicum* pCES-His-rppA strain, characterization of the malonyl-CoA biosensor was performed using cerulenin in the same manner as described in Example 1.2 above. As a result, it could be observed that the normalized signal and the normalized production of flaviolin also increased as the cerulenin concentration in the corresponding strain increased (FIGS. 6E and 6F).
[SEQ ID NO: 17] 5'- CCATTATAATTAGGCCTCGG-3'
[SEQ ID NO: 18] 5'- CCATGCTACTCCTACCAACC-3'
[SEQ ID NO: 19] 5'-GGTTGGTAGGAGTAGCATGGGATCCATGGCGACCCTGTGCCGACC-3'
[SEQ ID NO: 20] 5'-CCGAGGCCTAATTATAATGGATTAGCCGGACAGCGCAACGC-3'
[SEQ ID NO: 21] 5'-GGTTGGTAGGAGTAGCATGGGATCCATGCACCATCACCATCACCATGC-3'

In another example, in order to apply this biosensor to an *R. opacus* strain, an *rppA* expression plasmid was constructed using pCH as a base plasmid. An acetamide-inducible promoter (G. Roberts et al., FEMS Microbiol. Lett. 222:131-136, 2003) was used, which was amplified by PCR using the primers of SEQ ID NO: 22 and SEQ ID NO: 23. In addition, an *rppA* expression cassette was amplified by PCR from the genomic DNA of *S. griseus* using the primers of SEQ ID NO: 24 and SEQ ID NO: 25, and an *rppA* expression cassette for additional expression of the N-terminal His-tag was amplified by PCR using the primer of SEQ ID NO: 26 and SEQ ID NO: 25. Each *rppA* expression cassette, the acetamide-inducible promoter DNA fragment, and the pCH plasmid linearized by PstI were assembled together using Gibson assembly, thereby constructing two vectors (pCH-rppA and pCH-His-rppA). Each of the constructed plasmids was transformed into an *R. opacus* strain, and then inoculated into 5 mL of LB medium. Each of the resulting strains was cultured in an incubator at 30°C and 220 rpm for 18 hours, and then subcultured in 5 mL of LB medium for 48 hours, after which sampling was performed. It could be seen that red-colored flaviolin was produced only in the strain containing pCH-rppA (FIG. 6G).
[SEQ ID NO: 22] 5'-CTTGATCAGCTTGCATGCCTGCAGAAGCTTTCTAGCAGAAATAATTC-3'
[SEQ ID NO: 23] 5'- GTGCATGTGGACTCCCTTTCTCTTATC-3'
[SEQ ID NO: 24] 5'-GATAAGAGAAAGGGAGTCCACATGGCGACCCTGTGCCGACC-3'
[SEQ ID NO: 25] 5'-GGATCCTCTAGAGTCGACCTGCAGATTAGCCGGACAGCGCAACGC-3'
[SEQ ID NO: 26] 5'-GATAAGAGAAAGGGAGTCCACATGCACCATCACCATCACCATGC-3'

### Example 2. High-Throughput Screening of Strain Having Increased Malonyl-CoA Producing Ability by Use of RppA Biosensor

### 2.1. High-Throughput Screening of Strain Having Increased Malonyl-CoA Producing Ability by Introduction of E. coli Genome-Scale Synthetic Control sRNA Library

After construction of the RppA malonyl-CoA biosensor, confirmation of successful operation thereof, and demonstration of the applicability and scalability of the biosensor, synthetic control sRNA technology developed by the present inventors was introduced in order to screen a strain having increased malonyl-CoA producing ability by the use of the biosensor (KR 10-1575587, US 9388417, EP 13735942.8, CN 201380012767.X, KR 10-1690780, KR 10-1750855, US 15317939, CN 201480081132.X; Na D, et al. (2013), Nat Biotechnol 31:170-174; Yoo SM, Na D, Lee SY (2013), Nat Protoc 8:1694-1707). In addition, in order to include all major genes in *E. coli,* a previously constructed *E. coli* genome-scale synthetic-control sRNA library (including 1,858 genes in *E. coli* K-12 W3110 strain) was introduced and knocked down at the gene level, and knockdown gene targets effective for increased production of malonyl-CoA were screened. Thus, the *E. coli* genome-scale synthetic-control sRNA library was introduced into an *E. coli* BL21(DE3) strain containing a pTac-5'UTR_Sgr_rppA plasmid, and then high-throughput screening was performed (FIG. 7). In order to cover all 1,858 sRNAs, 11,488 colonies having a size equal to larger than 6 times the library size were selected from the obtained colonies, and then a robotic automatic high-throughput screening system was used. To this end, a K3 colony picker (KBiosystems, Basildon, UK) was used, and each colony was inoculated into LB medium (supplemented with antibiotic and 0.2 mM IPTG) in 96-well microplates by a robot (Korea Research Institute of Bioscience and Biotechnology (KRIBB); Jeongeup, Republic of Korea). The K3 colony picker illuminates and images the agar plates. Then, the intrinsic program recognizes and locates colonies and instructs a robot arm tipped with pins to pick the selected colonies. The inoculated cells were cultured in a HT-MegaGrow incubator (Bioneer, Republic of Korea) at 30°C and 500 rpm for 24 hours. After culture, 231 strains with relatively strong signals were selected. The selected strains were cultured in 14 mL disposable Falcon round-bottom tubes containing 3 mL of LB medium supplemented with antibiotics and 0.2 mM IPTG at 30°C and 250 rpm for 24 hours. Among them, 70 strains showed high signals than the control strain (biosensor strain containing no sRNA), and sRNAs contained in these strains were sequenced (FIG. 8). At this time, three sRNAs were observed twice (*argB, fabF, nudD*). In addition, among them, 26 strains showed an increase in signal of 45% or more compared to the control strain. Accordingly, sRNA vectors corresponding to the 26 strains were transformed back to the original sensor strain (FIG. 9), and cultured again in 3 mL of LB as described above. As a result, among them, 14 sRNAs showing an increase in signal of more than 70% compared to the control strain were finally selected. The 14 selected knockdown gene targets for increased malonyl-CoA production are listed in Table 3 below.

**[Table 3]**

| List of 14 finally selected knockdown target genes | | | |
|---|---|---|---|
| **No.** | **Target gene** | **Protein function** | **Essentiality^{‡}** |
| 1 | *fabH* | 3-oxoacyl-[acyl-carrier-protein] synthase III | NE |
| 2 | *fabF* | 3-oxoacyl-[acyl-carrier-protein] synthase II | NE |
| 3 | *cytR* | LacI family transcriptional regulator, repressor for *deo* operon, *udp, cdd, tsx, nupC* and *nupG* | NE |
| 4 | *yfcY* | beta-ketoacyl-CoA thiolase | NE |
| 5 | *fmt* | 10-formyltetrahydrofolate:L-methionyl-tRNA(fMet)N-formyl transferase | ND |
| 6 | *mqo* | malate dehydrogenase, FAD/NAD(P)-binding domain | ND |
| 7 | *fadR* | GntR family transcriptional regulator, negative regulator for fad regulon and positive regulator of *fabA* | NE |
| 8 | *yfiD* | pyruvate formate lyase subunit | NE |
| 9 | *purB* | adenylosuccinate lyase | NE |
| 10 | *xapR* | transcriptional activator of *xapAB* | NE |
| 11 | *araA* | L-arabinose isomerase | ND |
| 12 | *pyrF* | orotidine-5'-phosphate decarboxylase | E |
| 13 | *pabA* | aminodeoxychorismate synthase, subunit II | E |
| 14 | *hycI* | protease involved in processing C-terminal end of HycE | NE |

### 2.2. Confirmation of Overexpression Gene Targets for Increased Malonyl-CoA Production, Identified Using FVSEOF Algorithm, by RppA Biosensor

Not only knockdown gene targets, but also overexpression gene targets were demonstrated by the RppA biosensor. At this time, gene targets identified using an FVSEOF algorithm were used (Park JM, et al. (2012), BMC Syst Biol 6:106). *In silico* analysis was performed using the *E*. *coli* genome-scale metabolic model iJO1366. (Orth JD, et al. (2011), Mol Syst Biol 7:535). Thus identified 9 gene targets are as follows: *zwf, mdh, fumA, fumB, fumC, serA, serB, serC,* and *tpiA* (FIG. 10A and 10C). Each gene target was amplified in *E. coli* BL21(DE3), and inserted into a pTrc99A plasmid under the trc promoter, and transformed into a BL21(DE3) pTac-5'UTR-Sgr_rppA strain. The constructed sensor strains were cultured in test tubes in the same manner as described in Example 2.1 above, and at this time, signals were measured. As a result, increased signals compared to the control were observed in 8 gene targets (*serA, mdh, zwf, tpiA, serB, fumB, serC,* and *fumC*) among 9 gene targets (FIG. 10B).

### Example 3. Increased Production of Useful Products Using Knockdown Gene Targets Screened Using RppA Biosensor

Flask culture conditions used in this Example and the following Examples are as follows unless otherwise specified. A colony was inoculated into a test tube containing 10 mL of LB medium, and cultured in an incubator at 37°C and 200 rpm. 1 mL of the cell culture was inoculated into a baffled flask containing 50 mL of modified R/2 medium. When strains which have been grown at 37°C and 200 rpm reached an OD₆₀₀ value of 0.8, the strains were treated with 0.5 mM IPTG to induce production, and were cultured at 30°C and 200 rpm for 48 hours. As a carbon source, glycerol or glucose was added.

### 3.1. Increased Production of 6-Methylsalicylic Acid Using Selected Knockdown Gene Targets

In the next step, the knockdown gene targets, selected in the above Example and enabling increased malonyl-CoA production, were applied for production of useful products in order to demonstrate that the biosensor of the present invention could successfully screen effective knockdown gene targets. Accordingly, as the first product, 6-methylsalicylic acid (6MSA) reported to be produced from a *Penicilium griseofulvum* (or *Penicilium patulum*) strain was attempted to be produced from genetically engineered *E. coli.* 6-Methylsalicylic acid possesses antibiotic and antifungal activities (Dimroth P, Ringelmann E, Lynen F (1976), Eur J Biochem 68:591-596), and is produced from one molecule of acetyl-CoA and three molecules of malonyl-CoA by a 6-methylsalicylic acid synthase (6MSAS), type I iterative polyketide synthase, found in fungi (FIG. 11).

The sequence of 6-methylsalicylic acid synthase (6MSAS) that was used in the present invention is as follows:

6-methylsalicylic acid synthase (6MSAS) [SEQ ID NO:
123]:
mhsaatstyp sgktspapvg tpgteyseye fsndvavvgm acrvaggnhn
pellwqslls qksamgeipp mrwepyyrrd arnekflknt tsrgyfldrl
edfdcqffgi spkeaeqmdp qqrvslevas ealedagipa kslsgsdtav
fwgvnsddys klvledlpnv eawmgigtay cgvpnrisyh lnlmgpstav
daacasslva ihhgvqairl geskvaivgg vnalcgpglt rvldkagais
sdgscksfdd dahgyargeg agalvlkslh ralldhdnvl avikgsavcq

dgktngimap nsvaqqlaan nalsaanidp htvryveaha tstplgdpte
isaiasvyga drpaddpcyi gsikpnighl eagagvmgfi kavlaiqkgv
lppqanltkl nsridwktag vkvvqeatpw pesdpirrag vcsygyggtv
shavieefsp ilqpdplgng avsgpgllll sgpqekrlal qaktlrdwmt
aegkdhnlsd ilttlatrrd hhdyraalvv ddyrdaeqvl qslangvdht
fttqsrvlgs diskdvvwvf sghgaqwpdm gkqlihnpvf faaiqpldel
iqaeiglspi ellrtgdfes sdrvqiltyv mqiglsallq sngitpqavi
ghsvgeiaas vvagalspae galivtrral lyrqvmgkgg milvnlpsae
teeilgsrsd lvvaidssps scvvagdkel vaetaealka rgvktftvks
diafhsptln glvdplrdvl aetlspvspn vklystalad prgqdlrdve
ywagnmvnrv rltsavkaav edgyrlflev sthpvvshsi netlmdagme
dfaviptllr kkptekhilh siaqlhcrga evnwaaqmpg rwatgvpttt
wmhkpiwrki etaplhtglt hdvekhtllg qripvpgtdt yvyttrldnd
tkpfpgshpl hgteivpaag lintflkgtg gqmlqnvvlr vpvainaprs
vqvvvqqdqv kvvsrlipse psqldddasw vthttaywdr kvagsedrid
faavksrlvt kladnfsidy ldkvgvsamg fpwavtehyr ndkemlarvd
vnpaisgdap lpwdssswap vldaatsvgs tifptpalrm paqiervevf
tsqdppkisw lyvqeasdsv ptshvsvvse agevlakfta mrfseiegtp
gvsgsmeslv hqiawppatp aeeplsietv ilvspdattr alyaaslptr
vnsfqfsstq effsnasslp lekgtvvtyi pgevaslaev paasesftwn
llelikftvn gslpikvftl tanigegqtp talaqsplyg larviasehp
dlgtlidvee pviplstmry iqgadiirin dgiartsrfr slprnkllpa
segprllprp egtylitggl gvlglevadf lvekgarrll lisrralppr
rtwdqvsedl qptiakirll esrgasvhvl plditkpdav eqlttaldrl
slpsvqgvvh aagvldnelv mqttrdafnr vlapkiagal alhevfppks

vdffvmfssc gnlvgftgqa sygsgnafld tlathrarlg daavsfqwts
wrglgmgast dfinaelesk gitdvtrdea faawqhlaky dmdhgvvlrs
rafedgepip vsilndiavr rvgtvsntsp aaagssdavp tsgpelkayl
dekirgcvak vlqmtaedvd skaaladlgv dsvmtvtlrr qlqltlkiav
pptltwshpt vshlavwfae klak

In order to construct an *E. coli* strain capable of producing 6-methylsalicylic acid, a pTac15K plasmid was first linearized by inverse PCR using the primers of SEQ ID NO: 27/ SEQ ID NO: 28, and then a *Pg6MSAS* gene encoding 6-methylsalicylic acid synthase was amplified using the genomic DNA of *P. griseofulvum* as a template and sequentially using the primers of SEQ ID NO: 29 and SEQ ID NO: 30, and SEQ ID NO: 31 and SEQ ID NO: 30. The two DNA fragments were assembled using Gibson assembly, thereby constructing a pTac-Pg6MSAS plasmid (FIG. 12B). The 6MSAS enzyme contains an acyl-carrier protein domain at its terminus, and 4'-phosphopantetheinyl transferase (Sfp) is required to activate this domain.

The sequence of 4'-phosphopantetheinyl transferase (Sfp) that was used in the present invention is as follows:

4'-phosphopantetheinyl transferase (Sfp) [SEQ ID NO:
124]:
mkiygiymdr plsqeenerf msfispekre kcrrfyhked ahrtllgdvl
vrsvisrqyq ldksdirfst qeygkpcipd lpdahfnish sgrwvicafd
sqpigidiek tkpisleiak rffskteysd llakdkdeqt dyfyhlwsmk

esfikqegkg lslpldsfsv rlhqdgqvsi elpdshspcy iktyevdpgy
kmavcavhpd fpeditmvsy eell

*sfp* gene fragment encoding 4'-phosphopantetheinyl transferase was amplified by PCR from the genomic DNA of *Bacillus subtilis* using the primers of SEQ ID NO: 32 and SEQ ID NO: 33, and inserted into a pTac15K plasmid at the EcoRI site. The resulting plasmid was amplified again by PCR using the primers of SEQ ID NO: 34 and SEQ ID NO: 35, and then inserted into a pTac-Pg6MSAS plasmid at the SphI site, thereby constructing a pTac-Pg6MSAS-sfp plasmid (FIG. 12A). The constructed plasmid was transformed into an *E*. *coli* BL21(DE3) strain, and enzyme expression was checked by SDS-PAGE (FIG. 12C). Then, flask culture of the strain was performed in modified R/2 medium in the presence of different concentrations of glucose or glycerol, and as a result, it could be seen that when 100 g/L of glycerol was added, 4.7 mg/L of 6-methylsalicylic acid was produced (FIG. 13A). The authenticity of the produced 6-methylsalicylic acid was confirmed by LC-MS (FIGS. 13B and 13C). Before introduction of the sRNAs selected in the above Example, in order to examine the production of 6-methylsalicylic acid in different *E. coli* strains, the pTac-Pg6MSAS-sfp plasmid was introduced into the 16 *E. coli* strains shown in Table 1 above, and then test tube-scale culture of each of the strains was performed in 3 mL of modified R/2 medium. Among these strains, 6 strains (NM522, BL21, S17-1, JM110, HB101, and XL1-Blue) producing more than 1 mg/L of 6MSA were selected (FIG. 13D) . Then, 14 selected sRNAs were introduced into each of the selected strains, thereby constructing a total of 84 strains, and test tube-scale of each of the constructed strains was performed. As a result, the *pajbA*-knockdown BL21(DE3) strain produced the largest amount of 6-methylsalicylic acid (6.1 mg/L; FIG. 14). This strain was further subjected to flask culture (50 mL) under the same medium conditions, and as a result, it produced 8.0 mg/L of 6-methylsalicylic acid (FIG. 13E) .
[SEQ ID NO: 27] 5'-GCTGAGAAGCTTGCCAAATAATGGATCCTCTAGAGTCGACCTG-3' 
[SEQ ID NO: 28] 5'-
CTGGGGATGTTTTCCCAGAGGGGTATGTAGAAGTTGCAGCGGAATGCATGAATTCTGTTTC
CTGTGTGAAATTG-3'
[SEQ ID NO: 29] 5'- ACAGTGAATATGAATTCTCCAACG-3'
[SEQ ID NO: 30] 5'- ATTATTTGGCAAGCTTCTCAGC-3' 
[SEQ ID NO: 31] 5'-
CTCTGGGAAAACATCCCCAGCACCAGTCGGAACCCCTGGGACTGAGT
ACAGTGAATATGAATTCTCCAACG-3'
[SEQ ID NO: 32] 5'-TAATAAGAATTCATGAAGATTTACGGAATTTATATG-3'
[SEQ ID NO: 33] 5'- TTATTAGAATTCTTATAAAAGCTCTTCGTACGAG-3'
[SEQ ID NO: 34] 5'-CTAGAGTCGACCTGCAGGCATGCCACTCCCGTTCTGGATAATG-3'
[SEQ ID NO: 35] 5'- CAAAACAGCCAAGCTTGCATGC-3'

In order to further optimize the 6-methylsalicylic acid producing strain, only the *Pg6MSAS* gene having a long length (5.3 kb) was expressed with a plasmid (pTac-Pg6MSAS), and *sfp* was expressed using the strain BAP1 (Pfeifer BA, Admiraal SJ, Gramajo H, Cane DE, Khosla C (2001), Science 291:1790-1792), which is a strain that *sfp* is inserted into the genome of the BL21(DE3) strain. The results of flask culture showed an increased 6-methylsalicylic acid production of 17.6 mg/L, and from the results of SDS-PAGE analysis, this was believed to be because of improved 4'-phosphopantetheinyl transferase expression and the reduced proportion of inactivated 6-methylsalicylic acid synthase (FIG. 12C). Accordingly, when a *pajbA*-knockdown sRNA was introduced into an *E. coli* BAP1 pTac-Pg6MSAS strain, 6-methylsalicylic acid could be produced in an amount of 23.9 mg/L, which corresponding to an increase of about 35.8% (FIG. 13F). When fed-batch fermentation of this strain was performed, an increased 6-methylsalicylic acid concentration of 97.8 mg/L could be obtained after about 27 hours (FIG. 13G). The fed-batch fermentation was performed in 1.9 L of modified R/2 medium in a 6.6-L fermenter (BioFlo320, Eppendofr, Germany) in the presence of 50 g/L of glycerol as an initial carbon source. The colony was inoculated into a test tube containing 10 mL of LB medium, and then cultured at 37°C and 200 rpm for one day. Next, the cells were inoculated into two baffled flasks, each containing 50 mL of modified R/2 medium, and at this time, 50 g/L of glycerol was used as a carbon source. Flask culture was performed for about 9 hours until the OD₆₀₀ value reached about 2, and then the cells were inoculated into a fermenter. The culture pH was maintained at 6.8 by 28% (v/v) ammonia solution, and the dissolved oxygen (DO) concentration was controlled at 40% of air saturation by supplying air at 2 L/min and automatically controlling the agitation speed as 1000 rpm and by increasing the oxygen flow rate. The pH-stat feeding strategy was employed in order to supply nutrients. When the pH becomes higher than 6.86, the feeding solution was automatically added. The feed solution contained, per liter, the following components: 800 g glycerol, 6 mL trace metal solution and 12 g MgSO₄·7H₂O. Heterologous protein expression was induced with 0.5 mM IPTG when the OD₆₀₀ value after inoculation reached 2 to 3.

In order to further increase the production of 6-methylsalicylic acid, it was attempted to increase the expression of the previously selected three FVSEOF gene targets (P < 0.05; *zwf, mdh, serA;* FIG. 10) and the following five enzymes: *C. glutamicum* acetyl-CoA carboxylase (AccBC and AccD1), *E. coli* glyceraldehyde 3-phosphate dehydrogenase (GapA), *E. coli* phosphoglycerate kinase (Pgk), *E. coli* acetyl-CoA synthetase (Acs), and *E. coli* pyruvate dehydrogenase (PDH: AceEF and Lpd). At this time, the corresponding gene fragments were inserted and cloned into a pBBR1TaC plasmid. The pBBR1TaC plasmid is a tac promoter-based expression plasmid constructed from pBBR1MCS. For construction of pBBR1TaC, pBBR1MCS was first amplified by inverse PCR using the primers of SEQ ID NO: 147 and SEQ ID NO: 148, and a DNA fragment composed of tac promoter, MCS and rrnBT1T2 terminator was amplified using pTac15K as a template and the primers of SEQ ID NO: 149 and SEQ ID NO: 150, and cloned into the plasmid using Gibson assembly. Next, in order to transfer genes from the pTrc99A-zwf, pTrc99A-mdh and pTrc99A-serA plasmids constructed in Example 2.2 above to pBBR1TaC, the following experiment was performed. First, zwf, *mdh* and *serA* genes were each amplified using the primers of SEQ ID NO: 151 and SEQ ID NO: 152, and the pBBR1TaC plasmid was amplified using the primers of SEQ ID NO: 75 and SEQ ID NO: 76, and then subjected to Gibson assembly, thereby constructing pBBR1-zwf, pBBR1-mdh and pBBR1-serA plasmids. C. *glutamicum* acetyl-CoA carboxylase is composed of the alpha subunit AccBC and the beta subunit AccD1, and the corresponding genes were each cloned into pBBR1TaC, thereby constructing pBBR1-accBC and pBBR1-accD1. At this time, the *accBC* gene was amplified using the primers of SEQ ID NO: 153 and SEQ ID NO: 154, and the *accD1* gene was amplified using the primers of SEQ ID NO: 155 and SEQ ID NO: 156. The two gene fragments were each inserted by Gibson assembly into the pBBR1TaC plasmid amplified using the primers of SEQ ID NO: 9 and SEQ ID NO: 10. Using pBBR1-accD1 as a template among the two constructed plasmids, a DNA fragment containing tac promoter and *accD1* was amplified using the primers of SEQ ID NO: 157 and SEQ ID NO: 158, and then inserted by Gibson assembly into a pBBR1-accBC plasmid digested by HindIII restriction enzyme, thereby constructing a pBBR1-accBCD1 plasmid. *E. coli* pyruvate dehydrogenase (PDH) is composed of subunits E1 (AceE), E2 (AceF) and E3 (Lpd). First, an *aceEF* gene fragment and an *lpd* gene fragment were cloned into a pBBR1TaC plasmid, thereby constructing pBBR1-aceEF and pBBR1-lpd plasmids. The gene fragment *aceEF* was amplified using the primers of SEQ ID NO: 159 and SEQ ID NO: 160, and *lpd* was amplified using the primers of SEQ ID NO: 161 and SEQ ID NO: 162. The two gene fragments were each inserted by Gibson assembly into the linearized pBBR1TaC plasmid used for construction of the pBBR1-accBC and pBBR1-accD1 plasmids. Using pBBR1-lpd as a template among the two constructed plasmids, a gene fragment comprising tac promoter and *lpd* was amplified using the primers of SEQ ID NO: 163 and SEQ ID NO: 164, and then inserted into a pBBR1-aceEF plasmid digested by SalI restriction enzyme, thereby constructing a pBBR1-aceEF-lpd plasmid. For construction of pBBR1-gapA, pBBR1-pgk and pBBR1-acs plasmids containing *E. coli* glyceraldehyde 3-phosphate dehydrogenase (GapA), *E. coli* phosphoglycerate kinase (Pgk) and *E*. *coli* acetyl-CoA synthetase (Acs), respectively, *gapA, pgk* and acs gene fragments were amplified by PCR using the primers of SEQ ID NO: 165 and SEQ ID NO: 166, SEQ ID NO: 167 and SEQ ID NO: 168, and SEQ ID NO: 169 and SEQ ID NO: 170, and then each inserted by Gibson assembly into the linearized pBBR1TaC plasmid.
[SEQ ID NO: 147] 5'-GACGGATGGCCTTTTACTAGTGCCTGGGGTGCCTAATGAG-3'
[SEQ ID NO: 148] 5'-CGATGATTAATTGTCAACTGCTACGCCTGAATAAGTGATAATAAG-3'
[SEQ ID NO: 149] 5'-GTTGACAATTAATCATCGGCTC-3'
[SEQ ID NO: 150] 5'- ACTAGTAAAAGGCCATCCGTCAGGATG-3'
[SEQ ID NO: 151] 5'- GTTGACAATTAATCATCGGC-3'
[SEQ ID NO: 152] 5'- CGTTTCACTTCTGAGTTCGG-3'
[SEQ ID NO: 153] 5'-CAATTTCACACAGGAAACAGAATTCGTGTCAGTCGAGACTAGGAAG-3'
[SEQ ID NO: 154] 5'-CTCTAGAGGATCCCCGGGTACCATTACTTGATCTCGAGGAGAAC-3'
[SEQ ID NO: 155] 5'-CAATTTCACACAGGAAACAGAATTCATGACCATTTCCTCACCTTTG-3'
[SEQ ID NO: 156] 5'-CTCTAGAGGATCCCCGGGTACCATTACAGTGGCATGTTGCCGTG-3'
[SEQ ID NO: 157] 5'-GAGTCGACCTGCAGGCATGCATTGACAATTAATCATCGGCTCG-3'
[SEQ ID NO: 158] 5'-CTCATCCGCCAAAACAGCCAAGCTT-3'
[SEQ ID NO: 159] 5'-CAATTTCACACAGGAAACAGAATTCATGTCAGAACGTTTCCCAAATG-3'
[SEQ ID NO: 160] 5'-CTCTAGAGGATCCCCGGGTACCATTACATCACCAGACGGCGAATG-3'
[SEQ ID NO: 161] 5'-CAATTTCACACAGGAAACAGAATTCATGAGTACTGAAATCAAAACTC-3'
[SEQ ID NO: 162] 5'-CTCTAGAGGATCCCCGGGTACCATTACTTCTTCTTCGCTTTCGG-3'
[SEQ ID NO: 163] 5'-GTACCCGGGGATCCTCTAGAGTTGACAATTAATCATCGGCTCG-3'
[SEQ ID NO: 164] 5'- CAAGCTTGCATGCCTGCAGGTCGAC-3'
[SEQ ID NO: 165] 5'-CAATTTCACACAGGAAACAGAATTCATGACTATCAAAGTAGGTATCAAC-3'
[SEQ ID NO: 166] 5'-CTCTAGAGGATCCCCGGGTACCATTATTTGGAGATGTGAGCGATC-3'
[SEQ ID NO: 167] 5'-CAATTTCACACAGGAAACAGAATTCATGTCTGTAATTAAGATGACCGATC-3'
[SEQ ID NO: 168] 5'-CTCTAGAGGATCCCCGGGTACCATTACTTCTTAGCGCGCTCTTCG-3'
[SEQ ID NO: 169] 5'-CAATTTCACACAGGAAACAGAATTCATGAGCCAAATTCACAAACAC-3'
[SEQ ID NO: 170] 5'-CTCTAGAGGATCCCCGGGTACCATTACGATGGCATCGCGATAG-3'

The 8 plasmids constructed as described above were each transformed into the strain *E. coli* BAP1 pTac-Pg6MSAS pWAS-anti-pabA showing the highest production, and then flask culture of the strain was performed. The results of the flask culture are shown in FIG. 15A. When C. *glutamicum* acetyl-CoA carboxylase was overexpressed, the production of 6-methylsalicylic acid increased to 63.6 mg/L. Fed-batch fermentation of the corresponding strain was performed, and as a result, as shown in FIG. 15B, the production of 6-methylsalicylic acid from glycerol increased to 440.3 mg/L ± 30.2 mg/L.

### 3.2. Increased Production of Aloesone Using Selected Knockdown Gene Targets

As a second product, aloesone produced from *Rheum palmatum* (Abe I, Utsumi Y, Oguro S, Noguchi H (2004), FEBS Lett 562:171-176) or *Aloe arborescens* (Mizuuchi Y, et al. (2009), FEBS J 276:2391-2401) type III polyketide synthase was tested. Aloesone is a precursor of aloesin which is widely used in the cosmetic field due its whitening effect. However, a biosynthetic pathway for producing aloesin from aloesone has not yet been reported. It has been reported that aloesone is produced from one molecule of acetyl-CoA and six molecules of malonyl-CoA (FIG. 16) and is produced by R. *palmatum* aloesone synthase (ALS) or A. *arborescens* aloesone synthase (PKS3).

The sequence of *R. palmatum* aloesone synthase (ALS) is as follows.

R. palmatum aloesone synthase (ALS)[SEQ ID NO: 125]:
madvlqeirn sqkasgpatv laigtahppt cypqadypdf yfrvcksehm
tklkkkmqfi cdrsgirqrf mfhteenlgk npgmctfdgp slnarqdmli
mevpklgaea aekaikewgq dksrithlif ctttsndmpg adyqfatlfg
lnpgvsrtmv yqqgcfaggt vlrlvkdiae nnkgarvlvv cseivafafr
gphedhidsl igqllfgdga aalvvgtdid esverpifqi msatqatipn
slhtmalhlt eagltfhlsk evpkvvsdnm eelmleafkp lgitdwnsif
wqvhpggrai ldkieeklel tkdkmrdsry ilseygnlts acvlfvmdem
rkrsfregkq ttgdgyewgv aiglgpgltv etvvlrsvpi p

In addition, the sequence of A. *arborescens* aloesone synthase (PKS3) is as follows.

A. arborescens aloesone synthase, PKS3)[SEQ ID NO:
126]:
mgslsdstpl mkdvqgirka qkadgtatvm aigtahpphi isqdsyadfy
frvtnsehkv elkkkfdric kktmigkryf nfdeeflkky pnitsfdkps
lndrhdicip gvpalgaeaa vkaieewgrp kseithlvfc tsggvdmpsa
dfqcakllgl rtnvnkyciy mqgcyaggtv mryakdlaen nrgarvlmvc
aeltiialrg pndshidnai gnslfgdgaa alivgsdpii gvekpmfeiv

cakqtvipns eevihlhlre sglmfymtkd saatisnnie aclvdvfksv
gmtppedwns lfwiphpggr aildqveakl klrpekfsat rtvlwdygnm
isacvlyild emrrksaaeg letygeglew gvllgfgpgm tietillhsl ppv

Genes corresponding to the two enzymes were each synthesized by Integrated DNA Technologies Inc. (USA) and then inserted by Gibson assembly into a pCDFDuet-1 (Novagen) plasmid at the NcoI site. The two constructed plasmids pCDF-RpALS and pCDF-AaPKS3 were each transformed into an *E. coli* BL21(DE3) strain, and then the expression of the heterologous enzymes was analyzed by SDS-PAGE (FIG. 17A). Flask culture of the strains was performed, and as a result, 20.5 mg/L and 4.7 mg/L of aloesone were produced from 20 g/L of glucose (FIG. 17B). Accordingly, the plasmid pCDF-RpALS was used in the following experiments. The authenticity of the produced aloesone was confirmed by LC-MS and MS/MS analysis (FIG. 17C). Next, the 14 sRNAs selected in the above Example were introduced into various *E. coli* strains as described in Example 3.1 above. To this end, each sRNA was introduced into a BL21(DE3) strain and a W3110(DE3) strain, thereby constructing 28 strains. The results of test tube-scale culture of the constructed strains are shown in FIG. 17F. At this time, the *pabA*-knockdown BL21(DE3) strain showed the highest aloesone production (18.5 mg/L), and the aloesone production of the strain in flask culture was 27.1 mg/L, which corresponds to an increase of 32.2% compared to that of the control strain (containing no sRNA) (FIGS. 17D and 17E).

In order to further increase the production of aloesone, the expression of the three FVSEOF gene targets (*zwf, mdh,* and *serA;* FIG. 10) and the following five enzymes used for increased production of 6-methylsalicylic acid was increased: *C. glutamicum* acetyl-CoA carboxylase (AccBC and AccD1), *E*. *coli* glyceraldehyde 3-phosphate dehydrogenase (GapA), *E. coli* phosphoglycerate kinase (Pgk), *E. coli* acetyl-CoA synthetase (Acs), and *E. coli* pyruvate dehydrogenase (PDH: AceEF and Lpd). To this end, eight plasmids pBBR1-zwf, pBBR1-mdh, pBBR1-serA, pBBR1-accBCD1, pBBR1-gapA, pBBR1-pgk, pBBR1-acs, and pBBR1-aceEF-lpd containing the corresponding genes were each transformed into the strain showing the highest aloesone production (*E. coli* BL21(DE3) harboring pCDF-RpALS and pWAS-anti-pabA), and then flask culture of the resulting strains was performed. The results of the flask culture are shown in FIG. 18. When C. *glutamicum* acetyl-CoA carboxylase was overexpressed, the production of aloesone increased to 30.9 mg/L. In this case, 20 g/L of glucose was used as a carbon source.

### 3.3. Increased Production of Resveratrol Using Selected Knockdown Gene Targets

As a third product, resveratrol produced in plants was selected. Resveratrol, a compound belonging to the stilbenoid family among phenylpropanoid-based natural products, is a very useful natural product having antioxidant, anti-aging and anticancer effects, etc. Resveratrol is produced from one molecule of p-coumaroyl-CoA and three molecules of malonyl-CoA (FIG. 19). Accordingly, a strain that produces p-coumaric acid from a simple carbon source was constructed. To this end, a previously constructed tyrosine overproducing strain was used (Kim B, Binkley R, Kim HU, Lee SY (2018), Biotechnol Bioeng). This strain (BTY5.13) contains a pTac15K plasmid-based plasmid (pTY13) which overexpresses *Zymomonas mobilis tyrC, E. coli aroG*^{fbr}, and *aroL* in a *tyrR, tyrP*-knockdown strain (BTY5) based on *E. coli* BL21(DE3). To convert tyrosine to p-coumaric acid, tyrosine ammonia-lyase (TAL) should be expressed. Thus, the gene *SeTAL* encoding tyrosine ammonia-lyase was amplified from the genomic DNA of *Saccharothrix espanaensis* using the primers of SEQ ID NO: 36 and SEQ ID NO: 37, and then a pTrc99A plasmid was linearized by PCR using the primers of SEQ ID NO: 38 and SEQ ID NO: 39. Next, the two DNA fragments were assembled using Gibson assembly, thereby constructing the plasmid pTrc-SeTAL.

The sequence of tyrosine ammonia-lyase that was used in the present invention is as follows.

Tyrosine ammonia-lyase (TAL) [SEQ ID NO: 127]:

mtqvverqad rlssreylar vvrsagwdag ltsctdeeiv rmgasartie
eylksdkpiy gltqgfgplv lfdadseleq ggslishlgt gqgaplapev
srlilwlriq nmrkgysavs pvfwqkladl wnkgftpaip rhgtvsasgd
lqplahaala ftgvgeawtr dadgrwstvp avdalaalga epfdwpvrea
lafvngtgas lavavlnhrs alrlvracav lsarlatllg anpehydvgh
gvargqvgql taaewirqgl prgmvrdgsr plqepyslrc apqvlgavld
qldgagdvla revdgcqdnp ityegellhg gnfhampvgf asdqiglamh
maaylaerql gllvspvtng dlppmltpra grgaglagvq isatsfvsri
rqlvfpaslt tlptngwnqd hvpmalngan svfealelgw ltvgslavgv
aqlaamtgha aegvwaelag icppldadrp lgaevraard llsahadqll
vdeadgkdfg

In order to optimize the expression of tyrosine ammonia-lyase, His-tag and thioredoxin tag (TrxA) were each attached to the N-terminus. For attachment of His-tag, His-SeTAL was amplified by PCR using the genomic DNA of *S. espanaensis* and the primers of SEQ ID NO: 40 and SEQ ID NO: 37, and pTrc-SeTAL as a template was linearized by inverse PCR using the primers of SEQ ID NO: 38 and SEQ ID NO: 41. The two DNA fragments were assembled using Gibson assembly, thereby constructing pTrc-HisTAL. Next, *trxA* gene was amplified by PCR using the genomic DNA of *E. coli* W3110 as a template and the primers of SEQ ID NO: 42 and SEQ ID NO: 43, and TrxA-TAL was amplified by PCR using the genomic DNA of *S. espanaensis* as a template and the primers of SEQ ID NO: 44 and SEQ ID NO: 45. The two DNA fragments were subjected to extension PCR to obtain a single DNA fragment by PCR using the primers of SEQ ID NO: 46 and SEQ ID NO: 37. The obtained DNA fragment was assembled by Gibson assembly with the linearized pTrc-SeTAL DNA fragment used for construction of the pTrc-HisTAL, thereby constructing pTrc-TrxTAL. The three constructed plasmids pTrc-SeTAL, pTrc-HisTAL and pTrc-TrxTAL were each transformed into a BTY5.13 strain, and then inoculated into a flask containing 50 mL of modified MR medium supplemented with 20 g/L of glucose, and the strains were cultured at 30°C and 200 rpm. When the cells reached an OD₆₀₀ value of about 0.8, the cells were treated with 1 mM of IPTG, and then cultured for 36 hours. The modified MR medium contained, per liter, the following components: 6.67g KH₂PO₄, 4g (NH₄)₂HPO₄, 0.8g citric acid, 0.8g MgSO₄·7H₂O, 5 mL trace metal solution, 2g yeast extract and 15g (NH₄)₂SO₄. When His-TAL was expressed, the highest level of p-coumaric acid production (0.41 g/L) could be obtained (FIG. 20A). Thus, to construct a single plasmid by assembling pTrc-HisTAL with the pTY13 plasmid, the HisTAL portion was amplified using the primers of SEQ ID NO: 47 and SEQ ID NO: 48, and then inserted by Gibson assembly into the pTY13 plasmid at the NheI site, thereby constructing a pTY13-HisTAL plasmid. The constructed plasmid was transformed into a BTY5 strain, and then flask culture of the strain was performed. As a result, 0.35 g/L of p-coumaric acid was produced from 20 g/L of glycerol (FIG. 20A). 
[SEQ ID NO: 36] 5'-
GAATTGTGAGCGGATAACAAAGACCGAGGAAAAGGAGCATCGCAAATGACGCAGGTCGTGG
AACGTC-3'
[SEQ ID NO: 37] 5'-TAGAGGATCCCCGGGTACTCATCCGAAATCCTTCCCGTC-3'
[SEQ ID NO: 38] 5'-GTACCCGGGGATCCTCTAG-3'
[SEQ ID NO: 39] 5'-TTGTTATCCGCTCACAATTC-3' 
[SEQ ID NO: 40] 5'-
GAGGAAAAGGAGCATCGCAAATGCACCATCATCATCATCAT
ACGCAGGTCGTGGAACGTC-3'
[SEQ ID NO: 41] 5'-TTGCGATGCTCCTTTTCCTC-3'
[SEQ ID NO: 42] 5'-ATGAGCGATAAAATTATTCACCTG-3'
[SEQ ID NO: 43] 5'-CGCCAGGTTAGCGTCGAGG-3'
[SEQ ID NO: 44] 5'-CCTCGACGCTAACCTGGCGACGCAGGTCGTGGAACGTC-3'
[SEQ ID NO: 45] 5'- TCATCCGAAATCCTTCCCGTC-3'
[SEQ ID NO: 46] 5'-AGACCGAGGAAAAGGAGCATCGCAAATGAGCGATAAAATTATTCACCTG-3'
[SEQ ID NO: 47] 5'-GTAAGCCAGTATACACTCCGGACTGCACGGTGCACCAATG-3'
[SEQ ID NO: 48] 5'-CTGTTGGGCGCCATCTCCTTGTGTAGAAACGCAAAAAGGCCATC-3'

After successfully constructing the p-coumaric acid producing strain as described above, a downstream resveratrol biosynthetic pathway starting from p-coumaric acid was constructed, which is composed of *Arabidopsis thaliana* mutant 4-coumarate:CoA ligase (4CL) 1 (At4CL1m) and *Vitis vinifera* stilbene synthase (STS). First, At4CL1 gene was amplified from A. *thaliana* cDNA using the primers of SEQ ID NO: 49 and SEQ ID NO: 50, and then inserted by Gibson assembly into a pTac15K plasmid at EcoRI and KpnI sites, thereby constructing a pTac-At4CL1 plasmid. Next, pTac-At4CL1m (At4CL1m; I250L/N404K/I461V) was constructed by three consecutive rounds of site-directed mutagenesis in order to enhance substrate specificity for *p*-coumaroyl-CoA. At this time, for the three consecutive rounds of site-directed mutagenesis, the primer pairs of SEQ ID NO: 51 and SEQ ID NO: 52, SEQ ID NO: 53 and SEQ ID NO: 54, and SEQ ID NO: 55 and SEQ ID NO: 56 were used. Next, from A. *thaliana, At4CL3* and *At4CL4* genes that encode 4-coumarate:CoA ligase 3 and 4-coumarate:CoA ligase 4, respectively, were amplified by PCR using the primer pairs of SEQ ID NO: 57 and SEQ ID NO: 58, and SEQ ID NO: 59 and SEQ ID NO: 60, respectively, and from *S*. *coelicolor,* the *Sc4CL* gene encoding 4-coumarate:CoA ligase was amplified by PCR using the primer pair of SEQ ID NO: 61 and SEQ ID NO: 62. The amplified genes were cloned in the same manner as described above, thereby constructing pTac-At4CL3, pTac-At4CL4, and pTac-Sc4CL. For pTac-Sc4CL, one round of site-directed mutagenesis was performed using the primer pair of SEQ ID NO: 63 and SEQ ID NO: 64 in order to enhance substrate specificity for p-coumaroyl-CoA (Kaneko M, Ohnishi Y, Horinouchi S (2003), J Bacteriol 185:20-27), thereby constructing a pTac-Sc4CLm(Sc4CLm; A294G/A318G) plasmid. The *STS* gene encoding *Vitis vinifera* stilbene synthase was synthesized by Integrated DNA Technologies Inc., and it was amplified by PCR using the primer pair of SEQ ID NO: 65 and SEQ ID NO: 66, and then inserted into a pTac15K plasmid at EcoRI and KpnI sites using Gibson assembly, thereby constructing a pTac-VvSTS plasmid.

The sequence of *A*. *thaliana* 4-coumarate:CoA ligase 1 that was used in the present invention is as follows.

A. thaliana 4-coumarate:CoA ligase 1 [SEQ ID NO: 128]:
mapqeqavsq vmekqsnnnn sdvifrsklp diyipnhlsl hdyifqnise
fatkpcling ptghvytysd vhvisrqiaa nfhklgvnqn dvvmlllpnc
pefvlsflaa sfrgatataa npfftpaeia kqakasntkl iitearyvdk
ikplqnddgv vivciddnes vpipegclrf teltqsttea sevidsveis
pddvvalpys sgttglpkgv mlthkglvts vaqqvdgenp nlyfhsddvi
lcvlpmfhiy alnsimlcgl rvgaailimp kfeinlllel iqrckvtvap
mvppivlaia kssetekydl ssirvvksga aplgkeleda vnakfpnakl
gqgygmteag pvlamslgfa kepfpvksga cgtvvrnaem kivdpdtgds
lsrnqpgeic irghqimkgy lnnpaataet idkdgwlhtg digliddde
lfivdrlkel ikykgfqvap aelealligh pditdvavva mkeeaagevp
vafvvkskds elseddvkqf vskqvvfykr inkvfftesi pkapsgkilr
kdlraklang l

The sequence of *A*. *thaliana* 4-coumarate:CoA ligase 3 that was used in the present invention is as follows.

A. thaliana 4-coumarate:CoA ligase 3 [SEQ ID NO: 129]:
mitaalhepq ihkptdtsvv sddvlphspp tprifrsklp didipnhlpl
htycfeklss vsdkpclivg stgksytyge thlicrrvas glyklgirkg
dvimillqns aefvfsfmga smigavstta npfytsqely kqlkssgakl
iithsqyvdk lknlgenltl ittdeptpen clpfstlitd detnpfqetv
diggddaaal pfssgttglp kgvvlthksl itsvaqqvdg dnpnlylksn
dvilcvlplf hiyslnsvll nslrsgatvl lmhkfeigal ldliqrhrvt
iaalvpplvi alaknptvns ydlssvrfvl sgaaplgkel qdslrrrlpq
ailgqgygmt eagpvlsmsl gfakepiptk sgscgtvvrn aelkvvhlet
rlslgynqpg eicirgqqim keylndpeat satideegwl htgdigyvde
ddeifivdrl kevikfkgfq vppaelesll inhhsiadaa vvpqndevag
evpvafvvrs ngnditeedv keyvakqvvf ykrlhkvffv asipkspsgk
ilrkdlkakl c

The sequence of *A*. *thaliana* 4-coumarate:CoA ligase 4 that was used in the present invention is as follows.

A. thaliana 4-coumarate:CoA ligase 4 [SEQ ID NO: 130]:
mvlqqqthfl tkkidqedee eepshdfifr sklpdifipn hlpltdyvfq
rfsgdgdgds sttciidgat griltyadvq tnmrriaagi hrlgirhgdv
vmlllpnspe falsflavay lgavsttanp fytqpeiakq akasaakmii
tkkclvdklt nlkndgvliv clddddgngv vsssddgcvs fteltqadet
ellkpkispe dtvampyssg ttglpkgvmi thkglvtsia qkvdgenpnl
nftandvilc flpmfhiyal dalmlsamrt gaallivprf elnlvmeliq
rykvtvvpva ppvvlafiks peterydlss vrimlsgaat lkkeledavr
lkfpnaifgq gygmtesgtv akslafaknp fktksgacgt virnaemkvv
dtetgislpr nksgeicvrg hqlmkgylnd peatartidk dgwlhtgdig

fvddddeifi vdrlkelikf kgyqvapael eallishpsi ddaavvamkd
evadevpvaf varsqgsqlt eddvksyvnk qvvhykrikm vffievipka
vsgkilrkdl rakletmcsk

The sequence of *S. coelicolor* 4-coumarate:CoA ligase that was used in the present invention is as follows.

S. coelicolor 4-coumarate:CoA ligase [SEQ ID NO: 131]:
mfrseyadvp pvdlpihdav lggaaafgst palidgtdgt tltyeqvdrf
hrrvaaalae tgvrkgdvla lhspntvafp lafyaatrag asvttvhpla
taeefakqlk dsaarwivtv spllstarra aelaggvqei lvcdsapghr
slvdmlasta pepsvaidpa edvaalpyss gttgtpkgvm lthrqiatnl
aqlepsmpsa pgdrvlavlp ffhiygltal mnaplrlgat vvvlprfdle
qflaaiqnhr itslyvappi vlalakhplv adydlsslry ivsaaaplda
rlaaacsqrl glppvgqayg mtelspgthv vpldamadap pgtvgrliag
temrivsltd pgtdlpages geilirgpqi mkgylgrpda taamideegw
lhtgdvghvd adgwlfvvdr vkelikykgf qvapaeleah llthpgvada
avvgaydddg nevphafvvr qpaapglaes eimmyvaerv apykrvrrvt
fvdavpraas gkilrrqlre pr

The sequence of stilbene synthase that was used in the present invention is as follows.

Stilbene synthase [SEQ ID NO: 132]:
masveefrna qrakgpatil aigtatpdhc vyqsdyadfy frvtksehmt
alkkkfnric dksmikkryi hlteemleeh pnigaymaps lnirqeiita
evpklgkeaa lkalkewgqp kskithlvfc ttsgvempga dyklanllgl
epsvrrvmly hqgcyaggtv lrtakdlaen nagarvlvvc seitvvtfrg
psedaldslv gqalfgdgsa avivgsdpdi sierplfqlv saaqtfipns
agaiagnlre vgltfhlwpn vptlisenie kcltqafdpl gisdwnslfw

iahpggpail daveaklnld kkkleatrhv lseygnmssa cvlfildemr
kkslkgerat tgegldwgvl fgfgpgltie tvvlhsipmv tn

Each of the above-described plasmids was transformed into a BL21(DE3) strain, and then the expression of heterologous enzymes in the resulting strains was analyzed using SDS-PAGE. Then, in order to assemble the *4CL* gene with the *STS* gene into a single plasmid, the following operation was performed. First, a DNA fragment (containing tac promoter) for stilbene synthase expression was amplified by PCR from pTac-VvSTS using the primers of SEQ ID NO: 47 and SEQ ID NO: 48, and then inserted into pTac-At4CL3, pTac-At4CL4l and pTac-Sc4CLm plasmid at an NheI site by Gibson assembly, thereby constructing pTac-VvSTS-At4CL3, pTac-VvSTS-At4CL4 and pTac-VvSTS-Sc4CLm plasmids. For *At4CLlm,* a DNA fragment for 4-coumarate:CoA ligase expression was amplified by PCR from pTac-At4CL1m using the primers of SEQ ID NO: 67 and SEQ ID NO: 68, and then inserted into a pTac-VvSTS plasmid at the PvuII site, thereby constructing a pTac-VvSTS-At4CL1m plasmid. The constructed plasmids pTac-VvSTS-At4CL1m, pTac-VvSTS-At4CL3, pTac-VvSTS-At4CL4, and pTac-VvSTS-Sc4CLm was transformed into a BL21(DE3) strain, and flask culture of the strain was performed in medium supplemented with 2 mM p-coumaric acid). The results of the flask culture are shown in FIG. 20C. At this time, the BL21(DE3) pTac-VvSTS-At4CL1m strain showed the highest resveratrol production (18.0 mg/L). The fact that resveratrol was produced in this amount even though p-coumaric acid was added at a concentration of 2 mM (328.1 mg/L) was added was an indirect evidence that malonyl-CoA was a bottleneck. In the present invention, before solving the problem according to this assumption by increasing the concentration of malonyl-CoA, this problem was solved by regulating the expression of exogenous genes for resveratrol production in various ways, and then malonyl-CoA was addressed. Thus, in order to express the *At4CLlm* gene and the *STS* gene in a single operon, the *STS* gene was amplified by PCR using the primers of SEQ ID NO: 69 and SEQ ID NO: 70, and then inserted into a pTac-At4CL1m plasmid at the SphI site, thereby constructing a pTac-At4CL1m-opr-VvSTS plasmid. In addition, in order to facilitate the transfer of a substrate by expressing the two enzymes as a single fused enzyme, the *At4CLlm* gene was amplified by PCR using the primer pair of SEQ ID NO: 71 and SEQ ID NO: 72, and then inserted into a pTac-VvSTS plasmid at the NdeI site, thereby constructing a pTac-At4CL1m-fus-VvSTS plasmid. For expression of the fusion protein, a glycine-serine linker (Gly-Gly-Gly-Ser) was used. However, neither of the two strategies produced a higher level of resveratrol (FIG. 20D). Thus, pTac-VvSTS-At4CL1m was selected as a final plasmid, and this plasmid was not compatible with pTY13-HisTAL (the two plasmid all have a p15A replication origin), and thus was transferred to the above-mentioned pTacCDFS plasmid. To this end, an *STS* expression cassette was amplified by PCR using the primers of SEQ ID NO: 73 and SEQ ID NO: 74, and then assembled by Gibson assembly with a pTacCDFS plasmid linearized using the primers of SEQ ID NO: 75 and SEQ ID NO: 76. The constructed plasmid was treated with PstI restriction enzyme, and then assembled by Gibson assembly or T4 ligation with an *At4CLlm* expression cassette amplified using the primers of SEQ ID NO: 75 and SEQ ID NO: 76, thereby constructing a single plasmid (pTacCDF-VvSTS-At4CL1m). The constructed plasmid was transformed into a BL21(DE3) strain, and then flask culture of the strain was performed, thereby producing 21.2 mg/L of resveratrol (FIG. 20D). However, this is the result of supplying 2 mM p-coumaric acid. Thus, in order to produce resveratrol directly from glycerol, the pTacCDF-VvSTS-At4CL1m plasmid was transformed into a BTY5 pTY13-HisTAL strain, and flask culture of the strain was performed, and as a result, 12.4 mg/L of resveratrol was produced from 20 g/L of glycerol. The authenticity of the produced resveratrol was confirmed by LC-MS (FIG. 21).
[SEQ ID NO: 49] 5'-CAATTTCACACAGGAAACAGACATATGGCGCCACAAGAACAAGC-3'
[SEQ ID NO: 50] 5'-CTCTAGAGGATCCCCGGGTACCATTACAATCCATTTGCTAGTTTTG-3'
[SEQ ID NO: 51] 5'-CACAGCGATGACGTCCTACTCTGTGTTTTG-3'
[SEQ ID NO: 52] 5'-CAAAACACAGAGTAGGACGTCATCGCTGTG-3'
[SEQ ID NO: 53] 5'-CTCTTTCGAGGAAACAACCCGGTGAG-3'
[SEQ ID NO: 54] 5'-CTCACCGGGTTGTTTCCTCGAAAGAG-3'
[SEQ ID NO: 55] 5'-GATTGAAAGAACTTGTCAAGTATAAAGG-3'
[SEQ ID NO: 56] 5'-CCTTTATACTTGACAAGTTCTTTCAATC-3'
[SEQ ID NO: 57] 5'-CAATTTCACACAGGAAACAGACATATGATCACTGCAGCTCTACAC-3'
[SEQ ID NO: 58] 5'-CTCTAGAGGATCCCCGGGTACCATTAACAAAGCTTAGCTTTGAGG-3'
[SEQ ID NO: 59] 5'-CAATTTCACACAGGAAACAGACATATGGTGCTCCAACAACAAACG-3'
[SEQ ID NO: 60] 5'-CTCTAGAGGATCCCCGGGTACCATTATTTAGAGCACATGGTTTCC-3'
[SEQ ID NO: 61] 5'-CAATTTCACACAGGAAACAGACATATGTTCCGCAGCGAGTACGC-3'
[SEQ ID NO: 62] 5'-CTCTAGAGGATCCCCGGGTACCATTATCGCGGCTCCCTGAGCTGTC-3'
[SEQ ID NO: 63] 5'-GTACATCGTCAGCGGCGCCGCCCCGCTCGACG-3'
[SEQ ID NO: 64] 5'-CGTCGAGCGGGGCGGCGCCGCTGACGATGTAC-3'
[SEQ ID NO: 65] 5'-CAATTTCACACAGGAAACAGACATATGGCAAGTGTCGAGGAATTC-3'
[SEQ ID NO: 66] 5'-CTCTAGAGGATCCCCGGGTACCATTAATTGGTAACCATCGGAATGG-3'
[SEQ ID NO: 67] 5'-GTAAGCCAGTATACACTCCGGACTGCACGGTGCACCAATG-3'
[SEQ ID NO: 68] 5'-CTGTTGGGCGCCATCTCCTTGTGTAGAAACGCAAAAAGGCCATC-3'
[SEQ ID NO: 69] 5'-GAGTCGACCTGCAGGCATGCAATTTCACACAGGAAACAGA-3'
[SEQ ID NO: 70] 5'-CAAAACAGCCAAGCTTGCATG-3'
[SEQ ID NO: 71] 5'-TTTCACACAGGAAACAGACATATG-3'
[SEQ ID NO: 72] 5'-GAATTCCTCGACACTTGCCATACTACCACCACCCAATCCATTTGCTAGTTTTG-3'
[SEQ ID NO: 73] 5'-GTTGACAATTAATCATCGGC-3'
[SEQ ID NO: 74] 5'-CGTTTCACTTCTGAGTTCGG-3'
[SEQ ID NO: 75] 5'-CCGAACTCAGAAGTGAAACG-3'
[SEQ ID NO: 76] 5'-GCCGATGATTAATTGTCAAC-3'

The 14 sRNAs selected in Example 2.1 above were introduced into the final resveratrol producing strain constructed as described above, and flask culture of the strain was performed. The results of the flask culture are shown in FIG. 20E. As a result, when *pabA* was knocked down, 51.8 mg/L of resveratrol was produced from glycerol, and this production was 4.2-fold increase compared to that in the control strain (a strain having no sRNA). In addition, it is notable that *pabA* is a knockdown target which showed the greatest increase in the production of both 6-methylsalicylic acid and aloesone as mentioned above. Next, six knockdown gene targets enabling more than 2.5-fold increase in resveratrol titer were selected, and sRNA plasmids, each containing a combination of two among the gene targets, were constructed. Each of the sRNA plasmids was transformed into a resveratrol producing strain, and then flask culture of each of the resulting strains was performed. The results of the flask culture are shown in FIG. 20. However, an additional increase in resveratrol production was not achieved, because as the highest resveratrol production was 50.0 mg/L when *yfiD* and *purB* were simultaneously knocked down.

Here, the sRNA plasmids for combinatorial double knockdown were constructed as follows. A DNA fragment encoding a synthetic-control sRNA to be inserted was amplified from a parent plasmid using the primers of SEQ ID NO: 77 and SEQ ID NO: 78, and then assembled by Gibson assembly with a synthetic-control sRNA-containing parent plasmid linearized by PCR using the primers of SEQ ID NO: 79 and SEQ ID NO: 80.
[SEQ ID NO: 77] 5'-GAATTTTAACAAAATATTAACGAATTCTAACACCGTGCGTG-3'
[SEQ ID NO: 78] 5'-GTGCCACCTAAATTGTAAGCGGCGAATTGGGTACCTATAAAC-3'
[SEQ ID NO: 79] 5'- GCTTACAATTTAGGTGGCAC-3'
[SEQ ID NO: 80] 5'- GTTAATATTTTGTTAAAATTCGCG-3'

### 3.4. Increased Production of Naringenin Using Selected Knockdown Gene Targets

As a fourth product, naringenin produced in plants was selected. Naringenin is a common precursor of many pharmacologically useful products belonging to the flavonoid family among phenylpropanoid-based natural products. In addition, naringenin was reported to have anti-Alzheimer, anticancer, antioxidant and antifungal activities. Naringenin is produced from one molecule of *p*-coumaroyl-CoA and three molecules of malonyl-CoA, like resveratrol (FIG. 21). Since the *p*-coumaric acid producing strain was constructed in Example 3.3 above, a downstream naringenin biosynthetic pathway was constructed in the present example. Here, conversion from *p*-coumaric acid to *p*-coumaroyl-CoA was performed using *A. thaliana* mutant 4-coumarate:CoA ligase 1 (At4CL1m), and conversion from *p*-coumaroyl-CoA and malonyl-CoA to naringenin chalcone was performed using *Petunia x hybria* chalcone synthase (CHS), and conversion from naringenin chalcone to naringenin was performed using *A. thaliana* chalcone isomerase (CHI).

The sequence of chalcone synthase (CHS) that was used in the present invention is as follows.

Chalcone synthase (CHS) [SEQ ID NO: 133]:
mvtveeyrka qraegpatvm aigtatptnc vdqstypdyy fritnsehkt
dlkekfkrmc eksmikkrym hlteeilken psmceymaps ldarqdivvv

evpklgkeaa qkaikewgqp kskithlvfc ttsgvdmpgc dyqltkllgl
rpsvkrlmmy qqgcfaggtv lrlakdlaen nkgarvlvvc seitavtfrg
pndthldslv gqalfgdgag aiiigsdpip gverplfelv saaqtllpds
hgaidghlre vgltfhllkd vpglisknie ksleeafrpl sisdwnslfw
iahpggpail dqveiklglk peklkatrnv lsnygnmssa cvlfildemr
kasakeglgt tgeglewgvl fgfgpgltve tvvlhsvat

The sequence of chalcone isomerase (CHI) that was used in the present invention is as follows.

Chalcone isomerase (CHI) [SEQ ID NO: 134]:
msssnacasp spfpavtklh vdsvtfvpsv kspassnplf lggagvrgld
iqgkfviftv igvylegnav pslsvkwkgk tteeltesip ffreivtgaf
ekfikvtmkl pltgqqysek vtencvaiwk qlglytdcea kavekfleif
keetfppgss ilfalsptgs ltvafskdds ipetgiavie nkllaeavle
siigkngvsp gtrlsvaerl sqlmmknkde kevsdhsvee klaken

The *At4CL1m* gene encoding mutant 4-coumarate:CoA ligase 1 was amplified by PCR using *A*. *thaliana* cDNA as a template and the primers of SEQ ID NO: 81 and SEQ ID NO: 82, and the *AtCHI* gene encoding chalcone synthase was amplified by PCR using *A*. *thaliana* cDNA as a template and the primers of SEQ ID NO: 83 and SEQ ID NO: 84. Then, the two DNA fragments were assembled into a single DNA fragment by extension PCR using the primers of SEQ ID NO: 81 and SEQ ID NO: 84, and then inserted into a pTrc99A plasmid at KpnI and BamHI sites. In addition, the *PhCHS* gene encoding chalcone synthase was amplified by PCR using a DNA fragment (synthesized by Integrated DNA Technologies Inc.) as a template and the primers of SEQ ID NO: 85 and SEQ ID NO: 86, and then inserted into the BamHI and XbaI sites of the above-constructed plasmid, thereby constructing a pTrc-At4CL1m-AtCHI-PsCHS plasmid. For the same reason as described in Example 3.3 above, the plasmid pTrc-At4CL1m-AtCHI-PsCHS was digested with NcoI and PstI restriction enzymes so that it would be compatible with the plasmid in the *p*-coumaric acid producing strain. The produced DNA fragment was assembled by Gibson assembly with a pTrcCDFS plasmid linearized by inverse PCR using the primers of SEQ ID NO: 87 and SEQ ID NO: 88, thereby constructing a pTrcCDF-At4CL1m-AtCHI-PhCHS plasmid.
[SEQ ID NO: 81] 5'- AGACAGGGTACCATGGCGCCACAAGAACAAG-3'
[SEQ ID NO: 82] 5'-ATGTATATCTCCTTCTTAAAGTTAATTACAATCCATTTGCTAGTTTTGCCC-3'
[SEQ ID NO: 83] 5'-TTAACTTTAAGAAGGAGATATACATATGTCTTCATCCAACGCCTGC-3'
[SEQ ID NO: 84] 5'-AGACAGGGATCCTCAGTTCTCTTTGGCTAGTTTTTCC-3'
[SEQ ID NO: 85] 5'- AGAGAGGATCCATAACAATTCCCCATCTTAG-3'
[SEQ ID NO: 86] 5'-AGAGATCTAGATTAGGTAGCCACACTATGCAGAACC-3'
[SEQ ID NO: 87] 5'-GAAACTGCTTGTTCTTGTGGCGCCATGGTCTGTTTCCTGTGTG-3'
[SEQ ID NO: 88] 5'- CTAATCTAGAGTCGACCTGCAGGCATGCAAGCTTG-3'

The plasmid pTrcCDF-At4CL1m-AtCHI-PhCHS constructed as described above was introduced into a BTY5 pTY13-HisTAL strain, and then flask culture of the strain was performed. As a result, 37.2 mg/L and 64.5 mg/L of naringenin were produced from glucose and glycerol, respectively (FIG. 23A). Thus, the following experiments on naringenin production were all performed using glycerol. The expression of heterologous enzymes in the strain was analyzed by SDS-PAGE (FIG. 23B), and the authenticity of the produced naringenin was confirmed by LC-MS (FIG. 24). The 14 sRNAs selected in Example 2.1 above were introduced into the constructed naringenin producing strain, and flask culture of each of the resulting strains was performed. The results of the flask culture are shown in FIG. 25A. At this time, the *fadR*-knockdown strain showed the highest naringenin production (92.3 mg/L), which was 43% increase in naringenin production compared to that in the control strain (a naringenin producing strain having no sRNA). In addition, knockdown targets showing more than 15% increase compared to the control were combined and subjected to a combinatorial double knockdown experiment, and the results of the experiment are shown in FIG. 25B. At this time, in the strain in which both *fadR* and *xapR* were knocked down, 103.8 mg/L of naringenin was produced from glycerol, which was 61% increase in naringenin production compared to that in the control.

Here, the sRNA plasmid for combinatorial double knockdown was constructed as follows. A DNA fragment encoding a synthetic-control sRNA to be inserted was amplified from the parent plasmid using the primers of SEQ ID NO: 89 and SEQ ID NO: 90, and then assembled by Gibson assembly with a synthetic-control sRNA-containing parent plasmid linearized by PCR using the primers of SEQ ID NO: 91 and SEQ ID NO: 92.
[SEQ ID NO: 89] 5'-CACTAGATCTCAAATGTGCTGGAATTCTAACACCGTGCGTG-3'
[SEQ ID NO: 90] 5'-CCTTATAAATCAAACATGTGCGGCGAATTGGGTACCTATAAAC-3'
[SEQ ID NO: 91] 5'- GCACATGTTTGATTTATAAGGG-3'
[SEQ ID NO: 92] 5'- CAGCACATTTGAGATCTAGTGG-3'

As examined in Example 3 above, the knockdown targets for increased malonyl-CoA production, easily and rapidly screened by the RppA malonyl-CoA biosensor, all contributed greatly to the malonyl-CoA-based production of useful compounds. The four useful product-producing strains examined in the above Examples are not those constructed through many metabolic engineering strategies as reported in previous studies, but are those constructed in a short time by constructing basic production pathways and simply transforming sRNAs. The fact that the strains constructed in a sample manner as described above show remarkable producing ability is noteworthy in the art. In addition, it is obvious to those skilled in the art that the utility and applicability of the RppA biosensor are not limited only to the above-described examples.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

### INDUSTRIAL APPLICABILITY

Conventional methods for measuring malonyl-CoA concentration have various disadvantages and limitations in that they are time-consuming and labor-intensive, require skilled technology, have low data reliability, have difficulty in large-volume experiments, and require high-performance devices. However, the use of the biosensor according to the present invention provides single-step signal generation, utilization in various microorganisms, utilization in self-fluorescent microorganisms, a simple construction method, and a simple screening method. In addition, when the present invention is combined with high-throughput screening, it has advantages in that strains having increased malonyl-CoA producing ability can be screened very easily and rapidly (∼3 days) and can be applied directly to the malonyl-CoA-based production of useful compounds.

## Claims

1. A recombinant microorganism for malonyl-CoA detection in which a type III polyketide synthase-encoding gene is inserted in the genome of the microorganism or in which a recombinant vector containing the type III polyketide synthase-encoding gene is introduced.

2. The recombinant microorganism of claim 1, wherein the type III polyketide synthase is:
RppA derived from a microorganism selected from the group consisting of *Streptomyces griseus, Streptomyces coelicolor, Streptomyces avermitilis, Saccharopolyspora erythraea, Streptomyces peucetius,* and *Streptomyces aculeolatus;*
PhlD(polyketide synthase) derived from *Pseudomonas fluorescens;*
DpgA(polyketide synthase) derived from *Amycolatopsis mediterranei;*
ALS(aloesone synthase) derived from *Rheum palmatum;* or
PCS (5,7-dihydroxy-2-methylchromone synthase), OKS (octaketide synthase), PKS3 (aloesone synthase), PKS4 (octaketide synthase 2) or PKS5(octaketide synthase 3), which is derived from *Aloe arborescens.*

3. The recombinant microorganism of claim 1, wherein a gene encoding the type III polyketide synthase is operably linked to a promoter selected from the group consisting of tac, trc, T7, BAD, λPR an Anderson synthetic promoter.

4. The recombinant microorganism of claim 1, wherein the recombinant microorganism is selected from the group consisting of *E. coli, Rhizobium, Bifidobacterium, Rhodococcus, Candida, Erwinia, Enterobacter, Pasteurella, Mannheimia, Actinobacillus, Aggregatibacter, Xanthomonas, Vibrio, Pseudomonas, Azotobacter, Acinetobacter, Ralstonia, Agrobacterium, Rhodobacter, Zymomonas, Bacillus, Staphylococcus, Lactococcus, Streptococcus, Lactobacillus, Clostridium, Corynebacterium, Streptomyces, Bifidobacterium, Cyanobacterium,* and *Cyclobacterium.*

5. A method for screening a malonyl-CoA production-inducing substance, comprising the steps of:
(a) culturing the recombinant microorganism of claim 1;
(b) adding a candidate substance to the recombinant microorganism;
(c) comparing the color of a culture supernatant of the recombinant microorganism after addition of the candidate substance with the color of a culture supernatant of the recombinant microorganism without addition of the candidate substance; and
(d) selecting the candidate substance as the malonyl-CoA production-inducing substance, when the culture supernatant of the recombinant microorganism after addition of the candidate substance shows a deeper red color than the culture supernatant of the recombinant microorganism without addition of the candidate substance.

6. The method of claim 5, wherein the comparison between the colors in step (c) is made by the naked eyes.

7. The method of claim 5, wherein the comparison between the colors in step (c) is made by measuring absorbance, and step (d) comprises selecting the candidate substance as the malonyl-CoA production-inducing substance, when the absorbance measured after addition of the candidate substance is higher than that measured without addition of the candidate substance.

8. A method for screening a gene involved in increase of malonyl-CoA production, the method comprising the steps of:
(a) introducing a gene regulation library, which changes gene expression in a recombinant microorganism, into the recombinant microorganism of claim 1, thereby constructing a recombinant microorganism library in which gene expression in the recombinant microorganism is changed;
(b) culturing the constructed recombinant microorganism library, measuring the absorbance of the culture supernatant of the recombinant microorganism library, culturing the recombinant microorganism before introduction of the gene regulation library, and comparing the color of the culture supernatant of the recombinant microorganism library with the color of the culture supernatant of the recombinant microorganism before introduction of the gene regulation library; and
(c) selecting the gene introduced in the recombinant microorganism library as a gene involved in increase of malonyl-CoA production, when the culture supernatant of the recombinant microorganism library shows a deeper red color than the culture supernatant of the recombinant microorganism before introduction of the gene regulation library.

9. The method of claim 8, wherein the comparison between the colors in step (b) is made by the naked eyes.

10. The method of claim 8, wherein the comparison between the colors in step (b) is made by measuring absorbance, and step (c) comprises selecting the gene introduced in the recombinant microorganism library, when the absorbance of the culture supernatant of the recombinant microorganism library is higher than that of the culture supernatant of the recombinant microorganism before introduction of the gene regulation library.

11. The method of claim 8, wherein the gene regulation library is a library selected from an sRNA library, a genomic library, a cDNA library, a gRNA library, and an oligonucleotide library for construction of knockout or mutant strains.

12. A method for producing a recombinant microorganism having increased malonyl-CoA producing ability, the method comprising regulating expression of the gene, screened by the method of any one of claims 8 to 11, in a microorganism having malonyl-CoA producing ability.

13. The method of claim 12, wherein the screened gene is one or more genes selected from the group consisting of:
*fabF* (3-oxoacyl-[acyl-carrier-protein] synthase II);
*yfcY* (beta-ketoacyl-CoA thiolase);
*xapR* (transcriptional activator of *xapAB*);
*cytR* (transcriptional repressor for *deo* operon, *udp, cdd, tsx, nupC* and *nupG*);
*fabH* (3-oxoacyl-[acyl-carrier-protein] synthase III);
*mqo* (malate dehydrogenase);
*yfiD* (pyruvate formate lyase subunit);
*fmt* (10-formyltetrahydrofolate:L-methionyl-tRNA(fMet)N-formyltransferase);
*pyrF* (orotidine-5'-phosphate decarboxylase);
*araA* (L-arabinose isomerase);
*fadR* (negative regulator for fad regulon and positive regulator of *fabA*);
*pabA* (aminodeoxychorismate synthase, subunit II);
*purB* (adenylosuccinate lyase); and
*hycI* (protease involved in processing C-terminal end of HycE),
and expression of the gene is reduced.

14. The method of claim 12, wherein the microorganism is a microorganism selected from the group consisting of *E. coli, Rhizobium, Bifidobacterium, Rhodococcus, Candida, Erwinia, Enterobacter, Pasteurella, Mannheimia, Actinobacillus, Aggregatibacter, Xanthomonas, Vibrio, Pseudomonas, Azotobacter, Acinetobacter, Ralstonia, Agrobacterium, Rhodobacter, Zymomonas, Bacillus, Staphylococcus, Lactococcus, Streptococcus, Lactobacillus, Clostridium, Corynebacterium, Streptomyces, Bifidobacterium, Cyanobacterium,* and *Cyclobacterium.*

15. A recombinant microorganism having increased malonyl-CoA producing ability wherein expression of one or more genes selected from the group consisting of the following genes:
*fabF* (3-oxoacyl-[acyl-carrier-protein] synthase II);
*yfcY* (beta-ketoacyl-CoA thiolase);
*xapR* (transcriptional activator of *xapAB*);
*cytR* (transcriptional repressor for *deo* operon, *udp, cdd, tsx, nupC* and *nupG*);
*fabH* (3-oxoacyl-[acyl-carrier-protein] synthase III);
*mqo* (malate dehydrogenase);
*yfiD* (pyruvate formate lyase subunit);
*fmt* (10-formyltetrahydrofolate:L-methionyl-tRNA(fMet)N-formyltransferase);
*pyrF* (orotidine-5'-phosphate decarboxylase);
araA (L-arabinose isomerase);
*fadR* (negative regulator for fad regulon and positive regulator of *fabA*);
*pabA* (aminodeoxychorismate synthase, subunit II);
*purB* (adenylosuccinate lyase); and
*hycI* (protease involved in processing C-terminal end of HycE), in a microorganism having malonyl-CoA producing ability, is decreased compared to that in a wild-type microorganism.

16. A method of producing a useful substance using malonyl-CoA as a substrate or an intermediate, the method comprising the steps of:
(a) constructing a recombinant microorganism in which a gene involved in production of the useful substance is additionally introduced, or expression of the gene is increased, or the gene involved in production of the useful substance is additionally deleted, or expression of the gene is decreased in the recombinant microorganism of claim 15;
(b) culturing the constructed microorganism; and
(c) recovering the useful substance from the cultured microorganism.

17. The method of claim 16, wherein the useful substance is one or more selected from among:
a polyketide compound of actinorhodin, doxorubicin, daunorubicin, oxytetracycline, rapamycin, lovastatin, SEK4, SEK4b, SEK34, SEK15, SEK26, FK506, DMAC, aklavinone, aklanonic acid, epsilon-rhodomycinone, aloesin, aloenin, barbaloin, 5,7-dihydroxy-2-methylchromone, erythromycin, rifamycin, avermectin, geldanamycin, ivermectin, doxycycline, anthramycin, penicillic acid, calicheamicin, epothilone, tetracenomycin, frenolicin, triacetic acid lactone, 6-methylsalicylic acid, or aloesone;
a phenylpropanoid compound of pinocembrin, dihydrokaempferol, eriodictyol, dihydroquercetin, daidzein, genistein, apigenin, luteolin, kaempferol, quercetin, catechin, pelargonidin, cyanidin, afzelechin, myricetin, fisetin, galangin, hesperetin, tangeritin, delphinidin, epicatechin, chrysin, resveratrol or naringenin;
a biofuel group of pentane, hexane, heptane, octane, nonane, decane, undecane, dodecane, tridecane, tetradecane, pentadecane, hexadecane, heptadecane, octadecane, nonadecane, icosane, pentanol, hexanol, heptanol, octanol, nonanol, decanol, undecanol, dodecanol, tridecanol, tetradecanol, pentadecanol, hexadecanol, heptadecanol, octadecanol, nonadecanol, icosanol, methyl caprate, methyl laurate, methyl myristate, methyl palmitate, methyl palmitoleate, methyl stearate, methyl oleate, methyl linoleate, methyl linolenate, methyl arachidate, methyl paullinate, methyl erucate, ethyl caprate, ethyl laurate, ethyl myristate, ethyl palmitate, ethyl palmitoleate, ethyl stearate, ethyl oleate, ethyl linoleate, ethyl linolenate, ethyl arachidate, ethyl paullinate or methyl erucate;
a lipid compound of ceramide, palmitate or sphingosine; and
3-hydroxypropionic acid.

18. A recombinant microorganism for producing 6-methylsalicylic acid in which genes that encode 6-methylsalicylic acid synthase (6MSAS) and 4'-phosphopantetheinyl transferase (Sfp) are additionally introduced or expression of the genes is increased in the recombinant microorganism of claim 15.

19. The recombinant microorganism of claim 18, wherein the knocked down gene is one or more selected from the group consisting of *pabA, fabF, xapR* and *ytcY.*

20. The recombinant microorganism of claim 18, wherein genes that encode one or more enzymes selected from the group consisting of glucose-6-phosphate dehydrogenase (Zwf), malate dehydrogenase (Mdh), phosphoglycerate dehydrogenase (SerA), acetyl-CoA carboxylase (AccBC and AccD1), glyceraldehyde 3-phosphate dehydrogenase (GapA), phosphoglycerate kinase (Pgk), acetyl-CoA synthetase (Acs), and pyruvate dehydrogenase (AceEF and Lpd) are additionally introduced in the recombinant microorganism or expression of the genes is increased.

21. A method of producing 6-methylsalicylic acid, the method comprising the steps of:
(a) culturing the recombinant microorganism of claim 18; and
(b) recovering the 6-methylsalicylic acid from the cultured microorganism.

22. A recombinant microorganism for producing aloesone in which a gene that encodes aloesone synthase is additionally introduced or expression of the gene is increased in the recombinant microorganism of claim 15.

23. The recombinant microorganism of claim 22, wherein the knocked down gene is *pabA.*

24. The recombinant microorganism of claim 22, wherein genes that encode one or more enzymes selected from the group consisting of glucose-6-phosphate dehydrogenase (Zwf), malate dehydrogenase (Mdh), phosphoglycerate dehydrogenase (SerA), acetyl-CoA carboxylase (AccBC and AccD1), glyceraldehyde 3-phosphate dehydrogenase (GapA), phosphoglycerate kinase (Pgk), acetyl-CoA synthetase (Acs), and pyruvate dehydrogenase (AceEF and Lpd) are additionally introduced in the recombinant microorganism or expression of the genes is increased.

25. A method of producing aloesone, the method comprising the steps of:
(a) culturing the recombinant microorganism of claim 22; and
(b) recovering the aloesone from the cultured microorganism.

26. A recombinant microorganism for producing resveratrol in which genes that encode tyrosine ammonia-lyase (TAL), 4-coumarate:CoA ligase (4CL) and stilbene synthase (STS) are additionally introduced or expression of the genes is increased in the recombinant microorganism of claim 15.

27. The recombinant microorganism of claim 26, wherein the 4-coumarate:CoA ligase is either a mutant enzyme in which the amino acid is mutated to I250L/N404K/I461V in the amino acid sequence represented by SEQ ID NO: 128 or a mutant enzyme in which the amino acid is mutated to A294G/A318G in the amino acid sequence represented by SEQ ID NO: 131.

28. The recombinant microorganism of claim 26, wherein the knocked down gene is one or more selected from the group consisting of *pabA, yfiD, mqo, xapR, purB, fabH, fabF, yfcY,* , *fadR, cytR, fmt* and *pyrF.*

29. A method of producing resveratrol, the method comprising the steps of:
(a) culturing the recombinant microorganism of claim 26; and
(b) recovering the resveratrol from the cultured microorganism.

30. A recombinant microorganism for producing naringenin in which genes that encode tyrosine ammonia-lyase (TAL), 4-coumarate:CoA ligase (4CL), chalcone synthase (CHS), and chalcone isomerase (CHI) are additionally introduced or expression of the genes is increased in the recombinant microorganism of claim 15.

31. The recombinant microorganism of claim 30, wherein the 4-coumarate:CoA ligase is a mutant enzyme in which the amino acid is mutated to I250L/N404K/I461V in the amino acid sequence represented by SEQ ID NO: 128.

32. The recombinant microorganism of claim 30, wherein the knocked down gene is one or more selected from the group consisting of *fadR, hycI* and *xapR.*

33. A method of producing naringenin, the method comprising the steps of:
(a) culturing the recombinant microorganism of claim 30; and
(b) recovering the naringenin from the cultured microorganism.


SEQUENCE LISTING

<110>  KOREA ADVANCED INSTITUTE OF SCIENCE AND TECHNOLOGY

<120>  NOVEL MALONYL-COA BIOSENSOR BASED ON TYPE III POLYKETIDE SYNTHASE
        AND USE THEREOF

<130>  PF-B2212

<140>  PCT/KR2018/010087
<141>  2018-08-30

<150>  KR10-2018-0066323
<151>  2018-06-08

<160>  181

<170>  PatentIn version 3.5

<210>  1
<211>  41
<212>  DNA
<213>  Artificial Sequence

<220>
<223>  Primer

<400>  1
ctttaagaag gagatataca tatggcgacc ctgtgccgac c                          41


<210>  2
<211>  43
<212>  DNA
<213>  Artificial Sequence

<220>
<223>  Primer

<400>  2
cttgtcgacg gagctcgaat tcattagccg gacagcgcaa cgc                        43


<210>  3
<211>  40
<212>  DNA
<213>  Artificial Sequence

<220>
<223>  Primer

<400>  3
cgactcctgc attaggaaat gactgcacgg tgcaccaatg                            40


<210>  4
<211>  20
<212>  DNA
<213>  Artificial Sequence

<220>
<223>  Primer

<400>  4
gcgtttcact tctgagttcg                                                   20


<210>  5
<211>  41
<212>  DNA
<213>  Artificial Sequence

<220>
<223>  Primer

<400>  5
cgaactcaga agtgaaacgc ctgaaacctc aggcatttga g                           41


<210>  6
<211>  20
<212>  DNA
<213>  Artificial Sequence

<220>
<223>  Primer

<400>  6
atttcctaat gcaggagtcg                                                   20


<210>  7
<211>  41
<212>  DNA
<213>  Artificial Sequence

<220>
<223>  Primer

<400>  7
caatttcaca caggaaacag aatggcgacc ctgtgccgac c                           41


<210>  8
<211>  43
<212>  DNA
<213>  Artificial Sequence

<220>
<223>  Primer

<400>  8
ctctagagga tccccgggta ccattagccg gacagcgcaa cgc                         43


<210>  9
<211>  22
<212>  DNA
<213>  Artificial Sequence

<220>
<223>  Primer

<400>  9
ggtacccggg gatcctctag ag                                                22

<210>  10
<211>  21
<212>  DNA
<213>  Artificial Sequence

<220>
<223>  Primer

<400>  10
tctgtttcct gtgtgaaatt g                                                 21


<210>  11
<211>  44
<212>  DNA
<213>  Artificial Sequence

<220>
<223>  Primer

<400>  11
gatgctcctt tcttgttatt gaaattgtta tccgctcaca attc                        44


<210>  12
<211>  40
<212>  DNA
<213>  Artificial Sequence

<220>
<223>  Primer

<400>  12
cgaactcaga agtgaaacgc tgcctaatga gtgagctaac                             40


<210>  13
<211>  41
<212>  DNA
<213>  Artificial Sequence

<220>
<223>  Primer

<400>  13
cattggtgca ccgtgcagtc aaaattcgcg ttaaattttt g                           41


<210>  14
<211>  20
<212>  DNA
<213>  Artificial Sequence

<220>
<223>  Primer

<400>  14
gactgcacgg tgcaccaatg                                                   20


<210>  15
<211>  20

<212>  DNA
<213>  Artificial Sequence

<220>
<223>  Primer

<400>  15
gcgtttcact tctgagttcg                                                    20


<210>  16
<211>  59
<212>  DNA
<213>  Artificial Sequence

<220>
<223>  Primer

<400>  16
caatttcaca caggaaacag aatgcaccat caccatcacc atgcgaccct gtgccgacc         59


<210>  17
<211>  20
<212>  DNA
<213>  Artificial Sequence

<220>
<223>  Primer

<400>  17
ccattataat taggcctcgg                                                    20


<210>  18
<211>  20
<212>  DNA
<213>  Artificial Sequence

<220>
<223>  Primer

<400>  18
ccatgctact cctaccaacc                                                    20


<210>  19
<211>  45
<212>  DNA
<213>  Artificial Sequence

<220>
<223>  Primer

<400>  19
ggttggtagg agtagcatgg gatccatggc gaccctgtgc cgacc                        45


<210>  20
<211>  41
<212>  DNA
<213>  Artificial Sequence

<220>
<223>  Primer

<400>  20
ccgaggccta attataatgg attagccgga cagcgcaacg c                          41


<210>  21
<211>  48
<212>  DNA
<213>  Artificial Sequence

<220>
<223>  Primer

<400>  21
ggttggtagg agtagcatgg gatccatgca ccatcaccat caccatgc                   48


<210>  22
<211>  47
<212>  DNA
<213>  Artificial Sequence

<220>
<223>  Primer

<400>  22
cttgatcagc ttgcatgcct gcagaagctt tctagcagaa ataattc                    47


<210>  23
<211>  27
<212>  DNA
<213>  Artificial Sequence

<220>
<223>  Primer

<400>  23
gtgcatgtgg actccctttc tcttatc                                          27


<210>  24
<211>  41
<212>  DNA
<213>  Artificial Sequence

<220>
<223>  Primer

<400>  24
gataagagaa agggagtcca catggcgacc ctgtgccgac c                          41


<210>  25
<211>  45
<212>  DNA
<213>  Artificial Sequence

<220>
<223>  Primer

<400>  25
ggatcctcta gagtcgacct gcagattagc cggacagcgc aacgc                    45


<210>  26
<211>  44
<212>  DNA
<213>  Artificial Sequence

<220>
<223>  Primer

<400>  26
gataagagaa agggagtcca catgcaccat caccatcacc atgc                     44


<210>  27
<211>  43
<212>  DNA
<213>  Artificial Sequence

<220>
<223>  Primer

<400>  27
gctgagaagc ttgccaaata atggatcctc tagagtcgac ctg                      43


<210>  28
<211>  74
<212>  DNA
<213>  Artificial Sequence

<220>
<223>  Primer

<400>  28
ctggggatgt tttcccagag gggtatgtag aagttgcagc ggaatgcatg aattctgttt    60

cctgtgtgaa attg                                                      74


<210>  29
<211>  24
<212>  DNA
<213>  Artificial Sequence

<220>
<223>  Primer

<400>  29
acagtgaata tgaattctcc aacg                                           24


<210>  30
<211>  22
<212>  DNA
<213>  Artificial Sequence

<220>
<223>  Primer

<400>  30

attatttggc aagcttctca gc                                              22

<210>  31
<211>  71
<212>  DNA
<213>  Artificial Sequence

<220>
<223>  Primer

<400>  31
ctctgggaaa acatccccag caccagtcgg aacccctggg actgagtaca gtgaatatga      60

attctccaac g                                                          71

<210>  32
<211>  36
<212>  DNA
<213>  Artificial Sequence

<220>
<223>  Primer

<400>  32
taataagaat tcatgaagat ttacggaatt tatatg                               36

<210>  33
<211>  34
<212>  DNA
<213>  Artificial Sequence

<220>
<223>  Primer

<400>  33
ttattagaat tcttataaaa gctcttcgta cgag                                 34

<210>  34
<211>  43
<212>  DNA
<213>  Artificial Sequence

<220>
<223>  Primer

<400>  34
ctagagtcga cctgcaggca tgccactccc gttctggata atg                       43

<210>  35
<211>  22
<212>  DNA
<213>  Artificial Sequence

<220>
<223>  Primer

<400>  35
caaaacagcc aagcttgcat gc                                              22

<210>  36
<211>  67
<212>  DNA
<213>  Artificial Sequence

<220>
<223>  Primer

<400>  36
gaattgtgag cggataacaa agaccgagga aaaggagcat cgcaaatgac gcaggtcgtg      60

gaacgtc                                                                67


<210>  37
<211>  39
<212>  DNA
<213>  Artificial Sequence

<220>
<223>  Primer

<400>  37
tagaggatcc ccgggtactc atccgaaatc cttcccgtc                             39


<210>  38
<211>  19
<212>  DNA
<213>  Artificial Sequence

<220>
<223>  Primer

<400>  38
gtacccgggg atcctctag                                                   19


<210>  39
<211>  20
<212>  DNA
<213>  Artificial Sequence

<220>
<223>  Primer

<400>  39
ttgttatccg ctcacaattc                                                  20


<210>  40
<211>  60
<212>  DNA
<213>  Artificial Sequence

<220>
<223>  Primer

<400>  40
gaggaaaagg agcatcgcaa atgcaccatc atcatcatca tacgcaggtc gtggaacgtc      60

<210>  41
<211>  20
<212>  DNA
<213>  Artificial Sequence

<220>
<223>  Primer

<400>  41
ttgcgatgct cctttcctc                                                    20


<210>  42
<211>  24
<212>  DNA
<213>  Artificial Sequence

<220>
<223>  Primer

<400>  42
atgagcgata aaattattca cctg                                              24


<210>  43
<211>  19
<212>  DNA
<213>  Artificial Sequence

<220>
<223>  Primer

<400>  43
cgccaggtta gcgtcgagg                                                    19


<210>  44
<211>  38
<212>  DNA
<213>  Artificial Sequence

<220>
<223>  Primer

<400>  44
cctcgacgct aacctggcga cgcaggtcgt ggaacgtc                               38


<210>  45
<211>  21
<212>  DNA
<213>  Artificial Sequence

<220>
<223>  Primer

<400>  45
tcatccgaaa tccttcccgt c                                                 21


<210>  46
<211>  49

<212>  DNA
<213>  Artificial Sequence

<220>
<223>  Primer

<400>  46
agaccgagga aaaggagcat cgcaaatgag cgataaaatt attcacctg                    49


<210>  47
<211>  40
<212>  DNA
<213>  Artificial Sequence

<220>
<223>  Primer

<400>  47
gtaagccagt atacactccg gactgcacgg tgcaccaatg                              40


<210>  48
<211>  44
<212>  DNA
<213>  Artificial Sequence

<220>
<223>  Primer

<400>  48
ctgttgggcg ccatctcctt gtgtagaaac gcaaaaaggc catc                         44


<210>  49
<211>  44
<212>  DNA
<213>  Artificial Sequence

<220>
<223>  Primer

<400>  49
caatttcaca caggaaacag acatatggcg ccacaagaac aagc                         44


<210>  50
<211>  46
<212>  DNA
<213>  Artificial Sequence

<220>
<223>  Primer

<400>  50
ctctagagga tccccgggta ccattacaat ccatttgcta gttttg                       46


<210>  51
<211>  30
<212>  DNA
<213>  Artificial Sequence

<220>
<223>  Primer

<400>  51
cacagcgatg acgtcctact ctgtgttttg                                        30


<210>  52
<211>  30
<212>  DNA
<213>  Artificial Sequence

<220>
<223>  Primer

<400>  52
caaaacacag agtaggacgt catcgctgtg                                        30


<210>  53
<211>  26
<212>  DNA
<213>  Artificial Sequence

<220>
<223>  Primer

<400>  53
ctctttcgag gaaacaaccc ggtgag                                            26


<210>  54
<211>  26
<212>  DNA
<213>  Artificial Sequence

<220>
<223>  Primer

<400>  54
ctcaccgggt tgtttcctcg aaagag                                            26


<210>  55
<211>  28
<212>  DNA
<213>  Artificial Sequence

<220>
<223>  Primer

<400>  55
gattgaaaga acttgtcaag tataaagg                                          28


<210>  56
<211>  28
<212>  DNA
<213>  Artificial Sequence

<220>
<223>  Primer

<400>  56
cctttatact tgacaagttc tttcaatc                                          28


<210>  57
<211>  45
<212>  DNA
<213>  Artificial Sequence

<220>
<223>  Primer

<400>  57
caatttcaca caggaaacag acatatgatc actgcagctc tacac                       45


<210>  58
<211>  45
<212>  DNA
<213>  Artificial Sequence

<220>
<223>  Primer

<400>  58
ctctagagga tccccgggta ccattaacaa agcttagctt tgagg                       45


<210>  59
<211>  45
<212>  DNA
<213>  Artificial Sequence

<220>
<223>  Primer

<400>  59
caatttcaca caggaaacag acatatggtg ctccaacaac aaacg                       45


<210>  60
<211>  45
<212>  DNA
<213>  Artificial Sequence

<220>
<223>  Primer

<400>  60
ctctagagga tccccgggta ccattattta gagcacatgg tttcc                       45


<210>  61
<211>  44
<212>  DNA
<213>  Artificial Sequence

<220>
<223>  Primer

<400>  61
caatttcaca caggaaacag acatatgttc cgcagcgagt acgc                        44

<210>  62
<211>  46
<212>  DNA
<213>  Artificial Sequence

<220>
<223>  Primer

<400>  62
ctctagagga tccccgggta ccattatcgc ggctccctga gctgtc                    46


<210>  63
<211>  32
<212>  DNA
<213>  Artificial Sequence

<220>
<223>  Primer

<400>  63
gtacatcgtc agcggcgccg ccccgctcga cg                                   32


<210>  64
<211>  32
<212>  DNA
<213>  Artificial Sequence

<220>
<223>  Primer

<400>  64
cgtcgagcgg ggcggcgccg ctgacgatgt ac                                   32


<210>  65
<211>  45
<212>  DNA
<213>  Artificial Sequence

<220>
<223>  Primer

<400>  65
caatttcaca caggaaacag acatatggca agtgtcgagg aattc                     45


<210>  66
<211>  46
<212>  DNA
<213>  Artificial Sequence

<220>
<223>  Primer

<400>  66
ctctagagga tccccgggta ccattaattg gtaaccatcg gaatgg                    46


<210>  67
<211>  40

<212>  DNA
<213>  Artificial Sequence

<220>
<223>  Primer

<400>  67
gtaagccagt atacactccg gactgcacgg tgcaccaatg                              40


<210>  68
<211>  44
<212>  DNA
<213>  Artificial Sequence

<220>
<223>  Primer

<400>  68
ctgttgggcg ccatctcctt gtgtagaaac gcaaaaaggc catc                         44


<210>  69
<211>  40
<212>  DNA
<213>  Artificial Sequence

<220>
<223>  Primer

<400>  69
gagtcgacct gcaggcatgc aatttcacac aggaaacaga                              40


<210>  70
<211>  21
<212>  DNA
<213>  Artificial Sequence

<220>
<223>  Primer

<400>  70
caaaacagcc aagcttgcat g                                                  21


<210>  71
<211>  24
<212>  DNA
<213>  Artificial Sequence

<220>
<223>  Primer

<400>  71
tttcacacag gaaacagaca tatg                                               24


<210>  72
<211>  53
<212>  DNA
<213>  Artificial Sequence

<220>
<223>  Primer

<400>  72
gaattcctcg acacttgcca tactaccacc acccaatcca tttgctagtt ttg          53


<210>  73
<211>  20
<212>  DNA
<213>  Artificial Sequence

<220>
<223>  Primer

<400>  73
gttgacaatt aatcatcggc                                               20


<210>  74
<211>  20
<212>  DNA
<213>  Artificial Sequence

<220>
<223>  Primer

<400>  74
cgtttcactt ctgagttcgg                                               20


<210>  75
<211>  20
<212>  DNA
<213>  Artificial Sequence

<220>
<223>  Primer

<400>  75
ccgaactcag aagtgaaacg                                               20


<210>  76
<211>  20
<212>  DNA
<213>  Artificial Sequence

<220>
<223>  Primer

<400>  76
gccgatgatt aattgtcaac                                               20


<210>  77
<211>  41
<212>  DNA
<213>  Artificial Sequence

<220>
<223>  Primer

<400>  77
gaattttaac aaaatattaa cgaattctaa caccgtgcgt g                          41


<210>  78
<211>  42
<212>  DNA
<213>  Artificial Sequence

<220>
<223>  Primer

<400>  78
gtgccaccta aattgtaagc ggcgaattgg gtacctataa ac                         42


<210>  79
<211>  20
<212>  DNA
<213>  Artificial Sequence

<220>
<223>  Primer

<400>  79
gcttacaatt taggtggcac                                                  20


<210>  80
<211>  24
<212>  DNA
<213>  Artificial Sequence

<220>
<223>  Primer

<400>  80
gttaatattt tgttaaaatt cgcg                                             24


<210>  81
<211>  31
<212>  DNA
<213>  Artificial Sequence

<220>
<223>  Primer

<400>  81
agacagggta ccatggcgcc acaagaacaa g                                     31


<210>  82
<211>  51
<212>  DNA
<213>  Artificial Sequence

<220>
<223>  Primer

<400>  82
atgtatatct ccttcttaaa gttaattaca atccatttgc tagttttgcc c               51

<210>  83
<211>  46
<212>  DNA
<213>  Artificial Sequence

<220>
<223>  Primer

<400>  83
ttaactttaa gaaggagata tacatatgtc ttcatccaac gcctgc                    46


<210>  84
<211>  37
<212>  DNA
<213>  Artificial Sequence

<220>
<223>  Primer

<400>  84
agacagggat cctcagttct ctttggctag tttttcc                              37


<210>  85
<211>  31
<212>  DNA
<213>  Artificial Sequence

<220>
<223>  Primer

<400>  85
agagaggatc cataacaatt ccccatctta g                                    31


<210>  86
<211>  36
<212>  DNA
<213>  Artificial Sequence

<220>
<223>  Primer

<400>  86
agagatctag attaggtagc cacactatgc agaacc                               36


<210>  87
<211>  43
<212>  DNA
<213>  Artificial Sequence

<220>
<223>  Primer

<400>  87
gaaactgctt gttcttgtgg cgccatggtc tgtttcctgt gtg                       43


<210>  88
<211>  35

<212>  DNA
<213>  Artificial Sequence

<220>
<223>  Primer

<400>  88
ctaatctaga gtcgacctgc aggcatgcaa gcttg                                35


<210>  89
<211>  41
<212>  DNA
<213>  Artificial Sequence

<220>
<223>  Primer

<400>  89
cactagatct caaatgtgct ggaattctaa caccgtgcgt g                         41


<210>  90
<211>  43
<212>  DNA
<213>  Artificial Sequence

<220>
<223>  Primer

<400>  90
ccttataaat caaacatgtg cggcgaattg ggtacctata aac                       43


<210>  91
<211>  22
<212>  DNA
<213>  Artificial Sequence

<220>
<223>  Primer

<400>  91
gcacatgttt gatttataag gg                                              22


<210>  92
<211>  22
<212>  DNA
<213>  Artificial Sequence

<220>
<223>  Primer

<400>  92
cagcacattt gagatctagt gg                                              22


<210>  93
<211>  372
<212>  PRT
<213>  Streptomyces griseus

<400> 93

Met Ala Thr Leu Cys Arg Pro Ala Ile Ala Val Pro Glu His Val Ile
1               5                   10                  15

Thr Met Gln Gln Thr Leu Asp Leu Ala Arg Glu Thr His Ala Gly His
            20                  25                  30

Pro Gln Arg Asp Leu Val Leu Arg Leu Ile Gln Asn Thr Gly Val Gln
        35                  40                  45

Thr Arg His Leu Val Gln Pro Ile Glu Lys Thr Leu Ala His Pro Gly
    50                  55                  60

Phe Glu Val Arg Asn Gln Val Tyr Glu Ala Glu Ala Lys Thr Arg Val
65                  70                  75                  80

Pro Glu Val Val Arg Arg Ala Leu Ala Asn Ala Glu Thr Glu Pro Ser
                85                  90                  95

Glu Ile Asp Leu Ile Val Tyr Val Ser Cys Thr Gly Phe Met Met Pro
            100                 105                 110

Ser Leu Thr Ala Trp Ile Ile Asn Ser Met Gly Phe Arg Pro Glu Thr
        115                 120                 125

Arg Gln Leu Pro Ile Ala Gln Leu Gly Cys Ala Ala Gly Gly Ala Ala
    130                 135                 140

Ile Asn Arg Ala His Asp Phe Cys Val Ala Tyr Pro Asp Ser Asn Val
145                 150                 155                 160

Leu Ile Val Ser Cys Glu Phe Cys Ser Leu Cys Tyr Gln Pro Thr Asp
                165                 170                 175

Ile Gly Val Gly Ser Leu Leu Ser Asn Gly Leu Phe Gly Asp Ala Leu
            180                 185                 190

Ser Ala Ala Val Val Arg Gly Gln Gly Gly Thr Gly Met Arg Leu Glu
        195                 200                 205

Arg Asn Gly Ser His Leu Val Pro Asp Thr Glu Asp Trp Ile Ser Tyr
    210                 215                 220

Ala Val Arg Asp Thr Gly Phe His Phe Gln Leu Asp Lys Arg Val Pro
225                 230                 235                 240

Gly Thr Met Glu Met Leu Ala Pro Val Leu Leu Asp Leu Val Asp Leu

245                 250                 255

His Gly Trp Ser Val Pro Asn Met Asp Phe Phe Ile Val His Ala Gly
            260                 265                 270

Gly Pro Arg Ile Leu Asp Asp Leu Cys His Phe Leu Asp Leu Pro Pro
        275                 280                 285

Glu Met Phe Arg Tyr Ser Arg Ala Thr Leu Thr Glu Arg Gly Asn Ile
    290                 295                 300

Ala Ser Ser Val Val Phe Asp Ala Leu Ala Arg Leu Phe Asp Asp Gly
305                 310                 315                 320

Gly Ala Ala Glu Ser Ala Gln Gly Leu Ile Ala Gly Phe Gly Pro Gly
                325                 330                 335

Ile Thr Ala Glu Val Ala Val Gly Ser Trp Ala Lys Glu Gly Leu Gly
            340                 345                 350

Ala Asp Val Gly Arg Asp Leu Asp Glu Leu Glu Leu Thr Ala Gly Val
        355                 360                 365

Ala Leu Ser Gly
    370

<210>  94
<211>  374
<212>  PRT
<213>  Streptomyces coelicolor

<400>  94

Met Ala Thr Leu Cys Arg Pro Ser Val Ser Val Pro Glu His Val Ile
1               5                   10                  15

Thr Met Glu Glu Thr Leu Glu Leu Ala Arg Arg Arg His Thr Asp His
            20                  25                  30

Pro Gln Leu Pro Leu Ala Leu Arg Leu Ile Glu Asn Thr Gly Val Arg
        35                  40                  45

Thr Arg His Ile Val Gln Pro Ile Glu Asp Thr Leu Glu His Pro Gly
    50                  55                  60

Phe Glu Asp Arg Asn Lys Val Tyr Glu Arg Glu Ala Lys Ser Arg Val
65                  70                  75                  80

Pro Ala Val Ile Gln Arg Ala Leu Asp Asp Ala Glu Leu Leu Ala Thr

                85                  90                  95

Asp Ile Asp Val Ile Ile Tyr Val Ser Cys Thr Gly Phe Met Met Pro
            100                 105                 110

Ser Leu Thr Ala Trp Leu Ile Asn Glu Met Gly Phe Asp Ser Thr Thr
        115                 120                 125

Arg Gln Ile Pro Ile Ala Gln Leu Gly Cys Ala Ala Gly Gly Ala Ala
    130                 135                 140

Ile Asn Arg Ala His Asp Phe Cys Thr Ala Tyr Pro Glu Ala Asn Ala
145                 150                 155                 160

Leu Ile Val Ala Cys Glu Phe Cys Ser Leu Cys Tyr Gln Pro Thr Asp
                165                 170                 175

Leu Gly Val Gly Ser Leu Leu Cys Asn Gly Leu Phe Gly Asp Gly Ile
            180                 185                 190

Ala Ala Ala Val Val Arg Gly Arg Gly Gly Thr Gly Val Arg Leu Glu
        195                 200                 205

Arg Asn Gly Ser Tyr Leu Ile Pro Lys Thr Glu Asp Trp Ile Met Tyr
    210                 215                 220

Asp Val Lys Ala Thr Gly Phe His Phe Leu Leu Asp Lys Arg Val Pro
225                 230                 235                 240

Ala Thr Met Glu Pro Leu Ala Pro Ala Leu Lys Glu Leu Ala Gly Glu
                245                 250                 255

His Gly Trp Asp Ala Ser Asp Leu Asp Phe Tyr Ile Val His Ala Gly
            260                 265                 270

Gly Pro Arg Ile Leu Asp Asp Leu Ser Thr Phe Leu Glu Val Asp Pro
        275                 280                 285

His Ala Phe Arg Phe Ser Arg Ala Thr Leu Thr Glu Tyr Gly Asn Ile
    290                 295                 300

Ala Ser Ala Val Val Leu Asp Ala Leu Arg Arg Leu Phe Asp Glu Gly
305                 310                 315                 320

Gly Val Glu Glu Gly Ala Arg Gly Leu Leu Ala Gly Phe Gly Pro Gly
                325                 330                 335

Ile Thr Ala Glu Met Ser Leu Gly Cys Trp Gln Thr Ala Asp Val Arg
            340                 345                 350

Arg Gly Ile Arg Gln Asp Val Thr Arg Thr Ala Ala Arg Gly Val Ser
        355                 360                 365

Arg Arg Val Arg Gln Ala
    370

<210>  95
<211>  352
<212>  PRT
<213>  Streptomyces avermitilis

<400>  95

Met Ala Thr Leu Cys Lys Pro Ala Val Ser Val Pro Glu His Val Ile
1               5                   10                  15

Thr Met Glu Glu Thr Leu Glu Leu Ala Arg Ser Arg His Pro Asp His
            20                  25                  30

Pro Gln Leu Pro Leu Ala Leu Arg Leu Ile Glu Asn Thr Gly Val His
        35                  40                  45

Thr Arg His Ile Val Gln Pro Ile Glu Glu Thr Leu Lys His Pro Gly
    50                  55                  60

Phe Glu Glu Arg Asn His Val Tyr Glu Ala Glu Ala Lys Ala Arg Val
65                  70                  75                  80

Pro Ala Val Val Gln Arg Ala Leu Asp Glu Ala Glu Leu Leu Thr Thr
                85                  90                  95

Asp Ile Asp Val Ile Ile Tyr Val Ser Cys Thr Gly Phe Met Met Pro
            100                 105                 110

Ser Leu Thr Ala Tyr Leu Ile Asn Ser Met Asp Phe Ser Ser Asp Thr
        115                 120                 125

Arg Gln Ile Pro Ile Ala Gln Leu Gly Cys Ala Ala Gly Gly Ser Ala
    130                 135                 140

Ile Asn Arg Ala His Asp Phe Cys Thr Ala Tyr Pro Gln Ala Asn Ala
145                 150                 155                 160

Leu Ile Val Ala Cys Glu Phe Cys Ser Leu Cys Tyr Gln Pro Thr Asp
                165                 170                 175

Leu Gly Val Gly Ser Leu Leu Ser Asn Gly Leu Phe Gly Asp Gly Ile
        180                 185                 190

Ala Ala Ala Ala Val Arg Gly Lys Gly Gly Thr Gly Ile Thr Leu Glu
    195                 200                 205

Arg Asn Ala Ser Tyr Leu Ile Pro Lys Thr Asp Glu Trp Ile Ser Tyr
    210                 215                 220

Asp Val Arg Ala Thr Gly Phe His Phe Leu Leu Asp Lys Arg Val Pro
225                 230                 235                 240

Gly Thr Met Glu Pro Leu Ala Pro Ala Leu Gln Glu Leu Ala Ser Gln
                245                 250                 255

His Gly Trp Asp Ala Ser Asp Leu Asp Phe Tyr Ile Ile His Ala Gly
            260                 265                 270

Gly Pro Arg Ile Leu Asp Asp Leu Ser Lys Phe Leu Arg Val Pro Pro
        275                 280                 285

Glu Ala Phe Arg Phe Ser Arg Ala Thr Leu Thr Glu Tyr Gly Asn Ile
    290                 295                 300

Ala Ser Ala Val Val Leu Asp Ala Leu Arg Arg Leu Phe Asp Glu Gly
305                 310                 315                 320

Gly Ala Glu His Ala Ala Arg Gly Met Leu Ala Gly Phe Gly Pro Gly
                325                 330                 335

Ile Thr Ala Glu Met Ser Leu Gly Arg Trp His Arg Thr Asp Glu Ala
            340                 345                 350

<210>  96
<211>  367
<212>  PRT
<213>  Saccharopolyspora erythraea

<400>  96

Met Ala Val Leu Cys Thr Pro Ala Val Ala Val Pro Glu His Val Ile
1               5                   10                  15

Thr Val Glu Glu Thr Leu Asp Leu Ala Arg Arg Val His Ala Asp His
            20                  25                  30

Pro Gln Leu Pro Leu Val Leu Arg Leu Ile Ser Asn Thr Gly Val Arg
        35                  40                  45

Glu Arg His Leu Ile Arg Pro Ile Glu Asp Thr Leu Glu His Pro Gly
    50                  55                  60

Phe Glu Val Arg Asn Arg Ile Tyr Glu Glu Gln Ala Lys Gln Arg Val
65                  70                  75                  80

Pro Ala Val Val Arg Glu Ala Leu Asp Ser Ala Glu Leu Gly Pro Glu
                85                  90                  95

Asp Ile Asp Leu Ile Val Tyr Val Ser Cys Thr Gly Phe Met Met Pro
            100                 105                 110

Ser Leu Thr Ala Trp Leu Ile Asn Ser Met Gly Phe Arg Met Ser Thr
        115                 120                 125

Arg Gln Leu Pro Ile Ala Gln Leu Gly Cys Ala Ala Gly Gly Ala Ala
    130                 135                 140

Ile Asn Arg Ala His Asp Phe Cys Thr Ala Tyr Pro Asp Ala Asn Ala
145                 150                 155                 160

Leu Ile Val Ser Cys Glu Phe Cys Ser Leu Cys Tyr Gln Pro Thr Asp
                165                 170                 175

Asp Asp Ile Gly Ser Leu Leu Ser Asn Gly Leu Phe Gly Asp Ala Val
            180                 185                 190

Gly Ala Ala Val Val Arg Gly His Gly Gly Thr Gly Val Arg Leu Glu
        195                 200                 205

Arg Asn Ala Ser Ser Met Ile Pro Glu Thr Glu Asp Trp Ile Ser Tyr
    210                 215                 220

Ala Val Lys Ala Thr Gly Phe His Phe Gln Leu Asp Lys Arg Val Pro
225                 230                 235                 240

Lys Thr Met Glu Pro Leu Ala Pro Ala Leu Arg Ala Leu Ala Glu Asp
                245                 250                 255

His Arg Trp Asp Val Ala Gly Leu Asp Phe Tyr Val Ile His Ala Gly
            260                 265                 270

Gly Pro Arg Ile Leu Asp Asp Leu Thr Lys Phe Leu Gly Val Pro Ser
        275                 280                 285

Glu Ala Phe Arg His Ser Arg Ala Thr Leu Ala Gln Tyr Gly Asn Ile
    290                 295                 300

Ala Ser Ala Val Val Leu Asp Ala Leu Arg Arg Ile Ile Glu Glu Gly
305                 310                 315                 320

Arg Leu Glu Ser Gly Ala Arg Gly Met Ile Ala Gly Phe Gly Pro Gly
                325                 330                 335

Ile Thr Ala Glu Met Ser Val Gly Thr Trp Val Pro His Asp Val Leu
            340                 345                 350

Leu His Gly Glu His Ser Thr Thr Ser Ala Pro Gly Gly Asn Arg
        355                 360                 365

<210>  97
<211>  377
<212>  PRT
<213>  Streptomyces peucetius

<400>  97

Met Arg Val Pro Val Ala Val Asp Asp Leu Val Ala Pro Ser Thr Met
1               5                   10                  15

Gly Glu Arg His Thr Val Ile Asp Arg Gly Thr Ser Val Ala Ala Val
            20                  25                  30

His Thr Ala Leu Pro Pro His Arg Tyr Ala Gln Ser Asp Leu Thr Glu
        35                  40                  45

Leu Ile Ala Asp Leu Cys Leu Glu Pro Gly Ala Asp Arg Ala Leu Leu
    50                  55                  60

Arg Arg Leu His Thr Ser Ala Gly Val Arg Thr Arg His Leu Ala Leu
65                  70                  75                  80

Pro Ile Glu Gln Tyr Ala Gly Leu Gly Asp Phe Gly Gln Ala Asn Ala
                85                  90                  95

Ala Trp Leu Thr Val Gly Leu Ala Leu Ala Glu Glu Ala Leu Ser Gly
            100                 105                 110

Ala Leu Asp Ala Ala Gly Leu Thr Ala Ala Asp Ile Asp Leu Leu Val
        115                 120                 125

Cys Thr Ser Ile Thr Gly Val Ala Ala Pro Ser Leu Asp Ala Arg Leu
    130                 135                 140

Ala Val Arg Met Gly Met Arg Ala Asp Val Lys Arg Val Pro Val Phe
145                 150                 155                 160

Gly Leu Gly Cys Val Gly Gly Ala Ala Gly Leu Gly Arg Leu His Asp
                165                 170                 175

Tyr Leu Leu Gly His Pro Asp Asp Thr Ala Val Leu Leu Ser Val Glu
            180                 185                 190

Leu Cys Ser Leu Thr Leu Gln Arg Asp Gly Ser Leu Ala Asn Leu Val
        195                 200                 205

Ala Gly Ala Leu Phe Gly Asp Gly Ala Ala Ala Val Val Ala Arg Gly
    210                 215                 220

Gly Asp Ala Gly Arg Arg Gly Ala Gly Trp Pro Met Val Ala Ala Thr
225                 230                 235                 240

Arg Gly His Leu Tyr Pro Asp Thr Glu His Leu Leu Gly Trp Arg Ile
                245                 250                 255

Gly Ala Ser Gly Phe Arg Val Val Val Asp Ala Gly Ile Pro Asp Val
            260                 265                 270

Val Arg Thr His Leu Gly Gly Asp Leu Arg Asn Phe Leu Ala Thr His
        275                 280                 285

Gly Leu Val Pro Asp Asp Ile Gly Thr Trp Ile Cys His Pro Gly Gly
    290                 295                 300

Pro Lys Val Leu Ala Ala Val Gly Asp Ala Leu Glu Leu Pro Asp Gly
305                 310                 315                 320

Ala Leu Asp Ser Ser Trp Arg Ser Leu Ala Gly Val Gly Asn Leu Ser
                325                 330                 335

Ser Ala Ser Val Leu Arg Val Leu Glu Asp Val Ala Thr Arg Cys Arg
            340                 345                 350

Pro Asp Pro Gly Thr Trp Gly Val Leu Leu Ala Met Gly Pro Gly Phe
        355                 360                 365

Cys Ala Glu Phe Val Leu Leu Arg Trp
    370                 375

<210>  98
<211>  355
<212>  PRT
<213>  Streptomyces aculeolatus

<400>  98

Met Pro Arg Leu Cys Lys Pro Ala Val Ser Ala Pro Glu Tyr Thr Ile
1               5                   10                  15

Thr Met Glu Glu Thr Leu Glu Phe Ala Lys Gln Ala His Ala Gly Lys
            20                  25                  30

Pro Gln Leu Pro Leu Ala Leu Arg Leu Ile Arg Asn Thr Gly Val Leu
        35                  40                  45

Lys Arg His Ile Val Gln Pro Ile Glu Lys Thr Leu Gly His Pro Gly
    50                  55                  60

Leu Thr Glu Arg Asn Leu Ile Tyr Glu Ala Glu Ser Lys Lys Met Cys
65                  70                  75                  80

Pro Pro Val Ile Glu Glu Ala Leu Gln Asn Ala Asp Met Thr Ala Arg
                85                  90                  95

Asp Ile Asp Ala Ile Ile Tyr Val Ser Cys Thr Gly Phe Leu Met Pro
            100                 105                 110

Ser Leu Thr Ala Trp Leu Ile Asn Lys Met Gly Phe Arg Ser Asp Thr
        115                 120                 125

Arg Gln Ile Pro Ile Ala Gln Leu Gly Cys Ala Ala Gly Gly Ala Ala
    130                 135                 140

Val Asn Arg Ala His Asp Phe Cys Leu Ala His Pro Gly Ser Asn Val
145                 150                 155                 160

Leu Ile Val Ala Cys Glu Leu Cys Ser Leu Cys Tyr Gln Pro Thr Ala
                165                 170                 175

Asp Asp Ile Gly Ser Leu Leu Ser Asp Gly Leu Phe Gly Asp Ala Val
            180                 185                 190

Ala Ala Ala Val Val Arg Gly Asn Gly Gly Val Gly Ile Glu Val Glu
        195                 200                 205

Arg Asn Ala Ser Tyr Leu Ile Pro Asn Thr Glu Glu Trp Ile Ser Tyr
    210                 215                 220

Ser Val Arg Asp Thr Gly Phe His Phe Gln Leu Asp Arg Arg Val Pro
225                 230                 235                 240

Gly Thr Met Glu Pro Leu Ala Pro Val Leu Arg Glu Phe Ala Lys Asp
                245                 250                 255

His Ser Trp Asp Ala Gly Lys Leu Asp Phe Tyr Ile Val His Ala Gly
            260                 265                 270

Gly Pro Arg Ile Leu Asp Asp Leu Ala Arg Phe Leu Asp Val Asp Arg
        275                 280                 285

Gln Val Phe Arg His Ser Trp Ser Thr Leu Thr Glu Tyr Gly Asn Ile
    290                 295                 300

Ala Ser Ala Val Val Phe Asp Ala Ala Arg Arg Leu Phe Glu Glu Gly
305                 310                 315                 320

Ser Ala Lys Pro Asp Ala Thr Gly Met Ile Ala Gly Phe Gly Pro Gly
                325                 330                 335

Ile Thr Ala Glu Met Ala Leu Gly Thr Trp Gly Thr Asp Gly Thr Gly
            340                 345                 350

Thr Ser Asn
        355

<210> 99
<211> 349
<212> PRT
<213> Pseudomonas fluorescens

<400> 99

Met Ser Thr Leu Cys Lys Pro Ser Leu Leu Phe Pro His Tyr Lys Ile
1               5                   10                  15

Thr Gln Gln Gln Met Ile Asp His Leu Glu Gln Leu His Asp Asp His
            20                  25                  30

Pro Arg Met Ala Leu Ala Lys Arg Met Ile Gln Asn Thr Gln Val Asn
        35                  40                  45

Glu Arg Tyr Leu Val Leu Pro Ile Asp Glu Leu Ala Val His Thr Gly
    50                  55                  60

Phe Thr His Arg Ser Ile Val Tyr Glu Arg Glu Ala Arg Arg Met Ser
65                  70                  75                  80

Ser Ile Ala Ala Arg Gln Ala Ile Glu Asn Ala Gly Leu Thr Thr Asp
                85                  90                  95

Asp Ile Arg Met Val Ala Val Thr Ser Cys Thr Gly Phe Met Met Pro
            100                 105                 110

Ser Leu Thr Ala His Leu Ile Asn Asp Leu Gly Leu Arg Thr Ser Thr
        115                 120                 125

Val Gln Leu Pro Ile Ala Gln Leu Gly Cys Val Ala Gly Ala Ala Ala
    130                 135                 140

Ile Asn Arg Ala Asn Asp Phe Ala Ser Leu Ser Pro Asp Asn His Ala
145                 150                 155                 160

Leu Ile Val Ser Leu Glu Phe Ser Ser Leu Cys Tyr Gln Pro Gln Asp
                165                 170                 175

Thr Lys Leu His Ala Phe Ile Ser Ala Ala Leu Phe Gly Asp Ala Val
            180                 185                 190

Ser Ala Cys Val Met Arg Ala Asp Asp Lys Ala Pro Gly Phe Lys Ile
        195                 200                 205

Ala Lys Thr Gly Ser Tyr Phe Leu Pro Asp Ser Glu His Tyr Ile Lys
    210                 215                 220

Tyr Asp Val Lys Asp Ser Gly Phe His Phe Thr Leu Asp Lys Ala Val
225                 230                 235                 240

Met Asn Ser Ile Lys Asp Val Ala Pro Met Met Glu Glu Leu Asn Phe
                245                 250                 255

Glu Thr Phe Asn Gln His Cys Ala Gln Asn Asp Phe Phe Ile Phe His
            260                 265                 270

Thr Gly Gly Arg Lys Ile Leu Asp Glu Leu Val Leu Gln Leu Asp Leu
        275                 280                 285

Glu Pro Gly Arg Val Ala Gln Ser Arg Asp Ser Leu Ser Glu Ala Gly
    290                 295                 300

Asn Ile Ala Ser Val Val Val Phe Asp Val Leu Lys Arg Gln Phe Asp
305                 310                 315                 320

Ser Gly Pro Ala Asn Gly Ala Thr Gly Met Leu Ala Ala Phe Gly Pro
                325                 330                 335

Gly Phe Thr Ala Glu Met Ala Val Gly Lys Trp Val Ala
            340                 345

<210>  100

<211>  370
<212>  PRT
<213>  Amycolatopsis orientalis

<400>  100

Met Asp Val Ser Met Thr Thr Gly Ile Glu Leu Thr Glu Glu Leu Ser
1               5                   10                  15

Val Leu Asn Gly Leu Thr Glu Ile Thr Arg Phe Ala Gly Val Gly Thr
            20                  25                  30

Ala Val Ser Glu Thr Ser Tyr Ser Gln Thr Glu Leu Leu Asp Ile Leu
        35                  40                  45

Asp Val Glu Asp Pro Lys Ile Arg Ser Val Phe Leu Asn Ser Ala Ile
    50                  55                  60

Asp Arg Arg Phe Leu Thr Leu Pro Pro Glu Asn Pro Gly Gly Gly Arg
65                  70                  75                  80

Leu Ala Glu Pro Gln Gly Asp Leu Leu Asp Lys His Lys Lys Ile Ala
                85                  90                  95

Val Asp Met Gly Cys Arg Ala Leu Glu Ala Cys Leu Lys Ser Ala Gly
            100                 105                 110

Ala Thr Leu Ser Asp Leu Arg His Leu Cys Cys Val Thr Ser Thr Gly
        115                 120                 125

Phe Leu Thr Pro Gly Leu Ser Ala Leu Ile Ile Arg Glu Met Gly Ile
    130                 135                 140

Asp Pro His Cys Ser Arg Ser Asp Ile Val Gly Met Gly Cys Asn Ala
145                 150                 155                 160

Gly Leu Asn Ala Leu Asn Val Val Ser Gly Trp Ser Ala Ala His Pro
                165                 170                 175

Gly Glu Leu Gly Val Val Leu Cys Ser Glu Ala Cys Ser Ala Ala Tyr
            180                 185                 190

Ala Leu Asp Gly Thr Met Arg Thr Ala Val Val Asn Ser Leu Phe Gly
        195                 200                 205

Asp Gly Ser Ala Ala Leu Ala Val Ile Ser Gly Asp Gly Arg Val Ala
    210                 215                 220

Gly Pro Arg Val Leu Lys Phe Ala Ser Tyr Ile Ile Thr Asp Ala Val

225                 230                 235                 240

Asp Ala Met Arg Tyr Asp Trp Asp Arg Asp Gln Asp Arg Phe Ser Phe
                245                 250                 255

Phe Leu Asp Pro Gln Ile Pro Tyr Val Val Gly Ala His Ala Glu Ile
            260                 265                 270

Val Val Asp Arg Leu Leu Ser Gly Thr Gly Leu Arg Arg Ser Asp Ile
        275                 280                 285

Gly His Trp Leu Val His Ser Gly Gly Lys Lys Val Val Asp Ala Val
    290                 295                 300

Val Val Asn Leu Gly Leu Ser Arg His Asp Val Arg His Thr Thr Gly
305                 310                 315                 320

Val Leu Arg Asp Tyr Gly Asn Leu Ser Ser Gly Ser Phe Leu Phe Ser
                325                 330                 335

Tyr Glu Arg Leu Ser Glu Glu Asp Val Thr Arg Pro Gly Asp Tyr Gly
            340                 345                 350

Val Leu Met Thr Met Gly Pro Gly Ser Thr Ile Glu Met Ala Leu Ile
        355                 360                 365

Gln Trp
    370

<210>  101
<211>  391
<212>  PRT
<213>  Unknown

<220>
<223>  Rheum palmatum

<400>  101

Met Ala Asp Val Leu Gln Glu Ile Arg Asn Ser Gln Lys Ala Ser Gly
1               5                   10                  15

Pro Ala Thr Val Leu Ala Ile Gly Thr Ala His Pro Pro Thr Cys Tyr
            20                  25                  30

Pro Gln Ala Asp Tyr Pro Asp Phe Tyr Phe Arg Val Cys Lys Ser Glu
        35                  40                  45

His Met Thr Lys Leu Lys Lys Lys Met Gln Phe Ile Cys Asp Arg Ser
    50                  55                  60

Gly Ile Arg Gln Arg Phe Met Phe His Thr Glu Glu Asn Leu Gly Lys
65                  70                  75                  80

Asn Pro Gly Met Cys Thr Phe Asp Gly Pro Ser Leu Asn Ala Arg Gln
                85                  90                  95

Asp Met Leu Ile Met Glu Val Pro Lys Leu Gly Ala Glu Ala Ala Glu
            100                 105                 110

Lys Ala Ile Lys Glu Trp Gly Gln Asp Lys Ser Arg Ile Thr His Leu
        115                 120                 125

Ile Phe Cys Thr Thr Thr Ser Asn Asp Met Pro Gly Ala Asp Tyr Gln
    130                 135                 140

Phe Ala Thr Leu Phe Gly Leu Asn Pro Gly Val Ser Arg Thr Met Val
145                 150                 155                 160

Tyr Gln Gln Gly Cys Phe Ala Gly Gly Thr Val Leu Arg Leu Val Lys
                165                 170                 175

Asp Ile Ala Glu Asn Asn Lys Gly Ala Arg Val Leu Val Val Cys Ser
            180                 185                 190

Glu Ile Val Ala Phe Ala Phe Arg Gly Pro His Glu Asp His Ile Asp
        195                 200                 205

Ser Leu Ile Gly Gln Leu Leu Phe Gly Asp Gly Ala Ala Ala Leu Val
    210                 215                 220

Val Gly Thr Asp Ile Asp Glu Ser Val Glu Arg Pro Ile Phe Gln Ile
225                 230                 235                 240

Met Ser Ala Thr Gln Ala Thr Ile Pro Asn Ser Leu His Thr Met Ala
                245                 250                 255

Leu His Leu Thr Glu Ala Gly Leu Thr Phe His Leu Ser Lys Glu Val
            260                 265                 270

Pro Lys Val Val Ser Asp Asn Met Glu Glu Leu Met Leu Glu Ala Phe
        275                 280                 285

Lys Pro Leu Gly Ile Thr Asp Trp Asn Ser Ile Phe Trp Gln Val His
    290                 295                 300

Pro Gly Gly Arg Ala Ile Leu Asp Lys Ile Glu Glu Lys Leu Glu Leu

305                 310                 315                 320

Thr Lys Asp Lys Met Arg Asp Ser Arg Tyr Ile Leu Ser Glu Tyr Gly
                325                 330                 335

Asn Leu Thr Ser Ala Cys Val Leu Phe Val Met Asp Glu Met Arg Lys
            340                 345                 350

Arg Ser Phe Arg Glu Gly Lys Gln Thr Thr Gly Asp Gly Tyr Glu Trp
        355                 360                 365

Gly Val Ala Ile Gly Leu Gly Pro Gly Leu Thr Val Glu Thr Val Val
    370                 375                 380

Leu Arg Ser Val Pro Ile Pro
385                 390

<210>  102
<211>  403
<212>  PRT
<213>  Aloe arborescens

<400>  102

Met Ser Ser Leu Ser Asn Ser Leu Pro Leu Met Glu Asp Val Gln Gly
1               5                   10                  15

Ile Arg Lys Ala Gln Lys Ala Asp Gly Thr Ala Thr Val Met Ala Ile
            20                  25                  30

Gly Thr Ala His Pro Pro His Ile Phe Pro Gln Asp Thr Tyr Ala Asp
        35                  40                  45

Val Tyr Phe Arg Ala Thr Asn Ser Glu His Lys Val Glu Leu Lys Lys
    50                  55                  60

Lys Phe Asp His Ile Cys Lys Lys Thr Met Ile Gly Lys Arg Tyr Phe
65                  70                  75                  80

Asn Tyr Asp Glu Glu Phe Leu Lys Lys Tyr Pro Asn Ile Thr Ser Tyr
                85                  90                  95

Asp Glu Pro Ser Leu Asn Asp Arg Gln Asp Ile Cys Val Pro Gly Val
            100                 105                 110

Pro Ala Leu Gly Thr Glu Ala Ala Val Lys Ala Ile Glu Glu Trp Gly
        115                 120                 125

Arg Pro Lys Ser Glu Ile Thr His Leu Val Phe Cys Thr Ser Cys Gly

130                 135                 140

Val Asp Met Pro Ser Ala Asp Phe Gln Cys Ala Lys Leu Leu Gly Leu
145                 150                 155                 160

His Ala Asn Val Asn Lys Tyr Cys Ile Tyr Met Gln Gly Cys Tyr Ala
                165                 170                 175

Gly Gly Thr Val Met Arg Tyr Ala Lys Asp Leu Ala Glu Asn Asn Arg
            180                 185                 190

Gly Ala Arg Val Leu Val Val Cys Ala Glu Leu Thr Ile Met Met Leu
        195                 200                 205

Arg Ala Pro Asn Glu Thr His Leu Asp Asn Ala Ile Gly Ile Ser Leu
    210                 215                 220

Phe Gly Asp Gly Ala Ala Ala Leu Ile Ile Gly Ser Asp Pro Ile Ile
225                 230                 235                 240

Gly Val Glu Lys Pro Met Phe Glu Ile Val Cys Thr Lys Gln Thr Val
                245                 250                 255

Ile Pro Asn Thr Glu Asp Val Ile His Leu His Leu Arg Glu Thr Gly
            260                 265                 270

Met Met Phe Tyr Leu Ser Lys Gly Ser Pro Met Thr Ile Ser Asn Asn
        275                 280                 285

Val Glu Ala Cys Leu Ile Asp Val Phe Lys Ser Val Gly Ile Thr Pro
    290                 295                 300

Pro Glu Asp Trp Asn Ser Leu Phe Trp Ile Pro His Pro Gly Gly Arg
305                 310                 315                 320

Ala Ile Leu Asp Gln Val Glu Ala Lys Leu Lys Leu Arg Pro Glu Lys
                325                 330                 335

Phe Arg Ala Ala Arg Thr Val Leu Trp Asp Tyr Gly Asn Met Val Ser
            340                 345                 350

Ala Ser Val Gly Tyr Ile Leu Asp Glu Met Arg Arg Lys Ser Ala Ala
        355                 360                 365

Lys Gly Leu Glu Thr Tyr Gly Glu Gly Leu Glu Trp Gly Val Leu Leu
    370                 375                 380

Gly Phe Gly Pro Gly Ile Thr Val Glu Thr Ile Leu Leu His Ser Leu
385                 390                 395                 400

Pro Leu Met

<210>  103
<211>  403
<212>  PRT
<213>  Aloe arborescens

<400>  103

Met Ser Ser Leu Ser Asn Ala Ser His Leu Met Glu Asp Val Gln Gly
1               5                   10                  15

Ile Arg Lys Ala Gln Arg Ala Asp Gly Thr Ala Thr Val Met Ala Ile
            20                  25                  30

Gly Thr Ala His Pro Pro His Ile Phe Pro Gln Asp Thr Tyr Ala Asp
        35                  40                  45

Phe Tyr Phe Arg Ala Thr Asn Ser Glu His Lys Val Glu Leu Lys Lys
    50                  55                  60

Lys Phe Asp Arg Ile Cys Lys Lys Thr Met Ile Gly Lys Arg Tyr Phe
65                  70                  75                  80

Asn Tyr Asp Glu Glu Phe Leu Lys Lys Tyr Pro Asn Ile Thr Ser Phe
                85                  90                  95

Asp Glu Pro Ser Leu Asn Asp Arg Gln Asp Ile Cys Val Pro Gly Val
            100                 105                 110

Pro Ala Leu Gly Ala Glu Ala Ala Val Lys Ala Ile Ala Glu Trp Gly
        115                 120                 125

Arg Pro Lys Ser Glu Ile Thr His Leu Val Phe Cys Thr Ser Cys Gly
    130                 135                 140

Val Asp Met Pro Ser Ala Asp Phe Gln Cys Ala Lys Leu Leu Gly Leu
145                 150                 155                 160

Arg Thr Asn Val Asn Lys Tyr Cys Val Tyr Met Gln Gly Cys Tyr Ala
                165                 170                 175

Gly Gly Thr Val Met Arg Tyr Ala Lys Asp Leu Ala Glu Asn Asn Arg
            180                 185                 190

Gly Ala Arg Val Leu Val Val Cys Ala Glu Leu Thr Ile Ile Gly Leu
        195                 200                 205

Arg Gly Pro Asn Glu Ser His Leu Asp Asn Ala Ile Gly Asn Ser Leu
    210                 215                 220

Phe Gly Asp Gly Ala Ala Ala Leu Ile Val Gly Ser Asp Pro Ile Ile
225                 230                 235                 240

Gly Val Glu Lys Pro Met Phe Glu Ile Val Cys Ala Lys Gln Thr Val
                245                 250                 255

Ile Pro Asn Ser Glu Asp Val Ile His Leu His Met Arg Glu Ala Gly
            260                 265                 270

Leu Met Phe Tyr Met Ser Lys Asp Ser Pro Glu Thr Ile Ser Asn Asn
        275                 280                 285

Val Glu Ala Cys Leu Val Asp Val Phe Lys Ser Val Gly Met Thr Pro
    290                 295                 300

Pro Glu Asp Trp Asn Ser Leu Phe Trp Ile Pro His Pro Gly Gly Arg
305                 310                 315                 320

Ala Ile Leu Asp Gln Val Glu Ala Lys Leu Lys Leu Arg Pro Glu Lys
                325                 330                 335

Phe Arg Ala Thr Arg Thr Val Leu Trp Asp Cys Gly Asn Met Val Ser
            340                 345                 350

Ala Cys Val Leu Tyr Ile Leu Asp Glu Met Arg Arg Lys Ser Ala Asp
        355                 360                 365

Glu Gly Leu Glu Thr Tyr Gly Glu Gly Leu Glu Trp Gly Val Leu Leu
    370                 375                 380

Gly Phe Gly Pro Gly Met Thr Val Glu Thr Ile Leu Leu His Ser Leu
385                 390                 395                 400

Pro Leu Met

<210>  104
<211>  403
<212>  PRT
<213>  Aloe arborescens

<400>  104

Met Gly Ser Leu Ser Asp Ser Thr Pro Leu Met Lys Asp Val Gln Gly
1               5                   10                  15

Ile Arg Lys Ala Gln Lys Ala Asp Gly Thr Ala Thr Val Met Ala Ile
            20                  25                  30

Gly Thr Ala His Pro Pro His Ile Ile Ser Gln Asp Ser Tyr Ala Asp
        35                  40                  45

Phe Tyr Phe Arg Val Thr Asn Ser Glu His Lys Val Glu Leu Lys Lys
    50                  55                  60

Lys Phe Asp Arg Ile Cys Lys Lys Thr Met Ile Gly Lys Arg Tyr Phe
65                  70                  75                  80

Asn Phe Asp Glu Glu Phe Leu Lys Lys Tyr Pro Asn Ile Thr Ser Phe
                85                  90                  95

Asp Lys Pro Ser Leu Asn Asp Arg His Asp Ile Cys Ile Pro Gly Val
            100                 105                 110

Pro Ala Leu Gly Ala Glu Ala Ala Val Lys Ala Ile Glu Glu Trp Gly
        115                 120                 125

Arg Pro Lys Ser Glu Ile Thr His Leu Val Phe Cys Thr Ser Gly Gly
    130                 135                 140

Val Asp Met Pro Ser Ala Asp Phe Gln Cys Ala Lys Leu Leu Gly Leu
145                 150                 155                 160

Arg Thr Asn Val Asn Lys Tyr Cys Ile Tyr Met Gln Gly Cys Tyr Ala
                165                 170                 175

Gly Gly Thr Val Met Arg Tyr Ala Lys Asp Leu Ala Glu Asn Asn Arg
            180                 185                 190

Gly Ala Arg Val Leu Met Val Cys Ala Glu Leu Thr Ile Ile Ala Leu
        195                 200                 205

Arg Gly Pro Asn Asp Ser His Ile Asp Asn Ala Ile Gly Asn Ser Leu
    210                 215                 220

Phe Gly Asp Gly Ala Ala Ala Leu Ile Val Gly Ser Asp Pro Ile Ile
225                 230                 235                 240

Gly Val Glu Lys Pro Met Phe Glu Ile Val Cys Ala Lys Gln Thr Val
                245                 250                 255

Ile Pro Asn Ser Glu Glu Val Ile His Leu His Leu Arg Glu Ser Gly
        260                 265                 270

Leu Met Phe Tyr Met Thr Lys Asp Ser Ala Ala Thr Ile Ser Asn Asn
        275                 280                 285

Ile Glu Ala Cys Leu Val Asp Val Phe Lys Ser Val Gly Met Thr Pro
    290                 295                 300

Pro Glu Asp Trp Asn Ser Leu Phe Trp Ile Pro His Pro Gly Gly Arg
305                 310                 315                 320

Ala Ile Leu Asp Gln Val Glu Ala Lys Leu Lys Leu Arg Pro Glu Lys
                325                 330                 335

Phe Ser Ala Thr Arg Thr Val Leu Trp Asp Tyr Gly Asn Met Ile Ser
            340                 345                 350

Ala Cys Val Leu Tyr Ile Leu Asp Glu Met Arg Arg Lys Ser Ala Ala
        355                 360                 365

Glu Gly Leu Glu Thr Tyr Gly Glu Gly Leu Glu Trp Gly Val Leu Leu
    370                 375                 380

Gly Phe Gly Pro Gly Met Thr Ile Glu Thr Ile Leu Leu His Ser Leu
385                 390                 395                 400

Pro Pro Val

<210>  105
<211>  403
<212>  PRT
<213>  Aloe arborescens

<400>  105

Met Gly Ser Leu Ser Asn Tyr Ser Pro Val Met Glu Asp Val Gln Ala
1               5                   10                  15

Ile Arg Lys Ala Gln Lys Ala Asp Gly Thr Ala Thr Val Met Ala Ile
            20                  25                  30

Gly Thr Ala His Pro Pro His Ile Phe Pro Gln Asp Thr Tyr Ala Asp
        35                  40                  45

Phe Tyr Phe Arg Ala Thr Asn Ser Glu His Lys Val Glu Leu Lys Lys
    50                  55                  60

Lys Phe Asp Arg Ile Cys Lys Lys Thr Met Ile Gly Lys Arg Tyr Phe
65                  70                  75                  80

Asn Tyr Asp Glu Glu Phe Leu Lys Lys Tyr Pro Asn Ile Thr Ser Phe
                85                  90                  95

Asp Glu Pro Ser Leu Asn Asp Arg Gln Asp Ile Cys Val Pro Gly Val
            100                 105                 110

Pro Ala Leu Gly Ala Glu Ala Ala Val Lys Ala Ile Ala Glu Trp Gly
        115                 120                 125

Arg Pro Lys Ser Glu Ile Thr His Leu Val Phe Cys Thr Ser Cys Gly
    130                 135                 140

Val Asp Met Pro Ser Ala Asp Phe Gln Cys Ala Lys Leu Leu Gly Leu
145                 150                 155                 160

Arg Thr Asn Val Asn Lys Tyr Cys Val Tyr Met Gln Gly Cys Tyr Ala
                165                 170                 175

Gly Gly Thr Val Met Arg Tyr Ala Lys Asp Leu Ala Glu Asn Asn Arg
            180                 185                 190

Gly Ala Arg Val Leu Val Val Cys Ala Glu Leu Thr Ile Ile Gly Leu
        195                 200                 205

Arg Gly Pro Asn Glu Ser His Leu Asp Asn Ala Ile Gly Asn Ser Leu
    210                 215                 220

Phe Gly Asp Gly Ala Ala Ala Leu Ile Val Gly Ser Asp Pro Ile Ile
225                 230                 235                 240

Gly Val Glu Arg Pro Met Phe Glu Ile Val Cys Ala Lys Gln Thr Val
                245                 250                 255

Ile Pro Asn Ser Glu Asp Val Ile His Leu His Met Arg Glu Ala Gly
            260                 265                 270

Leu Met Phe Tyr Met Ser Lys Asp Ser Pro Glu Thr Ile Ser Asn Asn
        275                 280                 285

Val Glu Ala Cys Leu Val Asp Val Phe Lys Ser Val Gly Met Thr Pro
    290                 295                 300

Pro Glu Asp Trp Asn Ser Leu Phe Trp Ile Pro His Pro Gly Gly Arg
305                 310                 315                 320

Ala Ile Leu Asp Gln Val Glu Ala Arg Leu Lys Leu Arg Pro Glu Lys
                325                 330                 335

Phe Gly Ala Thr Arg Thr Val Leu Trp Asp Cys Gly Asn Met Val Ser
            340                 345                 350

Ala Cys Val Leu Tyr Ile Leu Asp Glu Met Arg Arg Lys Ser Val Ala
        355                 360                 365

Asp Gly Leu Ala Thr Tyr Gly Glu Gly Leu Glu Trp Gly Val Leu Leu
    370                 375                 380

Gly Phe Gly Pro Gly Met Thr Val Glu Thr Ile Leu Leu His Ser Leu
385                 390                 395                 400

Pro Pro Val

<210>  106
<211>  405
<212>  PRT
<213>  Aloe arborescens

<400>  106

Met Gly Ser Ile Ala Glu Ser Ser Pro Leu Met Ser Arg Glu Asn Val
1               5                   10                  15

Glu Gly Ile Arg Lys Ala Gln Arg Ala Glu Gly Thr Ala Thr Val Met
            20                  25                  30

Ala Ile Gly Thr Ala His Pro Pro His Ile Phe Pro Gln Asp Thr Tyr
        35                  40                  45

Ala Asp Phe Tyr Phe Arg Ala Thr Asn Ser Glu His Lys Val Glu Leu
    50                  55                  60

Lys Lys Lys Phe Asp Arg Ile Cys Lys Lys Thr Met Ile Gly Lys Arg
65                  70                  75                  80

Tyr Phe Asn Tyr Asp Glu Glu Phe Leu Lys Lys Tyr Pro Asn Ile Thr
                85                  90                  95

Ser Phe Asp Glu Pro Ser Leu Asn Asp Arg Gln Asp Ile Cys Val Pro
            100                 105                 110

Gly Val Pro Ala Leu Gly Lys Glu Ala Ala Leu Lys Ala Ile Glu Glu
        115                 120                 125

Trp Gly Gln Pro Leu Ser Lys Ile Thr His Leu Val Phe Cys Thr Ser
    130                 135                 140

Cys Gly Val Asp Met Pro Ser Ala Asp Phe Gln Leu Ala Lys Leu Leu
145                 150                 155                 160

Gly Leu Asn Thr Asn Val Asn Lys Tyr Cys Val Tyr Met Gln Gly Cys
                165                 170                 175

Tyr Ala Gly Gly Thr Val Leu Arg Tyr Ala Lys Asp Leu Ala Glu Asn
            180                 185                 190

Asn Arg Gly Ser Arg Val Leu Val Val Cys Ala Glu Leu Thr Ile Ile
        195                 200                 205

Gly Leu Arg Gly Pro Asn Glu Ser His Leu Asp Asn Ala Ile Gly Asn
    210                 215                 220

Ser Leu Phe Gly Asp Gly Ala Ala Ala Leu Ile Val Gly Ala Asp Pro
225                 230                 235                 240

Ile Val Gly Ile Glu Lys Pro Ile Phe Glu Ile Val Cys Ala Lys Gln
                245                 250                 255

Thr Val Ile Pro Asp Ser Glu Asp Val Ile His Leu His Leu Arg Glu
            260                 265                 270

Ala Gly Leu Met Phe Tyr Met Ser Lys Asp Ser Pro Glu Thr Ile Ser
        275                 280                 285

Asn Asn Val Glu Gly Cys Leu Val Asp Ile Phe Lys Ser Val Gly Met
    290                 295                 300

Thr Pro Pro Ala Asp Trp Asn Ser Leu Phe Trp Ile Pro His Pro Gly
305                 310                 315                 320

Gly Arg Ala Ile Leu Asp Glu Val Glu Ala Arg Leu Lys Leu Arg Pro
                325                 330                 335

Glu Lys Phe Arg Ala Thr Arg His Val Leu Trp Glu Tyr Gly Asn Met
            340                 345                 350

Val Ser Ala Cys Val Leu Tyr Ile Leu Asp Glu Met Arg Asn Lys Ser
        355                 360                 365

Ala Ala Asp Gly Leu Gly Thr Tyr Gly Glu Gly Leu Glu Trp Gly Val

370                 375                 380

Leu Leu Gly Phe Gly Pro Gly Met Thr Val Glu Thr Ile Leu Leu His
385                 390                 395                 400

Ser Leu Pro Pro Val
                405

<210>  107
<211>  413
<212>  PRT
<213>  Escherichia coli

<400>  107

Met Ser Lys Arg Arg Val Val Val Thr Gly Leu Gly Met Leu Ser Pro
1               5                   10                  15

Val Gly Asn Thr Val Glu Ser Thr Trp Lys Ala Leu Leu Ala Gly Gln
            20                  25                  30

Ser Gly Ile Ser Leu Ile Asp His Phe Asp Thr Ser Ala Tyr Ala Thr
        35                  40                  45

Lys Phe Ala Gly Leu Val Lys Asp Phe Asn Cys Glu Asp Ile Ile Ser
    50                  55                  60

Arg Lys Glu Gln Arg Lys Met Asp Ala Phe Ile Gln Tyr Gly Ile Val
65                  70                  75                  80

Ala Gly Val Gln Ala Met Gln Asp Ser Gly Leu Glu Ile Thr Glu Glu
                85                  90                  95

Asn Ala Thr Arg Ile Gly Ala Ala Ile Gly Ser Gly Ile Gly Gly Leu
            100                 105                 110

Gly Leu Ile Glu Glu Asn His Thr Ser Leu Met Asn Gly Gly Pro Arg
        115                 120                 125

Lys Ile Ser Pro Phe Phe Val Pro Ser Thr Ile Val Asn Met Val Ala
    130                 135                 140

Gly His Leu Thr Ile Met Tyr Gly Leu Arg Gly Pro Ser Ile Ser Ile
145                 150                 155                 160

Ala Thr Ala Cys Thr Ser Gly Val His Asn Ile Gly His Ala Ala Arg
                165                 170                 175

Ile Ile Ala Tyr Gly Asp Ala Asp Val Met Val Ala Gly Gly Ala Glu

180                 185                 190

Lys Ala Ser Thr Pro Leu Gly Val Gly Gly Phe Gly Ala Ala Arg Ala
        195                 200                 205

Leu Ser Thr Arg Asn Asp Asn Pro Gln Ala Ala Ser Arg Pro Trp Asp
    210                 215                 220

Lys Glu Arg Asp Gly Phe Val Leu Gly Asp Gly Ala Gly Met Leu Val
225                 230                 235                 240

Leu Glu Glu Tyr Glu His Ala Lys Lys Arg Gly Ala Lys Ile Tyr Ala
                245                 250                 255

Glu Leu Val Gly Phe Gly Met Ser Ser Asp Ala Tyr His Met Thr Ser
            260                 265                 270

Pro Pro Glu Asn Gly Ala Gly Ala Ala Leu Ala Met Ala Asn Ala Leu
        275                 280                 285

Arg Asp Ala Gly Ile Glu Ala Ser Gln Ile Gly Tyr Val Asn Ala His
    290                 295                 300

Gly Thr Ser Thr Pro Ala Gly Asp Lys Ala Glu Ala Gln Ala Val Lys
305                 310                 315                 320

Thr Ile Phe Gly Glu Ala Ala Ser Arg Val Leu Val Ser Ser Thr Lys
                325                 330                 335

Ser Met Thr Gly His Leu Leu Gly Ala Ala Gly Ala Val Glu Ser Ile
            340                 345                 350

Tyr Ser Ile Leu Ala Leu Arg Asp Gln Ala Val Pro Pro Thr Ile Asn
        355                 360                 365

Leu Asp Asn Pro Asp Glu Gly Cys Asp Leu Asp Phe Val Pro His Glu
    370                 375                 380

Ala Arg Gln Val Ser Gly Met Glu Tyr Thr Leu Cys Asn Ser Phe Gly
385                 390                 395                 400

Phe Gly Gly Thr Asn Gly Ser Leu Ile Phe Lys Lys Ile
                405                 410

<210>  108
<211>  436
<212>  PRT
<213>  Escherichia coli

<400>  108

Met Gly Gln Val Leu Pro Leu Val Thr Arg Gln Gly Asp Arg Ile Ala
1               5                   10                  15

Ile Val Ser Gly Leu Arg Thr Pro Phe Ala Arg Gln Ala Thr Ala Phe
            20                  25                  30

His Gly Ile Pro Ala Val Asp Leu Gly Lys Met Val Val Gly Glu Leu
        35                  40                  45

Leu Ala Arg Ser Glu Ile Pro Ala Glu Val Ile Glu Gln Leu Val Phe
    50                  55                  60

Gly Gln Val Val Gln Met Pro Glu Ala Pro Asn Ile Ala Arg Glu Ile
65                  70                  75                  80

Val Leu Gly Thr Gly Met Asn Val His Thr Asp Ala Tyr Ser Val Ser
                85                  90                  95

Arg Ala Cys Ala Thr Ser Phe Gln Ala Val Ala Asn Val Ala Glu Ser
            100                 105                 110

Leu Met Ala Gly Thr Ile Arg Ala Gly Ile Ala Gly Gly Ala Asp Ser
        115                 120                 125

Ser Ser Val Leu Pro Ile Gly Val Ser Lys Lys Leu Ala Arg Val Leu
    130                 135                 140

Val Asp Val Asn Lys Ala Arg Thr Met Ser Gln Arg Leu Lys Leu Phe
145                 150                 155                 160

Ser Arg Leu Arg Leu Arg Asp Leu Met Pro Val Pro Pro Ala Val Ala
                165                 170                 175

Glu Tyr Ser Thr Gly Leu Arg Met Gly Asp Thr Ala Glu Gln Met Ala
            180                 185                 190

Lys Thr Tyr Gly Ile Thr Arg Glu Gln Gln Asp Ala Leu Ala His Arg
        195                 200                 205

Ser His Gln Arg Ala Ala Gln Ala Trp Ser Asp Gly Lys Leu Lys Glu
    210                 215                 220

Glu Val Met Thr Ala Phe Ile Pro Pro Tyr Lys Gln Pro Leu Val Glu
225                 230                 235                 240

Asp Asn Asn Ile Arg Gly Asn Ser Ser Leu Ala Asp Tyr Ala Lys Leu
                245                 250                 255

Arg Pro Ala Phe Asp Arg Lys His Gly Thr Val Thr Ala Ala Asn Ser
            260                 265                 270

Thr Pro Leu Thr Asp Gly Ala Ala Ala Val Ile Leu Met Thr Glu Ser
        275                 280                 285

Arg Ala Lys Glu Leu Gly Leu Val Pro Leu Gly Tyr Leu Arg Ser Tyr
    290                 295                 300

Ala Phe Thr Ala Ile Asp Val Trp Gln Asp Met Leu Leu Gly Pro Ala
305                 310                 315                 320

Trp Ser Thr Pro Leu Ala Leu Glu Arg Ala Gly Leu Thr Met Ser Asp
                325                 330                 335

Leu Thr Leu Ile Asp Met His Glu Ala Phe Ala Ala Gln Thr Leu Ala
            340                 345                 350

Asn Ile Gln Leu Leu Gly Ser Glu Arg Phe Ala Arg Glu Ala Leu Gly
        355                 360                 365

Arg Ala His Ala Thr Gly Glu Val Asp Asp Ser Lys Phe Asn Val Leu
    370                 375                 380

Gly Gly Ser Ile Ala Tyr Gly His Pro Phe Ala Ala Thr Gly Ala Arg
385                 390                 395                 400

Met Ile Thr Gln Thr Leu His Glu Leu Arg Arg Arg Gly Gly Gly Phe
                405                 410                 415

Gly Leu Val Thr Ala Cys Ala Ala Gly Gly Leu Gly Ala Ala Met Val
            420                 425                 430

Leu Glu Ala Glu
        435

<210>  109
<211>  294
<212>  PRT
<213>  Escherichia coli

<400>  109

Met Glu Arg Val Tyr Arg Thr Asp Leu Lys Leu Leu Arg Tyr Phe Leu
1               5                   10                  15

Ala Val Ala Glu Glu Leu His Phe Gly Arg Ala Ala Ala Arg Leu Asn
            20                  25                  30

Met Ser Gln Pro Pro Leu Ser Ile His Ile Lys Glu Leu Glu Asn Gln
        35                  40                  45

Leu Gly Thr Gln Leu Phe Ile Arg His Ser Arg Ser Val Val Leu Thr
    50                  55                  60

His Ala Gly Lys Ile Leu Met Glu Glu Ser Arg Arg Leu Leu Val Asn
65                  70                  75                  80

Ala Asn Asn Val Leu Ala Arg Ile Glu Gln Ile Gly Arg Gly Glu Ala
                85                  90                  95

Gly Arg Ile Glu Leu Gly Val Val Gly Thr Ala Met Trp Gly Arg Met
            100                 105                 110

Arg Pro Val Met Arg Arg Phe Leu Arg Glu Asn Pro Asn Val Asp Val
        115                 120                 125

Leu Phe Arg Glu Lys Met Pro Ala Met Gln Met Ala Leu Leu Glu Arg
    130                 135                 140

Arg Glu Leu Asp Ala Gly Ile Trp Arg Met Ala Thr Glu Pro Pro Thr
145                 150                 155                 160

Gly Phe Thr Ser Leu Arg Leu His Glu Ser Ala Phe Leu Val Ala Met
                165                 170                 175

Pro Glu Glu His His Leu Ser Ser Phe Ser Thr Val Pro Leu Glu Ala
            180                 185                 190

Leu Arg Asp Glu Tyr Phe Val Thr Met Pro Pro Val Tyr Thr Asp Trp
        195                 200                 205

Asp Phe Leu Gln Arg Val Cys Gln Gln Val Gly Phe Ser Pro Val Val
    210                 215                 220

Ile Arg Glu Val Asn Glu Pro Gln Thr Val Leu Ala Met Val Ser Met
225                 230                 235                 240

Gly Ile Gly Ile Thr Leu Ile Ala Asp Ser Tyr Ala Gln Met Asn Trp
                245                 250                 255

Pro Gly Val Ile Phe Arg Pro Leu Lys Gln Arg Ile Pro Ala Asp Leu
            260                 265                 270

Tyr Ile Val Tyr Glu Thr Gln Gln Val Thr Pro Ala Met Val Lys Leu
        275                 280                 285

Leu Ala Ala Leu Thr Gln
    290

<210>  110
<211>  341
<212>  PRT
<213>  Escherichia coli

<400>  110

<210> 111
<211> 317
<212> PRT
<213> Escherichia coli

<400> 111

Met Tyr Thr Lys Ile Ile Gly Thr Gly Ser Tyr Leu Pro Glu Gln Val
1               5                   10                  15

Arg Thr Asn Ala Asp Leu Glu Lys Met Val Asp Thr Ser Asp Glu Trp
            20                  25                  30

Ile Val Thr Arg Thr Gly Ile Arg Glu Arg His Ile Ala Ala Pro Asn
        35                  40                  45

Glu Thr Val Ser Thr Met Gly Phe Glu Ala Ala Thr Arg Ala Ile Glu
    50                  55                  60

Met Ala Gly Ile Glu Lys Asp Gln Ile Gly Leu Ile Val Val Ala Thr
65                  70                  75                  80

Thr Ser Ala Thr His Ala Phe Pro Ser Ala Ala Cys Gln Ile Gln Ser
                85                  90                  95

Met Leu Gly Ile Lys Gly Cys Pro Ala Phe Asp Val Ala Ala Ala Cys
            100                 105                 110

Ala Gly Phe Thr Tyr Ala Leu Ser Val Ala Asp Gln Tyr Val Lys Ser
        115                 120                 125

Gly Ala Val Lys Tyr Ala Leu Val Val Gly Ser Asp Val Leu Ala Arg
    130                 135                 140

Thr Cys Asp Pro Thr Asp Arg Gly Thr Ile Ile Ile Phe Gly Asp Gly
145                 150                 155                 160

Ala Gly Ala Ala Val Leu Ala Ala Ser Glu Glu Pro Gly Ile Ile Ser
                165                 170                 175

Thr His Leu His Ala Asp Gly Ser Tyr Gly Glu Leu Leu Thr Leu Pro
            180                 185                 190

Asn Ala Asp Arg Val Asn Pro Glu Asn Ser Ile His Leu Thr Met Ala
        195                 200                 205

Gly Asn Glu Val Phe Lys Val Ala Val Thr Glu Leu Ala His Ile Val
    210                 215                 220

Asp Glu Thr Leu Ala Ala Asn Asn Leu Asp Arg Ser Gln Leu Asp Trp
225                 230                 235                 240

Leu Val Pro His Gln Ala Asn Leu Arg Ile Ile Ser Ala Thr Ala Lys
                245                 250                 255

Lys Leu Gly Met Ser Met Asp Asn Val Val Val Thr Leu Asp Arg His
            260                 265                 270

Gly Asn Thr Ser Ala Ala Ser Val Pro Cys Ala Leu Asp Glu Ala Val
        275                 280                 285

Arg Asp Gly Arg Ile Lys Pro Gly Gln Leu Val Leu Leu Glu Ala Phe
    290                 295                 300

Gly Gly Gly Phe Thr Trp Gly Ser Ala Leu Val Arg Phe
305                 310                 315

<210>  112
<211>  548
<212>  PRT
<213>  Escherichia coli

<400>  112

<210>  113
<211>  127
<212>  PRT
<213>  Escherichia coli

<400>  113

Met Ile Thr Gly Ile Gln Ile Thr Lys Ala Ala Asn Asp Asp Leu Leu
1               5                   10                  15

Asn Ser Phe Trp Leu Leu Asp Ser Glu Lys Gly Glu Ala Arg Cys Ile
            20                  25                  30

Val Ala Lys Ala Gly Tyr Ala Glu Asp Glu Val Val Ala Val Ser Lys
        35                  40                  45

Leu Gly Asp Ile Glu Tyr Arg Glu Val Pro Val Glu Val Lys Pro Glu
    50                  55                  60

Val Arg Val Glu Gly Gly Gln His Leu Asn Val Asn Val Leu Arg Arg
65                  70                  75                  80

Glu Thr Leu Glu Asp Ala Val Lys His Pro Glu Lys Tyr Pro Gln Leu
                85                  90                  95

Thr Ile Arg Val Ser Gly Tyr Ala Val Arg Phe Asn Ser Leu Thr Pro
            100                 105                 110

Glu Gln Gln Arg Asp Val Ile Ala Arg Thr Phe Thr Glu Ser Leu
        115                 120                 125

<210>  114

<211>  315
<212>  PRT
<213>  Escherichia coli

<400>  114

Met Ser Glu Ser Leu Arg Ile Ile Phe Ala Gly Thr Pro Asp Phe Ala
1               5                   10                  15

Ala Arg His Leu Asp Ala Leu Leu Ser Ser Gly His Asn Val Val Gly
            20                  25                  30

Val Phe Thr Gln Pro Asp Arg Pro Ala Gly Arg Gly Lys Lys Leu Met
        35                  40                  45

Pro Ser Pro Val Lys Val Leu Ala Glu Glu Lys Gly Leu Pro Val Phe
    50                  55                  60

Gln Pro Val Ser Leu Arg Pro Gln Glu Asn Gln Gln Leu Val Ala Glu
65                  70                  75                  80

Leu Gln Ala Asp Val Met Val Val Val Ala Tyr Gly Leu Ile Leu Pro
                85                  90                  95

Lys Ala Val Leu Glu Met Pro Arg Leu Gly Cys Ile Asn Val His Gly
            100                 105                 110

Ser Leu Leu Pro Arg Trp Arg Gly Ala Ala Pro Ile Gln Arg Ser Leu
        115                 120                 125

Trp Ala Gly Asp Ala Glu Thr Gly Val Thr Ile Met Gln Met Asp Val
    130                 135                 140

Gly Leu Asp Thr Gly Asp Met Leu Tyr Lys Leu Ser Cys Pro Ile Thr
145                 150                 155                 160

Ala Glu Asp Thr Ser Gly Thr Leu Tyr Asp Lys Leu Ala Glu Leu Gly
                165                 170                 175

Pro Gln Gly Leu Ile Thr Thr Leu Lys Gln Leu Ala Asp Gly Thr Ala
            180                 185                 190

Lys Pro Glu Val Gln Asp Glu Thr Leu Val Thr Tyr Ala Glu Lys Leu
        195                 200                 205

Ser Lys Glu Glu Ala Arg Ile Asp Trp Ser Leu Ser Ala Ala Gln Leu
    210                 215                 220

Glu Arg Cys Ile Arg Ala Phe Asn Pro Trp Pro Met Ser Trp Leu Glu

225                 230                 235                 240

Ile Glu Gly Gln Pro Val Lys Val Trp Lys Ala Ser Val Ile Asp Thr
                245                 250                 255

Ala Thr Asn Ala Ala Pro Gly Thr Ile Leu Glu Ala Asn Lys Gln Gly
            260                 265                 270

Ile Gln Val Ala Thr Gly Asp Gly Ile Leu Asn Leu Leu Ser Leu Gln
        275                 280                 285

Pro Ala Gly Lys Lys Ala Met Ser Ala Gln Asp Leu Leu Asn Ser Arg
    290                 295                 300

Arg Glu Trp Phe Val Pro Gly Asn Arg Leu Val
305                 310                 315

<210>  115
<211>  245
<212>  PRT
<213>  Escherichia coli

<400>  115

Met Thr Leu Thr Ala Ser Ser Ser Ser Arg Ala Val Thr Asn Ser Pro
1               5                   10                  15

Val Val Val Ala Leu Asp Tyr His Asn Arg Asp Asp Ala Leu Ala Phe
            20                  25                  30

Val Asp Lys Ile Asp Pro Arg Asp Cys Arg Leu Lys Val Gly Lys Glu
        35                  40                  45

Met Phe Thr Leu Phe Gly Pro Gln Phe Val Arg Glu Leu Gln Gln Arg
    50                  55                  60

Gly Phe Asp Ile Phe Leu Asp Leu Lys Phe His Asp Ile Pro Asn Thr
65                  70                  75                  80

Ala Ala His Ala Val Ala Ala Ala Ala Asp Leu Gly Val Trp Met Val
                85                  90                  95

Asn Val His Ala Ser Gly Gly Ala Arg Met Met Thr Ala Ala Arg Glu
            100                 105                 110

Ala Leu Val Pro Phe Gly Lys Asp Ala Pro Leu Leu Ile Ala Val Thr
        115                 120                 125

Val Leu Thr Ser Met Glu Ala Ser Asp Leu Val Asp Leu Gly Met Thr

130                 135                 140

Leu Ser Pro Ala Asp Tyr Ala Glu Arg Leu Ala Ala Leu Thr Gln Lys
145                 150                 155                 160

Cys Gly Leu Asp Gly Val Val Cys Ser Ala Gln Glu Ala Val Arg Phe
                165                 170                 175

Lys Gln Val Phe Gly Gln Glu Phe Lys Leu Val Thr Pro Gly Ile Arg
            180                 185                 190

Pro Gln Gly Ser Glu Ala Gly Asp Gln Arg Arg Ile Met Thr Pro Glu
        195                 200                 205

Gln Ala Leu Ser Ala Gly Val Asp Tyr Met Val Ile Gly Arg Pro Val
    210                 215                 220

Thr Gln Ser Val Asp Pro Ala Gln Thr Leu Lys Ala Ile Asn Ala Ser
225                 230                 235                 240

Leu Gln Arg Ser Ala
                245

<210>  116
<211>  500
<212>  PRT
<213>  Escherichia coli

<400>  116

Met Thr Ile Phe Asp Asn Tyr Glu Val Trp Phe Val Ile Gly Ser Gln
1               5                   10                  15

His Leu Tyr Gly Pro Glu Thr Leu Arg Gln Val Thr Gln His Ala Glu
            20                  25                  30

His Val Val Asn Ala Leu Asn Thr Glu Ala Lys Leu Pro Cys Lys Leu
        35                  40                  45

Val Leu Lys Pro Leu Gly Thr Thr Pro Asp Glu Ile Thr Ala Ile Cys
    50                  55                  60

Arg Asp Ala Asn Tyr Asp Asp Arg Cys Ala Gly Leu Val Val Trp Leu
65                  70                  75                  80

His Thr Phe Ser Pro Ala Lys Met Trp Ile Asn Gly Leu Thr Met Leu
                85                  90                  95

Asn Lys Pro Leu Leu Gln Phe His Thr Gln Phe Asn Ala Ala Leu Pro

            100                 105                 110

Trp Asp Ser Ile Asp Met Asp Phe Met Asn Leu Asn Gln Thr Ala His
        115                 120                 125

Gly Gly Arg Glu Phe Gly Phe Ile Gly Ala Arg Met Arg Gln Gln His
    130                 135                 140

Ala Val Val Thr Gly His Trp Gln Asp Lys Gln Ala His Glu Arg Ile
145                 150                 155                 160

Gly Ser Trp Met Arg Gln Ala Val Ser Lys Gln Asp Thr Arg His Leu
                165                 170                 175

Lys Val Cys Arg Phe Gly Asp Asn Met Arg Glu Val Ala Val Thr Asp
            180                 185                 190

Gly Asp Lys Val Ala Ala Gln Ile Lys Phe Gly Phe Ser Val Asn Thr
        195                 200                 205

Trp Ala Val Gly Asp Leu Val Gln Val Val Asn Ser Ile Ser Asp Gly
    210                 215                 220

Asp Val Asn Ala Leu Val Asp Glu Tyr Glu Ser Cys Tyr Thr Met Thr
225                 230                 235                 240

Pro Ala Thr Gln Ile His Gly Lys Lys Arg Gln Asn Val Leu Glu Ala
                245                 250                 255

Ala Arg Ile Glu Leu Gly Met Lys Arg Phe Leu Glu Gln Gly Gly Phe
            260                 265                 270

His Ala Phe Thr Thr Thr Phe Glu Asp Leu His Gly Leu Lys Gln Leu
        275                 280                 285

Pro Gly Leu Ala Val Gln Arg Leu Met Gln Gln Gly Tyr Gly Phe Ala
    290                 295                 300

Gly Glu Gly Asp Trp Lys Thr Ala Ala Leu Leu Arg Ile Met Lys Val
305                 310                 315                 320

Met Ser Thr Gly Leu Gln Gly Gly Thr Ser Phe Met Glu Asp Tyr Thr
                325                 330                 335

Tyr His Phe Glu Lys Gly Asn Asp Leu Val Leu Gly Ser His Met Leu
            340                 345                 350

Glu Val Cys Pro Ser Ile Ala Ala Glu Glu Lys Pro Ile Leu Asp Val
        355                 360                 365

Gln His Leu Gly Ile Gly Gly Lys Asp Asp Pro Ala Arg Leu Ile Phe
    370                 375                 380

Asn Thr Gln Thr Gly Pro Ala Ile Val Ala Ser Leu Ile Asp Leu Gly
385                 390                 395                 400

Asp Arg Tyr Arg Leu Leu Val Asn Cys Ile Asp Thr Val Lys Thr Pro
                405                 410                 415

His Ser Leu Pro Lys Leu Pro Val Ala Asn Ala Leu Trp Lys Ala Gln
            420                 425                 430

Pro Asp Leu Pro Thr Ala Ser Glu Ala Trp Ile Leu Ala Gly Gly Ala
        435                 440                 445

His His Thr Val Phe Ser His Ala Leu Asn Leu Asn Asp Met Arg Gln
    450                 455                 460

Phe Ala Glu Met His Asp Ile Glu Ile Thr Val Ile Asp Asn Asp Thr
465                 470                 475                 480

Arg Leu Pro Ala Phe Lys Asp Ala Leu Arg Trp Asn Glu Val Tyr Tyr
                485                 490                 495

Gly Phe Arg Arg
            500

<210>  117
<211>  334
<212>  PRT
<213>  Escherichia coli

<400>  117

Met Leu Asn Thr Leu Ile Val Gly Ala Ser Gly Tyr Ala Gly Ala Glu
1               5                   10                  15

Leu Val Thr Tyr Val Asn Arg His Pro His Met Asn Ile Thr Ala Leu
            20                  25                  30

Thr Val Ser Ala Gln Ser Asn Asp Ala Gly Lys Leu Ile Ser Asp Leu
        35                  40                  45

His Pro Gln Leu Lys Gly Ile Val Asp Leu Pro Leu Gln Pro Met Ser
    50                  55                  60

Asp Ile Ser Glu Phe Ser Pro Gly Val Asp Val Val Phe Leu Ala Thr
65                  70                  75                  80

Ala His Glu Val Ser His Asp Leu Ala Pro Gln Phe Leu Glu Ala Gly
                85                  90                  95

Cys Val Val Phe Asp Leu Ser Gly Ala Phe Arg Val Asn Asp Ala Thr
            100                 105                 110

Phe Tyr Glu Lys Tyr Tyr Gly Phe Thr His Gln Tyr Pro Glu Leu Leu
        115                 120                 125

Glu Gln Ala Ala Tyr Gly Leu Ala Glu Trp Cys Gly Asn Lys Leu Lys
    130                 135                 140

Glu Ala Asn Leu Ile Ala Val Pro Gly Cys Tyr Pro Thr Ala Ala Gln
145                 150                 155                 160

Leu Ala Leu Lys Pro Leu Ile Asp Ala Asp Leu Leu Asp Leu Asn Gln
                165                 170                 175

Trp Pro Val Ile Asn Ala Thr Ser Gly Val Ser Gly Ala Gly Arg Lys
            180                 185                 190

Ala Ala Ile Ser Asn Ser Phe Cys Glu Val Ser Leu Gln Pro Tyr Gly
        195                 200                 205

Val Phe Thr His Arg His Gln Pro Glu Ile Ala Thr His Leu Gly Ala
    210                 215                 220

Asp Val Ile Phe Thr Pro His Leu Gly Asn Phe Pro Arg Gly Ile Leu
225                 230                 235                 240

Glu Thr Ile Thr Cys Arg Leu Lys Ser Gly Val Thr Gln Ala Gln Val
                245                 250                 255

Ala Gln Val Leu Gln Gln Ala Tyr Ala His Lys Pro Leu Val Arg Leu
            260                 265                 270

Tyr Asp Lys Gly Val Pro Ala Leu Lys Asn Val Val Gly Leu Pro Phe
        275                 280                 285

Cys Asp Ile Gly Phe Ala Val Gln Gly Glu His Leu Ile Ile Val Ala
    290                 295                 300

Thr Glu Asp Asn Leu Leu Lys Gly Ala Ala Ala Gln Ala Val Gln Cys
305                 310                 315                 320

Ala Asn Ile Arg Phe Gly Tyr Ala Glu Thr Gln Ser Leu Ile
                325                 330

<210>  118
<211>  239
<212>  PRT
<213>  Escherichia coli

<400>  118

Met Val Ile Lys Ala Gln Ser Pro Ala Gly Phe Ala Glu Glu Tyr Ile
1               5                   10                  15

Ile Glu Ser Ile Trp Asn Asn Arg Phe Pro Pro Gly Thr Ile Leu Pro
            20                  25                  30

Ala Glu Arg Glu Leu Ser Glu Leu Ile Gly Val Thr Arg Thr Thr Leu
        35                  40                  45

Arg Glu Val Leu Gln Arg Leu Ala Arg Asp Gly Trp Leu Thr Ile Gln
    50                  55                  60

His Gly Lys Pro Thr Lys Val Asn Asn Phe Trp Glu Thr Ser Gly Leu
65                  70                  75                  80

Asn Ile Leu Glu Thr Leu Ala Arg Leu Asp His Glu Ser Val Pro Gln
                85                  90                  95

Leu Ile Asp Asn Leu Leu Ser Val Arg Thr Asn Ile Ser Thr Ile Phe
            100                 105                 110

Ile Arg Thr Ala Phe Arg Gln His Pro Asp Lys Ala Gln Glu Val Leu
        115                 120                 125

Ala Thr Ala Asn Glu Val Ala Asp His Ala Asp Ala Phe Ala Glu Leu
    130                 135                 140

Asp Tyr Asn Ile Phe Arg Gly Leu Ala Phe Ala Ser Gly Asn Pro Ile
145                 150                 155                 160

Tyr Gly Leu Ile Leu Asn Gly Met Lys Gly Leu Tyr Thr Arg Ile Gly
                165                 170                 175

Arg His Tyr Phe Ala Asn Pro Glu Ala Arg Ser Leu Ala Leu Gly Phe
            180                 185                 190

Tyr His Lys Leu Ser Ala Leu Cys Ser Glu Gly Ala His Asp Gln Val
        195                 200                 205

Tyr Glu Thr Val Arg Arg Tyr Gly His Glu Ser Gly Glu Ile Trp His
    210                 215                 220

Arg Met Gln Lys Asn Leu Pro Gly Asp Leu Ala Ile Gln Gly Arg
225                 230                 235

<210>  119
<211>  159
<212>  PRT
<213>  Escherichia coli

<400>  119

Met Phe Leu Arg Gln Glu Asp Phe Ala Thr Val Val Arg Ser Thr Pro
1               5                   10                  15

Leu Val Ser Leu Asp Phe Ile Val Glu Asn Ser Arg Gly Glu Phe Leu
            20                  25                  30

Leu Gly Lys Arg Thr Asn Arg Pro Ala Gln Gly Tyr Trp Phe Val Pro
        35                  40                  45

Gly Gly Arg Val Gln Lys Asp Glu Thr Leu Glu Ala Ala Phe Glu Arg
    50                  55                  60

Leu Thr Met Ala Glu Leu Gly Leu Arg Leu Pro Ile Thr Ala Gly Gln
65                  70                  75                  80

Phe Tyr Gly Val Trp Gln His Phe Tyr Asp Asp Asn Phe Ser Gly Thr
                85                  90                  95

Asp Phe Thr Thr His Tyr Val Val Leu Gly Phe Arg Phe Arg Val Ser
            100                 105                 110

Glu Glu Glu Leu Leu Leu Pro Asp Glu Gln His Asp Asp Tyr Arg Trp
        115                 120                 125

Leu Thr Ser Asp Ala Leu Leu Ala Ser Asp Asn Val His Ala Asn Ser
    130                 135                 140

Arg Ala Tyr Phe Leu Ala Glu Lys Arg Thr Gly Val Pro Gly Leu
145                 150                 155

<210>  120
<211>  187
<212>  PRT
<213>  Escherichia coli

<400>  120

Met Ile Leu Leu Ile Asp Asn Tyr Asp Ser Phe Thr Trp Asn Leu Tyr
1               5                   10                  15

Gln Tyr Phe Cys Glu Leu Gly Ala Asp Val Leu Val Lys Arg Asn Asp
            20                  25                  30

Ala Leu Thr Leu Ala Asp Ile Asp Ala Leu Lys Pro Gln Lys Ile Val
        35                  40                  45

Ile Ser Pro Gly Pro Cys Thr Pro Asp Glu Ala Gly Ile Ser Leu Asp
    50                  55                  60

Val Ile Arg His Tyr Ala Gly Arg Leu Pro Ile Leu Gly Val Cys Leu
65                  70                  75                  80

Gly His Gln Ala Met Ala Gln Ala Phe Gly Gly Lys Val Val Arg Ala
                85                  90                  95

Ala Lys Val Met His Gly Lys Thr Ser Pro Ile Thr His Asn Gly Glu
            100                 105                 110

Gly Val Phe Arg Gly Leu Ala Asn Pro Leu Thr Val Thr Arg Tyr His
        115                 120                 125

Ser Leu Val Val Glu Pro Asp Ser Leu Pro Ala Cys Phe Asp Val Thr
    130                 135                 140

Ala Trp Ser Glu Thr Arg Glu Ile Met Gly Ile Arg His Arg Gln Trp
145                 150                 155                 160

Asp Leu Glu Gly Val Gln Phe His Pro Glu Ser Ile Leu Ser Glu Gln
                165                 170                 175

Gly His Gln Leu Leu Ala Asn Phe Leu His Arg
            180                 185

<210>  121
<211>  456
<212>  PRT
<213>  Escherichia coli

<400>  121

Met Glu Leu Ser Ser Leu Thr Ala Val Ser Pro Val Asp Gly Arg Tyr
1               5                   10                  15

Gly Asp Lys Val Ser Ala Leu Arg Gly Ile Phe Ser Glu Tyr Gly Leu
            20                  25                  30

Leu Lys Phe Arg Val Gln Val Glu Val Arg Trp Leu Gln Lys Leu Ala
        35                  40                  45

Ala His Ala Ala Ile Lys Glu Val Pro Ala Phe Ala Ala Asp Ala Ile
    50                  55                  60

Gly Tyr Leu Asp Ala Ile Val Ala Ser Phe Ser Glu Glu Asp Ala Ala
65                  70                  75                  80

Arg Ile Lys Thr Ile Glu Arg Thr Thr Asn His Asp Val Lys Ala Val
                85                  90                  95

Glu Tyr Phe Leu Lys Glu Lys Val Ala Glu Ile Pro Glu Leu His Ala
            100                 105                 110

Val Ser Glu Phe Ile His Phe Ala Cys Thr Ser Glu Asp Ile Asn Asn
        115                 120                 125

Leu Ser His Ala Leu Met Leu Lys Thr Ala Arg Asp Glu Val Ile Leu
    130                 135                 140

Pro Tyr Trp Arg Gln Leu Ile Asp Gly Ile Lys Asp Leu Ala Val Gln
145                 150                 155                 160

Tyr Arg Asp Ile Pro Leu Leu Ser Arg Thr His Gly Gln Pro Ala Thr
                165                 170                 175

Pro Ser Thr Ile Gly Lys Glu Met Ala Asn Val Ala Tyr Arg Met Glu
            180                 185                 190

Arg Gln Tyr Arg Gln Leu Asn Gln Val Glu Ile Leu Gly Lys Ile Asn
        195                 200                 205

Gly Ala Val Gly Asn Tyr Asn Ala His Ile Ala Ala Tyr Pro Glu Val
    210                 215                 220

Asp Trp His Gln Phe Ser Glu Glu Phe Val Thr Ser Leu Gly Ile Gln
225                 230                 235                 240

Trp Asn Pro Tyr Thr Thr Gln Ile Glu Pro His Asp Tyr Ile Ala Glu
                245                 250                 255

Leu Phe Asp Cys Val Ala Arg Phe Asn Thr Ile Leu Ile Asp Phe Asp
            260                 265                 270

Arg Asp Val Trp Gly Tyr Ile Ala Leu Asn His Phe Lys Gln Lys Thr
        275                 280                 285

Ile Ala Gly Glu Ile Gly Ser Ser Thr Met Pro His Lys Val Asn Pro
    290                 295                 300

Ile Asp Phe Glu Asn Ser Glu Gly Asn Leu Gly Leu Ser Asn Ala Val
305                 310                 315                 320

Leu Gln His Leu Ala Ser Lys Leu Pro Val Ser Arg Trp Gln Arg Asp
                325                 330                 335

Leu Thr Asp Ser Thr Val Leu Arg Asn Leu Gly Val Gly Ile Gly Tyr
            340                 345                 350

Ala Leu Ile Ala Tyr Gln Ser Thr Leu Lys Gly Val Ser Lys Leu Glu
        355                 360                 365

Val Asn Arg Asp His Leu Leu Asp Glu Leu Asp His Asn Trp Glu Val
    370                 375                 380

Leu Ala Glu Pro Ile Gln Thr Val Met Arg Arg Tyr Gly Ile Glu Lys
385                 390                 395                 400

Pro Tyr Glu Lys Leu Lys Glu Leu Thr Arg Gly Lys Arg Val Asp Ala
                405                 410                 415

Glu Gly Met Lys Gln Phe Ile Asp Gly Leu Ala Leu Pro Glu Glu Glu
            420                 425                 430

Lys Ala Arg Leu Lys Ala Met Thr Pro Ala Asn Tyr Ile Gly Arg Ala
        435                 440                 445

Ile Thr Met Val Asp Glu Leu Lys
    450                 455

<210>  122
<211>  156
<212>  PRT
<213>  Escherichia coli

<400>  122

Met Thr Asp Val Leu Leu Cys Val Gly Asn Ser Met Met Gly Asp Asp
1               5                   10                  15

Gly Ala Gly Pro Leu Leu Ala Glu Lys Cys Ala Ala Ala Pro Lys Gly
            20                  25                  30

Asn Trp Val Val Ile Asp Gly Gly Ser Ala Pro Glu Asn Asp Ile Val
        35                  40                  45

Ala Ile Arg Glu Leu Arg Pro Thr Arg Leu Leu Ile Val Asp Ala Thr
    50                  55                  60

Asp Met Gly Leu Asn Pro Gly Glu Ile Arg Ile Ile Asp Pro Asp Asp
65                  70                  75                  80

Ile Ala Glu Met Phe Met Met Thr Thr His Asn Met Pro Leu Asn Tyr
                85                  90                  95

Leu Ile Asp Gln Leu Lys Glu Asp Ile Gly Glu Val Ile Phe Leu Gly
            100                 105                 110

Ile Gln Pro Asp Ile Val Gly Phe Tyr Tyr Pro Met Thr Gln Pro Ile
        115                 120                 125

Lys Asp Ala Val Glu Thr Val Tyr Gln Arg Leu Glu Gly Trp Glu Gly
    130                 135                 140

Asn Gly Gly Phe Ala Gln Leu Ala Val Glu Glu Glu
145                 150                 155

<210>  123
<211>  1774
<212>  PRT
<213>  Penicillium urticae

<400>  123

Met His Ser Ala Ala Thr Ser Thr Tyr Pro Ser Gly Lys Thr Ser Pro
1               5                   10                  15

Ala Pro Val Gly Thr Pro Gly Thr Glu Tyr Ser Glu Tyr Glu Phe Ser
            20                  25                  30

Asn Asp Val Ala Val Val Gly Met Ala Cys Arg Val Ala Gly Gly Asn
        35                  40                  45

His Asn Pro Glu Leu Leu Trp Gln Ser Leu Leu Ser Gln Lys Ser Ala
    50                  55                  60

Met Gly Glu Ile Pro Pro Met Arg Trp Glu Pro Tyr Tyr Arg Arg Asp
65                  70                  75                  80

Ala Arg Asn Glu Lys Phe Leu Lys Asn Thr Thr Ser Arg Gly Tyr Phe
                85                  90                  95

Leu Asp Arg Leu Glu Asp Phe Asp Cys Gln Phe Phe Gly Ile Ser Pro
            100                 105                 110

Lys Glu Ala Glu Gln Met Asp Pro Gln Gln Arg Val Ser Leu Glu Val
        115                 120                 125

Ala Ser Glu Ala Leu Glu Asp Ala Gly Ile Pro Ala Lys Ser Leu Ser
    130                 135                 140

Gly Ser Asp Thr Ala Val Phe Trp Gly Val Asn Ser Asp Asp Tyr Ser
145                 150                 155                 160

Lys Leu Val Leu Glu Asp Leu Pro Asn Val Glu Ala Trp Met Gly Ile
                165                 170                 175

Gly Thr Ala Tyr Cys Gly Val Pro Asn Arg Ile Ser Tyr His Leu Asn
            180                 185                 190

Leu Met Gly Pro Ser Thr Ala Val Asp Ala Ala Cys Ala Ser Ser Leu
        195                 200                 205

Val Ala Ile His His Gly Val Gln Ala Ile Arg Leu Gly Glu Ser Lys
    210                 215                 220

Val Ala Ile Val Gly Gly Val Asn Ala Leu Cys Gly Pro Gly Leu Thr
225                 230                 235                 240

Arg Val Leu Asp Lys Ala Gly Ala Ile Ser Ser Asp Gly Ser Cys Lys
                245                 250                 255

Ser Phe Asp Asp Asp Ala His Gly Tyr Ala Arg Gly Glu Gly Ala Gly
            260                 265                 270

Ala Leu Val Leu Lys Ser Leu His Arg Ala Leu Leu Asp His Asp Asn
        275                 280                 285

Val Leu Ala Val Ile Lys Gly Ser Ala Val Cys Gln Asp Gly Lys Thr
    290                 295                 300

Asn Gly Ile Met Ala Pro Asn Ser Val Ala Gln Gln Leu Ala Ala Asn
305                 310                 315                 320

Asn Ala Leu Ser Ala Ala Asn Ile Asp Pro His Thr Val Arg Tyr Val
                325                 330                 335

Glu Ala His Ala Thr Ser Thr Pro Leu Gly Asp Pro Thr Glu Ile Ser
            340                 345                 350

Ala Ile Ala Ser Val Tyr Gly Ala Asp Arg Pro Ala Asp Asp Pro Cys
        355                 360                 365

Tyr Ile Gly Ser Ile Lys Pro Asn Ile Gly His Leu Glu Ala Gly Ala
    370                 375                 380

Gly Val Met Gly Phe Ile Lys Ala Val Leu Ala Ile Gln Lys Gly Val
385                 390                 395                 400

Leu Pro Pro Gln Ala Asn Leu Thr Lys Leu Asn Ser Arg Ile Asp Trp
                405                 410                 415

Lys Thr Ala Gly Val Lys Val Val Gln Glu Ala Thr Pro Trp Pro Glu
            420                 425                 430

Ser Asp Pro Ile Arg Arg Ala Gly Val Cys Ser Tyr Gly Tyr Gly Gly
        435                 440                 445

Thr Val Ser His Ala Val Ile Glu Glu Phe Ser Pro Ile Leu Gln Pro
    450                 455                 460

Asp Pro Leu Gly Asn Gly Ala Val Ser Gly Pro Gly Leu Leu Leu Leu
465                 470                 475                 480

Ser Gly Pro Gln Glu Lys Arg Leu Ala Leu Gln Ala Lys Thr Leu Arg
                485                 490                 495

Asp Trp Met Thr Ala Glu Gly Lys Asp His Asn Leu Ser Asp Ile Leu
            500                 505                 510

Thr Thr Leu Ala Thr Arg Arg Asp His His Asp Tyr Arg Ala Ala Leu
        515                 520                 525

Val Val Asp Asp Tyr Arg Asp Ala Glu Gln Val Leu Gln Ser Leu Ala
    530                 535                 540

Asn Gly Val Asp His Thr Phe Thr Thr Gln Ser Arg Val Leu Gly Ser
545                 550                 555                 560

Asp Ile Ser Lys Asp Val Val Trp Val Phe Ser Gly His Gly Ala Gln
                565                 570                 575

Trp Pro Asp Met Gly Lys Gln Leu Ile His Asn Pro Val Phe Phe Ala
            580                 585                 590

Ala Ile Gln Pro Leu Asp Glu Leu Ile Gln Ala Glu Ile Gly Leu Ser
        595                 600                 605

Pro Ile Glu Leu Leu Arg Thr Gly Asp Phe Glu Ser Ser Asp Arg Val

610                 615                 620

Gln Ile Leu Thr Tyr Val Met Gln Ile Gly Leu Ser Ala Leu Leu Gln
625                 630                 635                 640

Ser Asn Gly Ile Thr Pro Gln Ala Val Ile Gly His Ser Val Gly Glu
                645                 650                 655

Ile Ala Ala Ser Val Val Ala Gly Ala Leu Ser Pro Ala Glu Gly Ala
            660                 665                 670

Leu Ile Val Thr Arg Arg Ala Leu Leu Tyr Arg Gln Val Met Gly Lys
        675                 680                 685

Gly Gly Met Ile Leu Val Asn Leu Pro Ser Ala Glu Thr Glu Glu Ile
    690                 695                 700

Leu Gly Ser Arg Ser Asp Leu Val Val Ala Ile Asp Ser Ser Pro Ser
705                 710                 715                 720

Ser Cys Val Val Ala Gly Asp Lys Glu Leu Val Ala Glu Thr Ala Glu
                725                 730                 735

Ala Leu Lys Ala Arg Gly Val Lys Thr Phe Thr Val Lys Ser Asp Ile
            740                 745                 750

Ala Phe His Ser Pro Thr Leu Asn Gly Leu Val Asp Pro Leu Arg Asp
        755                 760                 765

Val Leu Ala Glu Thr Leu Ser Pro Val Ser Pro Asn Val Lys Leu Tyr
    770                 775                 780

Ser Thr Ala Leu Ala Asp Pro Arg Gly Gln Asp Leu Arg Asp Val Glu
785                 790                 795                 800

Tyr Trp Ala Gly Asn Met Val Asn Arg Val Arg Leu Thr Ser Ala Val
                805                 810                 815

Lys Ala Ala Val Glu Asp Gly Tyr Arg Leu Phe Leu Glu Val Ser Thr
            820                 825                 830

His Pro Val Val Ser His Ser Ile Asn Glu Thr Leu Met Asp Ala Gly
        835                 840                 845

Met Glu Asp Phe Ala Val Ile Pro Thr Leu Leu Arg Lys Lys Pro Thr
    850                 855                 860

Glu Lys His Ile Leu His Ser Ile Ala Gln Leu His Cys Arg Gly Ala
865                 870                 875                 880

Glu Val Asn Trp Ala Ala Gln Met Pro Gly Arg Trp Ala Thr Gly Val
                885                 890                 895

Pro Thr Thr Thr Trp Met His Lys Pro Ile Trp Arg Lys Ile Glu Thr
            900                 905                 910

Ala Pro Leu His Thr Gly Leu Thr His Asp Val Glu Lys His Thr Leu
        915                 920                 925

Leu Gly Gln Arg Ile Pro Val Pro Gly Thr Asp Thr Tyr Val Tyr Thr
    930                 935                 940

Thr Arg Leu Asp Asn Asp Thr Lys Pro Phe Pro Gly Ser His Pro Leu
945                 950                 955                 960

His Gly Thr Glu Ile Val Pro Ala Ala Gly Leu Ile Asn Thr Phe Leu
                965                 970                 975

Lys Gly Thr Gly Gly Gln Met Leu Gln Asn Val Val Leu Arg Val Pro
            980                 985                 990

Val Ala Ile Asn Ala Pro Arg Ser  Val Gln Val Val Val  Gln Gln Asp
        995                 1000                1005

Gln Val  Lys Val Val Ser Arg  Leu Ile Pro Ser Glu  Pro Ser Gln
    1010                1015                1020

Leu Asp  Asp Asp Ala Ser Trp  Val Thr His Thr Thr  Ala Tyr Trp
    1025                1030                1035

Asp Arg  Lys Val Ala Gly Ser  Glu Asp Arg Ile Asp  Phe Ala Ala
    1040                1045                1050

Val Lys  Ser Arg Leu Val Thr  Lys Leu Ala Asp Asn  Phe Ser Ile
    1055                1060                1065

Asp Tyr  Leu Asp Lys Val Gly  Val Ser Ala Met Gly  Phe Pro Trp
    1070                1075                1080

Ala Val  Thr Glu His Tyr Arg  Asn Asp Lys Glu Met  Leu Ala Arg
    1085                1090                1095

Val Asp  Val Asn Pro Ala Ile  Ser Gly Asp Ala Pro  Leu Pro Trp
    1100                1105                1110

Asp Ser Ser Ser Trp Ala Pro Val Leu Asp Ala Ala Thr Ser Val
    1115                1120                1125

Gly Ser Thr Ile Phe Pro Thr Pro Ala Leu Arg Met Pro Ala Gln
    1130                1135                1140

Ile Glu Arg Val Glu Val Phe Thr Ser Gln Asp Pro Pro Lys Ile
    1145                1150                1155

Ser Trp Leu Tyr Val Gln Glu Ala Ser Asp Ser Val Pro Thr Ser
    1160                1165                1170

His Val Ser Val Val Ser Glu Ala Gly Glu Val Leu Ala Lys Phe
    1175                1180                1185

Thr Ala Met Arg Phe Ser Glu Ile Glu Gly Thr Pro Gly Val Ser
    1190                1195                1200

Gly Ser Met Glu Ser Leu Val His Gln Ile Ala Trp Pro Pro Ala
    1205                1210                1215

Thr Pro Ala Glu Glu Pro Leu Ser Ile Glu Thr Val Ile Leu Val
    1220                1225                1230

Ser Pro Asp Ala Thr Thr Arg Ala Leu Tyr Ala Ala Ser Leu Pro
    1235                1240                1245

Thr Arg Val Asn Ser Phe Gln Phe Ser Ser Thr Gln Glu Phe Phe
    1250                1255                1260

Ser Asn Ala Ser Ser Leu Pro Leu Glu Lys Gly Thr Val Val Thr
    1265                1270                1275

Tyr Ile Pro Gly Glu Val Ala Ser Leu Ala Glu Val Pro Ala Ala
    1280                1285                1290

Ser Glu Ser Phe Thr Trp Asn Leu Leu Glu Leu Ile Lys Phe Thr
    1295                1300                1305

Val Asn Gly Ser Leu Pro Ile Lys Val Phe Thr Leu Thr Ala Asn
    1310                1315                1320

Ile Gly Glu Gly Gln Thr Pro Thr Ala Leu Ala Gln Ser Pro Leu
    1325                1330                1335

Tyr Gly Leu Ala Arg Val Ile Ala Ser Glu His Pro Asp Leu Gly
    1340                1345                1350

Thr Leu Ile Asp Val Glu Glu Pro Val Ile Pro Leu Ser Thr Met
    1355                1360                1365

Arg Tyr Ile Gln Gly Ala Asp Ile Ile Arg Ile Asn Asp Gly Ile
    1370                1375                1380

Ala Arg Thr Ser Arg Phe Arg Ser Leu Pro Arg Asn Lys Leu Leu
    1385                1390                1395

Pro Ala Ser Glu Gly Pro Arg Leu Leu Pro Arg Pro Glu Gly Thr
    1400                1405                1410

Tyr Leu Ile Thr Gly Gly Leu Gly Val Leu Gly Leu Glu Val Ala
    1415                1420                1425

Asp Phe Leu Val Glu Lys Gly Ala Arg Arg Leu Leu Leu Ile Ser
    1430                1435                1440

Arg Arg Ala Leu Pro Pro Arg Arg Thr Trp Asp Gln Val Ser Glu
    1445                1450                1455

Asp Leu Gln Pro Thr Ile Ala Lys Ile Arg Leu Leu Glu Ser Arg
    1460                1465                1470

Gly Ala Ser Val His Val Leu Pro Leu Asp Ile Thr Lys Pro Asp
    1475                1480                1485

Ala Val Glu Gln Leu Thr Thr Ala Leu Asp Arg Leu Ser Leu Pro
    1490                1495                1500

Ser Val Gln Gly Val Val His Ala Ala Gly Val Leu Asp Asn Glu
    1505                1510                1515

Leu Val Met Gln Thr Thr Arg Asp Ala Phe Asn Arg Val Leu Ala
    1520                1525                1530

Pro Lys Ile Ala Gly Ala Leu Ala Leu His Glu Val Phe Pro Pro
    1535                1540                1545

Lys Ser Val Asp Phe Phe Val Met Phe Ser Ser Cys Gly Asn Leu
    1550                1555                1560

Val Gly Phe Thr Gly Gln Ala Ser Tyr Gly Ser Gly Asn Ala Phe
    1565                1570                1575

Leu Asp Thr Leu Ala Thr His Arg Ala Arg Leu Gly Asp Ala Ala

1580                1585                1590

Val Ser Phe Gln Trp Thr Ser Trp Arg Gly Leu Gly Met Gly Ala
    1595                1600                1605

Ser Thr Asp Phe Ile Asn Ala Glu Leu Glu Ser Lys Gly Ile Thr
    1610                1615                1620

Asp Val Thr Arg Asp Glu Ala Phe Ala Ala Trp Gln His Leu Ala
    1625                1630                1635

Lys Tyr Asp Met Asp His Gly Val Val Leu Arg Ser Arg Ala Phe
    1640                1645                1650

Glu Asp Gly Glu Pro Ile Pro Val Ser Ile Leu Asn Asp Ile Ala
    1655                1660                1665

Val Arg Arg Val Gly Thr Val Ser Asn Thr Ser Pro Ala Ala Ala
    1670                1675                1680

Gly Ser Ser Asp Ala Val Pro Thr Ser Gly Pro Glu Leu Lys Ala
    1685                1690                1695

Tyr Leu Asp Glu Lys Ile Arg Gly Cys Val Ala Lys Val Leu Gln
    1700                1705                1710

Met Thr Ala Glu Asp Val Asp Ser Lys Ala Ala Leu Ala Asp Leu
    1715                1720                1725

Gly Val Asp Ser Val Met Thr Val Thr Leu Arg Arg Gln Leu Gln
    1730                1735                1740

Leu Thr Leu Lys Ile Ala Val Pro Pro Thr Leu Thr Trp Ser His
    1745                1750                1755

Pro Thr Val Ser His Leu Ala Val Trp Phe Ala Glu Lys Leu Ala
    1760                1765                1770

Lys

<210>  124
<211>  224
<212>  PRT
<213>  Bacillus subtilis

<400>  124

Met Lys Ile Tyr Gly Ile Tyr Met Asp Arg Pro Leu Ser Gln Glu Glu

1               5                   10                  15

Asn Glu Arg Phe Met Ser Phe Ile Ser Pro Glu Lys Arg Glu Lys Cys
            20                  25                  30

Arg Arg Phe Tyr His Lys Glu Asp Ala His Arg Thr Leu Leu Gly Asp
        35                  40                  45

Val Leu Val Arg Ser Val Ile Ser Arg Gln Tyr Gln Leu Asp Lys Ser
    50                  55                  60

Asp Ile Arg Phe Ser Thr Gln Glu Tyr Gly Lys Pro Cys Ile Pro Asp
65                  70                  75                  80

Leu Pro Asp Ala His Phe Asn Ile Ser His Ser Gly Arg Trp Val Ile
                85                  90                  95

Cys Ala Phe Asp Ser Gln Pro Ile Gly Ile Asp Ile Glu Lys Thr Lys
            100                 105                 110

Pro Ile Ser Leu Glu Ile Ala Lys Arg Phe Phe Ser Lys Thr Glu Tyr
        115                 120                 125

Ser Asp Leu Leu Ala Lys Asp Lys Asp Glu Gln Thr Asp Tyr Phe Tyr
    130                 135                 140

His Leu Trp Ser Met Lys Glu Ser Phe Ile Lys Gln Glu Gly Lys Gly
145                 150                 155                 160

Leu Ser Leu Pro Leu Asp Ser Phe Ser Val Arg Leu His Gln Asp Gly
                165                 170                 175

Gln Val Ser Ile Glu Leu Pro Asp Ser His Ser Pro Cys Tyr Ile Lys
            180                 185                 190

Thr Tyr Glu Val Asp Pro Gly Tyr Lys Met Ala Val Cys Ala Val His
        195                 200                 205

Pro Asp Phe Pro Glu Asp Ile Thr Met Val Ser Tyr Glu Glu Leu Leu
    210                 215                 220

<210>  125
<211>  391
<212>  PRT
<213>  Unknown

<220>
<223>  Rheum palmatum

<400>  125

Met Ala Asp Val Leu Gln Glu Ile Arg Asn Ser Gln Lys Ala Ser Gly
1               5                   10                  15

Pro Ala Thr Val Leu Ala Ile Gly Thr Ala His Pro Pro Thr Cys Tyr
            20                  25                  30

Pro Gln Ala Asp Tyr Pro Asp Phe Tyr Phe Arg Val Cys Lys Ser Glu
        35                  40                  45

His Met Thr Lys Leu Lys Lys Lys Met Gln Phe Ile Cys Asp Arg Ser
    50                  55                  60

Gly Ile Arg Gln Arg Phe Met Phe His Thr Glu Glu Asn Leu Gly Lys
65                  70                  75                  80

Asn Pro Gly Met Cys Thr Phe Asp Gly Pro Ser Leu Asn Ala Arg Gln
                85                  90                  95

Asp Met Leu Ile Met Glu Val Pro Lys Leu Gly Ala Glu Ala Ala Glu
            100                 105                 110

Lys Ala Ile Lys Glu Trp Gly Gln Asp Lys Ser Arg Ile Thr His Leu
        115                 120                 125

Ile Phe Cys Thr Thr Thr Ser Asn Asp Met Pro Gly Ala Asp Tyr Gln
    130                 135                 140

Phe Ala Thr Leu Phe Gly Leu Asn Pro Gly Val Ser Arg Thr Met Val
145                 150                 155                 160

Tyr Gln Gln Gly Cys Phe Ala Gly Gly Thr Val Leu Arg Leu Val Lys
                165                 170                 175

Asp Ile Ala Glu Asn Asn Lys Gly Ala Arg Val Leu Val Val Cys Ser
            180                 185                 190

Glu Ile Val Ala Phe Ala Phe Arg Gly Pro His Glu Asp His Ile Asp
        195                 200                 205

Ser Leu Ile Gly Gln Leu Leu Phe Gly Asp Gly Ala Ala Ala Leu Val
    210                 215                 220

Val Gly Thr Asp Ile Asp Glu Ser Val Glu Arg Pro Ile Phe Gln Ile
225                 230                 235                 240

Met Ser Ala Thr Gln Ala Thr Ile Pro Asn Ser Leu His Thr Met Ala

245                 250                 255

Leu His Leu Thr Glu Ala Gly Leu Thr Phe His Leu Ser Lys Glu Val
            260                 265                 270

Pro Lys Val Val Ser Asp Asn Met Glu Glu Leu Met Leu Glu Ala Phe
        275                 280                 285

Lys Pro Leu Gly Ile Thr Asp Trp Asn Ser Ile Phe Trp Gln Val His
    290                 295                 300

Pro Gly Gly Arg Ala Ile Leu Asp Lys Ile Glu Glu Lys Leu Glu Leu
305                 310                 315                 320

Thr Lys Asp Lys Met Arg Asp Ser Arg Tyr Ile Leu Ser Glu Tyr Gly
                325                 330                 335

Asn Leu Thr Ser Ala Cys Val Leu Phe Val Met Asp Glu Met Arg Lys
            340                 345                 350

Arg Ser Phe Arg Glu Gly Lys Gln Thr Thr Gly Asp Gly Tyr Glu Trp
        355                 360                 365

Gly Val Ala Ile Gly Leu Gly Pro Gly Leu Thr Val Glu Thr Val Val
    370                 375                 380

Leu Arg Ser Val Pro Ile Pro
385                 390

<210>  126
<211>  403
<212>  PRT
<213>  Aloe arborescens

<400>  126

Met Gly Ser Leu Ser Asp Ser Thr Pro Leu Met Lys Asp Val Gln Gly
1               5                   10                  15

Ile Arg Lys Ala Gln Lys Ala Asp Gly Thr Ala Thr Val Met Ala Ile
            20                  25                  30

Gly Thr Ala His Pro Pro His Ile Ile Ser Gln Asp Ser Tyr Ala Asp
        35                  40                  45

Phe Tyr Phe Arg Val Thr Asn Ser Glu His Lys Val Glu Leu Lys Lys
    50                  55                  60

Lys Phe Asp Arg Ile Cys Lys Lys Thr Met Ile Gly Lys Arg Tyr Phe

65                  70                  75                  80

Asn Phe Asp Glu Glu Phe Leu Lys Lys Tyr Pro Asn Ile Thr Ser Phe
                85                  90                  95

Asp Lys Pro Ser Leu Asn Asp Arg His Asp Ile Cys Ile Pro Gly Val
            100                 105                 110

Pro Ala Leu Gly Ala Glu Ala Ala Val Lys Ala Ile Glu Glu Trp Gly
        115                 120                 125

Arg Pro Lys Ser Glu Ile Thr His Leu Val Phe Cys Thr Ser Gly Gly
    130                 135                 140

Val Asp Met Pro Ser Ala Asp Phe Gln Cys Ala Lys Leu Leu Gly Leu
145                 150                 155                 160

Arg Thr Asn Val Asn Lys Tyr Cys Ile Tyr Met Gln Gly Cys Tyr Ala
                165                 170                 175

Gly Gly Thr Val Met Arg Tyr Ala Lys Asp Leu Ala Glu Asn Asn Arg
            180                 185                 190

Gly Ala Arg Val Leu Met Val Cys Ala Glu Leu Thr Ile Ile Ala Leu
        195                 200                 205

Arg Gly Pro Asn Asp Ser His Ile Asp Asn Ala Ile Gly Asn Ser Leu
    210                 215                 220

Phe Gly Asp Gly Ala Ala Ala Leu Ile Val Gly Ser Asp Pro Ile Ile
225                 230                 235                 240

Gly Val Glu Lys Pro Met Phe Glu Ile Val Cys Ala Lys Gln Thr Val
                245                 250                 255

Ile Pro Asn Ser Glu Glu Val Ile His Leu His Leu Arg Glu Ser Gly
            260                 265                 270

Leu Met Phe Tyr Met Thr Lys Asp Ser Ala Ala Thr Ile Ser Asn Asn
        275                 280                 285

Ile Glu Ala Cys Leu Val Asp Val Phe Lys Ser Val Gly Met Thr Pro
    290                 295                 300

Pro Glu Asp Trp Asn Ser Leu Phe Trp Ile Pro His Pro Gly Gly Arg
305                 310                 315                 320

Ala Ile Leu Asp Gln Val Glu Ala Lys Leu Lys Leu Arg Pro Glu Lys
                325                 330                 335

Phe Ser Ala Thr Arg Thr Val Leu Trp Asp Tyr Gly Asn Met Ile Ser
            340                 345                 350

Ala Cys Val Leu Tyr Ile Leu Asp Glu Met Arg Arg Lys Ser Ala Ala
        355                 360                 365

Glu Gly Leu Glu Thr Tyr Gly Glu Gly Leu Glu Trp Gly Val Leu Leu
    370                 375                 380

Gly Phe Gly Pro Gly Met Thr Ile Glu Thr Ile Leu Leu His Ser Leu
385                 390                 395                 400

Pro Pro Val

<210>  127
<211>  510
<212>  PRT
<213>  Unknown

<220>
<223>  Saccharothrix espanaensis

<400>  127

Met Thr Gln Val Val Glu Arg Gln Ala Asp Arg Leu Ser Ser Arg Glu
1               5                   10                  15

Tyr Leu Ala Arg Val Val Arg Ser Ala Gly Trp Asp Ala Gly Leu Thr
            20                  25                  30

Ser Cys Thr Asp Glu Glu Ile Val Arg Met Gly Ala Ser Ala Arg Thr
        35                  40                  45

Ile Glu Glu Tyr Leu Lys Ser Asp Lys Pro Ile Tyr Gly Leu Thr Gln
    50                  55                  60

Gly Phe Gly Pro Leu Val Leu Phe Asp Ala Asp Ser Glu Leu Glu Gln
65                  70                  75                  80

Gly Gly Ser Leu Ile Ser His Leu Gly Thr Gly Gln Gly Ala Pro Leu
                85                  90                  95

Ala Pro Glu Val Ser Arg Leu Ile Leu Trp Leu Arg Ile Gln Asn Met
            100                 105                 110

Arg Lys Gly Tyr Ser Ala Val Ser Pro Val Phe Trp Gln Lys Leu Ala

115                 120                 125

Asp Leu Trp Asn Lys Gly Phe Thr Pro Ala Ile Pro Arg His Gly Thr
    130                 135                 140

Val Ser Ala Ser Gly Asp Leu Gln Pro Leu Ala His Ala Ala Leu Ala
145                 150                 155                 160

Phe Thr Gly Val Gly Glu Ala Trp Thr Arg Asp Ala Asp Gly Arg Trp
                165                 170                 175

Ser Thr Val Pro Ala Val Asp Ala Leu Ala Ala Leu Gly Ala Glu Pro
            180                 185                 190

Phe Asp Trp Pro Val Arg Glu Ala Leu Ala Phe Val Asn Gly Thr Gly
        195                 200                 205

Ala Ser Leu Ala Val Ala Val Leu Asn His Arg Ser Ala Leu Arg Leu
    210                 215                 220

Val Arg Ala Cys Ala Val Leu Ser Ala Arg Leu Ala Thr Leu Leu Gly
225                 230                 235                 240

Ala Asn Pro Glu His Tyr Asp Val Gly His Gly Val Ala Arg Gly Gln
                245                 250                 255

Val Gly Gln Leu Thr Ala Ala Glu Trp Ile Arg Gln Gly Leu Pro Arg
            260                 265                 270

Gly Met Val Arg Asp Gly Ser Arg Pro Leu Gln Glu Pro Tyr Ser Leu
        275                 280                 285

Arg Cys Ala Pro Gln Val Leu Gly Ala Val Leu Asp Gln Leu Asp Gly
    290                 295                 300

Ala Gly Asp Val Leu Ala Arg Glu Val Asp Gly Cys Gln Asp Asn Pro
305                 310                 315                 320

Ile Thr Tyr Glu Gly Glu Leu Leu His Gly Gly Asn Phe His Ala Met
                325                 330                 335

Pro Val Gly Phe Ala Ser Asp Gln Ile Gly Leu Ala Met His Met Ala
            340                 345                 350

Ala Tyr Leu Ala Glu Arg Gln Leu Gly Leu Leu Val Ser Pro Val Thr
        355                 360                 365

Asn Gly Asp Leu Pro Pro Met Leu Thr Pro Arg Ala Gly Arg Gly Ala
    370                 375                 380

Gly Leu Ala Gly Val Gln Ile Ser Ala Thr Ser Phe Val Ser Arg Ile
385                 390                 395                 400

Arg Gln Leu Val Phe Pro Ala Ser Leu Thr Thr Leu Pro Thr Asn Gly
                405                 410                 415

Trp Asn Gln Asp His Val Pro Met Ala Leu Asn Gly Ala Asn Ser Val
            420                 425                 430

Phe Glu Ala Leu Glu Leu Gly Trp Leu Thr Val Gly Ser Leu Ala Val
        435                 440                 445

Gly Val Ala Gln Leu Ala Ala Met Thr Gly His Ala Ala Glu Gly Val
    450                 455                 460

Trp Ala Glu Leu Ala Gly Ile Cys Pro Pro Leu Asp Ala Asp Arg Pro
465                 470                 475                 480

Leu Gly Ala Glu Val Arg Ala Ala Arg Asp Leu Leu Ser Ala His Ala
                485                 490                 495

Asp Gln Leu Leu Val Asp Glu Ala Asp Gly Lys Asp Phe Gly
            500                 505                 510

<210>  128
<211>  561
<212>  PRT
<213>  Arabidopsis thaliana

<400>  128

Met Ala Pro Gln Glu Gln Ala Val Ser Gln Val Met Glu Lys Gln Ser
1               5                   10                  15

Asn Asn Asn Asn Ser Asp Val Ile Phe Arg Ser Lys Leu Pro Asp Ile
            20                  25                  30

Tyr Ile Pro Asn His Leu Ser Leu His Asp Tyr Ile Phe Gln Asn Ile
        35                  40                  45

Ser Glu Phe Ala Thr Lys Pro Cys Leu Ile Asn Gly Pro Thr Gly His
    50                  55                  60

Val Tyr Thr Tyr Ser Asp Val His Val Ile Ser Arg Gln Ile Ala Ala
65                  70                  75                  80

Asn Phe His Lys Leu Gly Val Asn Gln Asn Asp Val Val Met Leu Leu
                85                  90                  95

Leu Pro Asn Cys Pro Glu Phe Val Leu Ser Phe Leu Ala Ala Ser Phe
            100                 105                 110

Arg Gly Ala Thr Ala Thr Ala Ala Asn Pro Phe Phe Thr Pro Ala Glu
        115                 120                 125

Ile Ala Lys Gln Ala Lys Ala Ser Asn Thr Lys Leu Ile Ile Thr Glu
    130                 135                 140

Ala Arg Tyr Val Asp Lys Ile Lys Pro Leu Gln Asn Asp Asp Gly Val
145                 150                 155                 160

Val Ile Val Cys Ile Asp Asp Asn Glu Ser Val Pro Ile Pro Glu Gly
                165                 170                 175

Cys Leu Arg Phe Thr Glu Leu Thr Gln Ser Thr Thr Glu Ala Ser Glu
            180                 185                 190

Val Ile Asp Ser Val Glu Ile Ser Pro Asp Asp Val Val Ala Leu Pro
        195                 200                 205

Tyr Ser Ser Gly Thr Thr Gly Leu Pro Lys Gly Val Met Leu Thr His
    210                 215                 220

Lys Gly Leu Val Thr Ser Val Ala Gln Gln Val Asp Gly Glu Asn Pro
225                 230                 235                 240

Asn Leu Tyr Phe His Ser Asp Asp Val Ile Leu Cys Val Leu Pro Met
                245                 250                 255

Phe His Ile Tyr Ala Leu Asn Ser Ile Met Leu Cys Gly Leu Arg Val
            260                 265                 270

Gly Ala Ala Ile Leu Ile Met Pro Lys Phe Glu Ile Asn Leu Leu Leu
        275                 280                 285

Glu Leu Ile Gln Arg Cys Lys Val Thr Val Ala Pro Met Val Pro Pro
    290                 295                 300

Ile Val Leu Ala Ile Ala Lys Ser Ser Glu Thr Glu Lys Tyr Asp Leu
305                 310                 315                 320

Ser Ser Ile Arg Val Val Lys Ser Gly Ala Ala Pro Leu Gly Lys Glu
                325                 330                 335

Leu Glu Asp Ala Val Asn Ala Lys Phe Pro Asn Ala Lys Leu Gly Gln
            340                 345                 350

Gly Tyr Gly Met Thr Glu Ala Gly Pro Val Leu Ala Met Ser Leu Gly
        355                 360                 365

Phe Ala Lys Glu Pro Phe Pro Val Lys Ser Gly Ala Cys Gly Thr Val
    370                 375                 380

Val Arg Asn Ala Glu Met Lys Ile Val Asp Pro Asp Thr Gly Asp Ser
385                 390                 395                 400

Leu Ser Arg Asn Gln Pro Gly Glu Ile Cys Ile Arg Gly His Gln Ile
                405                 410                 415

Met Lys Gly Tyr Leu Asn Asn Pro Ala Ala Thr Ala Glu Thr Ile Asp
            420                 425                 430

Lys Asp Gly Trp Leu His Thr Gly Asp Ile Gly Leu Ile Asp Asp Asp
        435                 440                 445

Asp Glu Leu Phe Ile Val Asp Arg Leu Lys Glu Leu Ile Lys Tyr Lys
    450                 455                 460

Gly Phe Gln Val Ala Pro Ala Glu Leu Glu Ala Leu Leu Ile Gly His
465                 470                 475                 480

Pro Asp Ile Thr Asp Val Ala Val Val Ala Met Lys Glu Glu Ala Ala
                485                 490                 495

Gly Glu Val Pro Val Ala Phe Val Val Lys Ser Lys Asp Ser Glu Leu
            500                 505                 510

Ser Glu Asp Asp Val Lys Gln Phe Val Ser Lys Gln Val Val Phe Tyr
        515                 520                 525

Lys Arg Ile Asn Lys Val Phe Phe Thr Glu Ser Ile Pro Lys Ala Pro
    530                 535                 540

Ser Gly Lys Ile Leu Arg Lys Asp Leu Arg Ala Lys Leu Ala Asn Gly
545                 550                 555                 560

Leu

<210>  129
<211>  561

<212>  PRT
<213>  Arabidopsis thaliana

<400>  129

Met Ile Thr Ala Ala Leu His Glu Pro Gln Ile His Lys Pro Thr Asp
1               5                   10                  15

Thr Ser Val Val Ser Asp Asp Val Leu Pro His Ser Pro Pro Thr Pro
            20                  25                  30

Arg Ile Phe Arg Ser Lys Leu Pro Asp Ile Asp Ile Pro Asn His Leu
        35                  40                  45

Pro Leu His Thr Tyr Cys Phe Glu Lys Leu Ser Ser Val Ser Asp Lys
    50                  55                  60

Pro Cys Leu Ile Val Gly Ser Thr Gly Lys Ser Tyr Thr Tyr Gly Glu
65                  70                  75                  80

Thr His Leu Ile Cys Arg Arg Val Ala Ser Gly Leu Tyr Lys Leu Gly
                85                  90                  95

Ile Arg Lys Gly Asp Val Ile Met Ile Leu Leu Gln Asn Ser Ala Glu
            100                 105                 110

Phe Val Phe Ser Phe Met Gly Ala Ser Met Ile Gly Ala Val Ser Thr
        115                 120                 125

Thr Ala Asn Pro Phe Tyr Thr Ser Gln Glu Leu Tyr Lys Gln Leu Lys
    130                 135                 140

Ser Ser Gly Ala Lys Leu Ile Ile Thr His Ser Gln Tyr Val Asp Lys
145                 150                 155                 160

Leu Lys Asn Leu Gly Glu Asn Leu Thr Leu Ile Thr Thr Asp Glu Pro
                165                 170                 175

Thr Pro Glu Asn Cys Leu Pro Phe Ser Thr Leu Ile Thr Asp Asp Glu
            180                 185                 190

Thr Asn Pro Phe Gln Glu Thr Val Asp Ile Gly Gly Asp Asp Ala Ala
        195                 200                 205

Ala Leu Pro Phe Ser Ser Gly Thr Thr Gly Leu Pro Lys Gly Val Val
    210                 215                 220

Leu Thr His Lys Ser Leu Ile Thr Ser Val Ala Gln Gln Val Asp Gly
225                 230                 235                 240

Asp Asn Pro Asn Leu Tyr Leu Lys Ser Asn Asp Val Ile Leu Cys Val
                245                 250                 255

Leu Pro Leu Phe His Ile Tyr Ser Leu Asn Ser Val Leu Leu Asn Ser
            260                 265                 270

Leu Arg Ser Gly Ala Thr Val Leu Leu Met His Lys Phe Glu Ile Gly
        275                 280                 285

Ala Leu Leu Asp Leu Ile Gln Arg His Arg Val Thr Ile Ala Ala Leu
    290                 295                 300

Val Pro Pro Leu Val Ile Ala Leu Ala Lys Asn Pro Thr Val Asn Ser
305                 310                 315                 320

Tyr Asp Leu Ser Ser Val Arg Phe Val Leu Ser Gly Ala Ala Pro Leu
                325                 330                 335

Gly Lys Glu Leu Gln Asp Ser Leu Arg Arg Arg Leu Pro Gln Ala Ile
            340                 345                 350

Leu Gly Gln Gly Tyr Gly Met Thr Glu Ala Gly Pro Val Leu Ser Met
        355                 360                 365

Ser Leu Gly Phe Ala Lys Glu Pro Ile Pro Thr Lys Ser Gly Ser Cys
    370                 375                 380

Gly Thr Val Val Arg Asn Ala Glu Leu Lys Val Val His Leu Glu Thr
385                 390                 395                 400

Arg Leu Ser Leu Gly Tyr Asn Gln Pro Gly Glu Ile Cys Ile Arg Gly
                405                 410                 415

Gln Gln Ile Met Lys Glu Tyr Leu Asn Asp Pro Glu Ala Thr Ser Ala
            420                 425                 430

Thr Ile Asp Glu Glu Gly Trp Leu His Thr Gly Asp Ile Gly Tyr Val
        435                 440                 445

Asp Glu Asp Asp Glu Ile Phe Ile Val Asp Arg Leu Lys Glu Val Ile
    450                 455                 460

Lys Phe Lys Gly Phe Gln Val Pro Pro Ala Glu Leu Glu Ser Leu Leu
465                 470                 475                 480

Ile Asn His His Ser Ile Ala Asp Ala Ala Val Val Pro Gln Asn Asp

485                 490                 495

Glu Val Ala Gly Glu Val Pro Val Ala Phe Val Val Arg Ser Asn Gly
            500                 505                 510

Asn Asp Ile Thr Glu Glu Asp Val Lys Glu Tyr Val Ala Lys Gln Val
        515                 520                 525

Val Phe Tyr Lys Arg Leu His Lys Val Phe Phe Val Ala Ser Ile Pro
    530                 535                 540

Lys Ser Pro Ser Gly Lys Ile Leu Arg Lys Asp Leu Lys Ala Lys Leu
545                 550                 555                 560

Cys

<210>  130
<211>  570
<212>  PRT
<213>  Arabidopsis thaliana

<400>  130

Met Val Leu Gln Gln Gln Thr His Phe Leu Thr Lys Lys Ile Asp Gln
1               5                   10                  15

Glu Asp Glu Glu Glu Glu Pro Ser His Asp Phe Ile Phe Arg Ser Lys
            20                  25                  30

Leu Pro Asp Ile Phe Ile Pro Asn His Leu Pro Leu Thr Asp Tyr Val
        35                  40                  45

Phe Gln Arg Phe Ser Gly Asp Gly Asp Gly Asp Ser Ser Thr Thr Cys
    50                  55                  60

Ile Ile Asp Gly Ala Thr Gly Arg Ile Leu Thr Tyr Ala Asp Val Gln
65                  70                  75                  80

Thr Asn Met Arg Arg Ile Ala Ala Gly Ile His Arg Leu Gly Ile Arg
                85                  90                  95

His Gly Asp Val Val Met Leu Leu Leu Pro Asn Ser Pro Glu Phe Ala
            100                 105                 110

Leu Ser Phe Leu Ala Val Ala Tyr Leu Gly Ala Val Ser Thr Thr Ala
        115                 120                 125

Asn Pro Phe Tyr Thr Gln Pro Glu Ile Ala Lys Gln Ala Lys Ala Ser

130                 135                 140

Ala Ala Lys Met Ile Ile Thr Lys Lys Cys Leu Val Asp Lys Leu Thr
145                 150                 155                 160

Asn Leu Lys Asn Asp Gly Val Leu Ile Val Cys Leu Asp Asp Asp Gly
                165                 170                 175

Asp Asn Gly Val Val Ser Ser Ser Asp Asp Gly Cys Val Ser Phe Thr
            180                 185                 190

Glu Leu Thr Gln Ala Asp Glu Thr Glu Leu Leu Lys Pro Lys Ile Ser
        195                 200                 205

Pro Glu Asp Thr Val Ala Met Pro Tyr Ser Ser Gly Thr Thr Gly Leu
    210                 215                 220

Pro Lys Gly Val Met Ile Thr His Lys Gly Leu Val Thr Ser Ile Ala
225                 230                 235                 240

Gln Lys Val Asp Gly Glu Asn Pro Asn Leu Asn Phe Thr Ala Asn Asp
                245                 250                 255

Val Ile Leu Cys Phe Leu Pro Met Phe His Ile Tyr Ala Leu Asp Ala
            260                 265                 270

Leu Met Leu Ser Ala Met Arg Thr Gly Ala Ala Leu Leu Ile Val Pro
        275                 280                 285

Arg Phe Glu Leu Asn Leu Val Met Glu Leu Ile Gln Arg Tyr Lys Val
    290                 295                 300

Thr Val Val Pro Val Ala Pro Pro Val Val Leu Ala Phe Ile Lys Ser
305                 310                 315                 320

Pro Glu Thr Glu Arg Tyr Asp Leu Ser Ser Val Arg Ile Met Leu Ser
                325                 330                 335

Gly Ala Ala Thr Leu Lys Lys Glu Leu Glu Asp Ala Val Arg Leu Lys
            340                 345                 350

Phe Pro Asn Ala Ile Phe Gly Gln Gly Tyr Gly Met Thr Glu Ser Gly
        355                 360                 365

Thr Val Ala Lys Ser Leu Ala Phe Ala Lys Asn Pro Phe Lys Thr Lys
    370                 375                 380

Ser Gly Ala Cys Gly Thr Val Ile Arg Asn Ala Glu Met Lys Val Val
385                 390                 395                 400

Asp Thr Glu Thr Gly Ile Ser Leu Pro Arg Asn Lys Ser Gly Glu Ile
                405                 410                 415

Cys Val Arg Gly His Gln Leu Met Lys Gly Tyr Leu Asn Asp Pro Glu
            420                 425                 430

Ala Thr Ala Arg Thr Ile Asp Lys Asp Gly Trp Leu His Thr Gly Asp
        435                 440                 445

Ile Gly Phe Val Asp Asp Asp Asp Glu Ile Phe Ile Val Asp Arg Leu
    450                 455                 460

Lys Glu Leu Ile Lys Phe Lys Gly Tyr Gln Val Ala Pro Ala Glu Leu
465                 470                 475                 480

Glu Ala Leu Leu Ile Ser His Pro Ser Ile Asp Asp Ala Ala Val Val
                485                 490                 495

Ala Met Lys Asp Glu Val Ala Asp Glu Val Pro Val Ala Phe Val Ala
            500                 505                 510

Arg Ser Gln Gly Ser Gln Leu Thr Glu Asp Asp Val Lys Ser Tyr Val
        515                 520                 525

Asn Lys Gln Val Val His Tyr Lys Arg Ile Lys Met Val Phe Phe Ile
    530                 535                 540

Glu Val Ile Pro Lys Ala Val Ser Gly Lys Ile Leu Arg Lys Asp Leu
545                 550                 555                 560

Arg Ala Lys Leu Glu Thr Met Cys Ser Lys
                565                 570

<210>  131
<211>  522
<212>  PRT
<213>  Streptomyces coelicolor

<400>  131

Met Phe Arg Ser Glu Tyr Ala Asp Val Pro Pro Val Asp Leu Pro Ile
1               5                   10                  15

His Asp Ala Val Leu Gly Gly Ala Ala Ala Phe Gly Ser Thr Pro Ala
            20                  25                  30

Leu Ile Asp Gly Thr Asp Gly Thr Thr Leu Thr Tyr Glu Gln Val Asp
        35                  40                  45

Arg Phe His Arg Arg Val Ala Ala Ala Leu Ala Glu Thr Gly Val Arg
    50                  55                  60

Lys Gly Asp Val Leu Ala Leu His Ser Pro Asn Thr Val Ala Phe Pro
65                  70                  75                  80

Leu Ala Phe Tyr Ala Ala Thr Arg Ala Gly Ala Ser Val Thr Thr Val
                85                  90                  95

His Pro Leu Ala Thr Ala Glu Glu Phe Ala Lys Gln Leu Lys Asp Ser
            100                 105                 110

Ala Ala Arg Trp Ile Val Thr Val Ser Pro Leu Leu Ser Thr Ala Arg
        115                 120                 125

Arg Ala Ala Glu Leu Ala Gly Gly Val Gln Glu Ile Leu Val Cys Asp
    130                 135                 140

Ser Ala Pro Gly His Arg Ser Leu Val Asp Met Leu Ala Ser Thr Ala
145                 150                 155                 160

Pro Glu Pro Ser Val Ala Ile Asp Pro Ala Glu Asp Val Ala Ala Leu
                165                 170                 175

Pro Tyr Ser Ser Gly Thr Thr Gly Thr Pro Lys Gly Val Met Leu Thr
            180                 185                 190

His Arg Gln Ile Ala Thr Asn Leu Ala Gln Leu Glu Pro Ser Met Pro
        195                 200                 205

Ser Ala Pro Gly Asp Arg Val Leu Ala Val Leu Pro Phe Phe His Ile
    210                 215                 220

Tyr Gly Leu Thr Ala Leu Met Asn Ala Pro Leu Arg Leu Gly Ala Thr
225                 230                 235                 240

Val Val Val Leu Pro Arg Phe Asp Leu Glu Gln Phe Leu Ala Ala Ile
                245                 250                 255

Gln Asn His Arg Ile Thr Ser Leu Tyr Val Ala Pro Pro Ile Val Leu
            260                 265                 270

Ala Leu Ala Lys His Pro Leu Val Ala Asp Tyr Asp Leu Ser Ser Leu
        275                 280                 285

Arg Tyr Ile Val Ser Ala Ala Ala Pro Leu Asp Ala Arg Leu Ala Ala
    290                 295                 300

Ala Cys Ser Gln Arg Leu Gly Leu Pro Pro Val Gly Gln Ala Tyr Gly
305                 310                 315                 320

Met Thr Glu Leu Ser Pro Gly Thr His Val Val Pro Leu Asp Ala Met
                325                 330                 335

Ala Asp Ala Pro Pro Gly Thr Val Gly Arg Leu Ile Ala Gly Thr Glu
            340                 345                 350

Met Arg Ile Val Ser Leu Thr Asp Pro Gly Thr Asp Leu Pro Ala Gly
        355                 360                 365

Glu Ser Gly Glu Ile Leu Ile Arg Gly Pro Gln Ile Met Lys Gly Tyr
    370                 375                 380

Leu Gly Arg Pro Asp Ala Thr Ala Ala Met Ile Asp Glu Glu Gly Trp
385                 390                 395                 400

Leu His Thr Gly Asp Val Gly His Val Asp Ala Asp Gly Trp Leu Phe
                405                 410                 415

Val Val Asp Arg Val Lys Glu Leu Ile Lys Tyr Lys Gly Phe Gln Val
            420                 425                 430

Ala Pro Ala Glu Leu Glu Ala His Leu Leu Thr His Pro Gly Val Ala
        435                 440                 445

Asp Ala Ala Val Val Gly Ala Tyr Asp Asp Asp Gly Asn Glu Val Pro
    450                 455                 460

His Ala Phe Val Val Arg Gln Pro Ala Ala Pro Gly Leu Ala Glu Ser
465                 470                 475                 480

Glu Ile Met Met Tyr Val Ala Glu Arg Val Ala Pro Tyr Lys Arg Val
                485                 490                 495

Arg Arg Val Thr Phe Val Asp Ala Val Pro Arg Ala Ala Ser Gly Lys
            500                 505                 510

Ile Leu Arg Arg Gln Leu Arg Glu Pro Arg
        515                 520

<210>  132
<211>  392

<212>  PRT
<213>  Vitis vinifera

<400>  132

Met Ala Ser Val Glu Glu Phe Arg Asn Ala Gln Arg Ala Lys Gly Pro
1               5                   10                  15

Ala Thr Ile Leu Ala Ile Gly Thr Ala Thr Pro Asp His Cys Val Tyr
            20                  25                  30

Gln Ser Asp Tyr Ala Asp Phe Tyr Phe Arg Val Thr Lys Ser Glu His
        35                  40                  45

Met Thr Ala Leu Lys Lys Lys Phe Asn Arg Ile Cys Asp Lys Ser Met
    50                  55                  60

Ile Lys Lys Arg Tyr Ile His Leu Thr Glu Glu Met Leu Glu Glu His
65                  70                  75                  80

Pro Asn Ile Gly Ala Tyr Met Ala Pro Ser Leu Asn Ile Arg Gln Glu
                85                  90                  95

Ile Ile Thr Ala Glu Val Pro Lys Leu Gly Lys Glu Ala Ala Leu Lys
            100                 105                 110

Ala Leu Lys Glu Trp Gly Gln Pro Lys Ser Lys Ile Thr His Leu Val
        115                 120                 125

Phe Cys Thr Thr Ser Gly Val Glu Met Pro Gly Ala Asp Tyr Lys Leu
    130                 135                 140

Ala Asn Leu Leu Gly Leu Glu Pro Ser Val Arg Arg Val Met Leu Tyr
145                 150                 155                 160

His Gln Gly Cys Tyr Ala Gly Gly Thr Val Leu Arg Thr Ala Lys Asp
                165                 170                 175

Leu Ala Glu Asn Asn Ala Gly Ala Arg Val Leu Val Val Cys Ser Glu
            180                 185                 190

Ile Thr Val Val Thr Phe Arg Gly Pro Ser Glu Asp Ala Leu Asp Ser
        195                 200                 205

Leu Val Gly Gln Ala Leu Phe Gly Asp Gly Ser Ala Ala Val Ile Val
    210                 215                 220

Gly Ser Asp Pro Asp Ile Ser Ile Glu Arg Pro Leu Phe Gln Leu Val
225                 230                 235                 240

Ser Ala Ala Gln Thr Phe Ile Pro Asn Ser Ala Gly Ala Ile Ala Gly
                245                 250                 255

Asn Leu Arg Glu Val Gly Leu Thr Phe His Leu Trp Pro Asn Val Pro
            260                 265                 270

Thr Leu Ile Ser Glu Asn Ile Glu Lys Cys Leu Thr Gln Ala Phe Asp
        275                 280                 285

Pro Leu Gly Ile Ser Asp Trp Asn Ser Leu Phe Trp Ile Ala His Pro
    290                 295                 300

Gly Gly Pro Ala Ile Leu Asp Ala Val Glu Ala Lys Leu Asn Leu Asp
305                 310                 315                 320

Lys Lys Lys Leu Glu Ala Thr Arg His Val Leu Ser Glu Tyr Gly Asn
                325                 330                 335

Met Ser Ser Ala Cys Val Leu Phe Ile Leu Asp Glu Met Arg Lys Lys
            340                 345                 350

Ser Leu Lys Gly Glu Arg Ala Thr Thr Gly Glu Gly Leu Asp Trp Gly
        355                 360                 365

Val Leu Phe Gly Phe Gly Pro Gly Leu Thr Ile Glu Thr Val Val Leu
    370                 375                 380

His Ser Ile Pro Met Val Thr Asn
385                 390

<210>  133
<211>  389
<212>  PRT
<213>  Petunia x hybrida

<400>  133

Met Val Thr Val Glu Glu Tyr Arg Lys Ala Gln Arg Ala Glu Gly Pro
1               5                   10                  15

Ala Thr Val Met Ala Ile Gly Thr Ala Thr Pro Thr Asn Cys Val Asp
            20                  25                  30

Gln Ser Thr Tyr Pro Asp Tyr Tyr Phe Arg Ile Thr Asn Ser Glu His
        35                  40                  45

Lys Thr Asp Leu Lys Glu Lys Phe Lys Arg Met Cys Glu Lys Ser Met
    50                  55                  60

Ile Lys Lys Arg Tyr Met His Leu Thr Glu Glu Ile Leu Lys Glu Asn
65                  70                  75                  80

Pro Ser Met Cys Glu Tyr Met Ala Pro Ser Leu Asp Ala Arg Gln Asp
                85                  90                  95

Ile Val Val Val Glu Val Pro Lys Leu Gly Lys Glu Ala Ala Gln Lys
            100                 105                 110

Ala Ile Lys Glu Trp Gly Gln Pro Lys Ser Lys Ile Thr His Leu Val
        115                 120                 125

Phe Cys Thr Thr Ser Gly Val Asp Met Pro Gly Cys Asp Tyr Gln Leu
    130                 135                 140

Thr Lys Leu Leu Gly Leu Arg Pro Ser Val Lys Arg Leu Met Met Tyr
145                 150                 155                 160

Gln Gln Gly Cys Phe Ala Gly Gly Thr Val Leu Arg Leu Ala Lys Asp
                165                 170                 175

Leu Ala Glu Asn Asn Lys Gly Ala Arg Val Leu Val Val Cys Ser Glu
            180                 185                 190

Ile Thr Ala Val Thr Phe Arg Gly Pro Asn Asp Thr His Leu Asp Ser
        195                 200                 205

Leu Val Gly Gln Ala Leu Phe Gly Asp Gly Ala Gly Ala Ile Ile Ile
    210                 215                 220

Gly Ser Asp Pro Ile Pro Gly Val Glu Arg Pro Leu Phe Glu Leu Val
225                 230                 235                 240

Ser Ala Ala Gln Thr Leu Leu Pro Asp Ser His Gly Ala Ile Asp Gly
                245                 250                 255

His Leu Arg Glu Val Gly Leu Thr Phe His Leu Leu Lys Asp Val Pro
            260                 265                 270

Gly Leu Ile Ser Lys Asn Ile Glu Lys Ser Leu Glu Glu Ala Phe Arg
        275                 280                 285

Pro Leu Ser Ile Ser Asp Trp Asn Ser Leu Phe Trp Ile Ala His Pro
    290                 295                 300

Gly Gly Pro Ala Ile Leu Asp Gln Val Glu Ile Lys Leu Gly Leu Lys

305                 310                 315                 320

Pro Glu Lys Leu Lys Ala Thr Arg Asn Val Leu Ser Asn Tyr Gly Asn
                325                 330                 335

Met Ser Ser Ala Cys Val Leu Phe Ile Leu Asp Glu Met Arg Lys Ala
            340                 345                 350

Ser Ala Lys Glu Gly Leu Gly Thr Thr Gly Glu Gly Leu Glu Trp Gly
        355                 360                 365

Val Leu Phe Gly Phe Gly Pro Gly Leu Thr Val Glu Thr Val Val Leu
    370                 375                 380

His Ser Val Ala Thr
385

<210>  134
<211>  391
<212>  PRT
<213>  Arabidopsis thaliana

<400>  134

Met Ala Asp Val Leu Gln Glu Ile Arg Asn Ser Gln Lys Ala Ser Gly
1               5                   10                  15

Pro Ala Thr Val Leu Ala Ile Gly Thr Ala His Pro Pro Thr Cys Tyr
            20                  25                  30

Pro Gln Ala Asp Tyr Pro Asp Phe Tyr Phe Arg Val Cys Lys Ser Glu
        35                  40                  45

His Met Thr Lys Leu Lys Lys Lys Met Gln Phe Ile Cys Asp Arg Ser
    50                  55                  60

Gly Ile Arg Gln Arg Phe Met Phe His Thr Glu Glu Asn Leu Gly Lys
65                  70                  75                  80

Asn Pro Gly Met Cys Thr Phe Asp Gly Pro Ser Leu Asn Ala Arg Gln
                85                  90                  95

Asp Met Leu Ile Met Glu Val Pro Lys Leu Gly Ala Glu Ala Ala Glu
            100                 105                 110

Lys Ala Ile Lys Glu Trp Gly Gln Asp Lys Ser Arg Ile Thr His Leu
        115                 120                 125

Ile Phe Cys Thr Thr Thr Ser Asn Asp Met Pro Gly Ala Asp Tyr Gln

130                 135                 140

Phe Ala Thr Leu Phe Gly Leu Asn Pro Gly Val Ser Arg Thr Met Val
145                 150                 155                 160

Tyr Gln Gln Gly Cys Phe Ala Gly Gly Thr Val Leu Arg Leu Val Lys
                165                 170                 175

Asp Ile Ala Glu Asn Asn Lys Gly Ala Arg Val Leu Val Val Cys Ser
            180                 185                 190

Glu Ile Val Ala Phe Ala Phe Arg Gly Pro His Glu Asp His Ile Asp
        195                 200                 205

Ser Leu Ile Gly Gln Leu Leu Phe Gly Asp Gly Ala Ala Ala Leu Val
    210                 215                 220

Val Gly Thr Asp Ile Asp Glu Ser Val Glu Arg Pro Ile Phe Gln Ile
225                 230                 235                 240

Met Ser Ala Thr Gln Ala Thr Ile Pro Asn Ser Leu His Thr Met Ala
                245                 250                 255

Leu His Leu Thr Glu Ala Gly Leu Thr Phe His Leu Ser Lys Glu Val
            260                 265                 270

Pro Lys Val Val Ser Asp Asn Met Glu Glu Leu Met Leu Glu Ala Phe
        275                 280                 285

Lys Pro Leu Gly Ile Thr Asp Trp Asn Ser Ile Phe Trp Gln Val His
    290                 295                 300

Pro Gly Gly Arg Ala Ile Leu Asp Lys Ile Glu Glu Lys Leu Glu Leu
305                 310                 315                 320

Thr Lys Asp Lys Met Arg Asp Ser Arg Tyr Ile Leu Ser Glu Tyr Gly
                325                 330                 335

Asn Leu Thr Ser Ala Cys Val Leu Phe Val Met Asp Glu Met Arg Lys
            340                 345                 350

Arg Ser Phe Arg Glu Gly Lys Gln Thr Thr Gly Asp Gly Tyr Glu Trp
        355                 360                 365

Gly Val Ala Ile Gly Leu Gly Pro Gly Leu Thr Val Glu Thr Val Val
    370                 375                 380

Leu Arg Ser Val Pro Ile Pro
385                 390

<210>  135
<211>  43
<212>  DNA
<213>  Artificial Sequence

<220>
<223>  Primer

<400>  135
ctttaagaag gagatataca tatgagttca ctctccaact ctc                43

<210>  136
<211>  44
<212>  DNA
<213>  Artificial Sequence

<220>
<223>  Primer

<400>  136
cttgtcgacg gagctcgaat tcattacatg agaggcaggc tgtg               44

<210>  137
<211>  44
<212>  DNA
<213>  Artificial Sequence

<220>
<223>  Primer

<400>  137
ctttaagaag gagatataca tatgagttca ctctccaacg cttc               44

<210>  138
<211>  44
<212>  DNA
<213>  Artificial Sequence

<220>
<223>  Primer

<400>  138
cttgtcgacg gagctcgaat tcattacatg agaggcaggc tgtg               44

<210>  139
<211>  44
<212>  DNA
<213>  Artificial Sequence

<220>
<223>  Primer

<400>  139
ctttaagaag gagatataca tatgggttca ctctccgact ctac               44

<210>  140
<211>  44
<212>  DNA
<213>  Artificial Sequence

<220>
<223>  Primer

<400>  140
cttgtcgacg gagctcgaat tcattacaca ggaggcaggc tatg                        44


<210>  141
<211>  42
<212>  DNA
<213>  Artificial Sequence

<220>
<223>  Primer

<400>  141
ctttaagaag gagatataca tatgggttca ctctccaact ac                          42


<210>  142
<211>  42
<212>  DNA
<213>  Artificial Sequence

<220>
<223>  Primer

<400>  142
cttgtcgacg gagctcgaat tcattacaca gggggcaggc tg                          42


<210>  143
<211>  39
<212>  DNA
<213>  Artificial Sequence

<220>
<223>  Primer

<400>  143
ctttaagaag gagatataca tatgggttcg atcgccgag                              39


<210>  144
<211>  42
<212>  DNA
<213>  Artificial Sequence

<220>
<223>  Primer

<400>  144
cttgtcgacg gagctcgaat tcattagaca ggaggcaggc tg                          42


<210>  145
<211>  32

<212>  DNA
<213>  Artificial Sequence

<220>
<223>  Primer.

<400>  145
gctcaccatc attgggctcc gtggccccaa tg                                    32


<210>  146
<211>  32
<212>  DNA
<213>  Artificial Sequence

<220>
<223>  Primer

<400>  146
cattggggcc acggagccca atgatggtga gc                                    32


<210>  147
<211>  40
<212>  DNA
<213>  Artificial Sequence

<220>
<223>  Primer

<400>  147
gacggatggc cttttactag tgcctggggt gcctaatgag                            40


<210>  148
<211>  45
<212>  DNA
<213>  Artificial Sequence

<220>
<223>  Primer

<400>  148
cgatgattaa ttgtcaactg ctacgcctga ataagtgata ataag                      45


<210>  149
<211>  22
<212>  DNA
<213>  Artificial Sequence

<220>
<223>  Primer

<400>  149
gttgacaatt aatcatcggc tc                                               22


<210>  150
<211>  27
<212>  DNA
<213>  Artificial Sequence

<220>
<223>  Primer

<400>  150
actagtaaaa ggccatccgt caggatg                                          27


<210>  151
<211>  20
<212>  DNA
<213>  Artificial Sequence

<220>
<223>  Primer

<400>  151
gttgacaatt aatcatcggc                                                  20


<210>  152
<211>  20
<212>  DNA
<213>  Artificial Sequence

<220>
<223>  Primer

<400>  152
cgtttcactt ctgagttcgg                                                  20


<210>  153
<211>  46
<212>  DNA
<213>  Artificial Sequence

<220>
<223>  Primer

<400>  153
caatttcaca caggaaacag aattcgtgtc agtcgagact aggaag                     46


<210>  154
<211>  44
<212>  DNA
<213>  Artificial Sequence

<220>
<223>  Primer

<400>  154
ctctagagga tccccgggta ccattacttg atctcgagga gaac                       44


<210>  155
<211>  46
<212>  DNA
<213>  Artificial Sequence

<220>
<223>  Primer

<400>  155
caatttcaca caggaaacag aattcatgac catttcctca cctttg                    46


<210>  156
<211>  44
<212>  DNA
<213>  Artificial Sequence

<220>
<223>  Primer

<400>  156
ctctagagga tccccgggta ccattacagt ggcatgttgc cgtg                      44


<210>  157
<211>  43
<212>  DNA
<213>  Artificial Sequence

<220>
<223>  Primer

<400>  157
gagtcgacct gcaggcatgc attgacaatt aatcatcggc tcg                       43


<210>  158
<211>  25
<212>  DNA
<213>  Artificial Sequence

<220>
<223>  Primer

<400>  158
ctcatccgcc aaaacagcca agctt                                           25


<210>  159
<211>  47
<212>  DNA
<213>  Artificial Sequence

<220>
<223>  Primer

<400>  159
caatttcaca caggaaacag aattcatgtc agaacgtttc ccaaatg                   47


<210>  160
<211>  45
<212>  DNA
<213>  Artificial Sequence

<220>
<223>  Primer

<400>  160
ctctagagga tccccgggta ccattacatc accagacggc gaatg                     45

<210>  161
<211>  47
<212>  DNA
<213>  Artificial Sequence

<220>
<223>  Primer

<400>  161
caatttcaca caggaaacag aattcatgag tactgaaatc aaaactc                    47


<210>  162
<211>  44
<212>  DNA
<213>  Artificial Sequence

<220>
<223>  Primer

<400>  162
ctctagagga tccccgggta ccattacttc ttcttcgctt tcgg                       44


<210>  163
<211>  43
<212>  DNA
<213>  Artificial Sequence

<220>
<223>  Primer

<400>  163
gtacccgggg atcctctaga gttgacaatt aatcatcggc tcg                        43


<210>  164
<211>  25
<212>  DNA
<213>  Artificial Sequence

<220>
<223>  Primer

<400>  164
caagcttgca tgcctgcagg tcgac                                            25


<210>  165
<211>  49
<212>  DNA
<213>  Artificial Sequence

<220>
<223>  Primer

<400>  165
caatttcaca caggaaacag aattcatgac tatcaaagta ggtatcaac                  49


<210>  166
<211>  45

<212>  DNA
<213>  Artificial Sequence

<220>
<223>  Primer

<400>  166
ctctagagga tccccgggta ccattatttg gagatgtgag cgatc                    45


<210>  167
<211>  50
<212>  DNA
<213>  Artificial Sequence

<220>
<223>  Primer

<400>  167
caatttcaca caggaaacag aattcatgtc tgtaattaag atgaccgatc               50


<210>  168
<211>  45
<212>  DNA
<213>  Artificial Sequence

<220>
<223>  Primer

<400>  168
ctctagagga tccccgggta ccattacttc ttagcgcgct cttcg                    45


<210>  169
<211>  46
<212>  DNA
<213>  Artificial Sequence

<220>
<223>  Primer

<400>  169
caatttcaca caggaaacag aattcatgag ccaaattcac aaacac                   46


<210>  170
<211>  43
<212>  DNA
<213>  Artificial Sequence

<220>
<223>  Primer

<400>  170
ctctagagga tccccgggta ccattacgat ggcatcgcga tag                      43


<210>  171
<211>  491
<212>  PRT
<213>  Escherichia coli

<400>  171

Met Ala Val Thr Gln Thr Ala Gln Ala Cys Asp Leu Val Ile Phe Gly
1               5                   10                  15

Ala Lys Gly Asp Leu Ala Arg Arg Lys Leu Leu Pro Ser Leu Tyr Gln
            20                  25                  30

Leu Glu Lys Ala Gly Gln Leu Asn Pro Asp Thr Arg Ile Ile Gly Val
        35                  40                  45

Gly Arg Ala Asp Trp Asp Lys Ala Ala Tyr Thr Lys Val Val Arg Glu
    50                  55                  60

Ala Leu Glu Thr Phe Met Lys Glu Thr Ile Asp Glu Gly Leu Trp Asp
65                  70                  75                  80

Thr Leu Ser Ala Arg Leu Asp Phe Cys Asn Leu Asp Val Asn Asp Thr
                85                  90                  95

Ala Ala Phe Ser Arg Leu Gly Ala Met Leu Asp Gln Lys Asn Arg Ile
            100                 105                 110

Thr Ile Asn Tyr Phe Ala Met Pro Pro Ser Thr Phe Gly Ala Ile Cys
        115                 120                 125

Lys Gly Leu Gly Glu Ala Lys Leu Asn Ala Lys Pro Ala Arg Val Val
    130                 135                 140

Met Glu Lys Pro Leu Gly Thr Ser Leu Ala Thr Ser Gln Glu Ile Asn
145                 150                 155                 160

Asp Gln Val Gly Glu Tyr Phe Glu Glu Cys Gln Val Tyr Arg Ile Asp
                165                 170                 175

His Tyr Leu Gly Lys Glu Thr Val Leu Asn Leu Leu Ala Leu Arg Phe
            180                 185                 190

Ala Asn Ser Leu Phe Val Asn Asn Trp Asp Asn Arg Thr Ile Asp His
        195                 200                 205

Val Glu Ile Thr Val Ala Glu Glu Val Gly Ile Glu Gly Arg Trp Gly
    210                 215                 220

Tyr Phe Asp Lys Ala Gly Gln Met Arg Asp Met Ile Gln Asn His Leu
225                 230                 235                 240

Leu Gln Ile Leu Cys Met Ile Ala Met Ser Pro Pro Ser Asp Leu Ser

                245                 250                 255

Ala Asp Ser Ile Arg Asp Glu Lys Val Lys Val Leu Lys Ser Leu Arg
            260                 265                 270

Arg Ile Asp Arg Ser Asn Val Arg Glu Lys Thr Val Arg Gly Gln Tyr
        275                 280                 285

Thr Ala Gly Phe Ala Gln Gly Lys Lys Val Pro Gly Tyr Leu Glu Glu
    290                 295                 300

Glu Gly Ala Asn Lys Ser Ser Asn Thr Glu Thr Phe Val Ala Ile Arg
305                 310                 315                 320

Val Asp Ile Asp Asn Trp Arg Trp Ala Gly Val Pro Phe Tyr Leu Arg
                325                 330                 335

Thr Gly Lys Arg Leu Pro Thr Lys Cys Ser Glu Val Val Val Tyr Phe
            340                 345                 350

Lys Thr Pro Glu Leu Asn Leu Phe Lys Glu Ser Trp Gln Asp Leu Pro
        355                 360                 365

Gln Asn Lys Leu Thr Ile Arg Leu Gln Pro Asp Glu Gly Val Asp Ile
    370                 375                 380

Gln Val Leu Asn Lys Val Pro Gly Leu Asp His Lys His Asn Leu Gln
385                 390                 395                 400

Ile Thr Lys Leu Asp Leu Ser Tyr Ser Glu Thr Phe Asn Gln Thr His
                405                 410                 415

Leu Ala Asp Ala Tyr Glu Arg Leu Leu Leu Glu Thr Met Arg Gly Ile
            420                 425                 430

Gln Ala Leu Phe Val Arg Arg Asp Glu Val Glu Glu Ala Trp Lys Trp
        435                 440                 445

Val Asp Ser Ile Thr Glu Ala Trp Ala Met Asp Asn Asp Ala Pro Lys
    450                 455                 460

Pro Tyr Gln Ala Gly Thr Trp Gly Pro Val Ala Ser Val Ala Met Ile
465                 470                 475                 480

Thr Arg Asp Gly Arg Ser Trp Asn Glu Phe Glu
                485                 490

<210>  172
<211>  312
<212>  PRT
<213>  Escherichia coli

<400>  172

Met Lys Val Ala Val Leu Gly Ala Ala Gly Gly Ile Gly Gln Ala Leu
1               5                   10                  15

Ala Leu Leu Leu Lys Thr Gln Leu Pro Ser Gly Ser Glu Leu Ser Leu
            20                  25                  30

Tyr Asp Ile Ala Pro Val Thr Pro Gly Val Ala Val Asp Leu Ser His
        35                  40                  45

Ile Pro Thr Ala Val Lys Ile Lys Gly Phe Ser Gly Glu Asp Ala Thr
    50                  55                  60

Pro Ala Leu Glu Gly Ala Asp Val Val Leu Ile Ser Ala Gly Val Ala
65                  70                  75                  80

Arg Lys Pro Gly Met Asp Arg Ser Asp Leu Phe Asn Val Asn Ala Gly
                85                  90                  95

Ile Val Lys Asn Leu Val Gln Gln Val Ala Lys Thr Cys Pro Lys Ala
            100                 105                 110

Cys Ile Gly Ile Ile Thr Asn Pro Val Asn Thr Thr Val Ala Ile Ala
        115                 120                 125

Ala Glu Val Leu Lys Lys Ala Gly Val Tyr Asp Lys Asn Lys Leu Phe
    130                 135                 140

Gly Val Thr Thr Leu Asp Ile Ile Arg Ser Asn Thr Phe Val Ala Glu
145                 150                 155                 160

Leu Lys Gly Lys Gln Pro Gly Glu Val Glu Val Pro Val Ile Gly Gly
                165                 170                 175

His Ser Gly Val Thr Ile Leu Pro Leu Leu Ser Gln Val Pro Gly Val
            180                 185                 190

Ser Phe Thr Glu Gln Glu Val Ala Asp Leu Thr Lys Arg Ile Gln Asn
        195                 200                 205

Ala Gly Thr Glu Val Val Glu Ala Lys Ala Gly Gly Gly Ser Ala Thr
    210                 215                 220

Leu Ser Met Gly Gln Ala Ala Ala Arg Phe Gly Leu Ser Leu Val Arg
225                 230                 235                 240

Ala Leu Gln Gly Glu Gln Gly Val Val Glu Cys Ala Tyr Val Glu Gly
                245                 250                 255

Asp Gly Gln Tyr Ala Arg Phe Phe Ser Gln Pro Leu Leu Leu Gly Lys
            260                 265                 270

Asn Gly Val Glu Glu Arg Lys Ser Ile Gly Thr Leu Ser Ala Phe Glu
        275                 280                 285

Gln Asn Ala Leu Glu Gly Met Leu Asp Thr Leu Lys Lys Asp Ile Ala
    290                 295                 300

Leu Gly Glu Glu Phe Val Asn Lys
305                 310

<210>  173
<211>  410
<212>  PRT
<213>  Escherichia coli

<400>  173

Met Ala Lys Val Ser Leu Glu Lys Asp Lys Ile Lys Phe Leu Leu Val
1               5                   10                  15

Glu Gly Val His Gln Lys Ala Leu Glu Ser Leu Arg Ala Ala Gly Tyr
            20                  25                  30

Thr Asn Ile Glu Phe His Lys Gly Ala Leu Asp Asp Glu Gln Leu Lys
        35                  40                  45

Glu Ser Ile Arg Asp Ala His Phe Ile Gly Leu Arg Ser Arg Thr His
    50                  55                  60

Leu Thr Glu Asp Val Ile Asn Ala Ala Glu Lys Leu Val Ala Ile Gly
65                  70                  75                  80

Cys Phe Cys Ile Gly Thr Asn Gln Val Asp Leu Asp Ala Ala Ala Lys
                85                  90                  95

Arg Gly Ile Pro Val Phe Asn Ala Pro Phe Ser Asn Thr Arg Ser Val
            100                 105                 110

Ala Glu Leu Val Ile Gly Glu Leu Leu Leu Leu Leu Arg Gly Val Pro
        115                 120                 125

Glu Ala Asn Ala Lys Ala His Arg Gly Val Trp Asn Lys Leu Ala Ala
    130                 135                 140

Gly Ser Phe Glu Ala Arg Gly Lys Lys Leu Gly Ile Ile Gly Tyr Gly
145                 150                 155                 160

His Ile Gly Thr Gln Leu Gly Ile Leu Ala Glu Ser Leu Gly Met Tyr
                165                 170                 175

Val Tyr Phe Tyr Asp Ile Glu Asn Lys Leu Pro Leu Gly Asn Ala Thr
            180                 185                 190

Gln Val Gln His Leu Ser Asp Leu Leu Asn Met Ser Asp Val Val Ser
        195                 200                 205

Leu His Val Pro Glu Asn Pro Ser Thr Lys Asn Met Met Gly Ala Lys
    210                 215                 220

Glu Ile Ser Leu Met Lys Pro Gly Ser Leu Leu Ile Asn Ala Ser Arg
225                 230                 235                 240

Gly Thr Val Val Asp Ile Pro Ala Leu Cys Asp Ala Leu Ala Ser Lys
                245                 250                 255

His Leu Ala Gly Ala Ala Ile Asp Val Phe Pro Thr Glu Pro Ala Thr
            260                 265                 270

Asn Ser Asp Pro Phe Thr Ser Pro Leu Cys Glu Phe Asp Asn Val Leu
        275                 280                 285

Leu Thr Pro His Ile Gly Gly Ser Thr Gln Glu Ala Gln Glu Asn Ile
    290                 295                 300

Gly Leu Glu Val Ala Gly Lys Leu Ile Lys Tyr Ser Asp Asn Gly Ser
305                 310                 315                 320

Thr Leu Ser Ala Val Asn Phe Pro Glu Val Ser Leu Pro Leu His Gly
                325                 330                 335

Gly Arg Arg Leu Met His Ile His Glu Asn Arg Pro Gly Val Leu Thr
            340                 345                 350

Ala Leu Asn Lys Ile Phe Ala Glu Gln Gly Val Asn Ile Ala Ala Gln
        355                 360                 365

Tyr Leu Gln Thr Ser Ala Gln Met Gly Tyr Val Val Ile Asp Ile Glu
    370                 375                 380

Ala Asp Glu Asp Val Ala Glu Lys Ala Leu Gln Ala Met Lys Ala Ile
385                 390                 395                 400

Pro Gly Thr Ile Arg Ala Arg Leu Leu Tyr
                405                 410

<210>  174
<211>  331
<212>  PRT
<213>  Escherichia coli

<400>  174

Met Thr Ile Lys Val Gly Ile Asn Gly Phe Gly Arg Ile Gly Arg Ile
1               5                   10                  15

Val Phe Arg Ala Ala Gln Lys Arg Ser Asp Ile Glu Ile Val Ala Ile
            20                  25                  30

Asn Asp Leu Leu Asp Ala Asp Tyr Met Ala Tyr Met Leu Lys Tyr Asp
        35                  40                  45

Ser Thr His Gly Arg Phe Asp Gly Thr Val Glu Val Lys Asp Gly His
    50                  55                  60

Leu Ile Val Asn Gly Lys Lys Ile Arg Val Thr Ala Glu Arg Asp Pro
65                  70                  75                  80

Ala Asn Leu Lys Trp Asp Glu Val Gly Val Asp Val Val Ala Glu Ala
                85                  90                  95

Thr Gly Leu Phe Leu Thr Asp Glu Thr Ala Arg Lys His Ile Thr Ala
            100                 105                 110

Gly Ala Lys Lys Val Val Met Thr Gly Pro Ser Lys Asp Asn Thr Pro
        115                 120                 125

Met Phe Val Lys Gly Ala Asn Phe Asp Lys Tyr Ala Gly Gln Asp Ile
    130                 135                 140

Val Ser Asn Ala Ser Cys Thr Thr Asn Cys Leu Ala Pro Leu Ala Lys
145                 150                 155                 160

Val Ile Asn Asp Asn Phe Gly Ile Ile Glu Gly Leu Met Thr Thr Val
                165                 170                 175

His Ala Thr Thr Ala Thr Gln Lys Thr Val Asp Gly Pro Ser His Lys
            180                 185                 190

Asp Trp Arg Gly Gly Arg Gly Ala Ser Gln Asn Ile Ile Pro Ser Ser
        195                 200                 205

Thr Gly Ala Ala Lys Ala Val Gly Lys Val Leu Pro Glu Leu Asn Gly
    210                 215                 220

Lys Leu Thr Gly Met Ala Phe Arg Val Pro Thr Pro Asn Val Ser Val
225                 230                 235                 240

Val Asp Leu Thr Val Arg Leu Glu Lys Ala Ala Thr Tyr Glu Gln Ile
                245                 250                 255

Lys Ala Ala Val Lys Ala Ala Ala Glu Gly Glu Met Lys Gly Val Leu
            260                 265                 270

Gly Tyr Thr Glu Asp Asp Val Val Ser Thr Asp Phe Asn Gly Glu Val
        275                 280                 285

Cys Thr Ser Val Phe Asp Ala Lys Ala Gly Ile Ala Leu Asn Asp Asn
    290                 295                 300

Phe Val Lys Leu Val Ser Trp Tyr Asp Asn Glu Thr Gly Tyr Ser Asn
305                 310                 315                 320

Lys Val Leu Asp Leu Ile Ala His Ile Ser Lys
                325                 330

<210>  175
<211>  387
<212>  PRT
<213>  Escherichia coli

<400>  175

Met Ser Val Ile Lys Met Thr Asp Leu Asp Leu Ala Gly Lys Arg Val
1               5                   10                  15

Phe Ile Arg Ala Asp Leu Asn Val Pro Val Lys Asp Gly Lys Val Thr
            20                  25                  30

Ser Asp Ala Arg Ile Arg Ala Ser Leu Pro Thr Ile Glu Leu Ala Leu
        35                  40                  45

Lys Gln Gly Ala Lys Val Met Val Thr Ser His Leu Gly Arg Pro Thr
    50                  55                  60

Glu Gly Glu Tyr Asn Glu Glu Phe Ser Leu Leu Pro Val Val Asn Tyr
65                  70                  75                  80

Leu Lys Asp Lys Leu Ser Asn Pro Val Arg Leu Val Lys Asp Tyr Leu
                85                  90                  95

Asp Gly Val Asp Val Ala Glu Gly Glu Leu Val Val Leu Glu Asn Val
            100                 105                 110

Arg Phe Asn Lys Gly Glu Lys Lys Asp Asp Glu Thr Leu Ser Lys Lys
        115                 120                 125

Tyr Ala Ala Leu Cys Asp Val Phe Val Met Asp Ala Phe Gly Thr Ala
    130                 135                 140

His Arg Ala Gln Ala Ser Thr His Gly Ile Gly Lys Phe Ala Asp Val
145                 150                 155                 160

Ala Cys Ala Gly Pro Leu Leu Ala Ala Glu Leu Asp Ala Leu Gly Lys
                165                 170                 175

Ala Leu Lys Glu Pro Ala Arg Pro Met Val Ala Ile Val Gly Gly Ser
            180                 185                 190

Lys Val Ser Thr Lys Leu Thr Val Leu Asp Ser Leu Ser Lys Ile Ala
        195                 200                 205

Asp Gln Leu Ile Val Gly Gly Gly Ile Ala Asn Thr Phe Ile Ala Ala
    210                 215                 220

Gln Gly His Asp Val Gly Lys Ser Leu Tyr Glu Ala Asp Leu Val Asp
225                 230                 235                 240

Glu Ala Lys Arg Leu Leu Thr Thr Cys Asn Ile Pro Val Pro Ser Asp
                245                 250                 255

Val Arg Val Ala Thr Glu Phe Ser Glu Thr Ala Pro Ala Thr Leu Lys
            260                 265                 270

Ser Val Asn Asp Val Lys Ala Asp Glu Gln Ile Leu Asp Ile Gly Asp
        275                 280                 285

Ala Ser Ala Gln Glu Leu Ala Glu Ile Leu Lys Asn Ala Lys Thr Ile
    290                 295                 300

Leu Trp Asn Gly Pro Val Gly Val Phe Glu Phe Pro Asn Phe Arg Lys
305                 310                 315                 320

Gly Thr Glu Ile Val Ala Asn Ala Ile Ala Asp Ser Glu Ala Phe Ser
                325                 330                 335

Ile Ala Gly Gly Gly Asp Thr Leu Ala Ala Ile Asp Leu Phe Gly Ile
            340                 345                 350

Ala Asp Lys Ile Ser Tyr Ile Ser Thr Gly Gly Gly Ala Phe Leu Glu
        355                 360                 365

Phe Val Glu Gly Lys Val Leu Pro Ala Val Ala Met Leu Glu Glu Arg
    370                 375                 380

Ala Lys Lys
385

<210> 176
<211> 652
<212> PRT
<213> Escherichia coli

<400> 176

Met Ser Gln Ile His Lys His Thr Ile Pro Ala Asn Ile Ala Asp Arg
1               5                   10                  15

Cys Leu Ile Asn Pro Gln Gln Tyr Glu Ala Met Tyr Gln Gln Ser Ile
            20                  25                  30

Asn Val Pro Asp Thr Phe Trp Gly Glu Gln Gly Lys Ile Leu Asp Trp
        35                  40                  45

Ile Lys Pro Tyr Gln Lys Val Lys Asn Thr Ser Phe Ala Pro Gly Asn
    50                  55                  60

Val Ser Ile Lys Trp Tyr Glu Asp Gly Thr Leu Asn Leu Ala Ala Asn
65                  70                  75                  80

Cys Leu Asp Arg His Leu Gln Glu Asn Gly Asp Arg Thr Ala Ile Ile
                85                  90                  95

Trp Glu Gly Asp Asp Ala Ser Gln Ser Lys His Ile Ser Tyr Lys Glu
            100                 105                 110

Leu His Arg Asp Val Cys Arg Phe Ala Asn Thr Leu Leu Glu Leu Gly
        115                 120                 125

Ile Lys Lys Gly Asp Val Val Ala Ile Tyr Met Pro Met Val Pro Glu
    130                 135                 140

Ala Ala Val Ala Met Leu Ala Cys Ala Arg Ile Gly Ala Val His Ser
145                 150                 155                 160

Val Ile Phe Gly Gly Phe Ser Pro Glu Ala Val Ala Gly Arg Ile Ile
                165                 170                 175

Asp Ser Asn Ser Arg Leu Val Ile Thr Ser Asp Glu Gly Val Arg Ala
            180                 185                 190

Gly Arg Ser Ile Pro Leu Lys Lys Asn Val Asp Asp Ala Leu Lys Asn
        195                 200                 205

Pro Asn Val Thr Ser Val Glu His Val Val Val Leu Lys Arg Thr Gly
    210                 215                 220

Gly Lys Ile Asp Trp Gln Glu Gly Arg Asp Leu Trp Trp His Asp Leu
225                 230                 235                 240

Val Glu Gln Ala Ser Asp Gln His Gln Ala Glu Glu Met Asn Ala Glu
                245                 250                 255

Asp Pro Leu Phe Ile Leu Tyr Thr Ser Gly Ser Thr Gly Lys Pro Lys
            260                 265                 270

Gly Val Leu His Thr Thr Gly Gly Tyr Leu Val Tyr Ala Ala Leu Thr
        275                 280                 285

Phe Lys Tyr Val Phe Asp Tyr His Pro Gly Asp Ile Tyr Trp Cys Thr
    290                 295                 300

Ala Asp Val Gly Trp Val Thr Gly His Ser Tyr Leu Leu Tyr Gly Pro
305                 310                 315                 320

Leu Ala Cys Gly Ala Thr Thr Leu Met Phe Glu Gly Val Pro Asn Trp
                325                 330                 335

Pro Thr Pro Ala Arg Met Ala Gln Val Val Asp Lys His Gln Val Asn
            340                 345                 350

Ile Leu Tyr Thr Ala Pro Thr Ala Ile Arg Ala Leu Met Ala Glu Gly
        355                 360                 365

Asp Lys Ala Ile Glu Gly Thr Asp Arg Ser Ser Leu Arg Ile Leu Gly
    370                 375                 380

Ser Val Gly Glu Pro Ile Asn Pro Glu Ala Trp Glu Trp Tyr Trp Lys
385                 390                 395                 400

Lys Ile Gly Asn Glu Lys Cys Pro Val Val Asp Thr Trp Trp Gln Thr

                405                 410                 415

Glu Thr Gly Gly Phe Met Ile Thr Pro Leu Pro Gly Ala Thr Glu Leu
            420                 425                 430

Lys Ala Gly Ser Ala Thr Arg Pro Phe Phe Gly Val Gln Pro Ala Leu
        435                 440                 445

Val Asp Asn Glu Gly Asn Pro Leu Glu Gly Ala Thr Glu Gly Ser Leu
    450                 455                 460

Val Ile Thr Asp Ser Trp Pro Gly Gln Ala Arg Thr Leu Phe Gly Asp
465                 470                 475                 480

His Glu Arg Phe Glu Gln Thr Tyr Phe Ser Thr Phe Lys Asn Met Tyr
                485                 490                 495

Phe Ser Gly Asp Gly Ala Arg Arg Asp Glu Asp Gly Tyr Tyr Trp Ile
            500                 505                 510

Thr Gly Arg Val Asp Asp Val Leu Asn Val Ser Gly His Arg Leu Gly
        515                 520                 525

Thr Ala Glu Ile Glu Ser Ala Leu Val Ala His Pro Lys Ile Ala Glu
    530                 535                 540

Ala Ala Val Val Gly Ile Pro His Asn Ile Lys Gly Gln Ala Ile Tyr
545                 550                 555                 560

Ala Tyr Val Thr Leu Asn His Gly Glu Glu Pro Ser Pro Glu Leu Tyr
                565                 570                 575

Ala Glu Val Arg Asn Trp Val Arg Lys Glu Ile Gly Pro Leu Ala Thr
            580                 585                 590

Pro Asp Val Leu His Trp Thr Asp Ser Leu Pro Lys Thr Arg Ser Gly
        595                 600                 605

Lys Ile Met Arg Arg Ile Leu Arg Lys Ile Ala Ala Gly Asp Thr Ser
    610                 615                 620

Asn Leu Gly Asp Thr Ser Thr Leu Ala Asp Pro Gly Val Val Glu Lys
625                 630                 635                 640

Leu Leu Glu Glu Lys Gln Ala Ile Ala Met Pro Ser
                645                 650

<210>  177
<211>  887
<212>  PRT
<213>  Escherichia coli

<400>  177

Met Ser Glu Arg Phe Pro Asn Asp Val Asp Pro Ile Glu Thr Arg Asp
1               5                   10                  15

Trp Leu Gln Ala Ile Glu Ser Val Ile Arg Glu Glu Gly Val Glu Arg
            20                  25                  30

Ala Gln Tyr Leu Ile Asp Gln Leu Leu Ala Glu Ala Arg Lys Gly Gly
        35                  40                  45

Val Asn Val Ala Ala Gly Thr Gly Ile Ser Asn Tyr Ile Asn Thr Ile
    50                  55                  60

Pro Val Glu Glu Gln Pro Glu Tyr Pro Gly Asn Leu Glu Leu Glu Arg
65                  70                  75                  80

Arg Ile Arg Ser Ala Ile Arg Trp Asn Ala Ile Met Thr Val Leu Arg
                85                  90                  95

Ala Ser Lys Lys Asp Leu Glu Leu Gly Gly His Met Ala Ser Phe Gln
            100                 105                 110

Ser Ser Ala Thr Ile Tyr Asp Val Cys Phe Asn His Phe Phe Arg Ala
        115                 120                 125

Arg Asn Glu Gln Asp Gly Gly Asp Leu Val Tyr Phe Gln Gly His Ile
    130                 135                 140

Ser Pro Gly Val Tyr Ala Arg Ala Phe Leu Glu Gly Arg Leu Thr Gln
145                 150                 155                 160

Glu Gln Leu Asp Asn Phe Arg Gln Glu Val His Gly Asn Gly Leu Ser
                165                 170                 175

Ser Tyr Pro His Pro Lys Leu Met Pro Glu Phe Trp Gln Phe Pro Thr
            180                 185                 190

Val Ser Met Gly Leu Gly Pro Ile Gly Ala Ile Tyr Gln Ala Lys Phe
        195                 200                 205

Leu Lys Tyr Leu Glu His Arg Gly Leu Lys Asp Thr Ser Lys Gln Thr
    210                 215                 220

Val Tyr Ala Phe Leu Gly Asp Gly Glu Met Asp Glu Pro Glu Ser Lys
225                 230                 235                 240

Gly Ala Ile Thr Ile Ala Thr Arg Glu Lys Leu Asp Asn Leu Val Phe
                245                 250                 255

Val Ile Asn Cys Asn Leu Gln Arg Leu Asp Gly Pro Val Thr Gly Asn
            260                 265                 270

Gly Lys Ile Ile Asn Glu Leu Glu Gly Ile Phe Glu Gly Ala Gly Trp
        275                 280                 285

Asn Val Ile Lys Val Met Trp Gly Ser Arg Trp Asp Glu Leu Leu Arg
    290                 295                 300

Lys Asp Thr Ser Gly Lys Leu Ile Gln Leu Met Asn Glu Thr Val Asp
305                 310                 315                 320

Gly Asp Tyr Gln Thr Phe Lys Ser Lys Asp Gly Ala Tyr Val Arg Glu
                325                 330                 335

His Phe Phe Gly Lys Tyr Pro Glu Thr Ala Ala Leu Val Ala Asp Trp
            340                 345                 350

Thr Asp Glu Gln Ile Trp Ala Leu Asn Arg Gly Gly His Asp Pro Lys
        355                 360                 365

Lys Ile Tyr Ala Ala Phe Lys Lys Ala Gln Glu Thr Lys Gly Lys Ala
    370                 375                 380

Thr Val Ile Leu Ala His Thr Ile Lys Gly Tyr Gly Met Gly Asp Ala
385                 390                 395                 400

Ala Glu Gly Lys Asn Ile Ala His Gln Val Lys Lys Met Asn Met Asp
                405                 410                 415

Gly Val Arg His Ile Arg Asp Arg Phe Asn Val Pro Val Ser Asp Ala
            420                 425                 430

Asp Ile Glu Lys Leu Pro Tyr Ile Thr Phe Pro Glu Gly Ser Glu Glu
        435                 440                 445

His Thr Tyr Leu His Ala Gln Arg Gln Lys Leu His Gly Tyr Leu Pro
    450                 455                 460

Ser Arg Gln Pro Asn Phe Thr Glu Lys Leu Glu Leu Pro Ser Leu Gln
465                 470                 475                 480

Asp Phe Gly Ala Leu Leu Glu Glu Gln Ser Lys Glu Ile Ser Thr Thr
                485                 490                 495

Ile Ala Phe Val Arg Ala Leu Asn Val Met Leu Lys Asn Lys Ser Ile
            500                 505                 510

Lys Asp Arg Leu Val Pro Ile Ile Ala Asp Glu Ala Arg Thr Phe Gly
        515                 520                 525

Met Glu Gly Leu Phe Arg Gln Ile Gly Ile Tyr Ser Pro Asn Gly Gln
    530                 535                 540

Gln Tyr Thr Pro Gln Asp Arg Glu Gln Val Ala Tyr Tyr Lys Glu Asp
545                 550                 555                 560

Glu Lys Gly Gln Ile Leu Gln Glu Gly Ile Asn Glu Leu Gly Ala Gly
                565                 570                 575

Cys Ser Trp Leu Ala Ala Ala Thr Ser Tyr Ser Thr Asn Asn Leu Pro
            580                 585                 590

Met Ile Pro Phe Tyr Ile Tyr Tyr Ser Met Phe Gly Phe Gln Arg Ile
        595                 600                 605

Gly Asp Leu Cys Trp Ala Ala Gly Asp Gln Gln Ala Arg Gly Phe Leu
    610                 615                 620

Ile Gly Gly Thr Ser Gly Arg Thr Thr Leu Asn Gly Glu Gly Leu Gln
625                 630                 635                 640

His Glu Asp Gly His Ser His Ile Gln Ser Leu Thr Ile Pro Asn Cys
                645                 650                 655

Ile Ser Tyr Asp Pro Ala Tyr Ala Tyr Glu Val Ala Val Ile Met His
            660                 665                 670

Asp Gly Leu Glu Arg Met Tyr Gly Glu Lys Gln Glu Asn Val Tyr Tyr
        675                 680                 685

Tyr Ile Thr Thr Leu Asn Glu Asn Tyr His Met Pro Ala Met Pro Glu
    690                 695                 700

Gly Ala Glu Glu Gly Ile Arg Lys Gly Ile Tyr Lys Leu Glu Thr Ile
705                 710                 715                 720

Glu Gly Ser Lys Gly Lys Val Gln Leu Leu Gly Ser Gly Ser Ile Leu
                725                 730                 735

Arg His Val Arg Glu Ala Ala Glu Ile Leu Ala Lys Asp Tyr Gly Val
        740                 745                 750

Gly Ser Asp Val Tyr Ser Val Thr Ser Phe Thr Glu Leu Ala Arg Asp
        755                 760                 765

Gly Gln Asp Cys Glu Arg Trp Asn Met Leu His Pro Leu Glu Thr Pro
    770                 775                 780

Arg Val Pro Tyr Ile Ala Gln Val Met Asn Asp Ala Pro Ala Val Ala
785                 790                 795                 800

Ser Thr Asp Tyr Met Lys Leu Phe Ala Glu Gln Val Arg Thr Tyr Val
                805                 810                 815

Pro Ala Asp Asp Tyr Arg Val Leu Gly Thr Asp Gly Phe Gly Arg Ser
            820                 825                 830

Asp Ser Arg Glu Asn Leu Arg His His Phe Glu Val Asp Ala Ser Tyr
        835                 840                 845

Val Val Val Ala Ala Leu Gly Glu Leu Ala Lys Arg Gly Glu Ile Asp
    850                 855                 860

Lys Lys Val Val Ala Asp Ala Ile Ala Lys Phe Asn Ile Asp Ala Asp
865                 870                 875                 880

Lys Val Asn Pro Arg Leu Ala
                885

<210>  178
<211>  630
<212>  PRT
<213>  Escherichia coli

<400>  178

Met Ala Ile Glu Ile Lys Val Pro Asp Ile Gly Ala Asp Glu Val Glu
1               5                   10                  15

Ile Thr Glu Ile Leu Val Lys Val Gly Asp Lys Val Glu Ala Glu Gln
            20                  25                  30

Ser Leu Ile Thr Val Glu Gly Asp Lys Ala Ser Met Glu Val Pro Ser
        35                  40                  45

Pro Gln Ala Gly Ile Val Lys Glu Ile Lys Val Ser Val Gly Asp Lys
    50                  55                  60

Thr Gln Thr Gly Ala Leu Ile Met Ile Phe Asp Ser Ala Asp Gly Ala
65                  70                  75                  80

Ala Asp Ala Ala Pro Ala Gln Ala Glu Glu Lys Lys Glu Ala Ala Pro
                85                  90                  95

Ala Ala Ala Pro Ala Ala Ala Ala Ala Lys Asp Val Asn Val Pro Asp
            100                 105                 110

Ile Gly Ser Asp Glu Val Glu Val Thr Glu Ile Leu Val Lys Val Gly
        115                 120                 125

Asp Lys Val Glu Ala Glu Gln Ser Leu Ile Thr Val Glu Gly Asp Lys
    130                 135                 140

Ala Ser Met Glu Val Pro Ala Pro Phe Ala Gly Thr Val Lys Glu Ile
145                 150                 155                 160

Lys Val Asn Val Gly Asp Lys Val Ser Thr Gly Ser Leu Ile Met Val
                165                 170                 175

Phe Glu Val Ala Gly Glu Ala Gly Ala Ala Ala Pro Ala Ala Lys Gln
            180                 185                 190

Glu Ala Ala Pro Ala Ala Ala Pro Ala Pro Ala Ala Gly Val Lys Glu
        195                 200                 205

Val Asn Val Pro Asp Ile Gly Gly Asp Glu Val Glu Val Thr Glu Val
    210                 215                 220

Met Val Lys Val Gly Asp Lys Val Ala Ala Glu Gln Ser Leu Ile Thr
225                 230                 235                 240

Val Glu Gly Asp Lys Ala Ser Met Glu Val Pro Ala Pro Phe Ala Gly
                245                 250                 255

Val Val Lys Glu Leu Lys Val Asn Val Gly Asp Lys Val Lys Thr Gly
            260                 265                 270

Ser Leu Ile Met Ile Phe Glu Val Glu Gly Ala Ala Pro Ala Ala Ala
        275                 280                 285

Pro Ala Lys Gln Glu Ala Ala Ala Pro Ala Pro Ala Ala Lys Ala Glu
    290                 295                 300

Ala Pro Ala Ala Ala Pro Ala Ala Lys Ala Glu Gly Lys Ser Glu Phe

305                 310                 315                 320

Ala Glu Asn Asp Ala Tyr Val His Ala Thr Pro Leu Ile Arg Arg Leu
                325                 330                 335

Ala Arg Glu Phe Gly Val Asn Leu Ala Lys Val Lys Gly Thr Gly Arg
            340                 345                 350

Lys Gly Arg Ile Leu Arg Glu Asp Val Gln Ala Tyr Val Lys Glu Ala
        355                 360                 365

Ile Lys Arg Ala Glu Ala Ala Pro Ala Ala Thr Gly Gly Gly Ile Pro
    370                 375                 380

Gly Met Leu Pro Trp Pro Lys Val Asp Phe Ser Lys Phe Gly Glu Ile
385                 390                 395                 400

Glu Glu Val Glu Leu Gly Arg Ile Gln Lys Ile Ser Gly Ala Asn Leu
                405                 410                 415

Ser Arg Asn Trp Val Met Ile Pro His Val Thr His Phe Asp Lys Thr
            420                 425                 430

Asp Ile Thr Glu Leu Glu Ala Phe Arg Lys Gln Gln Asn Glu Glu Ala
        435                 440                 445

Ala Lys Arg Lys Leu Asp Val Lys Ile Thr Pro Val Val Phe Ile Met
    450                 455                 460

Lys Ala Val Ala Ala Ala Leu Glu Gln Met Pro Arg Phe Asn Ser Ser
465                 470                 475                 480

Leu Ser Glu Asp Gly Gln Arg Leu Thr Leu Lys Lys Tyr Ile Asn Ile
                485                 490                 495

Gly Val Ala Val Asp Thr Pro Asn Gly Leu Val Val Pro Val Phe Lys
            500                 505                 510

Asp Val Asn Lys Lys Gly Ile Ile Glu Leu Ser Arg Glu Leu Met Thr
        515                 520                 525

Ile Ser Lys Lys Ala Arg Asp Gly Lys Leu Thr Ala Gly Glu Met Gln
    530                 535                 540

Gly Gly Cys Phe Thr Ile Ser Ser Ile Gly Gly Leu Gly Thr Thr His
545                 550                 555                 560

Phe Ala Pro Ile Val Asn Ala Pro Glu Val Ala Ile Leu Gly Val Ser
                565                 570                 575

Lys Ser Ala Met Glu Pro Val Trp Asn Gly Lys Glu Phe Val Pro Arg
            580                 585                 590

Leu Met Leu Pro Ile Ser Leu Ser Phe Asp His Arg Val Ile Asp Gly
        595                 600                 605

Ala Asp Gly Ala Arg Phe Ile Thr Ile Ile Asn Asn Thr Leu Ser Asp
    610                 615                 620

Ile Arg Arg Leu Val Met
625                 630

<210>  179
<211>  474
<212>  PRT
<213>  Escherichia coli

<400>  179

<210> 180
<211> 591
<212> PRT
<213> Corynebacterium glutamicum

<400> 180

Val Ser Val Glu Thr Arg Lys Ile Thr Lys Val Leu Val Ala Asn Arg
1               5                   10                  15

Gly Glu Ile Ala Ile Arg Val Phe Arg Ala Ala Arg Asp Glu Gly Ile
            20                  25                  30

Gly Ser Val Ala Val Tyr Ala Glu Pro Asp Ala Asp Ala Pro Phe Val
        35                  40                  45

Ser Tyr Ala Asp Glu Ala Phe Ala Leu Gly Gly Gln Thr Ser Ala Glu
    50                  55                  60

Ser Tyr Leu Val Ile Asp Lys Ile Ile Asp Ala Ala Arg Lys Ser Gly
65                  70                  75                  80

Ala Asp Ala Ile His Pro Gly Tyr Gly Phe Leu Ala Glu Asn Ala Asp
                85                  90                  95

Phe Ala Glu Ala Val Ile Asn Glu Gly Leu Ile Trp Ile Gly Pro Ser
            100                 105                 110

Pro Glu Ser Ile Arg Ser Leu Gly Asp Lys Val Thr Ala Arg His Ile
        115                 120                 125

Ala Asp Thr Ala Lys Ala Pro Met Ala Pro Gly Thr Lys Glu Pro Val
    130                 135                 140

Lys Asp Ala Ala Glu Val Val Ala Phe Ala Glu Glu Phe Gly Leu Pro
145                 150                 155                 160

Ile Ala Ile Lys Ala Ala Phe Gly Gly Gly Gly Arg Gly Met Lys Val
                165                 170                 175

Ala Tyr Lys Met Glu Glu Val Ala Asp Leu Phe Glu Ser Ala Thr Arg
            180                 185                 190

Glu Ala Thr Ala Ala Phe Gly Arg Gly Glu Cys Phe Val Glu Arg Tyr
        195                 200                 205

Leu Asp Lys Ala Arg His Val Glu Ala Gln Val Ile Ala Asp Lys His
    210                 215                 220

Gly Asn Val Val Val Ala Gly Thr Arg Asp Cys Ser Leu Gln Arg Arg
225                 230                 235                 240

Phe Gln Lys Leu Val Glu Glu Ala Pro Ala Pro Phe Leu Thr Asp Asp
                245                 250                 255

Gln Arg Glu Arg Leu His Ser Ser Ala Lys Ala Ile Cys Lys Glu Ala
            260                 265                 270

Gly Tyr Tyr Gly Ala Gly Thr Val Glu Tyr Leu Val Gly Ser Asp Gly
        275                 280                 285

Leu Ile Ser Phe Leu Glu Val Asn Thr Arg Leu Gln Val Glu His Pro
    290                 295                 300

Val Thr Glu Glu Thr Thr Gly Ile Asp Leu Val Arg Glu Met Phe Arg
305                 310                 315                 320

Ile Ala Glu Gly His Glu Leu Ser Ile Lys Glu Asp Pro Ala Pro Arg
                325                 330                 335

Gly His Ala Phe Glu Phe Arg Ile Asn Gly Glu Asp Ala Gly Ser Asn
            340                 345                 350

Phe Met Pro Ala Pro Gly Lys Ile Thr Ser Tyr Arg Glu Pro Gln Gly
        355                 360                 365

Pro Gly Val Arg Met Asp Ser Gly Val Val Glu Gly Ser Glu Ile Ser
    370                 375                 380

Gly Gln Phe Asp Ser Met Leu Ala Lys Leu Ile Val Trp Gly Asp Thr
385                 390                 395                 400

Arg Glu Gln Ala Leu Gln Arg Ser Arg Arg Ala Leu Ala Glu Tyr Val
                405                 410                 415

Val Glu Gly Met Pro Thr Val Ile Pro Phe His Gln His Ile Val Glu
            420                 425                 430

Asn Pro Ala Phe Val Gly Asn Asp Glu Gly Phe Glu Ile Tyr Thr Lys
        435                 440                 445

Trp Ile Glu Glu Val Trp Asp Asn Pro Ile Ala Pro Tyr Val Asp Ala
    450                 455                 460

Ser Glu Leu Asp Glu Asp Glu Asp Lys Thr Pro Ala Gln Lys Val Val
465                 470                 475                 480

Val Glu Ile Asn Gly Arg Arg Val Glu Val Ala Leu Pro Gly Asp Leu
                485                 490                 495

Ala Leu Gly Gly Thr Ala Gly Pro Lys Lys Lys Ala Lys Lys Arg Arg
            500                 505                 510

Ala Gly Gly Ala Lys Ala Gly Val Ser Gly Asp Ala Val Ala Ala Pro
        515                 520                 525

Met Gln Gly Thr Val Ile Lys Val Asn Val Glu Glu Gly Ala Glu Val
    530                 535                 540

Asn Glu Gly Asp Thr Val Val Val Leu Glu Ala Met Lys Met Glu Asn
545                 550                 555                 560

Pro Val Lys Ala His Lys Ser Gly Thr Val Thr Gly Leu Thr Val Ala
                565                 570                 575

Ala Gly Glu Gly Val Asn Lys Gly Val Val Leu Leu Glu Ile Lys
            580                 585                 590

<210> 181
<211> 543
<212> PRT
<213> Corynebacterium glutamicum

<400> 181

Met Thr Ile Ser Ser Pro Leu Ile Asp Val Ala Asn Leu Pro Asp Ile
1               5                   10                  15

Asn Thr Thr Ala Gly Lys Ile Ala Asp Leu Lys Ala Arg Arg Ala Glu
            20                  25                  30

Ala His Phe Pro Met Gly Glu Lys Ala Val Glu Lys Val His Ala Ala
        35                  40                  45

Gly Arg Leu Thr Ala Arg Glu Arg Leu Asp Tyr Leu Leu Asp Glu Gly
    50                  55                  60

Ser Phe Ile Glu Thr Asp Gln Leu Ala Arg His Arg Thr Thr Ala Phe
65                  70                  75                  80

Gly Leu Gly Ala Lys Arg Pro Ala Thr Asp Gly Ile Val Thr Gly Trp
                85                  90                  95

Gly Thr Ile Asp Gly Arg Glu Val Cys Ile Phe Ser Gln Asp Gly Thr
            100                 105                 110

Val Phe Gly Gly Ala Leu Gly Glu Val Tyr Gly Glu Lys Met Ile Lys
        115                 120                 125

Ile Met Glu Leu Ala Ile Asp Thr Gly Arg Pro Leu Ile Gly Leu Tyr
    130                 135                 140

Glu Gly Ala Gly Ala Arg Ile Gln Asp Gly Ala Val Ser Leu Asp Phe
145                 150                 155                 160

Ile Ser Gln Thr Phe Tyr Gln Asn Ile Gln Ala Ser Gly Val Ile Pro
                165                 170                 175

Gln Ile Ser Val Ile Met Gly Ala Cys Ala Gly Gly Asn Ala Tyr Gly
            180                 185                 190

Pro Ala Leu Thr Asp Phe Val Val Met Val Asp Lys Thr Ser Lys Met
        195                 200                 205

Phe Val Thr Gly Pro Asp Val Ile Lys Thr Val Thr Gly Glu Glu Ile
    210                 215                 220

Thr Gln Glu Glu Leu Gly Gly Ala Thr Thr His Met Val Thr Ala Gly
225                 230                 235                 240

Asn Ser His Tyr Thr Ala Ala Thr Asp Glu Glu Ala Leu Asp Trp Val
                245                 250                 255

Gln Asp Leu Val Ser Phe Leu Pro Ser Asn Asn Arg Ser Tyr Thr Pro
            260                 265                 270

Leu Glu Asp Phe Asp Glu Glu Glu Gly Gly Val Glu Glu Asn Ile Thr

275                 280                 285

Ala Asp Asp Leu Lys Leu Asp Glu Ile Ile Pro Asp Ser Ala Thr Val
    290                 295                 300

Pro Tyr Asp Val Arg Asp Val Ile Glu Cys Leu Thr Asp Asp Gly Glu
305                 310                 315                 320

Tyr Leu Glu Ile Gln Ala Asp Arg Ala Glu Asn Val Val Ile Ala Phe
                325                 330                 335

Gly Arg Ile Glu Gly Gln Ser Val Gly Phe Val Ala Asn Gln Pro Thr
            340                 345                 350

Gln Phe Ala Gly Cys Leu Asp Ile Asp Ser Ser Glu Lys Ala Ala Arg
        355                 360                 365

Phe Val Arg Thr Cys Asp Ala Phe Asn Ile Pro Ile Val Met Leu Val
    370                 375                 380

Asp Val Pro Gly Phe Leu Pro Gly Ala Gly Gln Glu Tyr Gly Gly Ile
385                 390                 395                 400

Leu Arg Arg Gly Ala Lys Leu Leu Tyr Ala Tyr Gly Glu Ala Thr Val
                405                 410                 415

Pro Lys Ile Thr Val Thr Met Arg Lys Ala Tyr Gly Gly Ala Tyr Cys
            420                 425                 430

Val Met Gly Ser Lys Gly Leu Gly Ser Asp Ile Asn Leu Ala Trp Pro
        435                 440                 445

Thr Ala Gln Ile Ala Val Met Gly Ala Ala Gly Ala Val Gly Phe Ile
    450                 455                 460

Tyr Arg Lys Glu Leu Met Ala Ala Asp Ala Lys Gly Leu Asp Thr Val
465                 470                 475                 480

Ala Leu Ala Lys Ser Phe Glu Arg Glu Tyr Glu Asp His Met Leu Asn
                485                 490                 495

Pro Tyr His Ala Ala Glu Arg Gly Leu Ile Asp Ala Val Ile Leu Pro
            500                 505                 510

Ser Glu Thr Arg Gly Gln Ile Ser Arg Asn Leu Arg Leu Leu Lys His
        515                 520                 525

Lys Asn Val Thr Arg Pro Ala Arg Lys His Gly Asn Met Pro Leu
    530                 535                 540